# EUROPEAN PATENT SPECIFICATION

(11) **EP 1 442 122 B1**
(45) Date of publication and mention of the grant of the patent: **28.09.2011**
(21) Application number: 02800506.4
(22) Date of filing: 04.10.2002
(51) Int. Cl.: C12N 15/29, C12N 15/52, C12N 15/53, C12N 15/55, C12N 15/60, C12N 15/61, A01H 5/00

(54) **MANIPULATION OF FLAVONOID BIOSYNTHESIS IN PLANTS**
MANIPULATION DER FLAVONOID-BIOSYNTHESE IN PFLANZEN
MANIPULATION DE LA BIOSYNTHESE DES FLAVONOIDES CHEZ LES PLANTES

(30) Priority: 05.10.2001 AU PR811301
(43) Date of publication of application: 04.08.2004
(73) Proprietor: Agriculture Victoria Services Pty Ltd, Attwood, Victoria 3049 (AU); AgResearch Limited, Hamilton 2001 (NZ)
(72) Inventor: SPANGENBERG, German, Bundoora, Victoria 3083 (AU); SAWBRIDGE, Timothy, Ivor, Collingwood, Victoria 3066 (AU); ONG, Eng, Kok, Vermont South, Victoria 3133 (AU); EMMERLING, Michael, Greensborough, Victoria 3088 (AU)
(74) Representative: Marshall, Cameron John
(86) International application number: PCT/AU2002/001345
(87) International publication number: WO 2003/031622

(56) References cited:
- EP-A- 0 316 797
- EP-A- 1 033 405
- WO-A-00/44909
- WO-A-01/05984
- WO-A-02/10210
- WO-A-02/18604
- WO-A-02/26994
- WO-A-95/27790
- WO-A-96/36716
- WO-A-97/12982
- WO-A-98/07836
- WO-A-99/14351
- WO-A-99/36543
- WO-A-02/063021
- US-A- 6 054 636
- DATABASE EMBL [Online] 31 July 2001 (2001-07-31), "EST5311934 GESD Medicago truncatula cDNA clone pGESD7I14 5' end, mRNA sequence." XP002413000 retrieved from EBI accession no. EM_EST:BI310184 Database accession no. BI310184
- DEVIC MARTINE ET AL: "The BANYULS gene encodes a DFR-like protein and is a marker of early seed coat development" PLANT JOURNAL, vol. 19, no. 4, August 1999 (1999-08), pages 387-398, XP002982937 ISSN: 0960-7412
- ARIOLI T ET AL: "IN TRIFOLIUM SUBTERRANEUM, CHALCONE SYNTHASE IS ENCODED BY A MULTIGENE FAMILY" GENE, ELSEVIER, AMSTERDAM, NL, vol. 138, 1994, pages 79-86, XP008059793 ISSN: 0378-1119
- DATABASE UniProt [Online] 1 March 2001 (2001-03-01), KANEKAO ET AL.: XP002402226 retrieved from EBI Database accession no. Q9FGH3
- BAVAGE ADRIAN D ET AL: "Expression of an antirrhinum dihydroflavonol reductase gene results in changes in condensed tannin structure and accumulation in root cultures of Lotus corniculatus (bird's foot trefoil)" PLANT MOLECULAR BIOLOGY, vol. 35, no. 4, 1997, pages 443-458, XP002402078 ISSN: 0167-4412
- CARRON T R ET AL: "GENETIC MODIFICATION OF CONDENSED TANNIN BIOSYNTHESIS IN LOTUS CORNICULATUS. 1 HETEROLOGOUS ANTISENSE DIHYDROFLAVONOL REDUCTASE DOWN-REGULATES TANNIN ACCUMULATION IN HAIRY ROOT CULTURES" THEORETICAL AND APPLIED GENETICS, SPRINGER, BERLIN, DE, vol. 87, no. 8, 1 March 1994 (1994-03-01), pages 1006-1015, XP002006034 ISSN: 0040-5752
- JOSEPH ROSLYN ET AL: "Proanthocyanidin synthesis in the forage legume Onobrychis viciifolia. A study of chalcone synthase, dihydroflavonol 4-reductase and leucoanthocyanidin 4-reductase in developing leaves" AUSTRALIAN JOURNAL OF PLANT PHYSIOLOGY, vol. 25, no. 3, 1998, pages 271-278, XP009073207 ISSN: 0310-7841
- DATABASE GENBANK [Online] 29 January 1997 XP002983366 Database accession no. (AAB41524)
- DATABASE GENBANK [Online] XP003009272 Database accession no. (CAA74847)
- DATABASE GENBANK [Online] 27 October 2000 XP003009273 Database accession no. (CAC14061)
- DATABASE GENBANK [Online] 30 January 1997 XP003009274 Database accession no. (AAB41556)
- DATABASE GENBANK [Online] 03 July 2001 XP003009275 Database accession no. (CAA11226)
- DATABASE GENBANK [Online] 14 May 2001 XP003009276 Database accession no. (AAK52955)
- DATABASE GENBANK [Online] 10 September 1999 XP003009277 Database accession no. (AAD54273)
- DATABASE GENBANK [Online] 07 December 1993 XP003009278 Database accession no. (CAA80265)
- DATABASE GENBANK [Online] 11 January 2000 XP003009279 Database accession no. (AAF23859)
- DATABASE GENBANK [Online] 27 December 2000 XP003009280 Database accession no. (BAB01697)
- DATABASE TREMBL [Online] 01 May 2000 XP003010314 Database accession no. (CAB63776)
- DATABASE GENBANK [Online] 16 March 2000 XP003009281 Database accession no. (CAB78172)
- DATABASE GENBANK [Online] 16 January 2001 XP003009282 Database accession no. (AAG49298)
- DATABASE GENBANK [Online] 15 May 1996 XP003009283 Database accession no. (AAA99500)
- DATABASE GENBANK [Online] 05 May 1995 XP003009284 Database accession no. (CAA41169)
- DATABASE GENBANK [Online] 30 January 1997 XP003009285 Database accession no. (AAB41550)

## Description

The present invention relates to nucleic acids and nucleic acid fragments encoding amino acid sequences for flavonoid biosynthetic enzymes in plants, and the use thereof for the modification of flavonoid biosynthesis in plants.

Flavonoids constitute a relatively diverse family of aromatic molecules that are derived from phenylalanine and malonyl-coenzyme A (CoA, via the fatty acid pathway). These compounds include six major subgroups that are found in most higher plants: the chalcones, flavones, flavonols, flavandiols, anthocyanins and condensed tannins (or proanthocyanidins). A seventh group, the aurones, is widespread, but not ubiquitous.

Some plant species also synthesize specialised forms of flavonoids, such as the isoflavonoids that are found in legumes and a small number of non-legume plants. Similarly, sorghum, maize and gloxinia are among the few species known to synthesize 3-deoxyanthocyanins (or phlobaphenes in the polymerised form). The stilbenes which are closely related to flavonoids, are synthesised by another group of unrelated species that includes grape, peanut and pine.

Besides providing pigmentation to flowers, fruits, seeds, and leaves, flavonoids also have key roles in signalling between plants and microbes, in male fertility of some species, in defense as antimicrobial agents and feeding deterrents, and in UV protection.

Flavonoids also have significant activities when ingested by animals, and there is great interest in their potential health benefits, particularly for compounds such as isoflavonoids, which have been linked to anticancer benefits, and stilbenes that are believed to contribute to reduced heart disease.

The major branch pathways of flavonoid biosynthesis start with general phenylpropanoid metabolism and lead to the nine major subgroups: the colorless chalcones, aurones, isoflavonoids, flavones, flavonols, flavandiols, anthocyanins, condensed tannins, and phlobaphene pigments. The enzyme phenylalanine ammonia-lyase (PAL) of the general phenylpropanoid pathway will lead to the production of cinnamic acid. Cinnamate-4-hydroxylase (C4H) will produce p-coumaric acid which will be converted through the action of 4-coumaroyl:CoA-ligase (4CL) to the production of 4-coumaroyl-CoA and malonyl-CoA. The first committed step in flavonoid biosynthesis is catalyzed by chalcone synthase (CHS), which uses malonyl CoA and 4-coumaryl CoA as substrates. Chalcone reductase (CHR) balances the production of 5-hydroxy- or 5-deoxyflavonoids. The next enzyme, chalcone isomerase (CHI) catalyses ring closure to form a flavanone, but the reaction can also occur spontaneously. Other enzymes in the pathway are: flavanone 3-hydroxylase (F3H), dihydroflavonol 4-reductase (DFR), flavonoid 3'-hydroxylase (F3'H) and flavonoid 3', 5' hydroxylase (F3'5'H).

The *Arabidopsis BANYULS* gene encodes a DFR-like protein that may be a leucoanthocyanidin reductase (LCR) that catalyzes an early step in condensed tannin biosynthesis. Condensed tannins are plant polyphenols with protein-precipitating and antioxidant properties, synthesized by the flavonoid pathway. Their chemical properties include protein binding, metal chelation, anti-oxidation, and UV-light absorption. As a result condensed tannins inhibit viruses, micro-organisms, insects, fungal pathogens, and monogastric digestion. Moderate amounts of tannins improve forage quality by disrupting protein foam and conferring protection from rumen pasture bloat. Bloat is a digestive disorder that occurs on some highly nutritious forage legumes such as alfalfa (*Medicago sativa*) and white clover (*Trifolium repens*). Moderate amounts of tannin can also reduce digestion rates in the rumen and can reduce parasitic load sufficiently to increase the titre of amino acids and small peptides in the small intestine without compromising total digestion.

Vestitone reductase (VR) is the penultimate enzyme in medicarpin biosynthesis. Medicarpin, a phytoalexin, has been associated with plant resistance to fungal pathogens.

While nucleic acid sequences encoding some flavonoid biosynthetic enzymes CHI, CHS, CHR, DFR, LCR, F3'5'H, F3H, F3'H, PAL and VR have been isolated for certain species of plants, there remains a need for materials useful in modifying flavonoid biosynthesis; in modifying protein binding, metal chelation, anti-oxidation, and UV-light absorption; in modifying plant pigment production; in modifying plant defense to biotic stresses such as viruses, micro-organisms, insects or fungal pathogens; in modifying forage quality, for example by disrupting protein foam and/or conferring protection from rumen pasture bloat, particularly in forage legumes and grasses, including alfalfa, medics, clovers, ryegrasses and fescues, and for methods for their use.

It is an object of the present invention to overcome, or at least alleviate, one or more of the difficulties or deficiencies associated with the prior art.

In one aspect, the present invention provides substantially purified or isolated nucleic acids or nucleic acid fragments encoding the flavonoid biosynthetic enzyme DFR from a clover (*Trifolium*) species and functionally active fragments and variants thereof.

The present invention also provides substantially purified or isolated nucleic acids or nucleic acid fragments encoding amino acid sequences for a class of proteins which are related to DFR and functionally active fragments and variants thereof. Such proteins are referred to herein as DFR-like.

The individual or simultaneous enhancement or otherwise manipulation of DFR or like gene activities in plants may enhance or otherwise alter flavonoid biosynthesis; may enhance or otherwise alter the plant capacity for protein binding, metal chelation, anti-oxidation or UV-light absorption; may enhance or reduce or otherwise alter plant pigment production; may modify plant defense to biotic stresses such as viruses, micro-organisms, insects or fungal pathogens; and/or may modify forage quality, for example by disrupting protein foam and/or conferring protection from rumen pasture bloat.

The individual or simultaneous enhancement or otherwise manipulation of DFR or like gene activities in plants has significant consequences for a range of applications in, for example, plant production and plant protection. For example, it has applications in increasing plant tolerance and plant defense to biotic stresses such as viruses, micro-organisms, insects and fungal pathogens; in improving plant forage quality, for example by disrupting protein foam and in conferring protection from rumen pasture bloat; in reducing digestion rates in the rumen and reducing parasitic load; in the production of plant compounds leading to health benefits, such as isoflavonoids, which have been linked to anticancer benefits, and stilbenes that are believed to contribute to reduced heart disease.

Methods for the manipulation of DFR or like gene activities in plants, including legumes such as clovers (*Trifolium* species) may facilitate the production of, for example, forage legumes and forage grasses and other crops with enhanced tolerance to biotic stresses such as viruses, micro-organisms, insects and fungal pathogens; altered pigmentation in flowers; forage legumes with enhanced herbage quality and bloat-safety; crops with enhanced isoflavonoid content leading to health benefits.

The clover (*Trifolium*) species may be of any suitable type, including white clover (*Trifolium repens*), red clover (*Trifolium pratense*) and subterranean clover (*Trifolium subterraneum*). Preferably the species is a clover, more preferably white clover (*T. repens*). White clover (*Trifolium repens* L.) are key pasture legumes, in temperate climates throughout the world.

The nucleic acid or nucleic acid fragment may be of any suitable type and includes DNA (such as cDNA or genomic DNA) and RNA (such as mRNA) that is single- or double-stranded, optionally containing synthetic, non-natural or altered nucleotide bases, and combinations thereof.

The term "isolated" means that the material is removed from its original environment (eg. the natural environment if it is naturally occurring). For example, a naturally occurring nucleic acid present in a living plant is not isolated, but the same nucleic acid separated from some or all of the coexisting materials in the natural system, is isolated. Such nucleic acids could be part of a vector and/or such nucleic acids could be part of a composition, and still be isolated in that such a vector or composition is not part of its natural environment.

Such nucleic acids or nucleic acid fragments could be assembled to form a consensus contig. As used herein, the term "consensus contig" refers to a nucleotide sequence that is assembled from two or more constituent nucleotide sequences that share common or overlapping regions of sequence homology. For example, the nucleotide sequence of two or more nucleic acids or nucleic acid fragments can be compared and aligned in order to identify common or overlapping sequences. Where common or overlapping sequences exist between two or more nucleic acids or nucleic acid fragments, the sequences (and thus their corresponding nucleic acids or nucleic acid fragments) can be assembled into a single contiguous nucleotide sequence.

In a further preferred embodiment of this aspect of the invention, the substantially purified or isolated nucleic acid or nucleic acid fragment encoding a DFR or DFR-like protein includes a nucleotide sequence selected from the group consisting of (a) sequences shown in Figure (Sequence ID No: 305); (b) complements of the sequences recited in (a); (c) sequences antisense to the sequences recited in (a) and (b); and (d) functionally active fragments and variants of the sequences recited in (a), (b) and (c).

By "functionally active" in relation to nucleic acids it is meant that the fragment or variant (such as an analogue, derivative or mutant) encodes a polypeptide which is capable of modifying flavonoid biosynthesis in a plant. Such variants include naturally occurring allelic variants and non-naturally occurring variants. Additions, deletions, substitutions and derivatizations of one or more of the nucleotides are contemplated so long as the modifications do not result in loss of functional activity of the fragment or variant. Preferably the functionally active fragment or variant has at least approximately 80% identity to the relevant part of the above mentioned nucleotide sequence, more preferably at least approximately 90% identity, even more preferably at least approximately 95% identity, most preferably at least approximately 98% homology. Such functionally active variants and fragments include, for example, those having nucleic acid changes which result in conservative amino acid substitutions of one or more residues in the corresponding amino acid sequence. Preferably the fragment has a size of at least 10 nucleotides, more preferably at least 15 nucleotides, most preferably at least 20 nucleotides.

Nucleic acids or nucleic acid fragments encoding at least a portion of several DFR have been isolated and identified. The nucleic acids or nucleic acid fragments of the present invention may be used to isolate cDNAs and genes encoding homologous proteins from the same or other plant species. Isolation of homologous genes using sequence-dependent protocols, such as methods of nucleic acid hybridisation, and methods of DNA and RNA amplification as exemplified by various uses of nucleic acid amplification technologies (e.g. polymerase chain reaction, ligase chain reaction), is well known in the art.

For example, genes encoding other DFR or DFR-like proteins, either as cDNAs or genomic DNAs, may be isolated directly by using all or a portion of the nucleic acids or nucleic acid fragments of the present invention as hybridisation probes to screen libraries from the desired plant employing the methodology well known to those skilled in the art. Specific oligonucleotide probes based upon the nucleic acid sequences of the present invention may be designed and synthesized by methods known in the art. Moreover, the entire sequences may be used directly to synthesize DNA probes by methods known to the skilled artisan such as random primer DNA labelling, nick translation, or end-labelling techniques, or RNA probes using available *in vitro* transcription systems. In addition, specific primers may be designed and used to amplify a part or all of the sequences of the present invention. The resulting amplification products may be labelled directly during amplification reactions or labelled after amplification reactions, and used as probes to isolate full-length cDNA or genomic fragments under conditions of appropriate stringency.

In addition, short segments of the nucleic acids or nucleic acid fragments of the present invention may be used in amplification protocols to amplify longer nucleic acids or nucleic acid fragments encoding homologous genes from DNA or RNA. For example, polymerase chain reaction may be performed on a library of cloned nucleic acid fragments wherein the sequence of one primer is derived from the nucleic acid sequences of the present invention, and the sequence of the other primer takes advantage of the presence of the polyadenylic acid tracts to the 3' end of the mRNA precursor encoding plant genes. Alternatively, the second primer sequence may be based upon sequences derived from the cloning vector. For example, those skilled in the art can follow the RACE protocol [Frohman et al. (1988) Proc. Natal. Acad Sci. USA 85:8998] to generate cDNAs by using PCR to amplify copies of the region between a single point in the transcript and the 3' or 5' end. Using commercially available 3' RACE and 5' RACE systems (BRL), specific 3' or 5' cDNA fragments may be isolated [Ohara et al. (1989) Proc. Natal. Acad Sci US 86:5673; Loh et al. (1989) Science 243:217]. Products generated by the 3' and 5' RACE procedures may be combined to generate full-length cDNAs.

In a second aspect of the present invention there is provided a substantially purified or isolated polypeptide from a clover (*Trifolium*), selected from DFR and DFR-like; and functionally active fragments and variants thereof.

The clover (*Trifolium*) may be of any suitable type, including white clover (*trifolium repens*), red clover (*trifolium pratense*) and subterranean clover (*Trifolium subterraneum*). Preferably the species is a clover, more preferably white clover (*T*. *repens*).

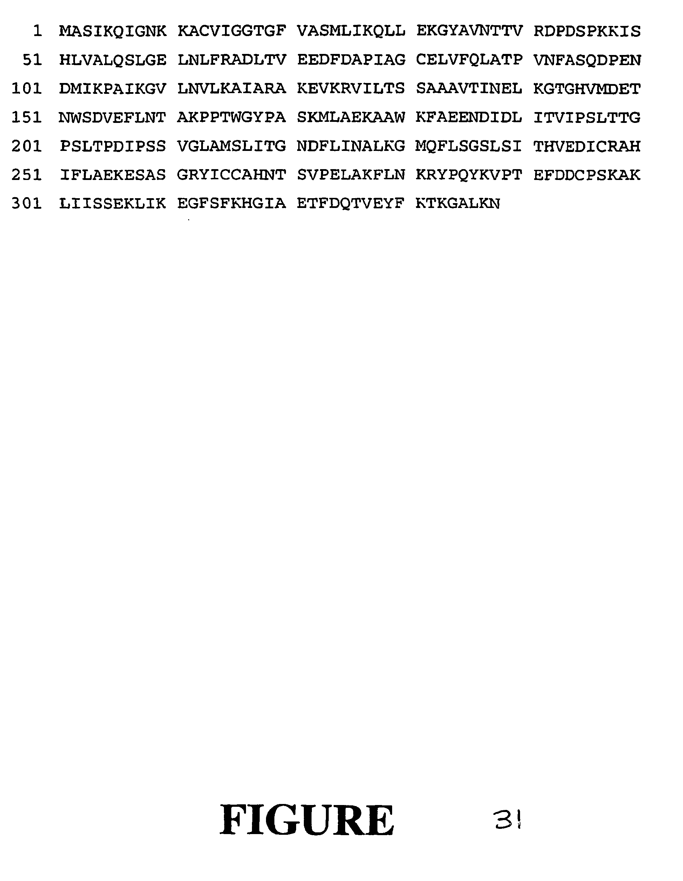

In a still further preferred embodiment of this aspect of the invention, the substantially purified or isolated DFR or DFR-like polypeptide includes an amino acid sequence selected from the sequence shown in Figure 31 (Sequence ID No: 306), and functionally active fragments and variants thereof.

By "functionally active" in relation to polypeptides it is meant that the fragment or variant has one or more of the biological properties of the proteins DFR and DFR-like. Additions, deletions, substitutions and derivatizations of one or more of the amino acids are contemplated so long as the modifications do not result in loss of functional activity of the fragment or variant. Preferably the functionally active fragment or variant has at least approximately 60% identity to the relevant part of the above mentioned amino acid sequence, more preferably at least approximately 80% identity, even more preferably at least approximately 90% identity most preferably at least approximately 95% homology. Such functionally active variants and fragments include, for example, those having conservative amino acid substitutions of one or more residues in the corresponding amino acid sequence. Preferably the fragment has a size of at least 10 amino acids, more preferably at least 15 amino acids, most preferably at least 20 amino acids.

In a further embodiment of this aspect of the invention, there is provided a polypeptide recombinantly produced from a nucleic acid or nucleic acid fragment according to the present invention. Techniques for recombinantly producing polypeptides are well known to those skilled in the art.

Availability of the nucleotide sequences of the present invention and deduced amino acid sequences facilitates immunological screening of cDNA expression libraries. Synthetic peptides representing portions of the instant amino acid sequences may be synthesized. These peptides may be used to immunise animals to produce polyclonal or monoclonal antibodies with specificity for peptides and/or proteins including the amino acid sequences. These antibodies may be then used to screen cDNA expression libraries to isolate full-length cDNA clones of interest.

A genotype is the genetic constitution of an individual or group. Variations in genotype are important in commercial breeding programs, in determining parentage, in diagnostics and fingerprinting, and the like. Genotypes can be readily described in terms of genetic markers. A genetic marker identifies a specific region or locus in the genome. The more genetic markers, the finer defined is the genotype. A genetic marker becomes particularly useful when it is allelic between organisms because it then may serve to unambiguously identify an individual. Furthermore, a genetic marker becomes particularly useful when it is based on nucleic acid sequence information that can unambiguously establish a genotype of an individual and when the function encoded by such nucleic acid is known and is associated with a specific trait. Such nucleic acids and/or nucleotide sequence information including single nucleotide polymorphisms (SNPs), variations in single nucleotides between allelic forms of such nucleotide sequence, may be used as perfect markers or candidate genes for the given trait.

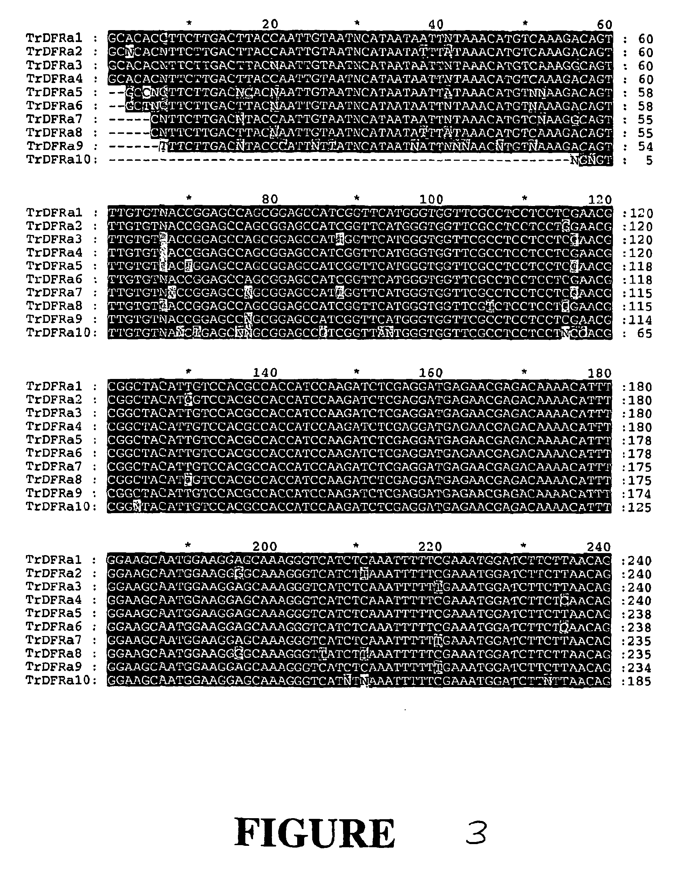

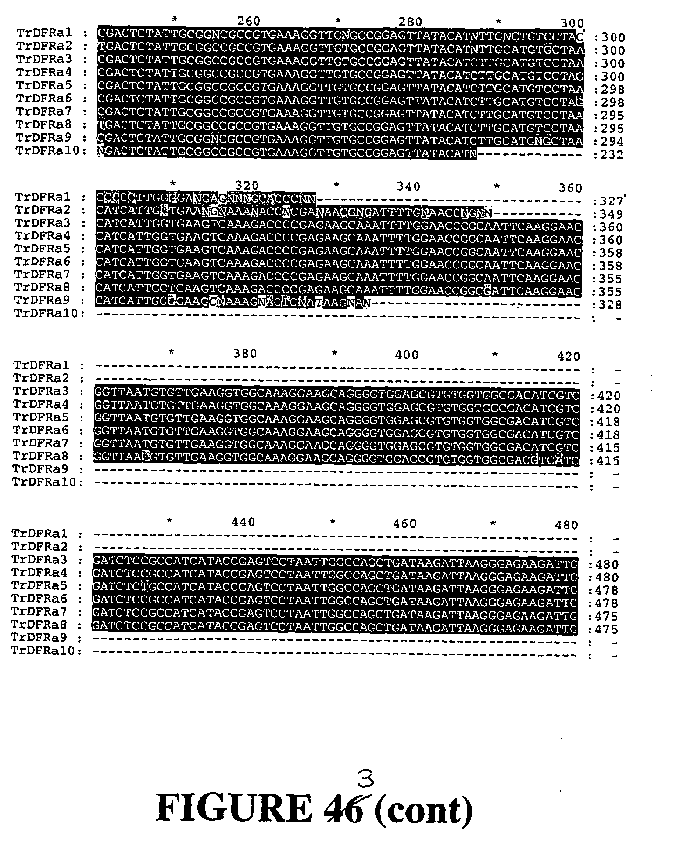

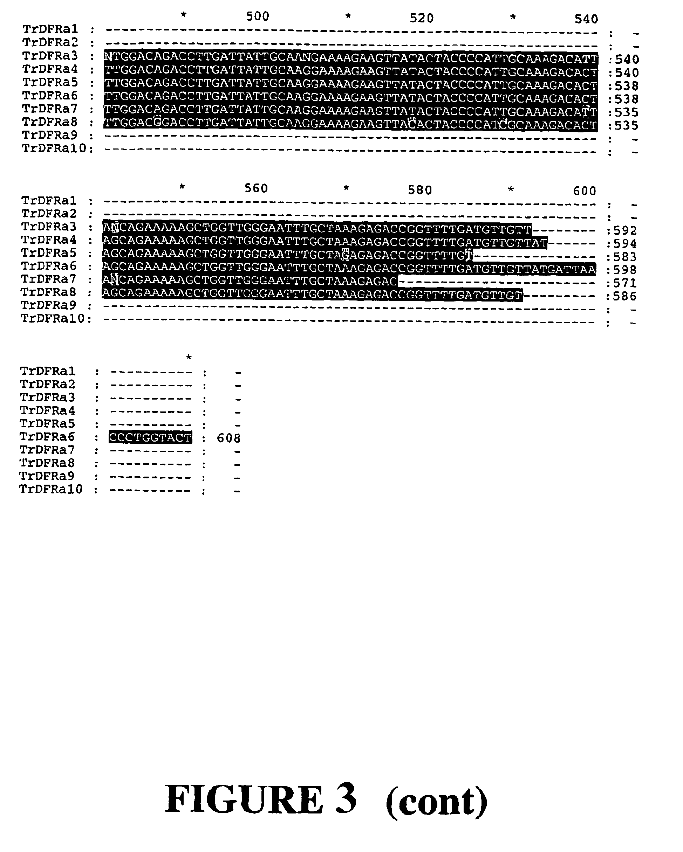

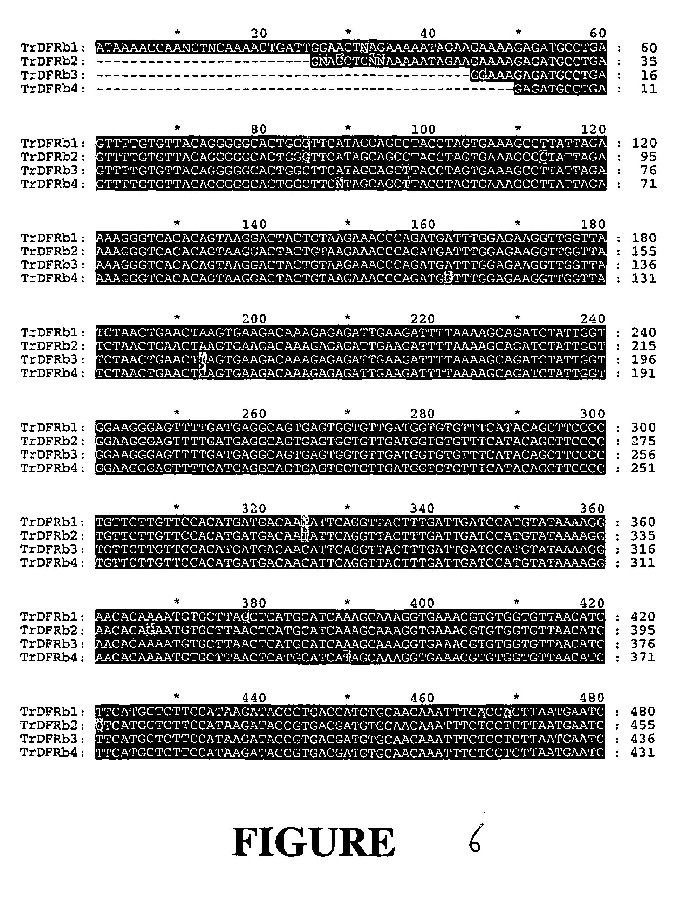

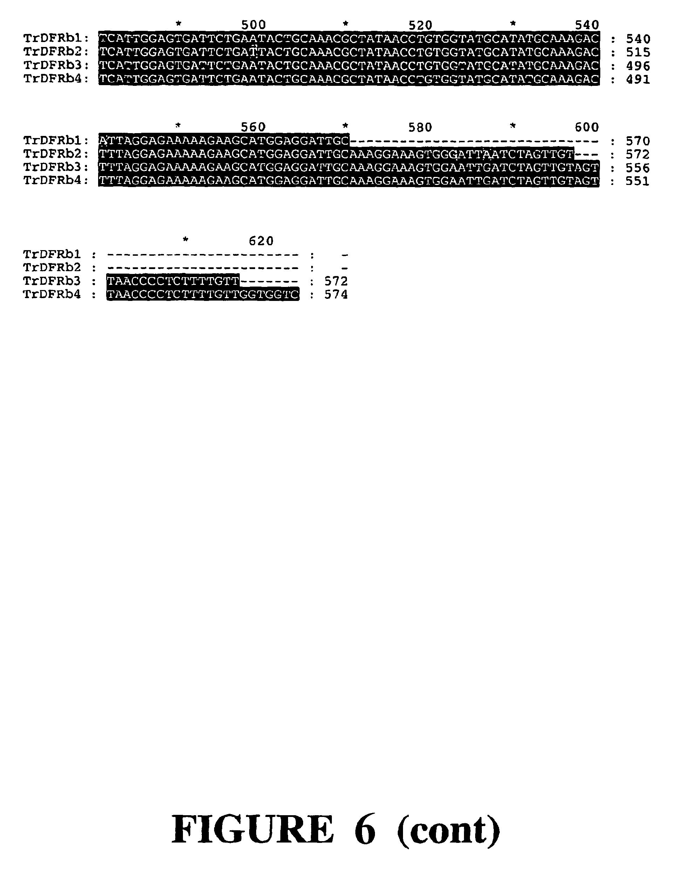

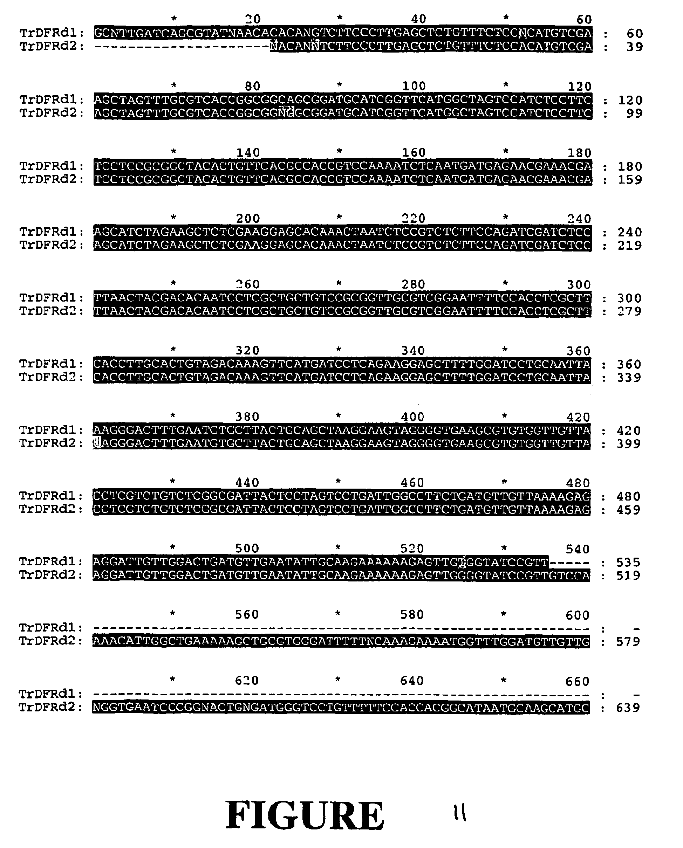

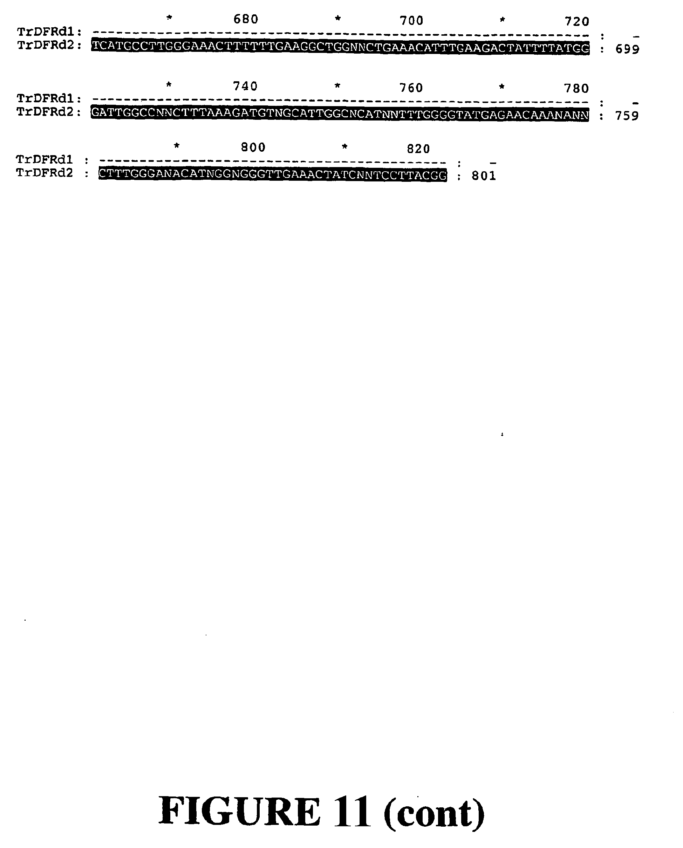

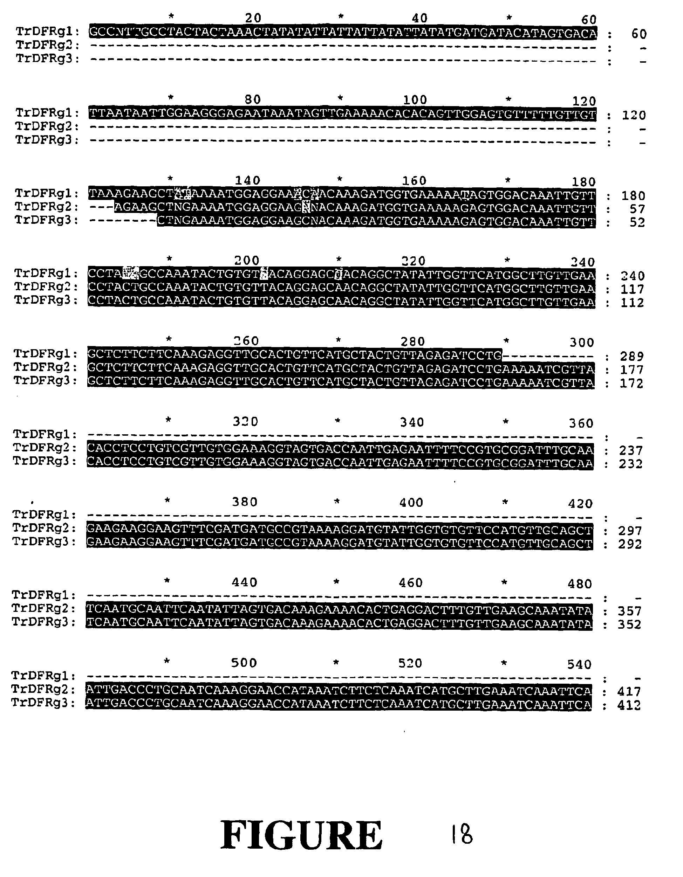

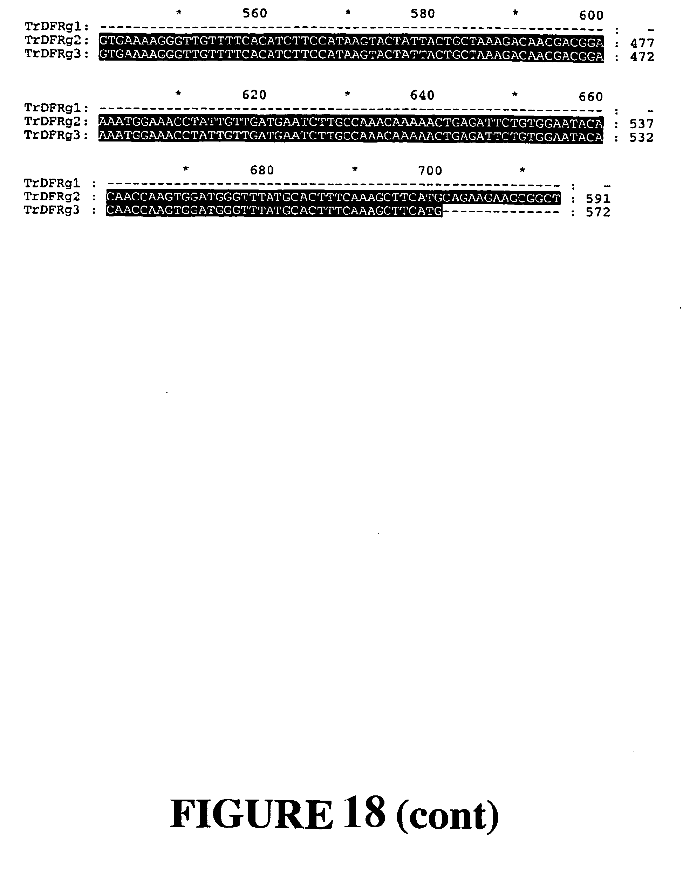

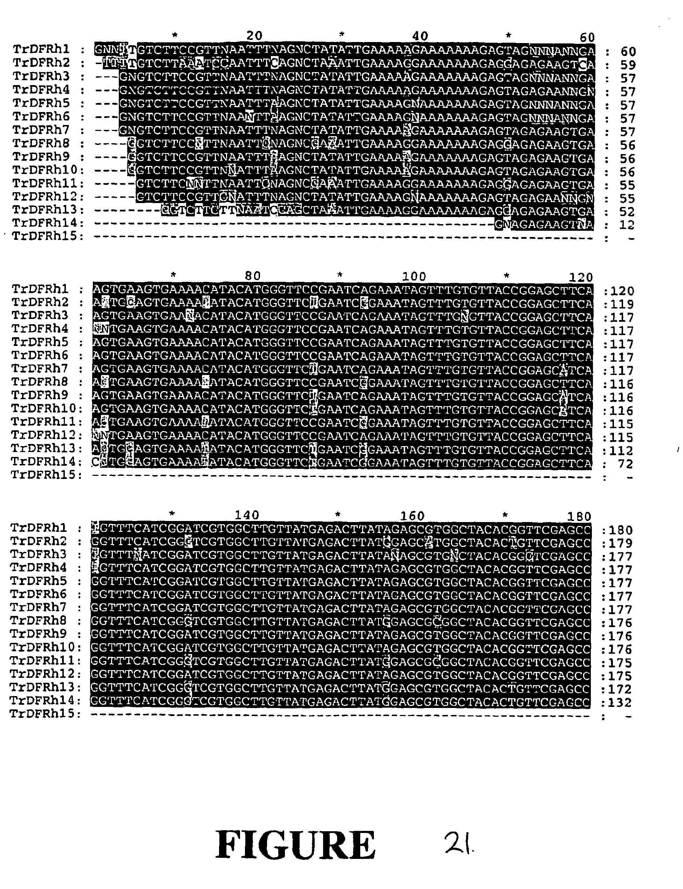

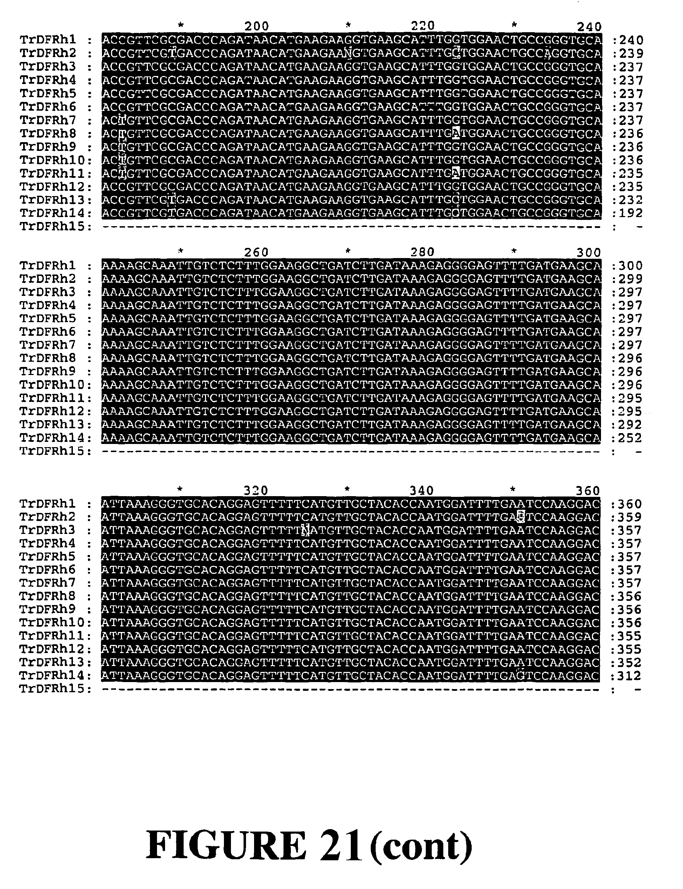

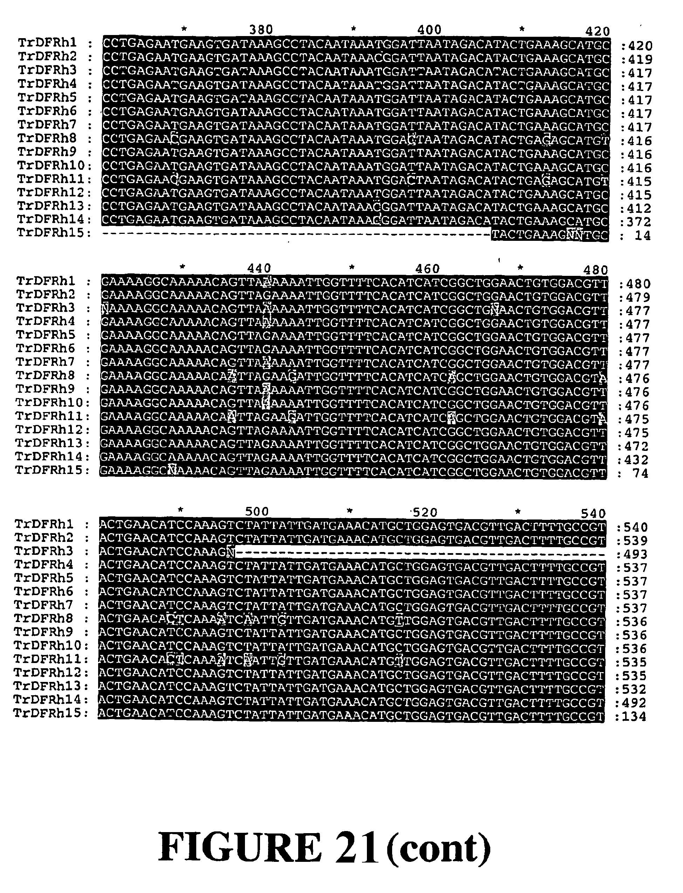

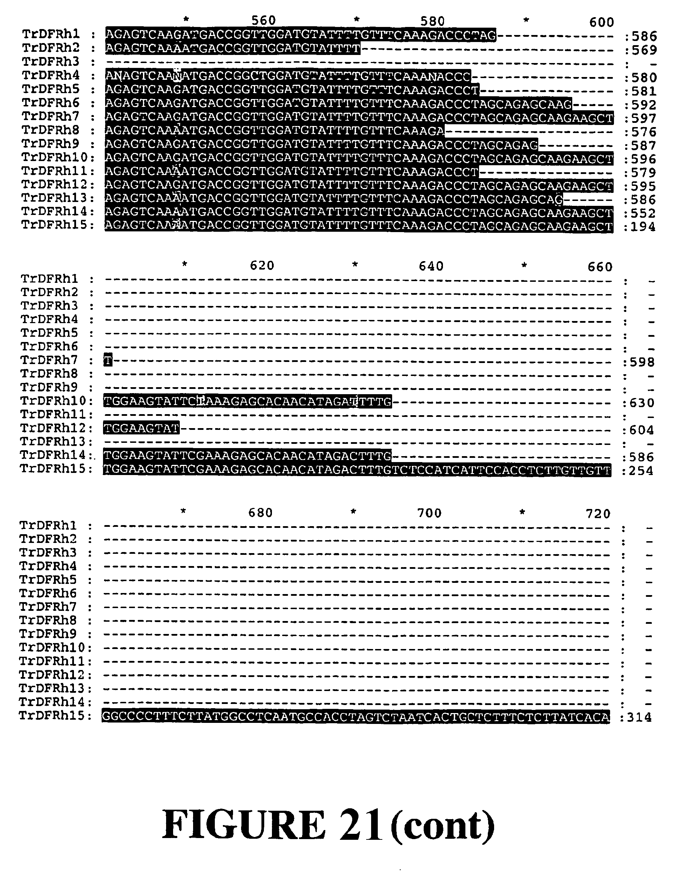

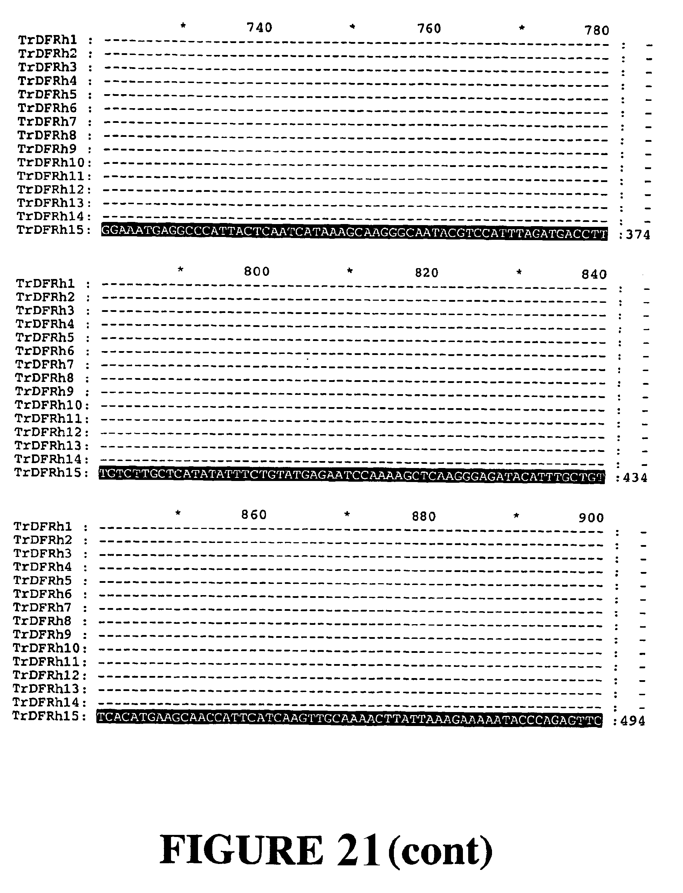

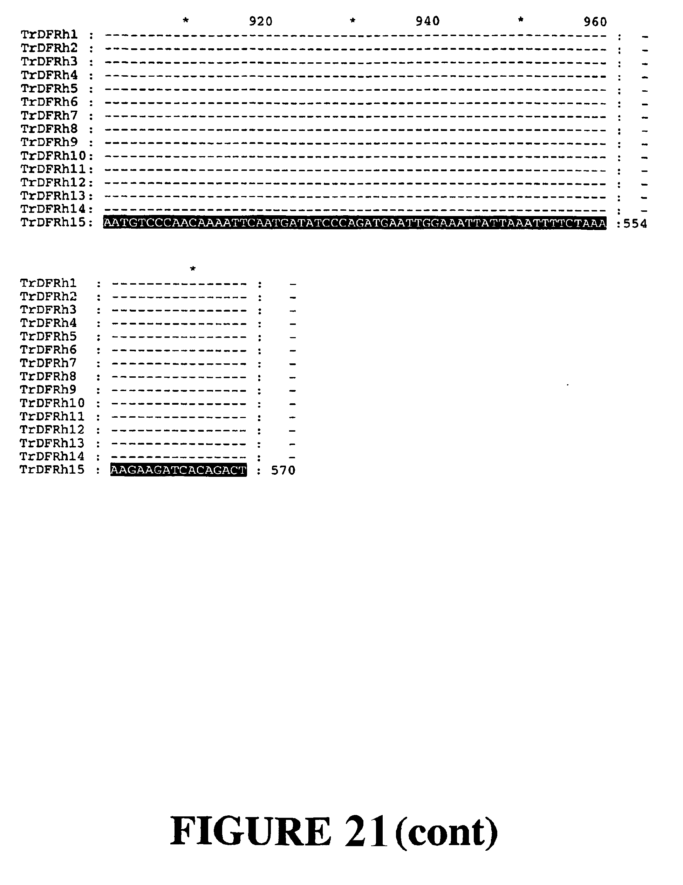

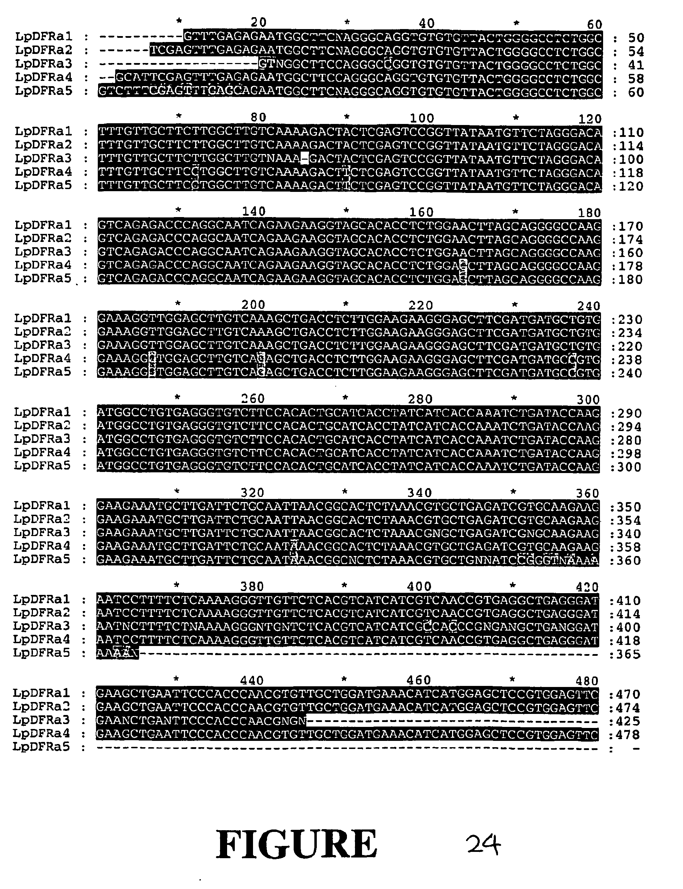

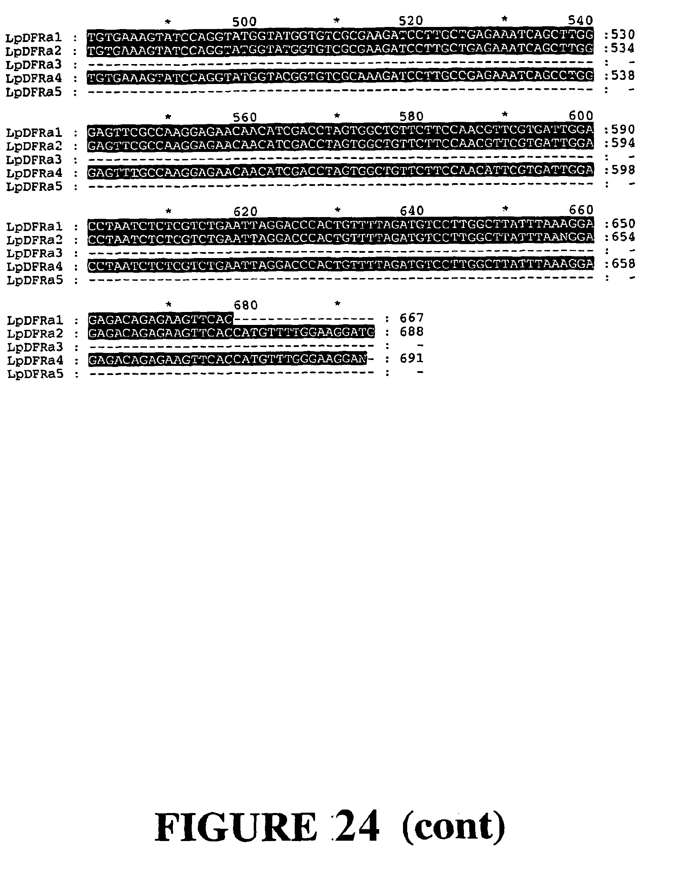

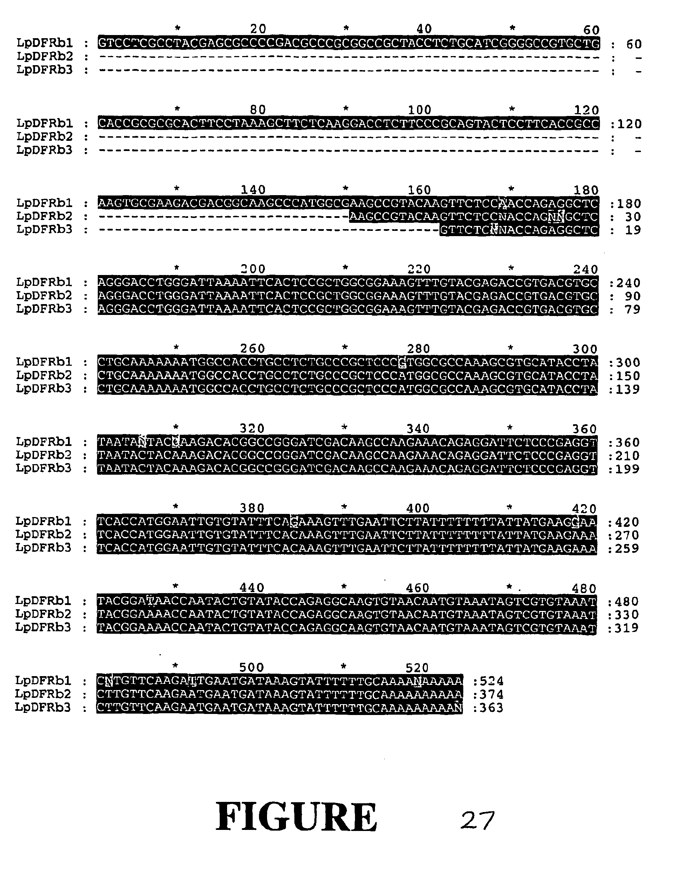

Applicants have identified a number of SNPs of the nucleic acids or nucleic acid fragments of the present invention. These are indicated (marked with grey on the black background) in the figures that show multiple alignments of nucleotide sequences of nucleic acid fragments contributing to consensus contig sequences. See for example, Figures 3, 6, 11, 18, 21, 24 and 27 hereto (Sequence ID Nos: 137 to 146, 149 to 152, 157 and 158, 165 to 167, 170 to 184, 288 to 292 and 295 to 297, respectively).

Accordingly, in a further aspect of the present invention, there is provided a substantially purified or isolated nucleic acid or nucleic acid fragment including a single nucleotide polymorphism (SNP) from a nucleic acid or nucleic acid fragment according to the present invention or complements or sequences antisense thereto, and functionally active fragments and variants thereof.

In a still further aspect of the present invention there is provided a method of isolating a nucleic acid or nucleic acid fragment of the present invention including a SNP, said method including sequencing nucleic acid fragments from a nucleic acid library.

The nucleic acid library may be of any suitable type and is preferably a cDNA library.

The nucleic acid or nucleic acid fragments may be isolated from a recombinant plasmid or may be amplified, for example using polymerase chain reaction.

The sequencing may be performed by techniques known to those skilled in the art.

In a still further aspect of the present invention, there is provided use of the nucleic acids or nucleic acid fragments of the present invention including SNPs, and/or nucleotide sequence information thereof, as molecular genetic markers.

In a still further aspect of the present invention there is provided use of a nucleic acid or nucleic acid fragment of the present invention, and/or nucleotide sequence information thereof, as a molecular genetic marker.

More particularly, nucleic acids or nucleic acid fragments according to the present invention and/or nucleotide sequence information thereof may be used as a molecular genetic marker for quantitative trait loci (QTL) tagging, QTL mapping, DNA fingerprinting and in marker assisted selection, particularly in clovers, alfalfa, ryegrasses and fescues. Even more particularly, nucleic acids or nucleic acid fragments according to the present invention and/or nucleotide sequence information thereof may be used as molecular genetic markers in plant improvement in relation to plant tolerance to biotic stresses such as viruses, micro-organisms, insects, fungal pathogens; in relation to forage quality; in relation to bloat safety; in relation to condensed tannin content; in relation to plant pigmentation. Even more particularly, sequence information revealing SNPs in allelic variants of the nucleic acids or nucleic acid fragments of the present invention and/or nucleotide sequence information thereof may be used as molecular genetic markers for QTL tagging and mapping and in marker assisted selection, particularly in clovers, alfalfa, ryegrasses and fescues.

In a still further aspect of the present invention there is provided a construct including a nucleic acid or nucleic acid fragment according to the present invention.

The term "construct" as used herein refers to an artificially assembled or isolated nucleic acid molecule which includes the gene of interest. In general a construct may include the gene or genes of interest, a marker gene which in some cases can also be the gene of interest and appropriate regulatory sequences. It should be appreciated that the inclusion of regulatory sequences in a construct is optional, for example, such sequences may not be required in situations where the regulatory sequences of a host cell are to be used. The term construct includes vectors but should not be seen as being limited thereto.

In a still further aspect of the present invention there is provided a vector including a nucleic acid or nucleic acid fragment according to the present invention.

The term "vector" as used herein encompasses both cloning and expression vectors. Vectors are often recombinant molecules containing nucleic acid molecules from several sources.

In a preferred embodiment of this aspect of the invention, the vector may include a regulatory element such as a promoter, a nucleic acid or nucleic acid fragment according to the present invention and a terminator; said regulatory element, nucleic acid or nucleic acid fragment and terminator being operatively linked.

By "operatively linked" is meant that said regulatory element is capable of causing expression of said nucleic acid or nucleic acid fragment in a plant cell and said terminator is capable of terminating expression of said nucleic acid or nucleic acid fragment in a plant cell. Preferably, said regulatory element is upstream of said nucleic acid or nucleic acid fragment and said terminator is downstream of said nucleic acid or nucleic acid fragment.

The vector may be of any suitable type and may be viral or non-viral. The vector may be an expression vector. Such vectors include chromosomal, non-chromosomal and synthetic nucleic acid sequences, eg. derivatives of plant viruses; bacterial plasmids; derivatives of the Ti plasmid from *Agrobacterium tumefaciens*, derivatives of the Ri plasmid from *Agrobacterium rhizogenes*; phage DNA; yeast artificial chromosomes; bacterial artificial chromosomes; binary bacterial artificial chromosomes; vectors derived from combinations of plasmids and phage DNA. However any other vector may be used as long as it is replicable, integrative or viable in the plant cell.

The regulatory element and terminator may be of any suitable type and may be endogenous to the target plant cell or may be exogenous, provided that they are functional in the target plant cell.

Preferably the regulatory element is a promoter. A variety of promoters which may be employed in the vectors of the present invention are well known to those skilled in the art. Factors influencing the choice of promoter include the desired tissue specificity of the vector, and whether constitutive or inducible expression is desired and the nature of the plant cell to be transformed (eg. monocotyledon or dicotyledon). Particularly suitable constitutive promoters include the Cauliflower Mosaic Virus 35S (CaMV 35S) promoter and derivatives thereof, the maize Ubiquitin promoter, and the rice Actin promoter.

A variety of terminators which may be employed in the vectors of the present invention are also well known to those skilled in the art. The terminator may be from the same gene as the promoter sequence or a different gene. Particularly suitable terminators are polyadenylation signals, such as the CaMV 35S polyA and other terminators from the nopaline synthase (nos), the octopine synthase (ocs) and the rbcS genes.

The vector, in addition to the regulatory element, the nucleic acid or nucleic acid fragment of the present invention and the terminator, may include further elements necessary for expression of the nucleic acid or nucleic acid fragment, in different combinations, for example vector backbone, origin of replication (ori), multiple cloning sites, spacer sequences, enhancers, introns (such as the maize Ubiquitin *Ubi* intron), antibiotic resistance genes and other selectable marker genes [such as the neomycin phosphotransferase (*npt2*) gene, the hygromycin phosphotransferase (*hph*) gene, the phosphinothricin acetyltransferase (*bar* or *pat*) gene and the gentamycin acetyl transferase (*aacC1*) gene], and reporter genes [such as beta-glucuronidase (GUS) gene (*gusA*) and green fluorescent protein (gfp)]. The vector may also contain a ribosome binding site for translation initiation. The vector may also include appropriate sequences for amplifying expression.

As an alternative to use of a selectable marker gene to provide a phenotypic trait for selection of transformed host cells, the presence of the vector in transformed cells may be determined by other techniques well known in the art, such as PCR (polymerase chain reaction), Southern blot hybridisation analysis, histochemical GUS assays, northern and Western blot hybridisation analyses.

Those skilled in the art will appreciate that the various components of the vector are operatively linked, so as to result in expression of said nucleic acid or nucleic acid fragment. Techniques for operatively linking the components of the vector of the present invention are well known to those skilled in the art. Such techniques include the use of linkers, such as synthetic linkers, for example including one or more restriction enzyme sites.

The constructs and vectors of the present invention may be incorporated into a variety of plants, including monocotyledons (such as grasses from the genera *Lolium, Festuca, Paspalum, Pennisetum, Panicum* and other forage and turfgrasses, corn, oat, sugarcane, wheat and barley), dicotyledons (such as Arabidopsis, tobacco, clovers, medics, eucalyptus, potato, sugarbeet, canola, soybean, chickpea) and gymnosperms. In a preferred embodiment, the constructs and vectors may be used to transform monocotyledons, preferably grass species such as ryegrasses (*Lolium* species) and fescues (*Festuca* species), more preferably perennial ryegrass, including forage- and turf-type cultivars. In an alternate preferred embodiment, the constructs and vectors may be used to transform dicotyledons, preferably forage legume species such as clovers (*Trifolium* species) and medics (*Medicago* species), more preferably white clover (*Trifolium repens*), red clover (*Trifolium pratense*), subterranean clover (*Trifolium subterraneum*) and alfalfa (*Medicago sativa*). Clovers, alfalfa and medics are key pasture legumes in temperate climates throughout the world.

Techniques for incorporating the constructs and vectors of the present invention into plant cells (for example by transduction, transfection or transformation) are well known to those skilled in the art. Such techniques include *Agrobacterium* mediated introduction, electroporation to tissues, cells and protoplasts, protoplast fusion, injection into reproductive organs, injection into immature embryos and high velocity projectile introduction to cells, tissues, calli, immature and mature embryos. The choice of technique will depend largely on the type of plant to be transformed.

Cells incorporating the constructs and vectors of the present invention may be selected, as described above, and then cultured in an appropriate medium to regenerate transformed plants, using techniques well known in the art. The culture conditions, such as temperature, pH and the like, will be apparent to the person skilled in the art. The resulting plants may be reproduced, either sexually or asexually, using methods well known in the art, to produce successive generations of transformed plants.

In a further aspect of the present invention there is provided a plant cell, plant, plant seed or other plant part, including, e.g. transformed with, a construct, vector, nucleic acid or nucleic acid fragment of the present invention.

The plant cell, plant, plant seed or other plant part may be from any suitable species, including monocotyledons, dicotyledons and gymnosperms. In a preferred embodiment the plant cell, plant, plant seed or other plant part may be from a monocotyledon, preferably a grass species, more preferably a ryegrass (*Lolium* species) or fescue (*Festuca* species), even more preferably perennial ryegrass, including both forage- and turf-type cultivars. In an alternate preferred embodiment the plant cell, plant, plant seed or other plant part may be from a dicotyledon, preferably forage legume species such as clovers (*Trifolium* species) and medics (*Medicago* species), more preferably white clover (*Trifolium repens*), red clover (*Trifolium pratense*), subterranean clover (*Trifolium subterraneum*) and alfalfa (*Medicago sativa*).

The present invention also provides a plant, plant seed or other plant part, or a plant extract derived from a plant cell of the present invention.

The present invention also provides a plant, plant seed or other plant part, or a plant extract derived from a plant of the present invention.

In a further aspect of the present invention there is provided a method of modifying flavonoid biosynthesis in a plant; said method including introducing into said plant an effective amount of a nucleic acid or nucleic acid fragment, construct and/or a vector according to the present invention.

In a further aspect of the present invention there is provided a method of modifying protein binding, metal chelation, anti-oxidation, and/or UV-light absorption in a plant, said method including introducing into said plant an effective amount of a nucleic acid or nucleic acid fragment, construct and/or a vector according to the present invention.

In a further aspect of the present invention there is provided a method of modifying pigment production in a plant, said method including introducing into said plant an effective amount of a nucleic acid or nucleic acid fragment, construct and/or a vector according to the present invention.

In a further aspect of the present invention there is provided a method of modifying plant defense to biotic stresses such as viruses, micro-organisms, insects and fungal pathogens, said method including introducing into said plant an effective amount of a nucleic acid or nucleic acid fragment, construct and/or a vector according to the present invention.

In a further aspect of the present invention there is provided a method of modifying forage quality of a plant by disrupting protein foam and/or conferring protection from rumen pasture bloat, said method including introducing into said plant an effective amount of a nucleic acid or nucleic acid fragment, construct and/or a vector according to the present invention.

By "an effective amount" it is meant an amount sufficient to result in an identifiable phenotypic trait in said plant, or a plant, plant seed or other plant part derived therefrom. Such amounts can be readily determined by an appropriately skilled person, taking into account the type of plant, the route of administration and other relevant factors. Such a person will readily be able to determine a suitable amount and method of administration. See, for example, Maniatis et al, Molecular Cloning: A Laboratory Manual, Cold Spring Harbor Laboratory, Cold Spring Harbor.

Using the methods and materials of the present invention, flavonoid biosynthesis, protein binding, metal chelation, anti-oxidation, UV-light absorption, tolerance to biotic stresses such as viruses, micro-organisms, insects and fungal pathogens; pigmentation in for example flowers and leaves; herbage quality and bloat-safety; and/or isoflavonoid content leading to health benefits, may be increased or otherwise modified, for example by incorporating additional copies of a sense nucleic acid or nucleic acid fragment of the present invention. They may be decreased or otherwise modified, for example by incorporating an antisense nucleic acid or nucleic acid fragment of the present invention.

The present invention will now be more fully described with reference to the accompanying Examples and drawings. It should be understood, however, that the description following is illustrative only and should not be taken in any way as a restriction on the generality of the invention described above.

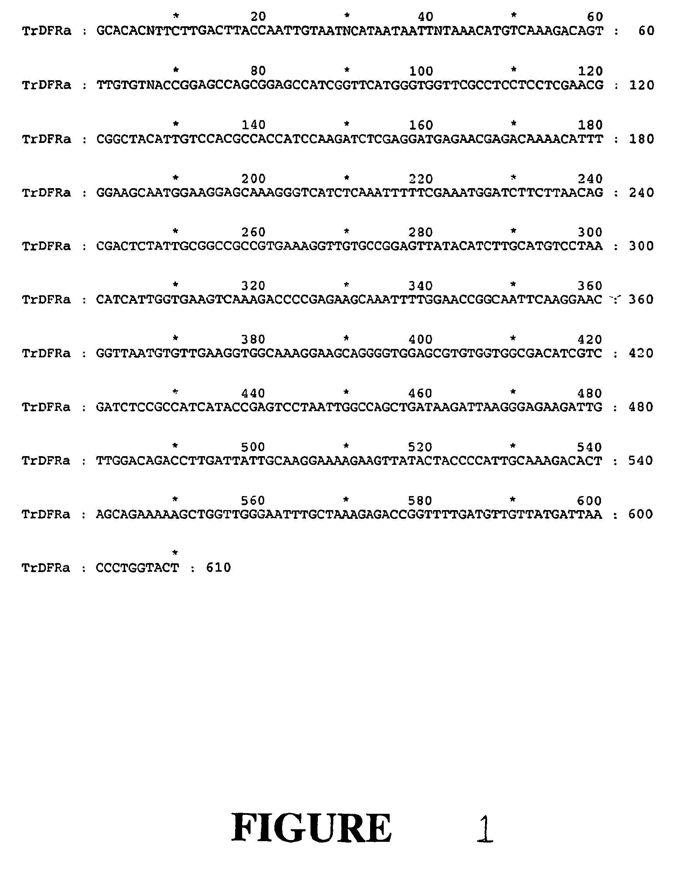

In the Figures
Figure 1 shows the consensus contig nucleotide sequence of TrDFRa (Sequence ID No: 135).

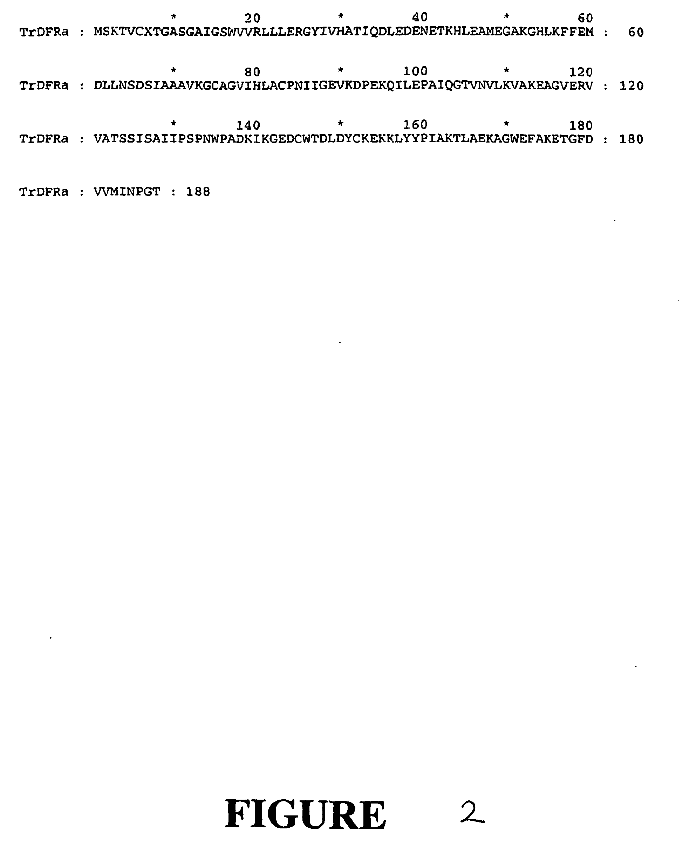

Figure 2 shows the deduced amino acid sequence of TrDFRa (Sequence ID No: 136).
Figure 3 shows the nucleotide sequences of the nucleic acid fragments contributing to the consensus contig sequence TrDFRa (Sequence ID Nos: 137 to 146).

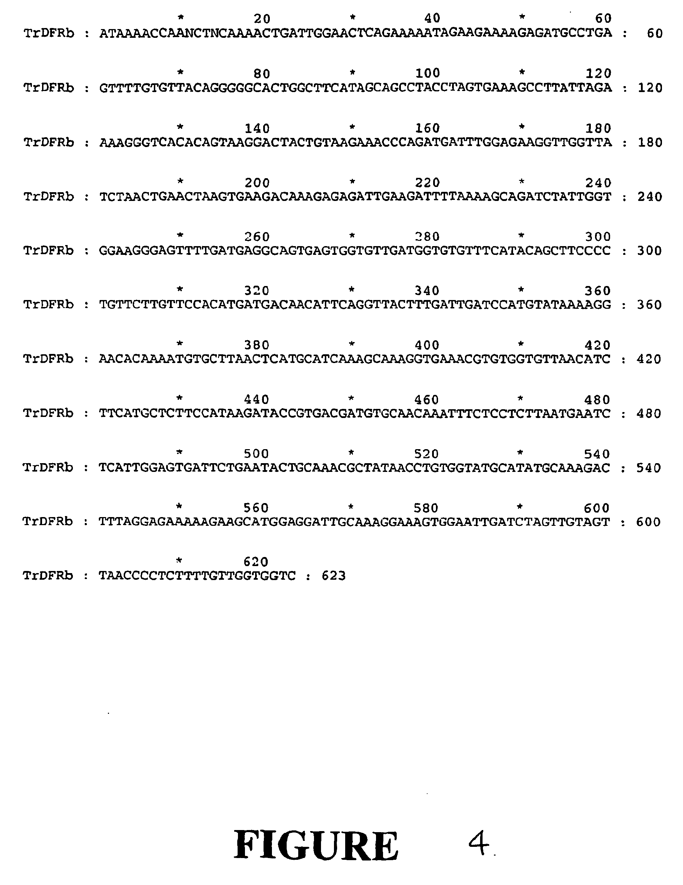

Figure 4 shows the consensus contig nucleotide sequence of TrDFRb (Sequence ID No: 147).

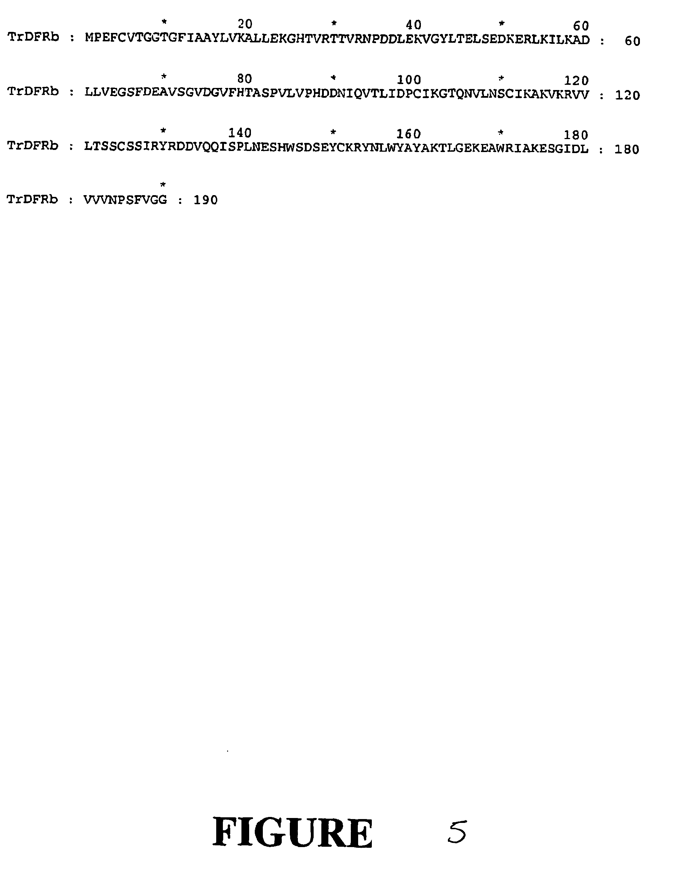

Figure 5 shows the deduced amino acid sequence of TrDFRb (Sequence ID No: 148).
Figure 6 shows the nucleotide sequences of the nucleic acid fragments contributing to the consensus contig sequence TrDFRb (Sequence ID Nos: 149 to 152).

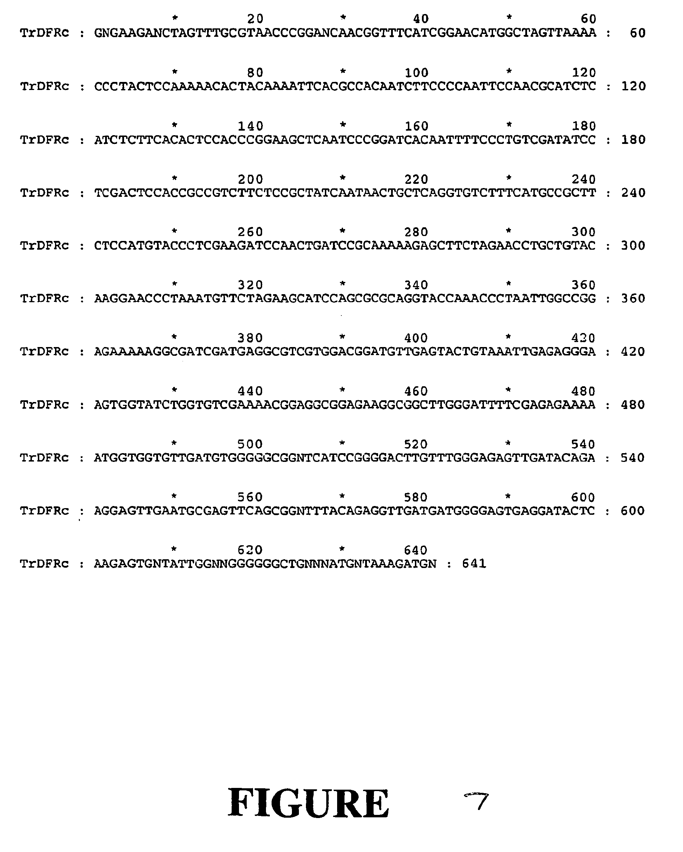

Figure 7 shows the nucleotide sequence of TrDFRc (Sequence ID No: 153).

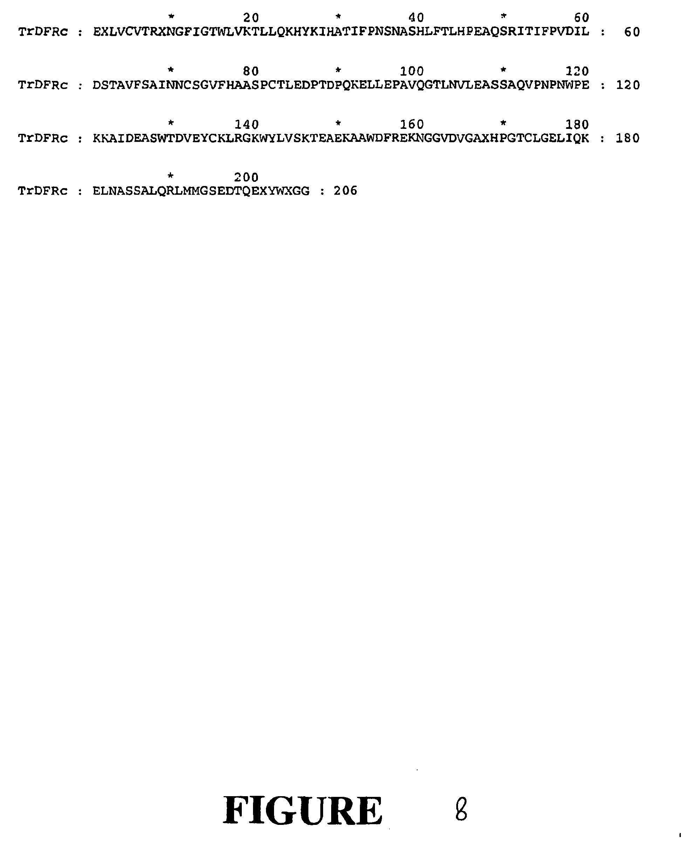

Figure 8 shows the deduced amino acid sequence of TrDFRc (Sequence ID No: 154).

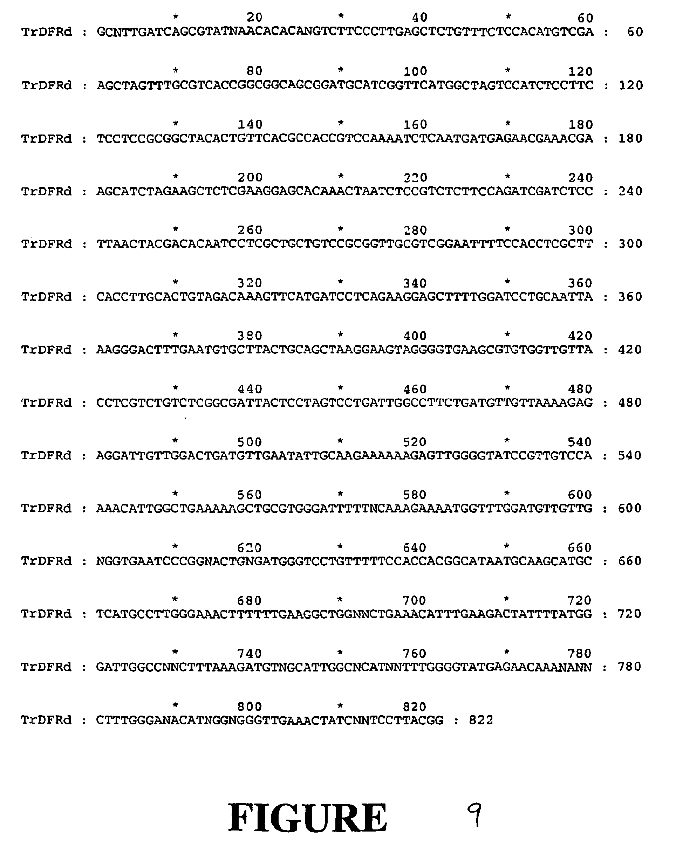

Figure 9 shows the consensus contig nucleotide sequence of TrDFRd (Sequence ID No: 155).

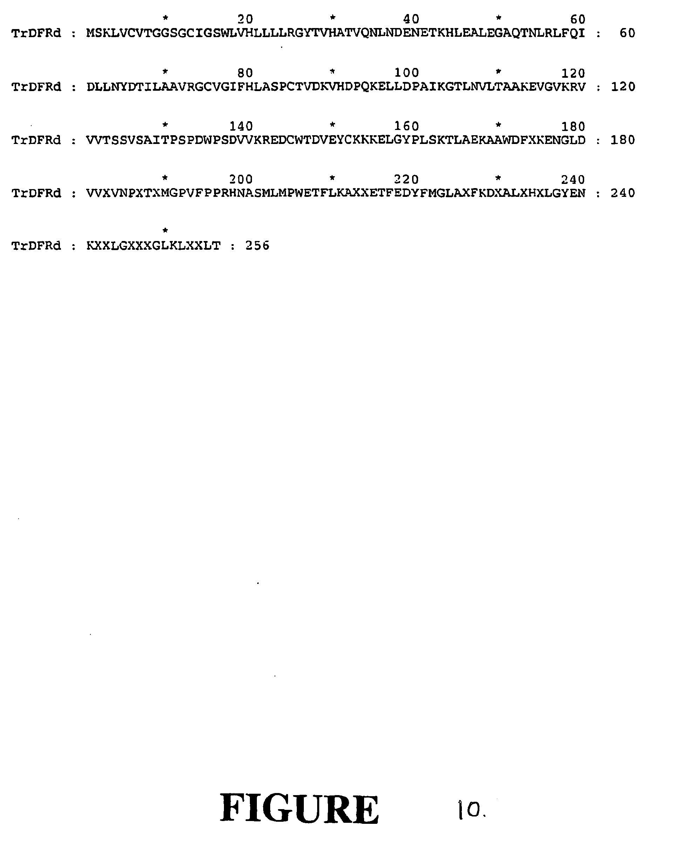

Figure 10 shows the deduced amino acid sequence of TrDFRd (Sequence ID No: 156).
Figure 11 shows the nucleotide sequences of the nucleic acid fragments contributing to the consensus contig sequence TrDFRd (Sequence ID Nos: 157 and 158).

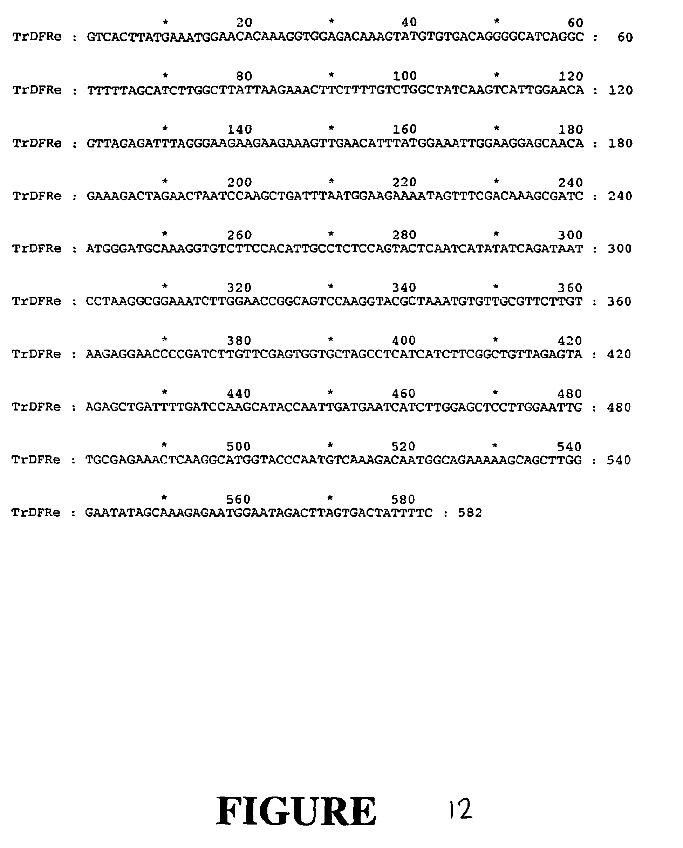

Figure 12 shows the nucleotide sequence of TrDFRe (Sequence ID No: 159).

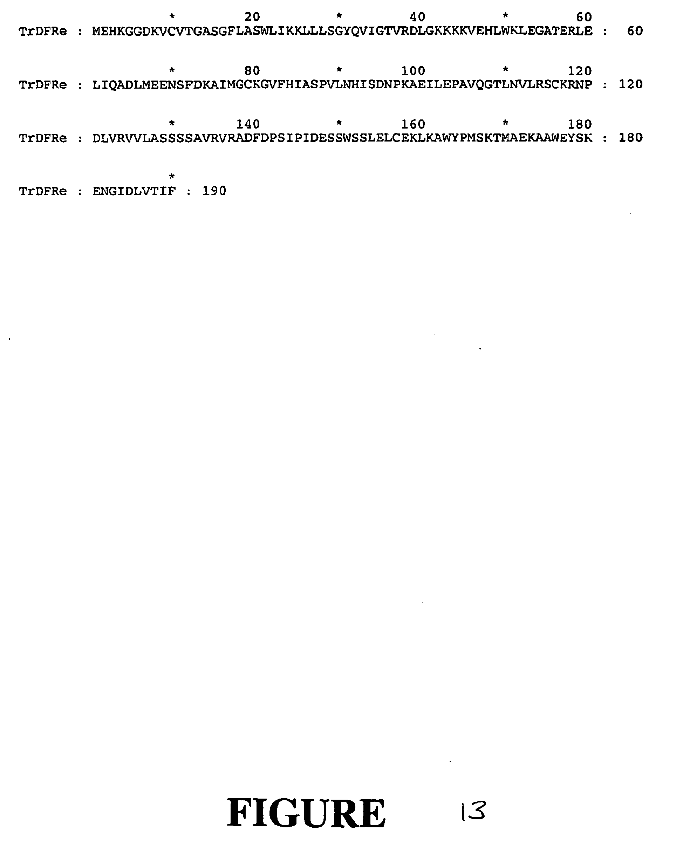

Figure 13 shows the deduced amino acid sequence of TrDFRe (Sequence ID No: 160).

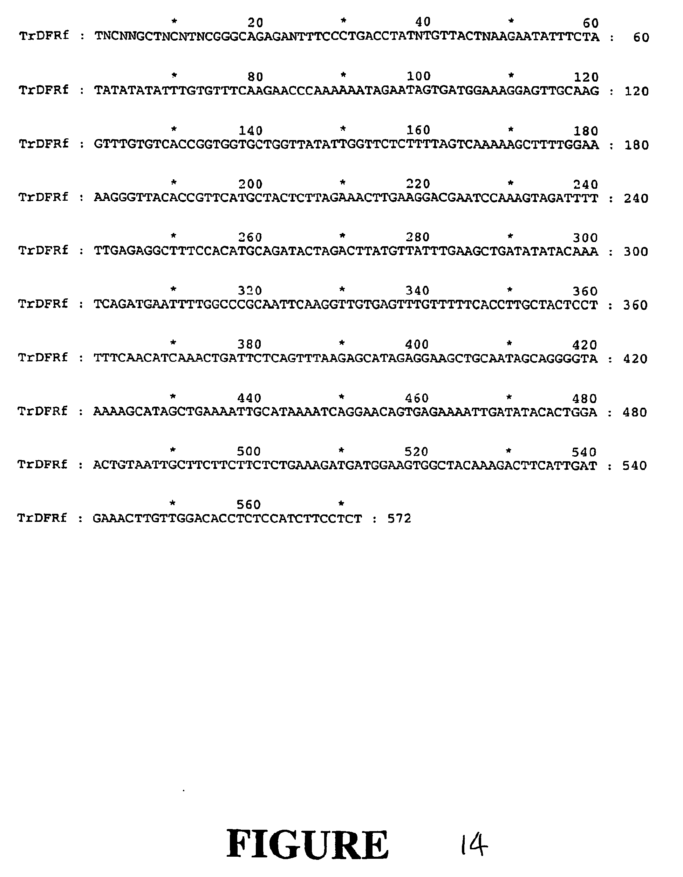

Figure 14 shows the nucleotide sequence of TrDFRf (Sequence ID No: 161).

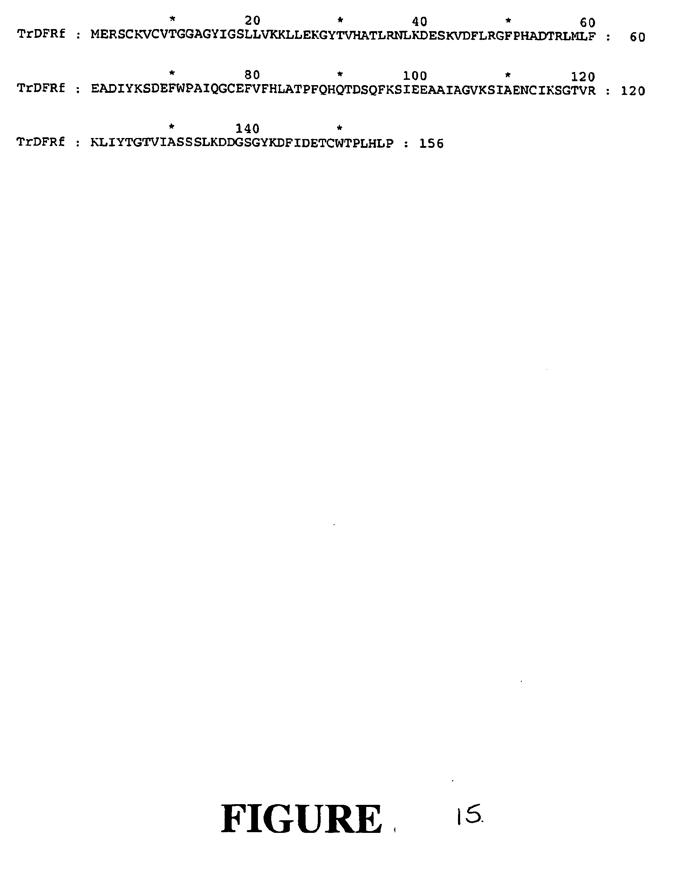

Figure 15 shows the deduced amino acid sequence of TrDFRf (Sequence ID No: 162).

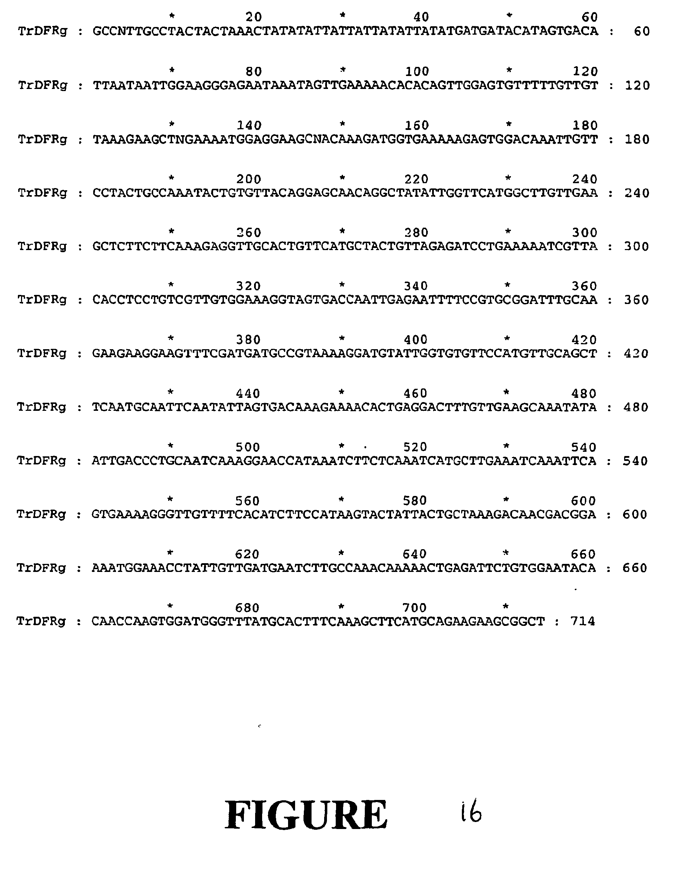

Figure 16 shows the consensus contig nucleotide sequence of TrDFRg (Sequence ID No: 163).

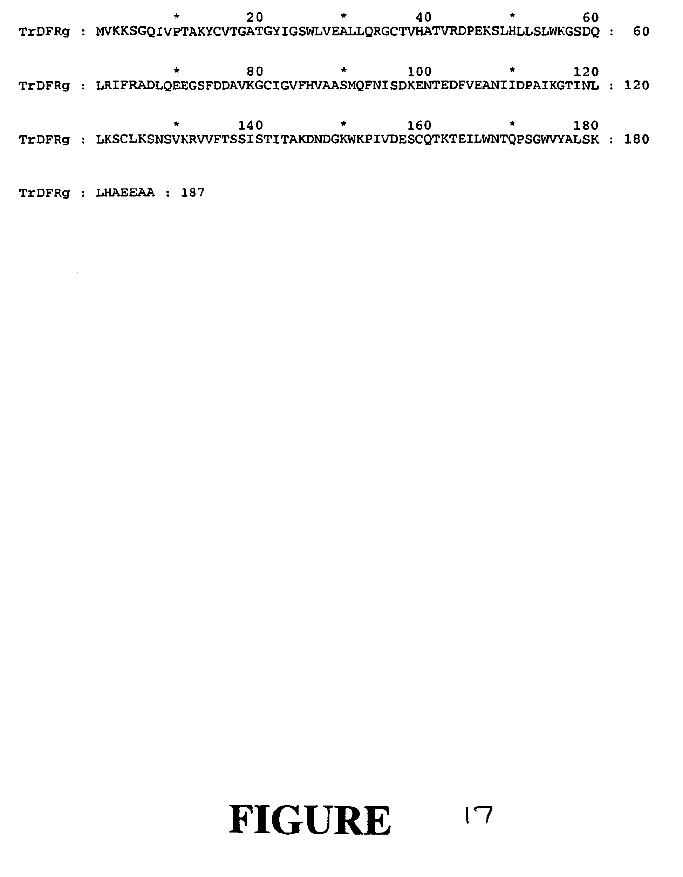

Figure 17 shows the deduced amino acid sequence of TrDFRg (Sequence ID No: 164).
Figure 18 shows the nucleotide sequences of the nucleic acid fragments contributing to the consensus contig sequence TrDFRg (Sequence ID Nos: 165 to 167).

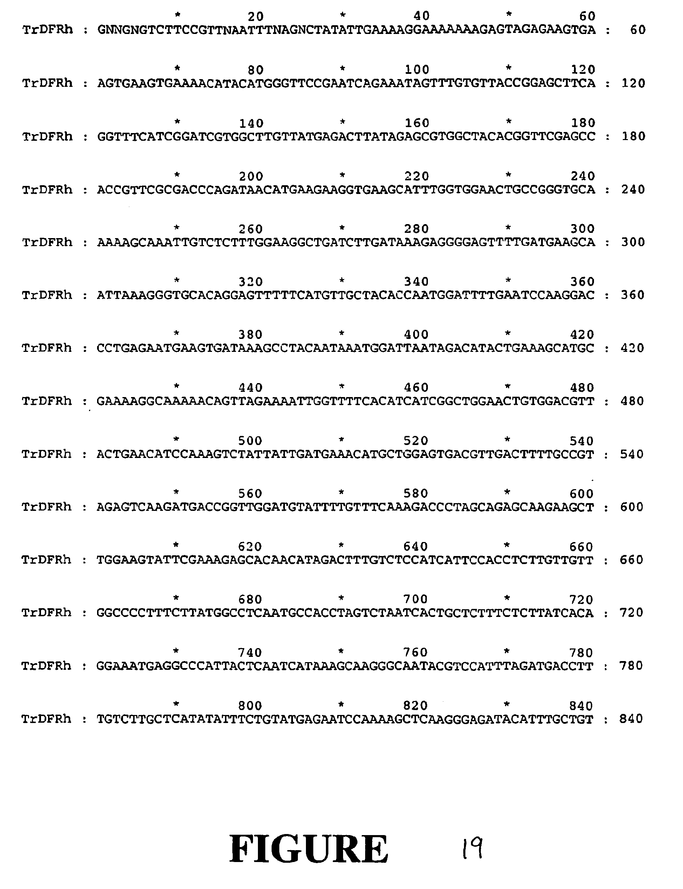

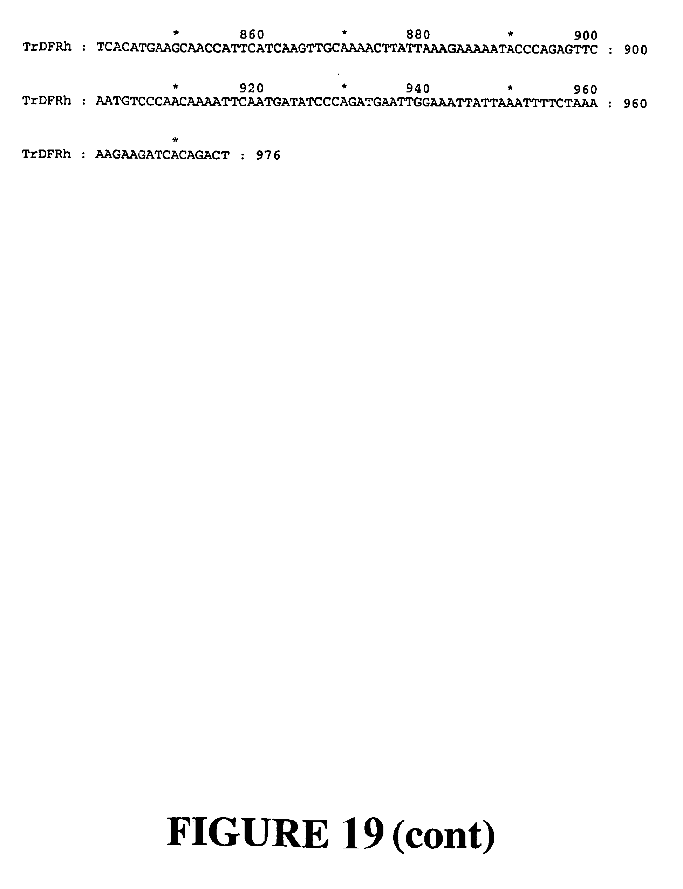

Figure 19 shows the consensus contig nucleotide sequence of TrDFRh (Sequence ID No: 168).

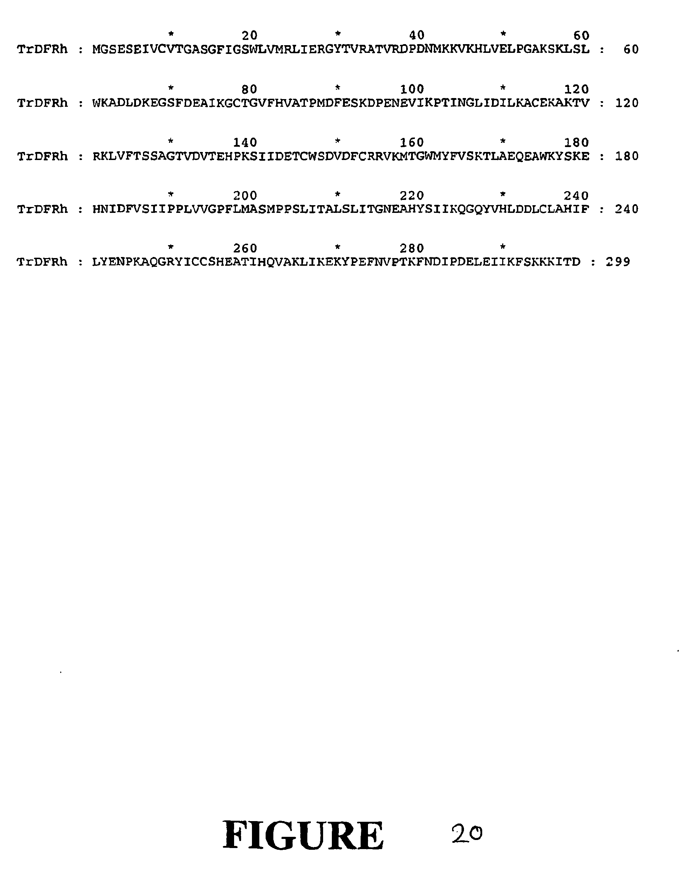

Figure 20 shows the deduced amino acid sequence of TrDFRh (Sequence ID No: 169).
Figure 21 shows the nucleotide sequences of the nucleic acid fragments contributing to the consensus contig sequence TrDFRh (Sequence ID Nos: 170 to 184).

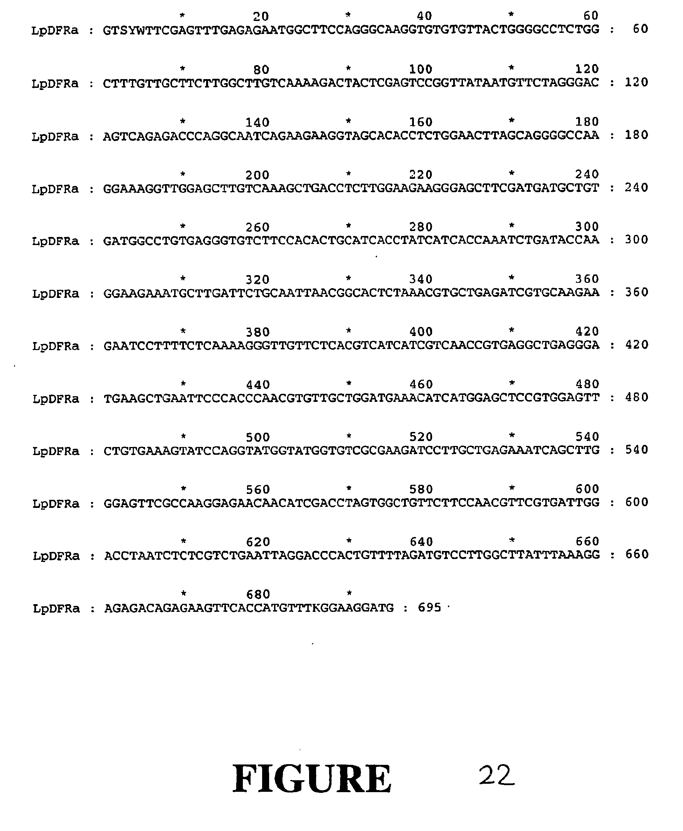

Figure 22 shows the consensus contig nucleotide sequence of LpDFRa (Sequence ID No: 286).

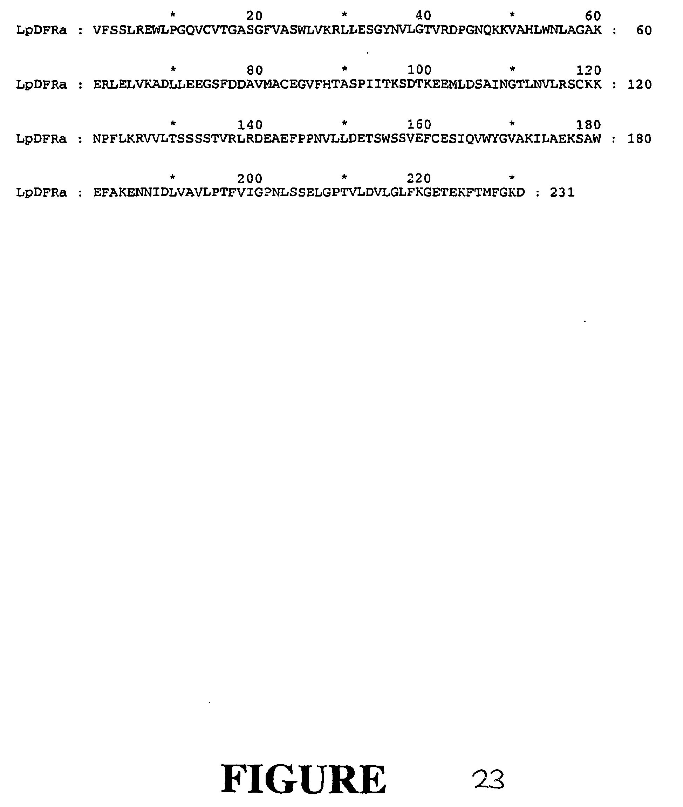

Figure 23 shows the deduced amino acid sequence of LpDFRa (Sequence ID No: 287).
Figure 24 shows the nucleotide sequences of the nucleic acid fragments contributing to the consensus contig sequence LpDFRa (Sequence ID Nos: 288 to 292).

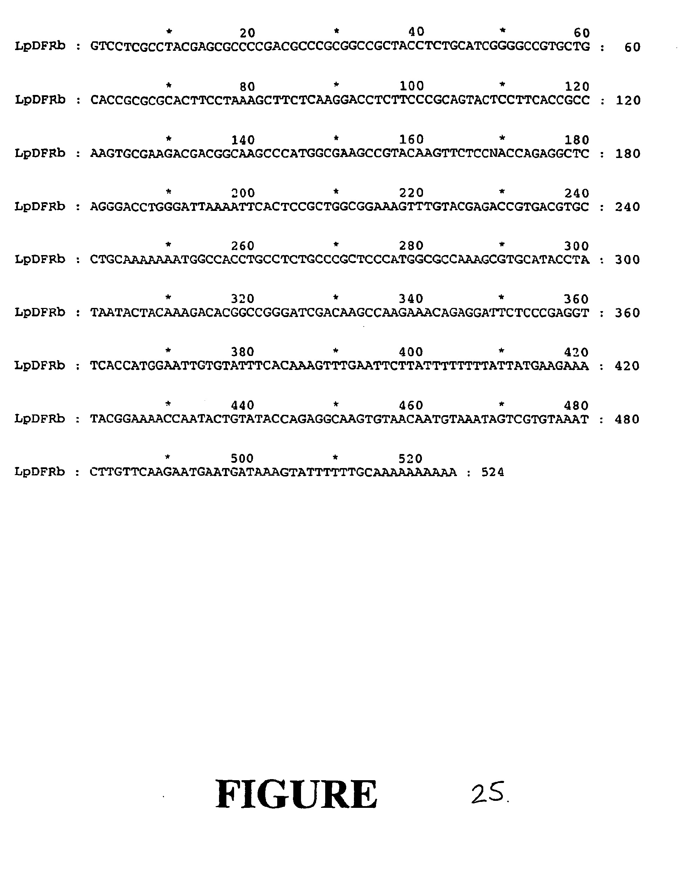

Figure 25 shows the consensus contig nucleotide sequence of LpDFRb (Sequence ID No: 293).

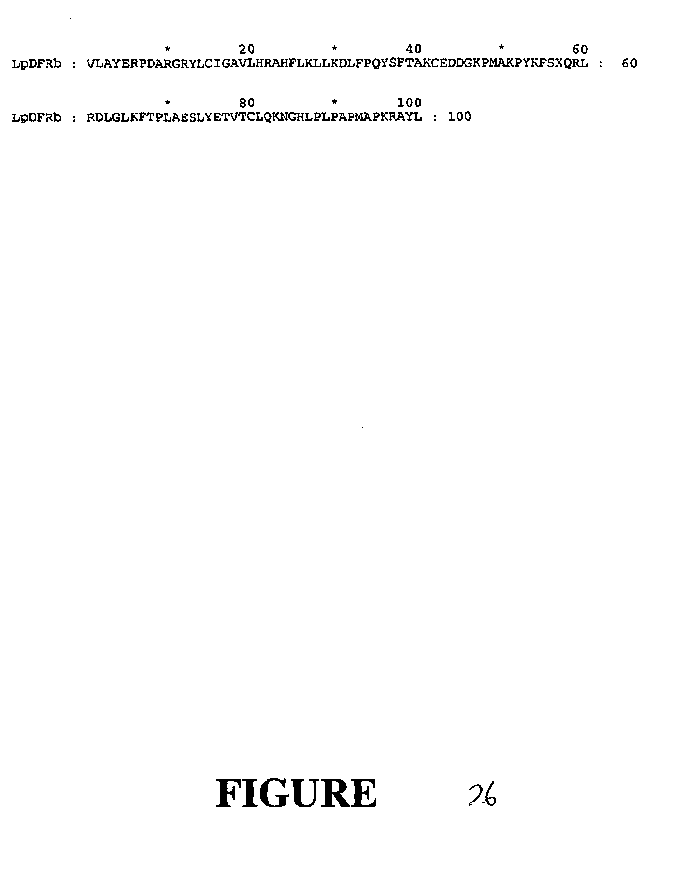

Figure 26 shows the deduced amino acid sequence of LpDFRb (Sequence ID No: 294).
Figure 27 shows the nucleotide sequences of the nucleic acid fragments contributing to the consensus contig sequence LpDFRb (Sequence ID Nos: 295 to 297).

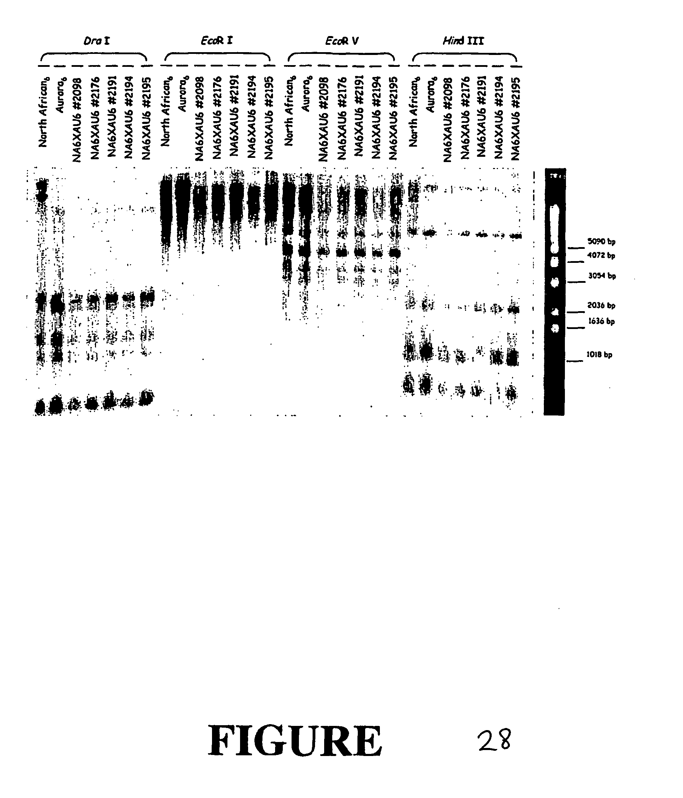

Figure 28 shows screening by Southern hybridisation for RFLPs using LpFSOH as a probe.
Figure 29 shows a plasmid map of the cDNA encoding white clover BANa.

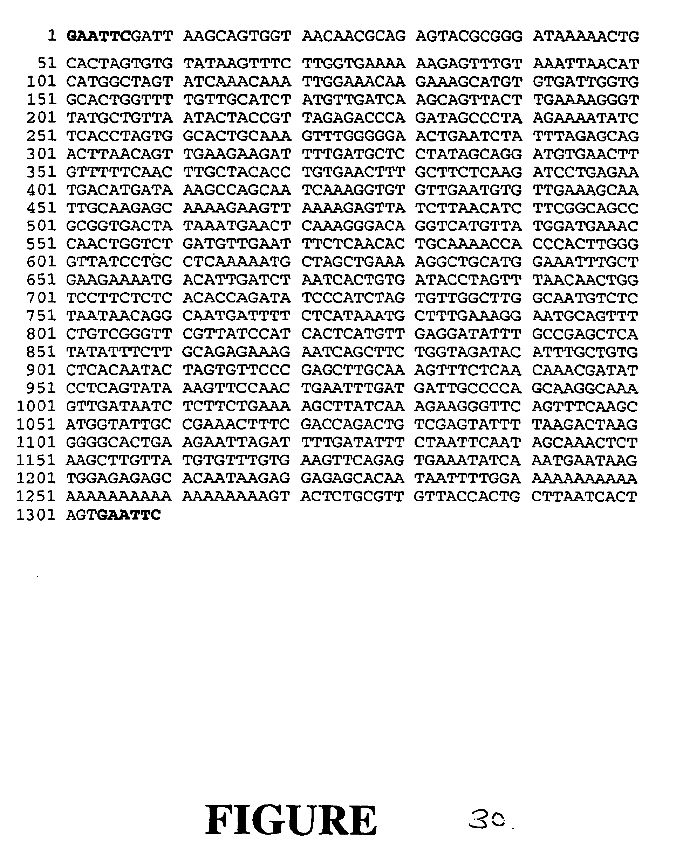

Figure 30 shows the full nucleotide sequence of white clover BANa cDNA (Sequence ID No: 305).
Figure 31 shows the deduced amino acid sequence of white clover BANa cDNA (Sequence ID No: 306).
Figure 32 shows plasmid maps of sense and antisense constructs of TrBANa in pDH51 transformation vector.
Figure 33 shows plasmid maps of sense and antisense constructs of TrBANa in pPZP221:35S² binary transformation vector.
Figure 34 shows a plasmid map of the cDNA encoding white clover DFRd.

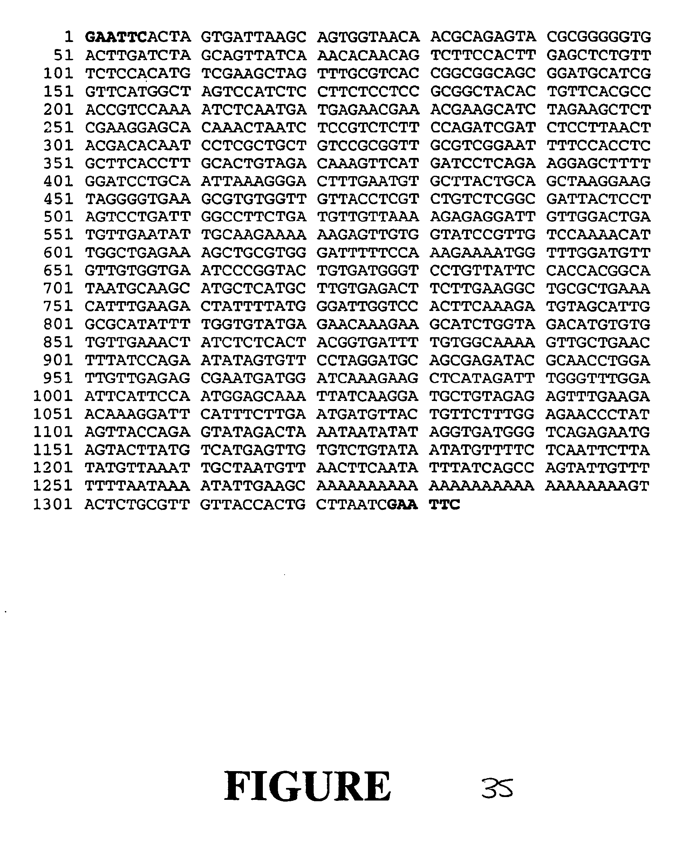

Figure 35 shows the full nucleotide sequence of white clover DFRd cDNA (Sequence ID No: 325).

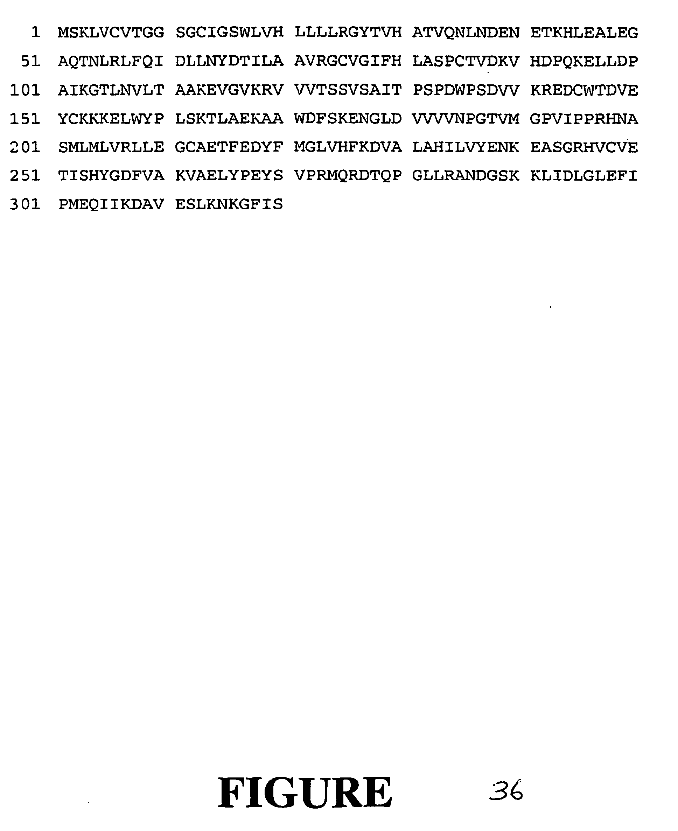

Figure 36 shows the deduced amino acid sequence of white clover DFRd cDNA (Sequence ID No: 326).
Figure 37 shows plasmid maps of sense and antisense constructs of TrDFRd in pDH51 transformation vector.
Figure 38 shows plasmid maps of sense and antisense constructs of TrDFRd in pPZP221:35S² binary transformation vector.

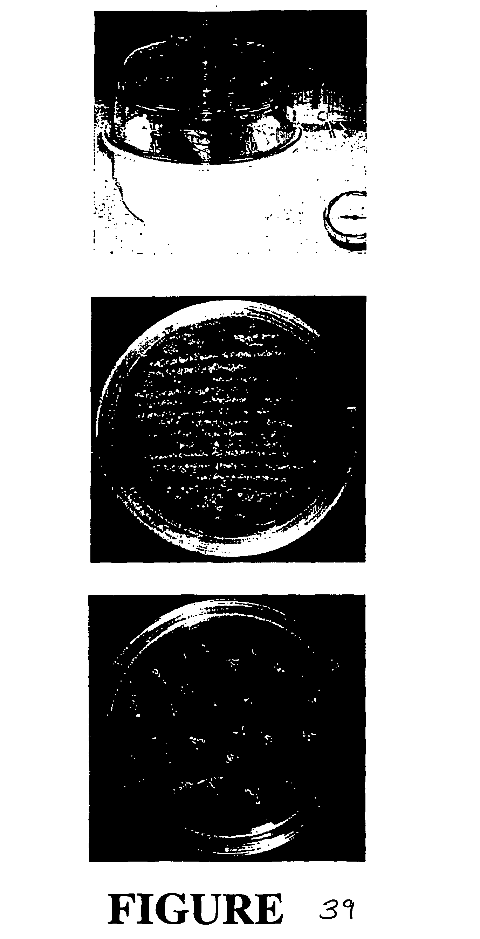

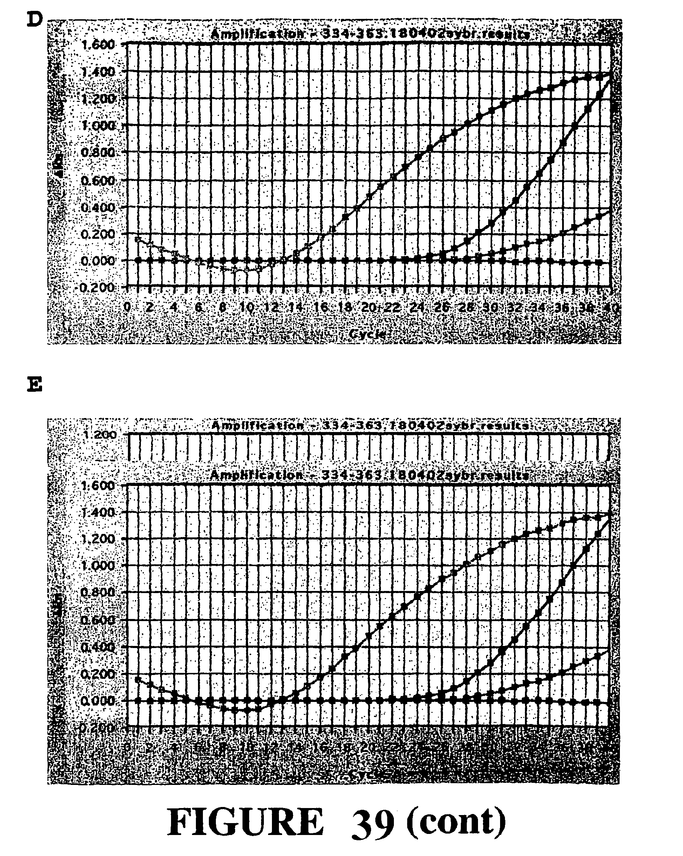

Figure 39 shows A, infiltration of Arabidopsis plants; B, selection of transgenic Arabidopsis plants on medium containing 75 µg/ml gentamycin; C, young transgenic Arabidopsis plants; D, E, two representative results of real-time PCR analysis of Arabidopsis transformed with chimeric genes involved in flavonoid biosynthesis.

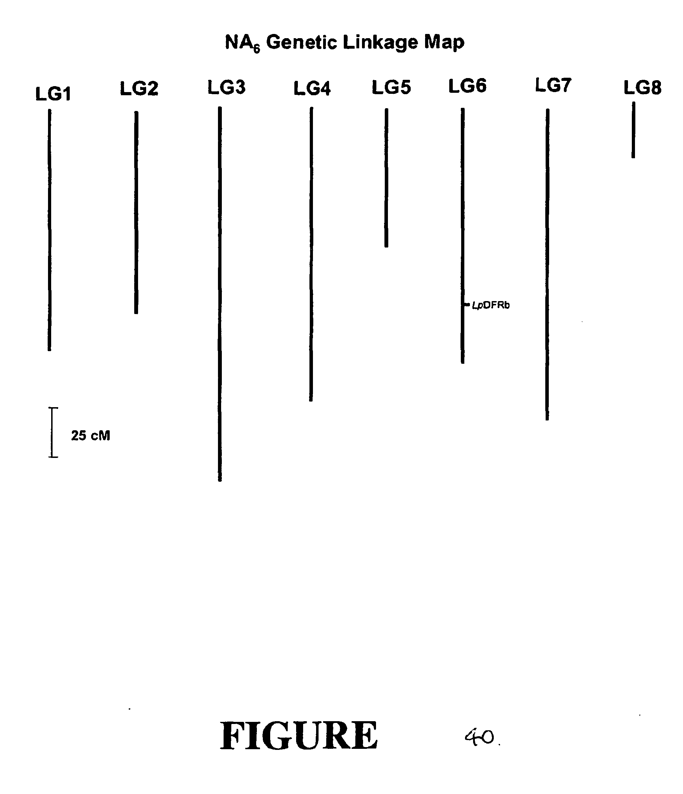

Figure 40 shows the genetic map detailing the relation of perennial ryegrass genes involved in flavonoid biosynthesis.

### EXAMPLE 1

### Preparation of cDNA libraries, isolation and sequencing of cDNAs coding for CHI, CHI-like, CHS, CHS-like, CHR, CHR-like, DFR, DFR-like, LCR, LCR-like, F3'5'H, F3'5'H-like, F3H, F3H-like, F3'H, F3'H-like, PAL, PAL-like, VR and VR-like proteins from white clover (Trifolium repens) and perennial ryegrass (Lolium perenne)

cDNA libraries representing mRNAs from various organs and tissues of white clover (*Trifolium repens*) and perennial ryegrass (*Lolium perenne*) were prepared. The characteristics of the white clover and perennial ryegrass libraries, respectively, are described below (Tables 1 and 2).

**TABLE 1**

| **cDNA libraries from white clover (*Trifolium repens*)** | |
|---|---|
| **Library** | **Organ/Tissue** |
| 01wc | Whole seedling, light grown |
| 02wc | Nodulated root 3, 5, 10, 14, 21 &28 day old seedling |
| 03wc | Nodules pinched off roots of 42 day old rhizobium inoculated plants |
| 04wc | Cut leaf and stem collected after 0, 1, 4, 6 &14 h after cutting |
| 05wc | Inflorescences: <50% open, not fully open and fully open |
| 06wc | Dark grown etiolated |
| 07wc | Inflorescence - very early stages, stem elongation, < 15 petals, 15-20 petals |
| 08wc | seed frozen at -80°C, imbibed in dark overnight at 10°C |
| 09wc | Drought stressed plants |
| 10wc | AMV infected leaf |
| 11wc | WCMV infected leaf |
| 12wc | Phosphorus starved plants |
| 13wc | Vegetative stolon tip |
| 14wc | stolon root initials |
| 15wc | Senescing stolon |
| 16wc | Senescing leaf |

**TABLE 2**

| **cDNA libraries from perennial ryegrass (*Lolium perenne*)** | |
|---|---|
| **Library** | **Organ/Tissue** |
| 01rg | Roots from 3-4 day old light-grown seedlings |
| 02rg | Leaves from 3-4 day old light-grown seedlings |
| 03rg | Etiolated 3-4 day old dark-grown seedlings |
| 04rg | Whole etiolated seedlings (1-5 day old and 17 days old) |
| 05rg | Senescing leaves from mature plants |

| **Library** | **Organ/Tissue** |
|---|---|
| 06rg | Whole etiolated seedlings (1-5 day old and 17 days old) |
| 07rg | Roots from mature plants grown in hydroponic culture |
| 08rg | Senescent leaf tissue |
| 09rg | Whole tillers and sliced leaves (0, 1, 3, 6, 12 and 24 h after harvesting) |
| 10rg | Embryogenic suspension-cultured cells |
| 11 rg | Non-embryogenic suspension-cultured cells |
| 12rg | Whole tillers and sliced leaves (0, 1, 3, 6, 12 and 24 h after harvesting) |
| 13rg | Shoot apices including vegetative apical meristems |
| 14rg | Immature inflorescences including different stages of inflorescence meristem and inflorescence development |
| 15rg | Defatted pollen |
| 16rg | Leaf blades and leaf sheaths (*rbcL, rbcS, cab, wir2A* subtracted) |
| 17rg | Senescing leaves and tillers |
| 18rg | Drought-stressed tillers (pseudostems from plants subjected to PEG-simulated drought stress) |
| 19rg | Non-embryogenic suspension-cultured cells subjected to osmotic stress (grown in media with half-strength salts) (1, 2, 3, 4, 5, 6, 24 and 48 h after transfer) |
| 20rg | Non-embryogenic suspension-cultured cells subjected to osmotic stress (grown in media with double-strength salts) (1, 2, 3, 4, 5, 6, 24 and 48 h after transfer) |
| 21 rg | Drought-stressed tillers (pseudostems from plants subjected to PEG-simulated drought stress) |
| 22rg | Spikelets with open and maturing florets |
| 23rg | Mature roots (specific subtraction with leaf tissue) |

The cDNA libraries may be prepared by any of many methods available. For example, total RNA may be isolated using the Trizol method (Gibco-BRL, USA) or the RNeasy Plant Mini kit (Qiagen, Germany), following the manufacturers' instructions. cDNAs may be generated using the SMART PCR cDNA synthesis kit (Clontech, USA), cDNAs may be amplified by long distance polymerase chain reaction using the Advantage 2 PCR Enzyme system (Clontech, USA), cDNAs may be cleaned using the GeneClean spin column (Bio 101, USA), tailed and size fractionated, according to the protocol provided by Clontech. The cDNAs may be introduced into the pGEM-T Easy Vector system 1 (Promega, USA) according to the protocol provided by Promega. The cDNAs in the pGEM-T Easy plasmid vector are transfected into *Escherichia coli* Epicurian coli XL10-Gold ultra competent cells (Stratagene, USA) according to the protocol provided by Stratagene.

Alternatively, the cDNAs may be introduced into plasmid vectors for first preparing the cDNA libraries in Uni-ZAP XR vectors according to the manufacturer's protocol (Stratagene Cloning Systems, La Jolla, CA, USA). The Uni-ZAP XR libraries are converted into plasmid libraries according to the protocol provided by Stratagene. Upon conversion, cDNA inserts will be contained in the plasmid vector pBluescript. In addition, the cDNAs may be introduced directly into precut pBluescript II SK(+) vectors (Stratagene) using T4 DNA ligase (New England Biolabs), followed by transfection into *E*. *coli* DH10B cells according to the manufacturer's protocol (GIBCO BRL Products).

Once the cDNA inserts are in plasmid vectors, plasmid DNAs are prepared from randomly picked bacterial colonies containing recombinant plasmids, or the insert cDNA sequences are amplified via polymerase chain reaction using primers specific for vector sequences flanking the inserted cDNA sequences. Plasmid DNA preparation may be performed robotically using the Qiagen QiaPrep Turbo kit (Qiagen, Germany) according to the protocol provided by Qiagen. Amplified insert DNAs are sequenced in dye-terminator sequencing reactions to generate partial cDNA sequences (expressed sequence tags or "ESTs"). The resulting ESTs are analyzed using an Applied Biosystems ABI 3700 sequence analyser.

### EXAMPLE 2

### DNA sequence analyses

The cDNA clones encoding CHI, CHI-like, CHS, CHS-like, CHR, CHR-like, DFR, DFR-like, LCR, LCR-like, F3'5'H, F3'S'H-like, F3H, F3H-like, F3'H, F3'H-like, PAL, PAL-like, VR and VR-like proteins were identified by conducting BLAST (Basic Local Alignment Search Tool; Altschul et al. (1993) J. Mol. Biol. 215:403-410) searches. The cDNA sequences obtained were analysed for similarity to all publicly available DNA sequences contained in the eBioinformatics nucleotide database using the BLASTN algorithm provided by the National Center for Biotechnology Information (NCBI). The DNA sequences were translated in all reading frames and compared for similarity to all publicly available protein sequences contained in the SWISS-PROT protein sequence database using BLASTx algorithm (v 2.0.1) (Gish and States (1993) Nature Genetics 3:266-272) provided by the NCBI.

The cDNA sequences obtained and identified were then used to identify additional identical and/or overlapping cDNA sequences generated using the BLASTN algorithm. The identical and/or overlapping sequences were subjected to a multiple alignment using the CLUSTALw algorithm, and to generate a consensus contig sequence derived from this multiple sequence alignment. The consensus contig sequence was then used as a query for a search against the SWISS-PROT protein sequence database using the BLASTx algorithm to confirm the initial identification.

### EXAMPLE 3

### Identification and full-length sequencing of cDNAs encoding perennial ryegrass F30H and white clover BANa, CHIa, CHId, CHRc, CHSa1, CHSa3, CHSc, CHSd2, CHSf, CHSh, DFRd, F3Ha, PALa, PALb, PALf and VRa proteins

To fully characterise for the purposes of the generation of probes for hybridisation experiments and the generation of transformation vectors, a set of cDNAs encoding perennial ryegrass F3OH and white clover BANa, CHIa, CHId, CHRc, CHSa1, CHSa3, CHSc, CHSd2, CHSf, CHSh, DFRd, F3Ha, PALa, PALb, PALf and VRa proteins was identified and fully sequenced.

Full-length cDNAs were identified from our EST sequence database using relevant published sequences (NCBI databank) as queries for BLAST searches. Full-length cDNAs were identified by alignment of the query and hit sequences using Sequencher (Gene Codes Corp., Ann Arbor, MI 48108, USA). The original plasmid was then used to transform chemically competent XL-1 cells (prepared in-house, CaCl₂ protocol). After colony PCR (using HotStarTaq, Qiagen) a minimum of three PCR-positive colonies per transformation were picked for initial sequencing with M13F and M13R primers. The resulting sequences were aligned with the original EST sequence using Sequencher to confirm identity and one of the three clones was picked for full-length sequencing, usually the one with the best initial sequencing result.

Sequencing was completed by primer walking, i.e. oligonucleotide primers were designed to the initial sequence and used for further sequencing. In most cases the sequencing could be done from both 5' and 3' end. The sequences of the oligonucleotide primers are shown in Table 2. In some instances, however, an extended poly-A tail necessitated the sequencing of the cDNA to be completed from the 5' end.

Contigs were then assembled in Sequencher. The contigs include the sequences of the SMART primers used to generate the initial cDNA library as well as pGEM-T Easy vector sequence up to the EcoRI cut site both at the 5' and 3' end.

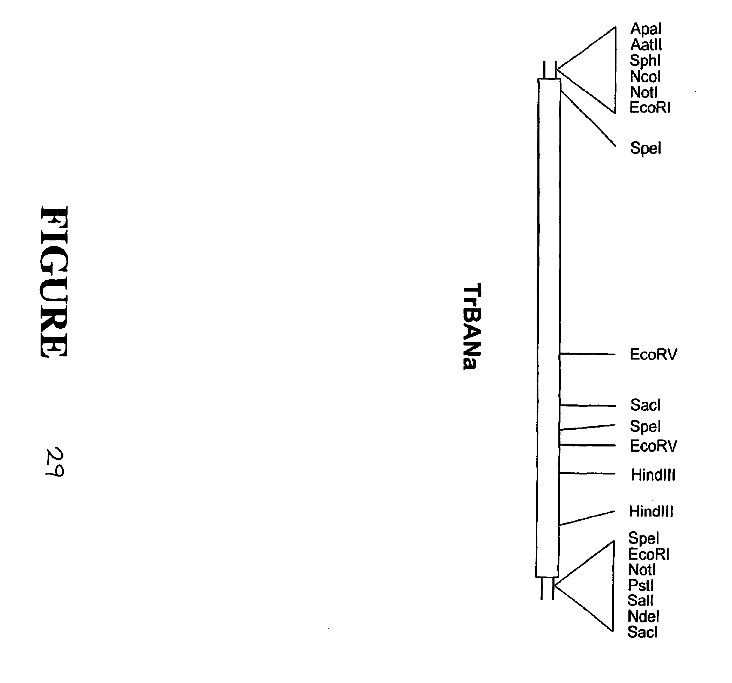

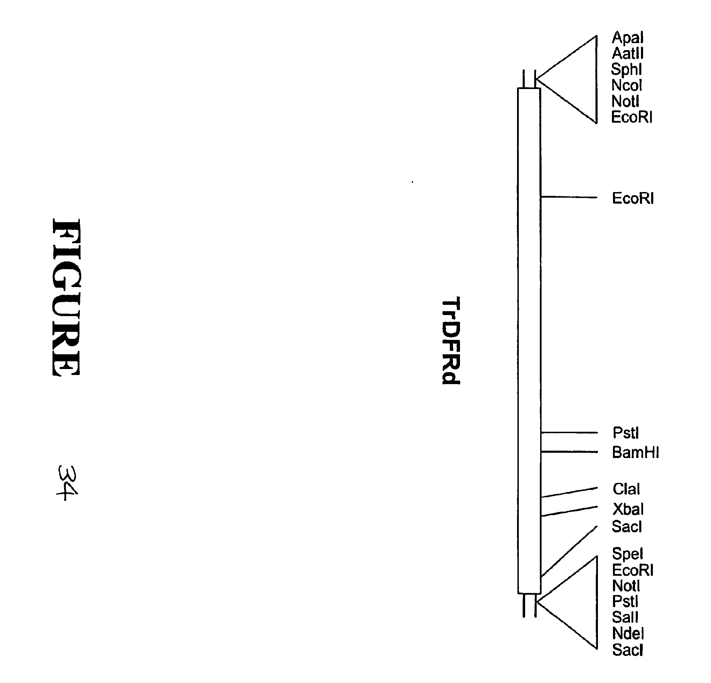

Plasmid maps and the full cDNA sequences of white clover BANa and DFRd proteins were obtained (Figures 29 and 34).

**TABLE 2**

| **List of primers used for sequencing of the full-length cDNAs** | | | |
|---|---|---|---|
| **gene name** | **clone ID** | **sequencing primer** | **primer sequence (5'>3')** |
| LpF3OH | 08rg1YsF07 | 08rg1YsF07.f1 | TTGAGAGCTTCGTCGACC |
| | | 08rg1YsF07.r1 | AACTCCTCGTAGTACTCC |
| TrCHRc | 11wc1IsD03 | 11wc1IsD03.f1 | TTCAATTGGAGTACTTGG |
| | | 11wc1IsD03.r1 | ACTCCTTGTTCATATAACC |
| TrCHSa1 | 02wc2FsD07 | 02wc2FsD07.f1 | ACATGGTGGTGGTTGAGG |
| | | 02wc2FsD07.f2 | TGCTGCACTCATTGTTGG |
| | | 02wc2FsD07.f3 | ACATTGATAAGGCATTGG |
| TrCHSa3 | 05wc1RsB06 | 05wc1RsB06.f1 | AGGAGGCTGCAGTCAAGG |
| | | 05wc1 RsB06.f2 | TGCCTGAAATTGAGAAACC |
| | | 05wc1 RsB06.f3 | AAAGCTAGCCTTGAAGCC |
| TrCHSc | 07wc1TsE12 | 07wc1TsE12.f1 | TCGGACATAACTCATGTGG |
| | | 07wc1TsE12.f2 | TTGGGTTGGAGAATAAGG |
| | | 07wc1TsE12.r1 | TGGACATTTATTGGTTGC |
| | | 07wc1TsE12.r2 | TATCATGTCTGGAAATGC |
| TrCHSd2 | 07wc1XsD03 | 07wc1XsD03.f1 | TTTATGTGAGTACATGGC |
| | | 07wc1XsD03.f2 | AGCAGCTGTGATTGTAGG |
| | | 07wc1XsD03.f3 | TGAGAAAGCTCTTGTTGAGG |
| TrCHSf | 07wc1UsD07 | 07wc1UsD07.f1 | AGATTGCATCAAAGAATGG |
| | | 07wc1UsD07.r1 | GGTCCAAAAGCCAATCC |
| TrCHSh | 13wc21sG04 | 13wc21sG04.f1 | TAAGACGAGACATAGTGG |
| | | 13wc21sG04.r1 | TATTCACTAAGCACATGC |
| TrDFRd | 12wc1CsE09 | 12wc1CsE09.f1 | TTACCTCGTCTGTCTCG |
| | | 12wc1CsE09.r1 | AACACACACATGTCTACC |
| TrF3Ha | 07wc1LsG03 | 07wc1LsG03.f1 | TGAAGGATTGGAGAGAGC |
| | | 07wc1LsG03.r1 | TACACAGTTGCATCTGG |
| TrPALa | 04wc1UsB03 | 04wc1UsB03.f1 | ATCGGAATCTGCTAGAGC |
| | | 04wc1UsB03.f2 | TGTTGGTTCTGGTTTAGC |
| | | 04wc1UsB03.r1 | TTCATATGCAATCCTTGC |
| | | 04wc1UsB03.r2 | TCTTGGTTGTGTTGTTCC |
| TrPALb | 05wc1PsH02 | 05wc1 PsH02.f1 | TGGGACTGATAGTTATGG |
| | | 05wc1PsH02.f2 | TCTTGCTCTTGTTAATGG |
| | | 05wc1 PsH02.r1 | AGCACCATTCCACTCTCC |
| | | 05wc1PsH02.r2 | TTCTCTTCGCTACTTGGC |
| TrPALf | 13wc2AsD12 | 13wc2AsD12.f1 | ATAGTGGTGTGAGGGTGG |
| | | 13wc2AsD12.f2 | TCTTGTTAATGGTACTGC |
| | | 13wc2AsD12.r1 | ATTTATCGCACTCTTCGC |
| | | 13wc2AsD 12.r2 | AAAGTGGAAGACATGAGC |
| TrVRa | 11wc1NsA07 | 11wc1NsA07.f1 | AAGAACAGTGGATGGAGC |
| | | 11wc1NsA07.r1 | TCAACTCATCTACTGATAG |

### EXAMPLE 4

### Development of transformation vectors containing chimeric genes with cDNA sequences from perennial ryegrass F30H and white clover BANa, CHIa, CHId, CHRc, CHSa1, CHSa3, CHSc, CHSd2, CHSf, CHSh, DFRd, F3Ha, PALa, PALb, PALf and VRa

To alter the expression of the proteins involved in flavonoid biosynthesis, protein binding, metal chelation, anti-oxidation, UV-light absorption, tolerance to biotic stresses such as viruses, micro-organisms, insects and fungal pathogens; pigmentation in for example flowers and leaves; herbage quality and bloat-safety and isoflavonoid content leading to health benefits, perennial ryegrass F3OH and white clover BANa, CHIa, CHId, CHRc, CHSa1, CHSa3, CHSc, CHSd2, CHSf, CHSh, DFRd, F3Ha, PALa, PALb, PALf and VRa, through antisense and/or sense suppression technology and for over-expression of these key enzymes in transgenic plants, a set of sense and antisense transformation vectors was produced.

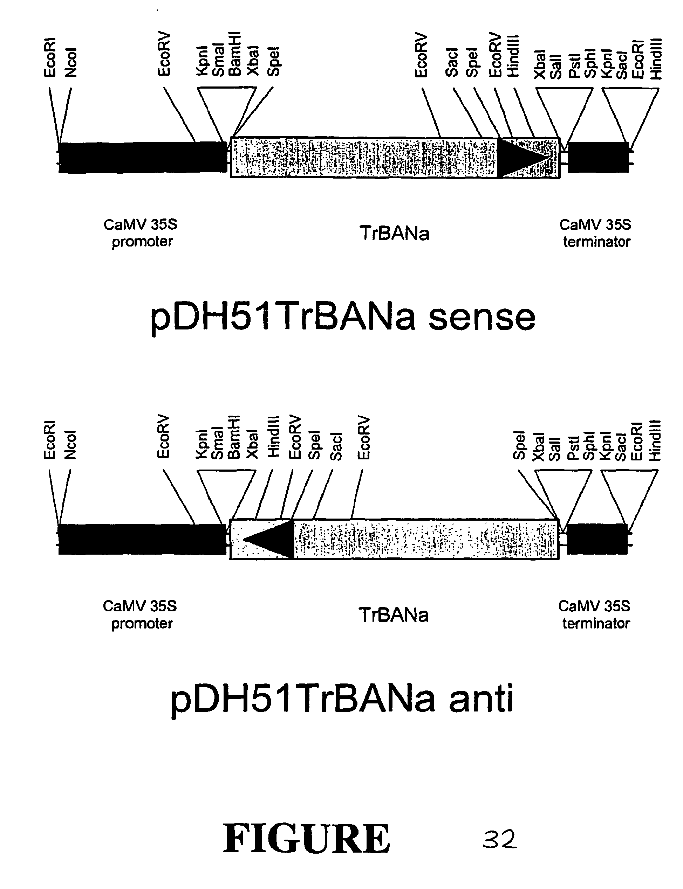

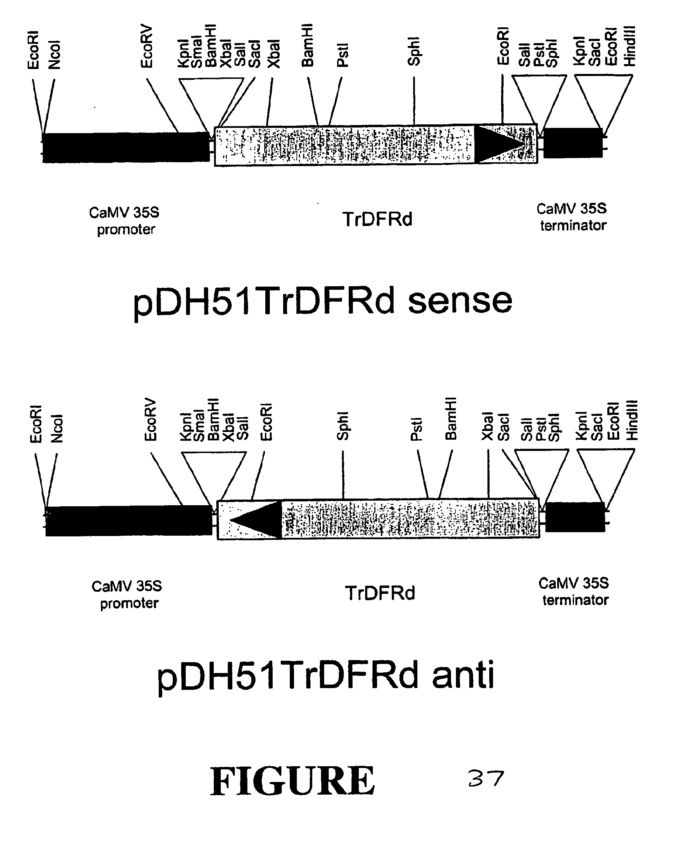

cDNA fragments were generated by high fidelity PCR using the original pGEM-T Easy plasmid cDNA as a template. The primers used (Table 3) contained recognition sites for appropriate restriction enzymes, for example EcoRI and Xbal, for directional and non-directional cloning into the target vector. After PCR amplification and restriction digest with the appropriate restriction enzyme (usually XbaI), the cDNA fragments were cloned into the corresponding site in pDH51, a pUC18-based transformation vector containing a CaMV 35S expression cassette. The orientation of the constructs (sense or antisense) was checked by DNA sequencing through the multi-cloning site of the vector. Transformation vectors containing chimeric genes using full-length open reading frame cDNAs encoding perennial ryegrass F30H and white clover BANa, CHIa, CHId, CHRc, CHSa1, CHSa3, CHSc, CHSd2, CHSF, CHSh, DFRd, F3Ha, PALa, PALb, PALf and VRa proteins in sense and antisense orientations under the control of the CaMV 35S promoter were generated (Figures 32 and 37).

**TABLE 3**

| **List of primers used to PCR-amplify the open reading frames** | | | |
|---|---|---|---|
| **gene name** | **clone ID** | **primer** | **primer sequence (5'->3')** |
| LpF3OH | 08rg1YsF07 | 08rg1YsF07f | GAATTCTAGAAGCAGAAAGTACGGACATCAGC |
| | | 08rg1YsF07r | GAATTCTAGAACCATATGGCGACACATCG |
| TrBANa | 05wc2XsG02 | 05wc2XsG02f | |
| | | 05wc2XsG02r | GGATCCTCTAGACCCCCTTAGTCTTAAAATACTCG |
| TrCHIa | 06wc2AsF12 | 06wc2AsF12f | GAATTCTAGAGATCTGAAACAACATAGTCACC |
| | | 06wc2AsF12r | GAATTCTAGATCAATCTTGTGCTGCAATGC |
| TrCHId | 12wc1FsG04 | 12wc1FsG04f | GAATTCTAGAAAGTTCAACGAGATCAATGG |
| | | 12wc1 FsG04r | GAATTCTAGATTCCGCTTGGTCTTTATTGC |
| TrCHRc | 11wc1IsD03 | 11wc1IsD03f | GAATTCTAGAACATGGGTAGTGTTGAAATTCC |
| | | 11wc1ISD03r | GAATTCTAGAAGATATTGAGTGAGCTTAAGG |
| TrCHSa1 | 02wc2FsD07 | 02wc2FsD07f | GACGTCGACATTACATACATAGCAGGAAC |
| | | 02wc2FsD07r | GACGTCGACAGTCTCTCATTCTCATATAGC |
| TrCHSa3 | 05wc1AsB06 | 05wc1RsB06f | GAATTCTAGAAGATATGGTGAGTGTAGCTG |
| | | 05wc1RsB06r | GAATTCTAGAATCACACATCTTATATAGCC |
| TrCHSc | 07wc1TsE12 | 07wc1TsE12f | GAATTCTAGAAGAAGAAATATGGGAGACGAAGG |
| | | 07wc1TsE12r | GAATTCTAGAAAGACTTCATGCACACAAGTTCC |
| TrCHSd2 | 07wc1XsD03 | 07wc1XsD03f | GAATTCTAGAATAACCTATCAGTACTCACC |
| | | 07wc1XsD03r | GAATTCTAGAATCTAGGCAATTTAAGTGGC |
| TrCHSf | 07wc1UsD07 | 07wc1UsD07f | GAATTCTAGATGATTCATTGTTTGTTTCCATAAC |
| | | 07wc1 UsD07r | GAATTCTAGAACATATTCATCTTCCTATCAC |
| TrCHSh | 13wc2IsG04 | 13wc2IsG04f | GAATTCTAGATCCAAATTCTCGTACCTCACC |
| | | 13wc2IsG04r | GAATTCTAGATAGTTCACATCTCTCGGCAGG |
| TrDFRd | 12wc1CsE09 | 12wc1CsE09f | GACGTCGACACAACAGTCTTCCACTTGAGC |
| | | 12wc1CsE09r | GACGTCGACTCTATACTCTGGTAACTATAGG |
| TrF3Ha | 07wc1 LsG03 | 07wc1 LsG03f | GAATTCTAGAACCACACAACACACAAACACC |
| | | 07wc1LsG03r | GAATTCTAGAACCAAGCAGCTTAATACACG |
| TrPALa | 04wc1UsB03 | 04wc1UsB03f | AGTACTGCAGAGATATGGAAGTAGTAGCAGCAGC |
| | | 04wc1 UsB03r | AGTACTGCAGTAGCAAACCAGTTCCCAACTCC |
| TrPALb | 05wc1PsH02 | 05wc1 PsH02f | AGTACTGCAGATAATGGAGGGAATTACCAATGG |
| | | 05wc1 PsH02r | AGTACTGCAGTGCTAATTAACATATTGGTAGAGG |
| TrPALf | 13wc2AsD12 | 13wc2AsD12f | AGTACTGCAGATAATGGAGGGAATTACCAATGG |
| | | 13wc2AsD12r | AGTACTGCAGTGCTAATTAACATATTGGTAGAGG |
| TrVRa | 11wc1NsA07 | 11wc1NsA07f | AGTACTGCAGATAAAGAGAGTCAAAAATGGC |
| | | 11wc1NsA07r | AGTACTGCAGAACACATACTTAGAGATAGCC |

### EXAMPLE 5

### Development of binary transformation vectors containing chimeric genes with cDNA sequences from perennial ryegrass F30H and white clover BANa, CHIa, CHId, CHRc, CHSa1, CHSa3, CHSc, CHSd2, CHSf, CHSh, DFRd, F3Ha, PALa, PALb, PALf and VRa

To alter the expression of the proteins involved in flavonoid biosynthesis, protein binding, metal chelation, anti-oxidation, UV-light absorption, tolerance to biotic stresses such as viruses, micro-organisms, insects and fungal pathogens; pigmentation in for example flowers and leaves; herbage quality and bloat-safety and isoflavonoid content leading to health benefits, perennial ryegrass F3OH and white clover BANa, CHIa, CHId, CHRc, CHSa1, CHSa3, CHSc, CHSd2, CHSf, CHSh, DFRd, F3Ha, PALa, PALb, PALf and VRa, through antisense and/or sense suppression technology and for over-expression of these key proteins in transgenic plants, a set of sense and antisense binary transformation vectors was produced.

cDNA fragments were generated by high fidelity PCR using the original pGEM-T Easy plasmid cDNA as a template. The primers used (Table 3) contained recognition sites for appropriate restriction enzymes, for example EcoRI and Xbal, for directional and non-directional cloning into the target vector. After PCR amplification and restriction digest with the appropriate restriction enzyme (usually Xbal), the cDNA fragments were cloned into the corresponding site in a modified pPZP binary vector (Hajdukiewicz *et al.,* 1994). The pPZP221 vector was modified to contain the 35S² cassette from pKYLX71:35S² as follows. pKYLX71:35S² was cut with ClaI. The 5' overhang was filled in using Klenow and the blunt end was A-tailed with Taq polymerase. After cutting with EcoRI, the 2kb fragment with an EcoRI-compatible and a 3'-A tail was gel-purified. pPZP221 was cut with HindIII and the resulting 5' overhang filled in and T-tailed with Taq polymerase. The remainder of the original pPZP221 multi-cloning site was removed by digestion with EcoRI, and the expression cassette cloned into the EcoRI site and the 3' T overhang restoring the Hindlll site. This binary vector contains between the left and right border the plant selectable marker gene aaaC1 under the control of the 35S promoter and 35S terminator and the pKYLX71:35S²-derived expression cassette with a CaMV 35S promoter with a duplicated enhancer region and an rbcS terminator.

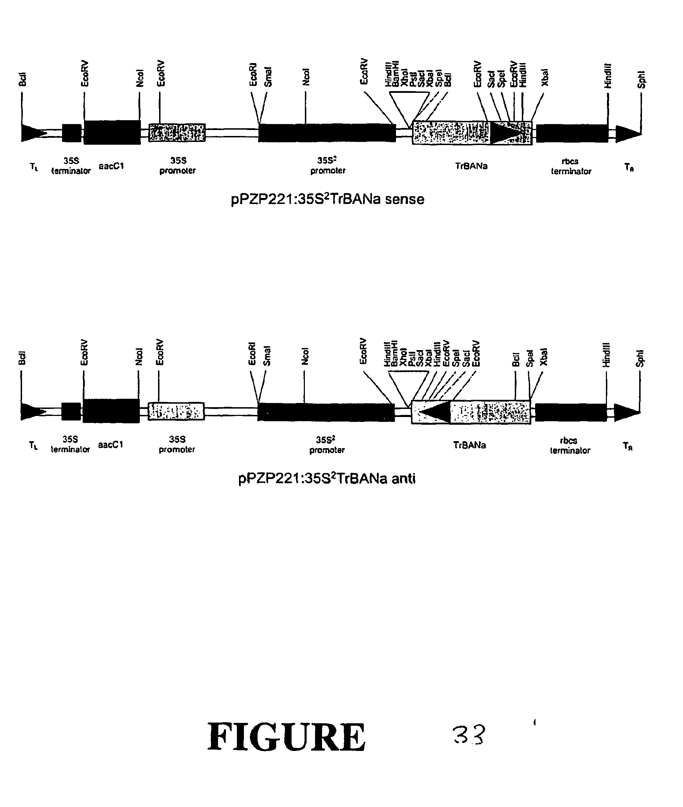

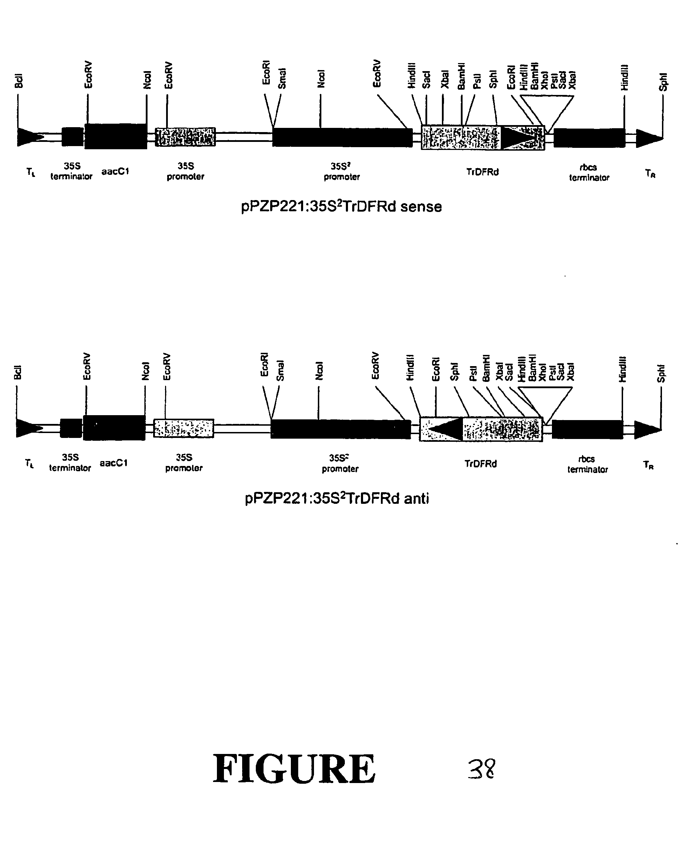

The orientation of the constructs (sense or antisense) was checked by restriction enzyme digest. Transformation vectors containing chimeric genes using full-length open reading frame cDNAs encoding perennial ryegrass F3OH and white clover BANa and DFRd proteins in sense and antisense orientations under the control of the CaMV 35S² promoter were generated (Figures 33 and 38).

### EXAMPLE 6

### Production and analysis of transgenic Arabidopsis plants carrying chimeric perennial ryegrass F30H and white clover BANa, CHIa, CHId, CHRc, CHSa1, CHSa3, CHSc, CHSd2, CHSf, CHSh, DFRd, F3Ha, PALa, PALb, PALf and VRa genes involved in flavonoid biosynthesis

A set of transgenic Arabidopsis plants carrying chimeric perennial ryegrass and white clover genes involved in flavonoid biosynthesis, protein binding, metal chelation, anti-oxidation, UV-light absorption, tolerance to biotic stresses such as viruses, micro-organisms, insects and fungal pathogens; pigmentation in for example flowers and leaves; herbage quality and bloat-safety and isoflavonoid content leading to health benefits, were produced.

pPZP221-based transformation vectors with *Lp*F3OH and *Tr*BANa, *Tr*CHIa, *Tr*CHId, *Tr*CHRc, *Tr*CHSa1, *Tr*CHSa3, *Tr*CHSc, *Tr*CHSd2, *Tr*CHSf, *Tr*CHSh, *Tr*DFRd, *Tr*F3Ha, *Tr*PALa, *Tr*PALb, *Tr*PALf and *Tr*VRa cDNAs comprising the full open reading frame sequences in sense and antisense orientations under the control of the CaMV 35S promoter with duplicated enhancer region (35S²) were generated as detailed in Example 6.

Agrobacterium-mediated gene transfer experiments were performed using these transformation vectors.

The production of transgenic Arabidopsis plants carrying the perennial ryegrass F3OH and white clover BANa, CHIa, CHId, CHRc, CHSa1, CHSa3, CHSc, CHSd2, CHSf, CHSh, DFRd, F3Ha, PALa, PALb, PALf and VRa cDNAs under the control of the CaMV 35S promoter with duplicated enhancer region (35S²) is described here in detail.

### Preparation of Arabidopsis plants

Seedling punnets were filled with Debco seed raising mixture (Debco Pty. Ltd.) to form a mound. The mound was covered with two layers of anti-bird netting secured with rubber bands on each side. The soil was saturated with water and enough seeds (*Arabidopsis thaliana* ecotype Columbia, Lehle Seeds #WT-02) sown to obtain approximately 15 plants per punnet. The seeds were then vernalised by placing the punnets at 4 °C. After 48 hours the punnets were transferred to a growth room at 22 °C under fluorescent light (constant illumination, 55 µmolm⁻²s⁻¹) and fed with Miracle-Gro (Scotts Australia Pty. Ltd.) once a week. Primary bolts were removed as soon as they appeared. After 4 - 6 days the secondary bolts were approximately 6 cm tall, and the plants were ready for vacuum infiltration.

### Preparation of Agrobacterium

*Agrobacterium tumefaciens* strain AGL-1 were streaked on LB medium containing 50 µg/ml rifampicin and 50 µg/ml kanamycin and grown at 27 °C for 48 hours. A single colony was used to inoculate 5 ml of LB medium containing 50 µg/ml rifampicin and 50 µg/ml kanamycin and grown over night at 27 °C and 250 rpm on an orbital shaker. The overnight culture was used as an inoculum for 500 ml of LB medium containing 50 µg/ml kanamycin only. Incubation was over night at 27 °C and 250 rpm on an orbital shaker in a 2 l Erlenmeyer flask.

The overnight cultures were centrifuged for 15 min at 5500 xg and the supernatant discarded. The cells were resuspended in 1 l I of infiltration medium [5% (w/v) sucrose, 0.03% (v/v) Silwet-L77 (Vac-In-Stuff, Lehle Seeds #VIS-01)] and immediately used for infiltration.

### Vacuum infiltration

The *Agrobacterium* suspension was poured into a container (Décor Tellfresh storer, #024) and the container placed inside the vacuum desiccator (Bel Art, #42020-0000). A punnet with *Arabidopsis* plants was inverted and dipped into the Agrobacterium suspension and a gentle vacuum (250 mm Hg) was applied for 2 min. After infiltration, the plants were returned to the growth room where they were kept away from direct light overnight. The next day the plants were returned to full direct light and allowed to grow until the siliques were fully developed. The plants were then allowed to dry out, the seed collected from the siliques and either stored at room temperature in a dry container or used for selection of transformants.

### Selection of transformants

Prior to plating the seeds were sterilised as follows. Sufficient seeds for one 150 mm petri dish (approximately 40 mg or 2000 seeds) were placed in a 1.5 ml microfuge tube. 500 µl 70% ethanol were added for 2 min and replaced by 500 µl sterilisation solution (H₂O:4% chlorine:5% SDS, 15:8:1). After vigorous shaking, the tube was left for 10 min after which time the sterilisation solution was replaced with 500 µl sterile water. The tube was shaken and spun for 5 sec to sediment the seeds. The washing step was repeated 3 times and the seeds were left covered with approximately 200 µl sterile water.

The seeds were then evenly spread on 150 mm petri dishes containing germination medium (4.61 g Murashige & Skoog salts, 10 g sucrose, 1 ml 1 M KOH, 2 g Phytagel, 0.5 g MES and 1 ml 1000x Gamborg's B-5 vitamins per litre) supplemented with 250 µg/ml timetin and 75 µg/ml gentamycin. After vernalisation for 48 hours at 4 °C the plants were grown under continuous fluorescent light (55 µmol m-2s-1) at 22 °C to the 6 - 8 leaf stage and transferred to soil.

### Preparation of genomic DNA

3 - 4 leaves of Arabidopsis plants regenerated on selective medium were harvested and freeze-dried. The tissue was homogenised on a Retsch MM300 mixer mill, then centrifuged for 10 min at 1700xg to collect cell debris. Genomic DNA was isolated from the supernatant using Wizard Magnetic 96 DNA Plant System kits (Promega) on a Biomek FX (Beckman Coulter). 5 µl of the sample (50 µl) were then analysed on an agarose gel to check the yield and the quality of the genomic DNA.

### Analysis of DNA using real-time PCR

Genomic DNA was analysed for the presence of the transgene by real-time PCR using SYBR Green chemistry. PCR primer pairs (Table 4) were designed using MacVector (Accelrys). The forward primer was located within the 35S² promoter region and the reverse primer within the transgene to amplify products of approximately 150 - 250 bp as recommended. The positioning of the forward primer within the 35S² promoter region guaranteed that homologous genes in Arabidopsis were not detected.

5 µl of each genomic DNA sample was run in a 50 µl PCR reaction including SYBR Green on an ABI (Applied Biosystems) together with samples containing DNA isolated from wild type Arabidopsis plants (negative control), samples containing buffer instead of DNA (buffer control) and samples containing the plasmid used for transformation (positive plasmid control).

Plants were obtained after transformation with all chimeric constructs and selection on medium containing gentamycin. The selection process and two representative real-time PCR analyses are shown in Figure xx.

**TABLE 4**

| **List of primers used for Real-time PCR analysis of Arabidopsis plants transformed with chimeric perennial ryegrass genes involved in flavonoid biosynthesis** | | |
|---|---|---|
| **construct** | **primer 1 (forward)** | **primer 2 (reverse)** |
| pPZP221LpF30Hsense | TTGGAGAGGACACGCTGAAATC | AGGAGAGGGTTGGACATCGC |
| PPZP221LpF3OHanti | CATTTCATTTGGAGAGGACACGC | ACGAGGAGTTCTGGAAGATGGG |
| pPZP221TrBANasense | TTGGAGAGGACACGCTGAAATC | GCAACAAAACCAGTGCCACC |
| pPZP221TrBANaanti | TCATTTGGAGAGGACACGCTG | GATGATTGCCCCAGCAAGG |
| pPZP221TrCHlasense | CATTTCATTTGGAGAGGACACGC | CAAGGTTCTCGACTTGGATTGC |
| pPZP221TrCHlaanti | TCATTTGGAGAGGACACGCTG | AGATTACCTGCCTTGTTGAACGAG |
| pPZP221TrCHldsense | TCATTTGGAGAGGACACGCTG | GACGGTAGGAGGGAATAGATTGTTC |
| pPZP221TrCHldanti | TCATTTGGAGAGGACACGCTG | CCAGGTTATCCGAGTTATTCAACG |
| pPZP221TrCHRcsense | CCACTATCCTTCGCAAGACCC | TCCCATTCCAACCACAGGC |
| pPZP221TrCHRcanti | TCATTTGGAGAGGACACGCTG | CAAGCCAGGACTCAGTGACCTATG |
| pPZP221TrCHSa1sense | TCATTTGGAGAGGACACGCTG | CTGGTCAACACGATTTGCTGG |
| pPZP221TrCHSa1anti | TCATTTGGAGAGGACACGCTG | AACCACAGGAGAAGGACTTGACTG |
| pPZP221TrCHSa3sense | CATTTCATTTGGAGAGGACACGC | AACACGGTTTGGTGGATTTGC |
| pPZP221 TrCHSa3anti | TCATTTGGAGAGGACACGCTG | ACAACTGGAGAAGGACTTGATTGG |
| pPZP221TrCHScsense | TTGGAGAGGACACGCTGAAATC | ACAAGTTGGTGAGGGAATGCC |
| pPZP221TrCHScanti | TCATTTGGAGAGGACACGCTG | GGGATTGATACTTGCTTTTGGACC |
| pPZP221TrCHSd2sense | CCCACTATCCTTCGCAAGACC | AGTTGCAGTGCCGATTGCC |
| pPZP221TrCHSd2anti | CATTTCATTTGGAGAGGACACGC | AAGATGGACTTGCCACAACAGG |
| pPZP221 TrCHSfsense | CATTTCATTTGGAGAGGACACGC | TCGTTGCCTTTCCCTGAGTAGG |
| pPZP221 TrCHSfanti | TCATTTGGAGAGGACACGCTG | GATTGGCTTTTGGACCAGGG |
| pPZP221TrCHShsense | TCATTTGGAGAGGACACGCTG | CGGTCACCATTTTTTTGTTGGAGG |
| pPZP221TrCHShanti | TCATTTGGAGAGGACACGCTG | TGTTGTTTGGGTTTGGACCG |
| pPZP221 TrDFRdsense | CATTTCATTTGGAGAGGACACGC | ATTGAGATTTTGGACGGTGGC |
| pPZP221 TrDFRdanti | CATTTCATTTGGAGAGGACACGC | CGCAACCTGGATTGTTGAGAGC |
| pPZP221TrF3Hasense | TCATTTGGAGAGGACACGCTG | CTTCCCTAACGAAACTTGACTCG |
| pPZP221 TrF3Haanti | TCATTTGGAGAGGACACGCTG | GAACAACAACTTAGGGACTTGGAGG |
| pPZP221TrPALasense | ATGACGCACAATCCCACTATCC | TTGCCTCAGCAGCCACACC |
| pPZP221TrPALaanti | GGAGAGGACACGCTGAAATCAC | TGCCAAAAGAGGTTGAAAGTGC |
| pPZP221TrPALbsense | ATCCCACTATCCTTCGCAAGACCC | AATGACTCCCCATCAACGACTCCG |
| pPZP221 TrPALbanti | TTGGAGAGGACACGCTGAAATC | GACAAATTGTTCACAGCTATGTGCC |
| pPZP221 TrPALfsense | ATCCCACTATCCTTCGCAAGACCC | CACCATACGCTTCACCTCATCC |
| pPZP221 TrPALfanti | TCATTTGGAGAGGACACGCTG | TTGTTAGAGAGGAGTTAGGAACCGC |
| pPZP221 TrVRasense | CCACTATCCTTCGCAAGACCC | GCTTACATCCCTCTTACGTTCTGG |
| pPZP221 TrVRaanti | CCACTATCCTTCGCAAGACCC | AAAAGCTCGTGGACGCTGG |

### EXAMPLE 7

### Genetic mapping of perennial ryegrass genes involved in flavonoid biosynthesis, protein binding, metal chelation, anti-oxidation, UV-light absorption, tolerance to biotic stresses such as viruses, micro-organisms, insects and fungal pathogens; pigmentation in for example flowers and leaves; herbage quality and bloat-safety and isoflavonoid content leading to health benefits

The cDNAs representing genes involved in flavonoid biosynthesis, protein binding, metal chelation, anti-oxidation, UV-light absorption, tolerance to biotic stresses such as viruses, micro-organisms, insects and fungal pathogens; pigmentation in for example flowers and leaves; herbage quality and bloat-safety and isoflavonoid content leading to health benefits, were amplified by PCR from their respective plasmids, gel-purified and radio-labelled for use as probes to detect restriction fragment length polymorphisms (RFLPs). RFLPs were mapped in the F₁ (first generation) population, NA₆ x AU₆. This population was made by crossing an individual (NA₆) from a North African ecotype with an individual (AU₆) from the cultivar Aurora, which is derived from a Swiss ecotype. Genomic DNA of the 2 parents and 114 progeny was extracted using the 1 x CTAB method of Fulton et al. (1995).

Probes were screened for their ability to detect polymorphism using the DNA (10 µg) of both parents and 5 F₁ progeny restricted with the enzymes DraI, EcoRI, EcoRV or HindIII. Hybridisations were carried out using the method of Sharp et al. (1988). Polymorphic probes were screened on a progeny set of 114 individuals restricted with the appropriate enzyme (Figure 115).

RFLP bands segregating within the population were scored and the data was entered into an Excel spreadsheet. Alleles showing the expected 1:1 ratio were mapped using MAPMAKER 3.0 (Lander et al. 1987). Alleles segregating from, and unique to, each parent, were mapped separately to give two different linkage maps. Markers were grouped into linkage groups at a LOD of 5.0 and ordered within each linkage group using a LOD threshold of 2.0.

Loci representing genes involved in flavonoid biosynthesis mapped to the linkage groups as indicated in Table 5 and in Figure 197. These gene locations can now be used as candidate genes for quantitative trait loci associated with flavonoid biosynthesis, protein binding, metal chelation, anti-oxidation, UV-light absorption, tolerance to biotic stresses such as viruses, micro-organisms, insects and fungal pathogens; pigmentation in for example flowers and leaves; herbage quality and bloat-safety and isoflavonoid content leading to health benefits.

**TABLE 5**

| **Map locations of ryegrass genes involved in flavonoid biosynthesis across two genetic linkage maps of perennial ryegrass** | | | | | |
|---|---|---|---|---|---|
| **Probe** | **Polymorphic** | **Mapped with** | **Locus** | **Linkage group** | |
| | | | | **NA₆** | **AU₆** |
| *Lp*DFRb | Y | *Hind* III | *Lp*DFRb | 6 | 6 |

### REFERENCES

An, G., Watson, B.D., Stachel, S., Gordon, M.P., Nester, E.W. (1985) New cloning vehicles for transformation of higher plants. The EMBO Journal 4, 227-284
Feinberg, A.P., Vogelstein, B. (1984). A technique for radiolabelling DNA restriction endonuclease fragments to high specific activity. Anal. Biochem. 132: 6-13.
Frohman et al. (1988) Rapid production of full-length cDNAs from rare transcripts: amplification using a single gene-specific oligonucleotide primer. Proc. Natl. Acad Sci. USA 85:8998
Gish and States (1993) Identification of protein coding regions by database similarity search. Nature Genetics 3:266-272
Lander, E.S., Green P., Abrahamson, J., Barlow, A., Daly, M.J., Lincoln, S.E., Newburg, L. (1987). MAPMAKER: an interactive computer package for constructing primary linkage maps of experimental and natural populations. Genomics 1: 174-181.
Loh, E.Y., Elliott, J.F., Cwirla, S., Lanier, L.L., Davis, M.M. (1989). Polymerase chain reaction with single-sided specificity: Analysis of T-cell receptor delta chain. Science 243:217-220
Ohara, O., Dorit, R.L., Gilbert, W. (1989). One-sided polymerase chain reaction: The amplification of cDNA. Proc. Natl. Acad Sci USA 86:5673-5677
Sambrook, J., Fritsch, E.F., Maniatis, T. (1989). Molecular Cloning. A Laboratory Manual. Cold Spring Harbour Laboratory Press
Schardl, C.L., Byrd, A.D., Benzion, G., Altschuler, M.A., Hildebrand, D.F., Hunt, A.G. (1987) Design and construction of a versatile system for the expression of foreign genes in plants. Gene 61, 1-11
Sharp, P.J., Kreis, M., Shewry, P.R., Gale, M.D. (1988). Location of α-amylase sequences in wheat and its relatives. Theor. Appl. Genet. 75:286-290.

It will also be understood that the term "comprises" (or its grammatical variants) as used in this specification is equivalent to the term "includes" and should not be taken as excluding the presence of other elements or features.

Documents cited in this specification are for reference purposes only and their inclusion is not acknowledgment that they form part of the common general knowledge in the relevant art.

## Claims

1. A substantially purified or isolated nucleic acid or nucleic acid fragment encoding a DFR or DFR-like protein or complementary or antisense to a sequence encoding a DFR or DFR-like protein and including a nucleotide sequence selected from the group consisting of
(a) sequence shown in SEQ ID No: 305;
(b) complement of the sequence recited in (a);
(c) sequences antisense to the sequences recited in (a) and (b); and
(d) functionally active variants of the sequences recited in (a), (b) or (c), having at least 90% identity to the sequence recited in (a), (b) or (c), respectively.

2. A nucleic acid or nucleic acid fragment according to Claim 1 from a clover (*Trifolium*) species.

3. A nucleic acid or nucleic acid fragment according to Claim 2, wherein said clover species is white clover (*Trifolium repens*).

4. A nucleic acid or nucleic acid fragment according to any one of Claims 1-3, wherein said nucleic acid or nucleic acid fragment includes the nucleotide sequence shown in Sequence ID No. 305.

5. A polypeptide encoded by a nucleic acid or nucleic acid fragment according to any one of Claims 1 to 4.

6. A construct including a nucleic acid or nucleic acid fragment according to any one of Claims 1 to 4.

7. A vector including a nucleic acid or nucleic acid fragment according to any one of Claims 1 to 4.

8. A vector according to Claim 7, further including a promoter and a terminator, said promoter, nucleic acid or nucleic acid fragment and terminator being operatively linked.

9. A plant cell, plant, plant seed or other plant part, including a construct according to Claim 6 or a vector according to Claim 7 or 8.

10. A plant, plant seed or other plant part derived from a plant cell or plant according to Claim 9 and including a construct according to Claim 6 or a vector according to Claim 7 or 8.

11. A method of modifying flavonoid biosynthesis in a plant, said method including introducing into said plant an effective amount of a nucleic acid or nucleic acid fragment according to any one of Claims 1 to 4, a construct according to Claim 6 and/or a vector according to Claim 7 or 8.

12. A substantially purified or isolated DFR or DFR-like polypeptide including an amino acid sequence selected from the group consisting of
(a) the sequence shown in Sequence ID No: 306, and
(b) functionally active variants of SEQ ID NO: 306, having at least 90% identity to SEQ ID No: 306.

13. A polypeptide according to Claim 12 from a clover (*Trifolium*) species.

14. A polypeptide according to Claim 13, wherein said clover species is white clover (*Trifolium repens*).

15. A polypeptide according to any one of Claims 12 to 14, wherein said polypeptide includes the amino acid sequence shown in SEQ ID No: 306.

## Patentansprüche

1. Im Wesentlichen gereinigte(s) oder isolierte(s) Nukleinsäure oder Nukleinsäurefragment, die/das eine DFR oder ein DFR-ähnliches Protein kodiert oder komplementär oder eine Antisense-Sequenz zu einer Sequenz ist, die eine DFR oder ein DFR-ähnliches Protein kodiert, und eine Nukleotidsequenz enthält, die aus der Gruppe ausgewählt ist, bestehend aus
(a) der in SEQ ID NR: 305 dargestellten Sequenz;
(b) dem Komplement der Sequenz unter (a);
(c) Sequenzen, die Antisense-Sequenzen der Sequenzen unter (a) und (b) sind, und
(d) funktionell aktiven Varianten der unter (a), (b) oder (c) genannten Sequenzen mit mindestens 90% Identität zu der unter (a), (b) bzw. (c) genannten Sequenz.

2. Nukleinsäure oder Nukleinsäurefragment nach Anspruch 1 von einer Klee- (*Trifolium-*) Spezies.

3. Nukleinsäure oder Nukleinsäurefragment nach Anspruch 2, wobei die Klee-Spezies Weißklee (*Trifolium repens*) ist.

4. Nukleinsäure oder Nukleinsäurefragment nach einem der Ansprüche 1-3, wobei die Nukleinsäure oder das Nukleinsäurefragment die in der Sequenz ID NR: 305 dargestellte Nukleotidsequenz enthält.

5. Polypeptid, das von einer Nukleinsäure oder einem Nukleinsäurefragment nach einem der Ansprüche 1 bis 4 kodiert wird.

6. Konstrukt, das eine Nukleinsäure oder ein Nukleinsäurefragment nach einem der Ansprüche 1 bis 4 enthält.

7. Vektor, der eine Nukleinsäure oder ein Nukleinsäurefragment nach einem der Ansprüche 1 bis 4 enthält.

8. Vektor nach Anspruch 7, der außerdem einen Promotor und einen Terminator enthält, wobei der Promotor, die Nukleinsäure oder das Nukleinsäurefragment und der Terminator funktionsfähig miteinander verknüpft sind.

9. Pflanzenzelle, Pflanze, Pflanzensamen oder anderer Pflanzenteil, die/der ein Konstrukt nach Anspruch 6 oder einen Vektor nach Anspruch 7 oder 8 enthält.

10. Pflanze, Pflanzensamen oder anderer Pflanzenteil, die/der von einer Pflanzenzelle oder Pflanze nach Anspruch 9 stammt und ein Konstrukt nach Anspruch 6 oder einen Vektor nach Anspruch 7 oder 8 enthält.

11. Verfahren zum Modifizieren der Flavonoidbiosynthese in einer Pflanze, bei dem man in die Pflanze eine wirksame Menge einer Nukleinsäure oder eines Nukleinsäurefragments nach einem der Ansprüche 1 bis 4, ein Konstrukt nach Anspruch 6 und/oder einen Vektor nach Anspruch 7 oder 8 einbringt.

12. Im Wesentlichen gereinigte(s) oder isolierte(s) DFR oder DFR-ähnliches Polypeptid, die/das eine Aminosäuresequenz enthält, die aus der Gruppe ausgewählt ist, bestehend aus
(a) der in SEQ ID NR: 306 dargestellten Sequenz und
(b) funktionell aktiven Varianten der SEQ ID NR: 306 mit mindestens 90% Identität zu SEQ ID NR: 306.

13. Polypeptid nach Anspruch 12 aus einer Klee-(*Trifolium-*) Spezies.

14. Polypeptid nach Anspruch 13, wobei die Klee-Spezies Weißklee (*Trifolium repens*) ist.

15. Polypeptid nach einem der Ansprüche 12 bis 14, wobei das Polypeptid die in SEQ ID NR: 306 dargestellte Aminosäuresequenz enthält.

## Revendications

1. Acide nucléique ou fragment d'acide nucléique sensiblement purifié ou isolé codant pour une protéine DFR ou de type DFR ou complémentaire ou antisens d'une séquence codant pour une protéine DFR ou de type DFR et comprenant une séquence nucléotidique choisie dans le groupe constitué de
(a) la séquence décrite dans SEQ ID NO: 305 ;
(b) le complément de la séquence décrite dans (a) ;
(c) des séquences antisens pour les séquences décrites dans (a) et (b); et
(d) des variants fonctionnellement actifs des séquences décrites dans (a), (b) ou (c), ayant au moins 90 % d'identité avec la séquence décrite dans (a), (b) ou (c), respectivement.

2. Acide nucléique ou fragment d'acide nucléique selon la revendication 1 d'une espèce de trèfle *(Trifolium).*

3. Acide nucléique ou fragment d'acide nucléique selon la revendication 2, où ladite espèce de trèfle est le trèfle blanc (*Trifolium repens*).

4. Acide nucléique ou fragment d'acide nucléique selon l'une quelconque des revendications 1 à 3, où ledit acide nucléique ou fragment d'acide nucléique comprend la séquence nucléotidique décrite dans Séquence ID n° 305.

5. Polypeptide codé par un acide nucléique ou fragment d'acide nucléique selon l'une quelconque des revendications 1 à 4.

6. Construction comprenant un acide nucléique ou fragment d'acide nucléique selon l'une quelconque des revendications 1 à 4.

7. Vecteur comprenant un acide nucléique ou fragment d'acide nucléique selon l'une quelconque des revendications 1 à 4.

8. Vecteur selon la revendication 7, comprenant en outre un promoteur et un terminateur, lesdits promoteur, acide nucléique ou fragment d'acide nucléique et terminateur étant fonctionnellement liés.

9. Cellule de plante, plante, semence ou autre partie de plante, comprenant une construction selon la revendication 6 ou un vecteur selon la revendication 7 ou 8.

10. Plante, semence de plante ou autre partie de plante dérivée d'une cellule de plante selon la revendication 9 et comprenant une construction selon la revendication 6 ou un vecteur selon la revendication 7 ou 8.

11. Procédé de modification de biosynthèse de flavonoïde dans une plante, ledit procédé comprenant l'introduction dans ladite plante d'une quantité efficace d'un acide nucléique ou fragment d'acide nucléique selon l'une quelconque des revendications 1 à 4, une construction selon la revendication 6 et/ou un vecteur selon la revendication 7 ou 8.

12. Polypeptide DFR ou de type DFR sensiblement purifié ou isolé comprenant une séquence d'acides aminés choisie dans le groupe constitué de
(a) la séquence décrite dans Séquence ID n° 306, et
(b) des variants fonctionnellement actifs de SEQ ID NO: 306, ayant au moins 90 % d'identité avec SEQ ID NO: 306.

13. Polypeptide selon la revendication 12 d'une espèce de trèfle (*Trifolium*).

14. Polypeptide selon la revendication 13, où ladite espèce de trèfle est le trèfle blanc (*Trifolium repens*).

15. Polypeptide selon l'une quelconque des revendications 12 à 14, où ledit polypeptide comprend la séquence d'acides aminés décrite dans SEQ ID No: 306.


### SEQUENCE LISTING

<110> Agriculture Victoria Services Pty Ltd
   AgResearch Limited
<120> Manipulation of flavonoid biosynthesis in plants
<130> M80393510
<150> PR8113
   <151> 2001-10-05
<160> 336
<170> PatentIn version 3.1
   <210> 1
   <211> 636
   <212> DNA
   <213> Trifolium repens
<220>
   <221> misc_feature
   <222> (11) .. (11)
   <223> Any nucleotide
<220>
   <221> misc_feature
   <222> (17) .. (17)
   <223> Any nucleotide
<400> 1
   gcattaaaca ntgaaanttg accagtccca acaaagatct gaaacacata gctccccatt 60
   ttttaacatt aaactaaaaa tatgtcggcc atcaccgcaa tccaagtcga gaaccttgaa 120
   tttccggctg tgattacttc tccggccacc ggtaagtcat attttcttgg tggtgcaggg 180
   gagagaggtt tgactattga aggaaacttc atcaagttca ctgccatagg agtatatttg 240
   gaagatgtag cagtggcttc acttgccact aaatggaagg gcaaatcctc tgaagagttg 300
   cttgagaccc ttgacttcta cagagatatc atttcaggac catttgagaa gttgattcga 360
   ggatcgaaga ttagggaatt gagtggtcct gagtactcaa ggaaggttaa tgaaaactgt 420
   gtggcacact taaaatctgt tgggacttat ggagatgcag aagttgaagc tatgcaaaaa 480
   tttgttgaag ccttcaagcc tattaatttt ccacctggtg cctctgtttt ttacaggcaa 540
   tcacctgatg gaatattagg gcttagtttc tctcaagatg caagtatacc agaaaaggaa 600
   gctgcagtaa tagagaacaa gggagcttca tcggcg 636
<210> 2
   <211> 185
   <212> PRT
   <213> Trifolium repens
<400> 2
   Met Ser Ala Ile Thr Ala Ile Gln Val Glu Asn Leu Glu Phe Pro Ala
   1               5                   10                  15
   Val Ile Thr Ser Pro Ala Thr Gly Lys Ser Tyr Phe Leu Gly Gly Ala
               20                  25                  30
   Gly Glu Arg Gly Leu Thr Ile Glu Gly Asn Phe Ile Lys Phe Thr Ala
           35                  40                  45
   Ile Gly Val Tyr Leu Glu Asp Val Ala Val Ala Ser Leu Ala Thr Lys
       50                  55                  60
   Trp Lys Gly Lys Ser Ser Glu Glu Leu Leu Glu Thr Leu Asp Phe Tyr
   65                  70                  75                  80
   Arg Asp Ile Ile Ser Gly Pro Phe Glu Lys Leu Ile Arg Gly Ser Lys
                   85                  90                  95
   Ile Arg Glu Leu Ser Gly Pro Glu Tyr Ser Arg Lys Val Asn Glu Asn
               100                 105                 110
   Cys Val Ala His Leu Lys Ser Val Gly Thr Tyr Gly Asp Ala Glu Val
           115                 120                 125
   Glu Ala Met Gln Lys Phe Val Glu Ala Phe Lys Pro Ile Asn Phe Pro
       130                 135                 140
   Pro Gly Ala Ser Val Phe Tyr Arg Gln Ser Pro Asp Gly Ile Leu Gly
   145                 150                 155                 160
   Leu Ser Phe Ser Gln Asp Ala Ser Ile Pro Glu Lys Glu Ala Ala Val
                   165                 170                 175
   Ile Glu Asn Lys Gly Ala Ser Ser Ala
               180                 185
<210> 3
   <211> 244
   <212> DNA
   <213> Trifolium repens
<220>
   <221> misc_feature
   <222> (17) .. (17)
   <223> Any nucleotide
<220>
   <221> misc_feature
   <222> (23) .. (23)
   <223> Any nucleotide
<220>
   <221> misc_feature
   <222> (50) .. (50)
   <223> Any nucleotide
<220>
   <221> misc_feature
   <222> (207) .. (208)
   <223> Any nucleotides
<220>
   <221> misc_feature
   <222> (213) .. (213)
   <223> Any nucleotide
<220>
   <221> misc_feature
   <222> (222) .. (222)
   <223> Any nucleotide
<220>
   <221> misc_feature
   <222> (233) .. (233)
   <223> Any nucleotide
<220>
   <221> misc_feature
   <222> (236) .. (236)
   <223> Any nucleotide
<220>
   <221> misc_feature
   <222> (238) .. (238)
   <223> Any nucleotide
<220>
   <221> misc_feature
   <222> (241) .. (241)
   <223> Any nucleotide
<220>
   <221> misc_feature
   <222> (244) .. (244)
   <223> Any nucleotide
<400> 3
   gcattaaaca ttgacanagt ccnaataaaa aagatctgaa acacatagtn ccccattttt
   60
   taacattaaa ctaaaaatat gtcggccatc accgcaatcc aagtcgagaa ccttgaattt
   120
   ccggctgtgg ttacttctcc ggccaccggt aagtcatatt ttcttggtgg tgcaggggag
   180
   agaggtttga ctattgaagg aaacttnntc aanggcgctg gnatacgacc ctnttngnag
   240
   nagn
   244
<210> 4
   <211> 606
   <212> DNA
   <213> Trifolium repens
<220>
   <221> misc_feature
   <222> (2) .. (2)
   <223> Any nucleotide
<220>
   <221> misc_feature
   <222> (605) .. (606)
   <223> Any nucleotides
<400> 4
   gnttgcccgg tcccaacaaa gatctgaaac acatagcccc ccatttttta acattaaact 60
   aaaaatatgt ctgccatcac cgcaatccaa gtcgagaacc ttgaattccc ggcggtgatt 120
   acttctccgg tcaacggtaa gtcatatttt cttggtggtg caggggagag aggtttgact 180
   attgaaggaa acttcatcaa gttcactgcc ataggagtat atttggaaga tgtagcagtg 240
   gcttcacttg ccactaaatg gaagggcaaa tcctctgaag agttgcttga gacccttgac 300
   ttctacagag atatcatttc aggaccattt gagaagttga ttcgaggatc gaagattagg 360
   gaattgagtg gtcctgagta ctcaaggaag gttaatgaaa actgtgtggc acacttaaaa 420
   tctgttggga cttacggaga tgcagaagtt gaagctatgc aaaaatttgt tgaagccttc 480
   aagcctatta attttccacc tggtgcctct gttttttaca ggcaatcacc tgatggaata 540
   ttagggctta gtttctctca agatgcaagt ataccagaaa aggaagctgc agtaatagag 600
   aacann 606
<210> 5
   <211> 599
   <212> DNA
   <213> Trifolium repens
<220>
   <221> misc_feature
   <222> (19) .. (19)
   <223> Any nucleotide
<400> 5
   ggatctgaaa cacatagtnc cccttttaac attaaactaa aaatatgtcg gccatcaccg 60
   caatccaagt cgagaacctt gaatttccag ctgtgattac ttctccggcc accggtaagt 120
   catattttct tggtggtgca ggggagagag gtttgactat tgaaggaaac ttcatcaagt 180
   tcactgccat aggagtatat ttggaagatg tagcagtggc ttcacttgcc actaaatgga 240
   agggtaaatc ctctgaggag ttgcttgaga ctcttgactt ctatagagac atcatttcag 300
   gaccctttga aaagttgatt cgaggatcga agattaggga attgagtggt cctgagtact 360
   caaggaaggt taatgaaaac tgcgtggccc acttaaaatc tgttgggact tatggagatg 420
   ctgaagctga agctatgcaa aaatttgttg aagccttcaa gcctattaat tttccacctg 480
   gtgcctctgt tttttacagg caatcacctg atggaatatt agggcttagt ttctctcaag 540
   atgcaagtat accagaaaag gaggctgcag taatagagaa caagggagct tcatcggcg 599
<210> 6
   <211> 228
   <212> DNA
   <213> Trifolium repens
<220>
   <221> misc_feature
   <222> (2) .. (2)
   <223> Any nucleotide
<220>
   <221> misc_feature
   <222> (15) .. (15)
   <223> Any nucleotide
<220>
   <221> misc_feature
   <222> (31) .. (31)
   <223> Any nucleotide
<220>
   <221> misc_feature
   <222> (54) .. (54)
   <223> Any nucleotide
<220>
   <221> misc_feature
   <222> (212) .. (212)
   <223> Any nucleotide
<220>
   <221> misc_feature
   <222> (214).. (215)
   <223> Any nucleotides
<220>
   <221> misc_feature
   <222> (217) .. (217)
   <223> Any nucleotide
<220>
   <221> misc_feature
   <222> (221) .. (221)
   <223> Any nucleotide
<220>
   <221> misc_feature
   <222> (223) .. (223)
   <223> Any nucleotide
<220>
   <221> misc_feature
   <222> (225) .. (22)
   <223> Any nucleotide
<220>
   <221> misc_feature
   <222> (225) .. (226)
   <223> Any nucleotides
<220>
   <221> misc_feature
   <222> (228) .. (228)
   <223> Any nucleotide
<400> 6
   gngatctgaa aaacntagta cccattttaa nattaaacta aaaatatgtc ggcnatcacc
   60
   gcaatccaag tcgagaacct tgaatttcca gctgtgatta cttctccggc caccggtaag
   120
   tcatattttc ttggtggtgc aggggagaga ggtttgacta ttgaaggaaa cttcatcaag
   180
   ttcactgcca taggagtata tttggaagat gnannanggg ntncnntn
   228
<210> 7
   <211> 589
   <212> DNA
   <213> Trifolium repens
<220>
   <221> misc_feature
   <222> (2) .. (2)
   <223> Any nucleotide
<220>
   <221> misc_feature
   <222> (8) .. (8)
   <223> Any nucleotide
<220>
   <221> misc_feature
   <222> (20) .. (20)
   <223> Any nucleotide
<220>
   <221> misc_feature
   <222> (589) .. (589)
   <223> Any nucleotide
<400> 7
   cnttaaantt gaccagtccn aacaaagatc tgaaacacat agccccccat tttttaacat
   60
   taaactaaaa atatgtctgc catcaccgca atccaagtcg agaaccttga cttcccggcg
   120
   gtgattactt ctccggtcaa cggtaagtca tattttcttg gtggtgcagg ggagagaggt
   180
   ttgactattg aaggaaactt catcaagttc actgccatag gagtatattt ggaagatgta
   240
   gcagtggctt cacttgccac taaatggaag ggcaaatcct ctgaagagtt gcttcagacc
   300
   cttgacttct acagagatat catttcagga ccatttgaga agttgattcg aggatccaag
   360
   attagggaat tgagtggtcc tgagtactca aggaaggtta atgaaaactg tgtggcacac
   420
   ttaaaatctg ttgggactta tggagatgca gaagttgaag ctatgcaaaa atttgttgaa
   480
   gccttcaagc ctattaattt tccacctggt gcctctgttt tttacaggca atcacctgat
   540
   ggaatattag ggcttagttt ctctcaagat gcaagtatac cagaaaagn
   589
<210> 8
   <211> 332
   <212> DNA
   <213> Trifolium repens
<220>
   <221> misc_feature
   <222> (29) .. (29)
   <223> Any nucleotide
<220>
   <221> misc_feature
   <222> (36) .. (36)
   <223> Any nucleotide
<220>
   <221> misc_feature
   <222> (206) .. (206)
   <223> Any nucleotide
<220>
   <221> misc_feature
   <222> (313) .. (313)
   <223> Any nucleotide
<220>
   <221> misc_feature
   <222> (326) .. (326)
   <223> Any nucleotide
<220>
   <221> misc_feature
   <222> (331) .. (331)
   <223> Any nucleotide
<400> 8
   ttaaaattga cacagtccca accttaaant tgaccnggtc ccaaacaaag atctgaaaca
   60
   acatagcccc ccatttttta acattaaact aaaaatatgt ctgccatcac cgcaatccaa
   120
   gtcgagaacc ttgaattccc ggcggtgatt acttctccgg tcaacggtaa gtcatatttt
   180
   cttggtggtg caggggagag aggttngact attgaaggaa acttcatcaa gttcactgcc
   240
   ataggagtat atttggaaga tgtagcaggg gcttcacttg ccactaaatg gaagggcaga
   300
   tcctctgaag agngcttgag accctngact nc
   332
<210> 9
   <211> 78
   <212> PRT
   <213> Trifolium repens
<220>
   <221> MISC_FEATURE
   <222> (37) .. (37)
   <223> Any amino acid
<220>
   <221> MISC_FEATURE
   <222> (73) .. (73)
   <223> Any amino acid
<220>
   <221> MISC_FEATURE
   <222> (77) .. (77)
   <223> Any amino acid
<400> 9
   Met Ser Ala Ile Thr Ala Ile Gln Val Glu Asn Leu Glu Phe Pro Ala
   1               5                   10                  15
   Val Ile Thr Ser Pro Val Asn Gly Lys Ser Tyr Phe Leu Gly Gly Ala
               20                  25                  30
   Gly Glu Arg Gly Xaa Thr Ile Glu Gly Asn Phe Ile Lys Phe Thr Ala
           35                  40                  45
   Ile Gly Val Tyr Leu Glu Asp Val Ala Gly Ala Ser Leu Ala Thr Lys
       50                  55                  60
   Trp Lys Gly Arg Ser Ser Glu Glu Xaa Leu Arg Pro Xaa Thr
   65                  70                  75
<210> 10
   <211> 332
   <212> DNA
   <213> Trifolium repens
<220>
   <221> misc_feature
   <222> (13) .. (13)
   <223> Any nucleotide
<220>
   <221> misc_feature
   <222> (19) .. (19)
   <223> Any nucleotide
<220>
   <221> misc_feature
   <222> (29) .. (29)
   <223> Any nucleotide
<220>
   <221> misc_feature
   <222> (36) .. (36)
   <223> Any nucleotide
<220>
   <221> misc_feature
   <222> (297) .. (297)
   <223> Any nucleotide
<220>
   <221> misc_feature
   <222> (310) .. (310)
   <223> Any nucleotide
<220>
   <221> misc_feature
   <222> (313) .. (313)
   <223> Any nucleotide
<220>
   <221> misc_feature
   <222> (315) .. (315)
   <223> Any nucleotide
<220>
   <221> misc_feature
   <222> (320) .. (320)
   <223> Any nucleotide
<220>
   <221> misc_feature
   <222> (324) .. (324)
   <223> Any nucleotide
<220>
   <221> misc_feature
   <222> (326) .. (326)
   <223> Any nucleotide
<220>
   <221> misc_feature
   <222> (331) .. (332)
   <223> Any nucleotides
<400> 10
   ttaaaattga ccnagtccna accttaaant tgaccnggtc ccaaacaaag atctgaaaca
   60
   acatagcccc ccatttttta acattaaact aaaaatatgt ctgccatcac cgcaatccaa
   120
   gtcgagaacc ttgaattccc ggcggtgatt acttctccgg tcaacggtaa gtcatatttt
   180
   cttggtggtg caggggagag aggtttgact attgaaggaa acttcatcaa gttcactgcc
   240
   ataggagtat atttggaaga tgtagcaggg gcttcacttg ccactaaatg gaagggnaga
   300
   tcctctgaan agngnttgan accntngact nn
   332

<210> 11
   <211> 331
   <212> DNA
   <213> Trifolium repens
<220>
   <221> misc_feature
   <222> (5) .. (5)
   <223> Any nucleotide
<220>
   <221> misc_feature
   <222> (28) .. (28)
   <223> Any nucleotide
<220>
   <221> misc_feature
   <222> (35) .. (35)
   <223> Any nucleotide
<220>
   <221> misc_feature
   <222> (205) .. (205)
   <223> Any nucleotide
<220>
   <221> misc_feature
   <222> (298) .. (298)
   <223> Any nucleotide
<220>
   <221> misc_feature
   <222> (301) .. (301)
   <223> Any nucleotide
<400> 11
   ttaanttgac acagtcccaa ccttaaantt gaccnggtcc caaacaaaga tctgaaacaa
   60
   catagccccc cattttttaa cattaaacta aaaatatgtc tgccatcacc gcaatccaag
   120
   tcgagaacct tgaattcccg gcggtgatta cttctccggt caacggtaag tcatattttc
   180
   ttggtggtgc aggggagaga ggttngacta ttgaaggaaa cttcatcaag ttcactgcca
   240
   taggagtata tttggaagat gtagcagggg cttcacttgc cactaaatgg aagggcanat
   300
   nctctgaaga gtgcttgaga cccttgactt c
   331
<210> 12
   <211> 186
   <212> DNA
   <213> Trifolium repens
<220>
   <221> misc_feature
   <222> (22) .. (22)
   <223> Any nucleotide
<220>
   <221> misc_feature
   <222> (135) .. (135)
   <223> Any nucleotide
<220>
   <221> misc_feature
   <222> (143) .. (143)
   <223> Any nucleotide
<220>
   <221> misc_feature
   <222> (157) .. (157)
   <223> Any nucleotide
<220>
   <221> misc_feature
   <222> (167) .. (167)
   <223> Any nucleotide
<220>
   <221> misc_feature
   <222> (173) .. (174)
   <223> Any nucleotides
<220>
   <221> misc_feature
   <222> (178) .. (179)
   <223> Any nucleotides
<220>
   <221> misc_feature
   <222> (181) .. (182)
   <223> Any nucleotides
<220>
   <221> misc_feature
   <222> (184) .. (184)
   <223> Any nucleotide
<220>
   <221> misc_feature
   <222> (186) .. (186)
   <223> Any nucleotide
<400> 12
   gcgttaaaat tgacccagtc cnaacaaaga tctgaaacac atagcccccc attttttaac
   60
   attaaactaa aaatatgtct gccatcaccg caatccaagt cgagaacctt gacttcccgg
   120
   cggagattac ttctncgggg aangggaagg gatattntct tggtggngga ggnnaganng
   180
   nntngn
   186
<210> 13
   <211> 274
   <212> DNA
   <213> Trifolium repens
<220>
   <221> misc_feature
   <222> (6) . . (6)
   <223> Any nucleotide
<220>
   <221> misc_feature
   <222> (9) . . (9)
   <223> Any nucleotide
<220>
   <221> misc_feature
   <222> (12) . . (12)
   <223> Any nucleotide
<220>
   <221> misc_feature
   <222> (15) . . (15)
   <223> Any nucleotide
<220>
   <221> misc_feature
   <222> (18) . . (18)
   <223> Any nucleotide
<400> 13
   gttagnagna gnatntcngg caccctttga aaagttgatt cgaggatcga agattaggga
   60
   attgagtggt cctgagtact caaggaaggt taatgaaaac tgcgtggcac acttaaaatc
   120
   tgttgggact tatggagatg cagaagctga agctatgcaa aaatttgttg aagccttcaa
   180
   gcctattaat tttccacctg gtgcctctgt tttttacagg caatcacctg atggaatatt
   240
   aggggttagt attgccaatt catttttttt aact
   274
<210> 14
   <211> 94
   <212> PRT
   <213> Trifolium repens
<400> 14
   Ala Pro Phe Glu Lys Leu Ile Arg Gly Ser Lys Ile Arg Glu Leu Ser
   1               5                   10                  15
   Gly Pro Glu Tyr Ser Arg Lys Val Asn Glu Asn Cys Val Ala His Leu
               20                  25                  30
   Lys Ser Val Gly Thr Tyr Gly Asp Ala Glu Ala Glu Ala Met Gln Lys
           35                  40                  45
   Phe Val Glu Ala Phe Lys Pro Ile Asn Phe Pro Pro Gly Ala Ser Val
       50                  55                  60
   Phe Tyr Arg Gln Ser Pro Asp Gly Ile Leu Gly Val Ser Ile Ala Asn
   65                  70                  75                  80
   Ser Phe Phe Leu Thr Ile Leu Ile Arg Val Arg Phe Asp Cys
                   85                  90
<210> 15
   <211> 274
   <212> DNA
   <213> Trifolium repens
<220>
   <221> misc_feature
   <222> (6) . . (6)
   <223> Any nucleotide
<220>
   <221> misc_feature
   <222> (9) . . (9)
   <223> Any nucleotide
<220>
   <221> misc_feature
   <222> (12) . . (13)
   <223> Any nucleotides
<220>
   <221> misc_feature
   <222> (18) . . (18)
   <223> Any nucleotide
<400> 15
   gttagnagna tnntttcngg caccctttga aaagttgatt cgaggatcga agattaggga 60
   attgagtggt cctgagtact caaggaaggt taatgaaaac tgcgtggcac acttaaaatc 120
   tgttgggact tatggagatg cagaagctga agctatgcaa aaatttgttg aagccttcaa 180
   gcctattaat tttccacctg gtgcctctgt tttttacagg caatcacctg atggaatatt
   240
   aggggttagt attgccaatt catttttttt aact 274
<210> 16
   <211> 264
   <212> DNA
   <213> Trifolium repens
<220>
   <221> misc_feature
   <222> (2) . . (2)
   <223> Any nucleotide
<220>
   <221> misc_feature
   <222> (5) . . (5)
   <223> Any nucleotide
<220>
   <221> misc_feature
   <222> (9) . . (9)
   <223> Any nucleotide
<400> 16
   gnatntttng gaccctttga aaagttgatt cgaggatcga agattaggga attgagtggt 60
   cctgagtact caaggaaggt taatgaaaac tgcgtggcac acttaaaatc tgttgggact 120
   tatggagatg cagaagctga agctatgcaa aaatttgttg aagccttcaa gcctattaat 180
   tttccacctg gtgcctctgt tttttacagg caatcacctg atggaatatt aggggttagt 240
   attgccaatt catttttttt aact 264
<210> 17
   <211> 825
   <212> DNA
   <213> Trifolium repens
<220>
   <221> misc_feature
   <222> (3) . . (3)
   <223> Any nucleotide
<220>
   <221> misc_feature
   <222> (5) . . (5)
   <223> Any nucleotide
<220>
   <221> misc_feature
   <222> (7) . . (10)
   <223> Any nucleotides
<220>
   <221> misc_feature
   <222> (13) . . (14)
   <223> Any nucleotides
<220>
   <221> misc_feature
   <222> (487) . . (488)
   <223> Any nucleotides
<220>
   <221> misc_feature
   <222> (507) . . (507)
   <223> Any nucleotide
<220>
   <221> misc_feature
   <222> (545) . . (545)
   <223> Any nucleotide
<220>
   <221> misc_feature
   <222> (579) . . (579)
   <223> Any nucleotide
<220>
   <221> misc_feature
   <222> (654) . . (654)
   <223> Any nucleotide
<220>
   <221> misc_feature
   <222> (716) . . (716)
   <223> Any nucleotide
<220>
   <221> misc_feature
   <222> (719) . . (719)
   <223> Any nucleotide
<220>
   <221> misc_feature
   <222> (725) . . (725)
   <223> Any nucleotide
<220>
   <221> misc_feature
   <222> (738) .. (738)
   <223> Any nucleotide
<220>
   <221> misc_feature
   <222> (762) .. (762)
   <223> Any nucleotide
<220>
   <221> misc_feature
   <222> (787) .. (787)
   <223> Any nucleotide
<400> 17
   ttnantnnnn ttnncgggca attacaacta cacaacacct tctccattac catctatctt
   60
   ctactaagtt caacgagatc aatggcactt ccttctgtca ccgctttgaa tatcgagaac
   120
   aatctattcc ctcctaccgt cacaccaccg ggatccacca acaatttctt cctcggcggt
   180
   gcaggagagc ggggtcttca aattcaagac aaatttgtca aattcaccgc tattggtgtt
   240
   tatctacagg acattgctgt tccttacctc gccactaaat ggaagggtaa gactgctcaa
   300
   gagctaacgg aaactgttcc tttcttcagg gacatcgtta caggtccatt tgagaaattt
   360
   atgcaggtga caatgatctt gccattgact gggcaacaat actcagagaa agtgtcagaa
   420
   aattgtgtag ctatttggaa gtctcttggg atttataccg acgaagaagc caaagcaatt
   480
   gagaagnntg tttctgtctt caaagangaa acattcccac caggctcctc tatccttttc
   540
   acagnattac ccaaaggatt aggatcacta acgataagnt tctctaaaga tggatccatt
   600
   ccagagaccg agtctgcagt tatagagaat aagctactct cacaagctgt gctngagtcg
   660
   atgatagggg cacacggtgt ctcccctgca gcaaaacaga gttttggcca ccaggntanc
   720
   cgagntattc aacgaggntg gctgatgcct agcaacttga tnatatcaac aaaacgaaaa
   780
   tgaaagncct tttctgcaat aaagaacaag cggaaatttt atttt
   825
<210> 18
   <211> 230
   <212> PRT
   <213> Trifolium repens
<220>
   <221> MISC_FEATURE
   <222> (163) .. (163)
   <223> Any amino acid
<220>
   <221> MISC_FEATURE
   <222> (188) .. (188)
   <223> Any amino acid
<220>
   <221> MISC_FEATURE
   <222> (209) . . (210)
   <223> Any amino acids
<220>
   <221> MISC_FEATURE
   <222> (212) . . (212)
   <223> Any amino acid
<220>
   <221> MISC_FEATURE
   <222> (216) .. (216)
   <223> Any amino acid
<220>
   <221> MISC_FEATURE
   <222> (224) .. (224)
   <223> Any amino acid
<400> 18
   Met Ala Leu Pro Ser Val Thr Ala Leu Asn Ile Glu Asn Asn Leu Phe
   1 5 10 15
   Pro Pro Thr Val Thr Pro Pro Gly Ser Thr Asn Asn Phe Phe Leu Gly
   20 25 30
   Gly Ala Gly Glu Arg Gly Leu Gln Ile Gln Asp Lys Phe Val Lys Phe
   35 40 45
   Thr Ala Ile Gly Val Tyr Leu Gln Asp Ile Ala Val Pro Tyr Leu Ala
   50 55 60
   Thr Lys Trp Lys Gly Lys Thr Ala Gln Glu Leu Thr Glu Thr Val Pro
   65 70 75 80
   Phe Phe Arg Asp Ile Val Thr Gly Pro Phe Glu Lys Phe Met Gln Val
   85 90 95
   Thr Met Ile Leu Pro Leu Thr Gly Gln Gln Tyr Ser Glu Lys Val Ser
   100 105 110
   Glu Asn Cys Val Ala Ile Trp Lys Ser Leu Gly Ile Tyr Thr Asp Glu
   115 120 125
   Glu Ala Lys Ala Ile Glu Lys Val Ser Val Phe Lys Glu Thr Phe Pro
   130 135 140
   Pro Gly Ser Ser Ile Leu Phe Thr Leu Pro Lys Gly Leu Gly Ser Leu
   145 150 155 160
   Thr Ile Xaa Phe Ser Lys Asp Gly Ser Ile Pro Glu Thr Glu Ser Ala
   165 170 175
   Val Ile Glu Asn Lys Leu Leu Ser Gln Ala Val Xaa Glu Ser Met Ile
   180 185 190
   Gly Ala His Gly Val Ser Pro Ala Ala Lys Gln Ser Phe Gly His Gln
   195 200 205
   Xaa Xaa Arg Xaa Ile Gln Arg Xaa Trp Leu Met Pro Ser Asn Leu Xaa
   210 215 220
   Ile Ser Thr Lys Arg Lys
   225 230
<210> 19
   <211> 807
   <212> DNA
   <213> Trifolium repens
<220>
   <221> misc_feature
   <222> (3) . . (3)
   <223> Any nucleotide
<220>
   <221> misc_feature
   <222> (5) . . (5)
   <223> Any nucleotide
<220>
   <221> misc_feature
   <222> (7) . . (10)
   <223> Any nucleotides
<220>
   <221> misc_feature
   <222> (13) . . (14)
   <223> Any nucleotides
<220>
   <221> misc_feature
   <222> (21) . . (21)
   <223> Any nucleotide
<220>
   <221> misc_feature
   <222> (23) . . (23)
   <223> Any nucleotide
<220>
   <221> misc_feature
   <222> (25) . . (25)
   <223> Any nucleotide
<220>
   <221> misc_feature
   <222> (99) . . (99)
   <223> Any nucleotide
<220>
   <221> misc_feature
   <222> (147) . . (147)
   <223> Any nucleotide
<220>
   <221> misc_feature
   <222> (229) . . (229)
   <223> Any nucleotide
<220>
   <221> misc_feature
   <222> (288) . . (288)
   <223> Any nucleotide
<220>
   <221> misc_feature
   <222> (316) . . (316)
   <223> Any nucleotide
<220>
   <221> misc_feature
   <222> (335) .. (335)
   <223> Any nucleotide
<220>
   <221> misc_feature
   <222> (337) . . (338)
   <223> Any nucleotides
<220>
   <221> misc_feature
   <222> (417) . . (417)
   <223> Any nucleotide
<220>
   <221> misc_feature
   <222> (434) .. (434)
   <223> Any nucleotide
<220>
   <221> misc_feature
   <222> (437) . . (437)
   <223> Any nucleotide

<220>
   <221> misc_feature
   <222> (486) . . (487)
   <223> Any nucleotides
<220>
   <221> misc_feature
   <222> (506) .. (506)
   <223> Any nucleotide
<220>
   <221> misc_feature
   <222> (544) .. (544)
   <223> Any nucleotide
<220>
   <221> misc_feature
   <222> (561) . . (561)
   <223> Any nucleotide
<220>
   <221> misc_feature
   <222> (578) . . (578)
   <223> Any nucleotide
<220>
   <221> misc_feature
   <222> (625) . . (625)
   <223> Any nucleotide
<220>
   <221> misc_feature
   <222> (653) .. (653)
   <223> Any nucleotide
<220>
   <221> misc_feature
   <222> (672) .. (673)
   <223> Any nucleotides
<220>
   <221> misc_feature
   <222> (682) . . (683)
   <223> Any nucleotides
<220>
   <221> misc_feature
   <222> (685) .. (685)
   <223> Any nucleotide
<220>
   <221> misc_feature
   <222> (690) .. (690)
   <223> Any nucleotide
<220>
   <221> misc_feature
   <222> (695) .. (695)
   <223> Any nucleotide
<220>
   <221> misc_feature
   <222> (706) .. (707)
   <223> Any nucleotides
<220>
   <221> misc_feature
   <222> (715) . . (715)
   <223> Any nucleotide
<220>
   <221> misc_feature
   <222> (718) . . (718)
   <223> Any nucleotide
<220>
   <221> misc_feature
   <222> (721) . . (721)
   <223> Any nucleotide
<220>
   <221> misc_feature
   <222> (724) . . (724)
   <223> Any nucleotide
<220>
   <221> misc_feature
   <222> (737) .. (737)
   <223> Any nucleotide
<220>
   <221> misc_feature
   <222> (754) . . (754)
   <223> Any nucleotide
<220>
   <221> misc_feature
   <222> (761) . . (762)
   <223> Any nucleotides
<220>
   <221> misc_feature
   <222> (764) . . (764)
   <223> Any nucleotide
<220>
   <221> misc_feature
   <222> (766) . . (766)
   <223> Any nucleotide
<220>
   <221> misc_feature
   <222> (775) . . (775)
   <223> Any nucleotide
<220>
   <221> misc_feature
   <222> (782) . . (782)
   <223> Any nucleotide
<220>
   <221> misc_feature
   <222> (784) .. (784)
   <223> Any nucleotide
<220>
   <221> misc_feature
   <222> (786) . . (786)
   <223> Any nucleotide
<400> 19
   ttnantnnnn ttnncggttt ntnanaacta cacaacacct tcttttttcc atttatcttc
   60
   tactaagttc aacgagatca atggcacttc cttctgtcnc cgctttgaat atcgagaaca
   120
   atctattccc tcctaccgtc acaccancgg gatccaccaa caatttcttc ctcggcggtg
   180
   caggagagcg gggtcttcaa attcaagaca aatttgtcaa attcaccgnt attggtgttt
   240
   atctacagga cattgctgtt ccttacctcg ccactaaatg gaagggtnag actgctcaag
   300
   agctaacgga aactgnccct ttcttcaggg acatngnnac aggtccattt gagaaattta
   360
   tgcaggtgac aatgatcttg ccattgactg ggcaacaata ctcagagaaa gtgtcanaaa
   420
   attgtgtacc tatntgnaag tctcttcgga tttataccga ccaagaagcc aaagcaattg
   480
   agaagnntgt ttctgtcttc aaagangaaa cattcccacc aggctcctct atccttttca
   540
   cagnattacc caaaggatta ngatcactaa cgataagntt ctctaaagat ggatccattc
   600
   cagagaccga gtctgcagtt atagngaata agctactctc acaagctgtg ctngagtcga
   660
   tgataggggc anncggtgtc tnncntgcan caaancatag ttttgnncac caggntancc
   720
   nagntattca acgaggntgg ctgatgccta gcancttgat nntntnaaca aaacnaaaat
   780
   gnangncctt ttctgcatta aagaaca
   807
<210> 20
   <211> 266
   <212> DNA
   <213> Trifolium repens
<220>
   <221> misc_feature
   <222> (14) . . (15)
   <223> Any nucleotides
<220>
   <221> misc_feature
   <222> (33) . . (33)
   <223> Any nucleotide
<220>
   <221> misc_feature
   <222> (52) . . (52)
   <223> Any nucleotide
<220>
   <221> misc_feature
   <222> (200) . . (200)
   <223> Any nucleotide
<220>
   <221> misc_feature
   <222> (211) . . (211)
   <223> Any nucleotide
<220>
   <221> misc_feature
   <222> (229) . . (229)
   <223> Any nucleotide
<220>
   <221> misc_feature
   <222> (241) . . (242)
   <223> Any nucleotides
<220>
   <221> misc_feature
   <222> (244) . . (244)
   <223> Any nucleotide
<220>
   <221> misc_feature
   <222> (247) . . (247)
   <223> Any nucleotide
<220>
   <221> misc_feature
   <222> (254) . . (254)
   <223> Any nucleotide
<220>
   <221> misc_feature
   <222> (256) . . (256)
   <223> Any nucleotide
<220>
   <221> misc_feature
   <222> (266) .. (266)
   <223> Any nucleotide
<400> 20
   gcaattacaa cctnncaaca ccttctcctt acntctatct tctactaagt tnaacgagat 60
   caatggcact tccttctgtg accgctttga atatcgagaa caatctattc cctcctaccg 120
   tcacaccacc gggatccacc aacaatttct tcctcggcgg tgcaggagag cggggtcttc 180
   aaattcaaga caaatttgtn aaattgaccg ntattggtgt ttatgtacng gacgttggtg 240
   nngnttncgt gggngntggc tggatn 266
<210> 21
   <211> 797
   <213> DNA
   <213> Trifolium repens
<400> 21
   ttacactcac aacaccttct ccattaccat ctatcttcta ctaagttcaa cgagatcaat 60
   ggcacttcct tctgtcaccg ctttgaatat cgagaacaat ctattccctc ctaccgtcac 120
   accaccggga tccaccaaca atttcttcct cggcggtgca ggagagcggg gtcttcaaat 180
   tcaagacaaa tttgtcaaat tcaccgctat tggtgtttat ctacaggaca ttgctgttcc 240
   ttacctcgcc actaaatgga agggtaagac tgctcaagag ctaacggaaa ctgttccttt 300
   cttcagggac atcgttacag gtccatttga gaaatttatg caggtgacaa tgatcttgcc 360
   attgactggg caacaatact cagagaaagt gtcagaaaat tgtgtagcta tttggaagtc 420
   tcttgggatt tataccgacg aagaagccaa agcaattgag aagtttgttt ctgtcttcaa 480
   agatgaaaca ttcccaccag gctcctctat ccttttcaca gtattaccca aaggattagg 540
   atcactaacg ataagtttct ctaaagatgg atccattcca gagaccgagt ctgcagttat 600
   agagaataag ctactctcac aagctgtgct tgagtcgatg ataggggcgc acggtgtctc
   660
   ccctgcagca aaacagagtt tggccaccag gttatccgag ttattcaacg aggttggtga
   720
   tgctagcaac tgattatatc aacaaaacga aaatgaaagt cctttctgca ataaagacca
   780
   agcggaaatt ttatttt
   797
<210> 22
   <211> 468
   <212> DNA
   <213> Trifolium repens
<220>
   <221> misc_feature
   <222> (30) . . (30)
   <223> Any nucleotide
<220>
   <221> misc_feature
   <222> (47) . . (47)
   <223> Any nucleotide
<220>
   <221> misc_feature
   <222> (436) . . (436)
   <223> Any nucleotide
<220>
   <221> misc_feature
   <222> (442) .. (442)
   <223> Any nucleotide
<220>
   <221> misc_feature
   <222> (450) . . (450)
   <223> Any nucleotide
<220>
   <221> misc_feature
   <222> (453) . . (455)
   <223> Any nucleotides
<220>
   <221> misc_feature
   <222> (458) . . (458)
   <223> Any nucleotide
<220>
   <221> misc_feature
   <222> (464) . . (464)
   <223> Any nucleotide
<220>
   <221> misc_feature
   <222> (467) .. (468)
   <223> Any nucleotides
<400> 22
   tcacattatt acaattacaa cttaacattn actcgtaaaa agagatnaat ggcacttcct 60
   tctgtcaccg ctttggatat cgagaacaat ctattccctc ccaccgtcac accaccggga 120
   tccactgaca acttcttcct cggcggtgca ggagagaggg gtcttcaaat ccaagacaaa 180
   ttcgtcaaat tcaccgctat tggtgtttat ctgcaggaca ttgctgttcc ttacctcgca 240
   gctaaatgga agggtaagac tgctcaagag ctaactcaaa ctgttccttt cttcagggac 300
   atcgttacag gtccatttga gaaatttatg caggtgacaa tgatcttgcc attgactggg 360
   caacaatact cagagaaagt gtcagaaaat tgtgtagcta tttggaagtc tcttgggata 420
   tatacggacg aacaanccaa ancaattgan aannntgntt ctgnttnn 468
<210> 23
   <211> 1130
   <212> DNA
   <213> Trifolium repens
<220>
   <221> misc_feature
   <222> (5) . . (5)
   <223> Any nucleotide
<220>
   <221> misc_feature
   <222> (7) . . (8)
   <223> Any nucleotides
<220>
   <221> misc_feature
   <222> (28) . . (28)
   <223> Any nucleotide
<220>
   <221> misc_feature
   <222> (38) . . (38)
   <223> Any nucleotide
<220>
   <221> misc_feature
   <222> (53) . . (53)
   <223> Any nucleotide
<220>
   <221> misc_feature
   <222> (61) . . (61)
   <223> Any nucleotide
<220>
   <221> misc_feature
   <222> (91) . . (91)
   <223> Any nucleotide
<220>
   <221> misc_feature
   <222> (101) .. (101)
   <223> Any nucleotide
<220>
   <221> misc_feature
   <222> (107) . . (107)
   <223> Any nucleotide
<220>
   <221> misc_feature
   <222> (115) . . (115)
   <223> Any nucleotide
<220>
   <221> misc_feature
   <222> (138) .. (138)
   <223> Any nucleotide
<220>
   <221> misc_feature
   <222> (154) .. (154)
   <223> Any nucleotide
<220>
   <221> misc_feature
   <222> (191) .. (191)
   <223> Any nucleotide
<400> 23
   tattntnnga aaccacttgt gttgaagncg tgaacttngc taccctccat atnatactat 60
   nacctcttct gagacccttc atcatagaaa nacaacacac ntcagcnctt tgctntttct 120
   acaacaacct ataactanac atattatttt tatntattta gtatataatt gaaataaact 180
   gctaaagata nttattaaga tatggtgagt gtagctgaaa ttcgcaaggc tcagagggct 240
   gaaggccctg caaccatttt ggccattggc actgcaaatc cagcaaaccg tgttgagcag 300
   agcacatatc ctgatttcta cttcaaaatc acaaacagtg agcacaagac tgagctcaaa 360
   gagaaattcc agcgcatgtg tgacaaatct atgatcaaga gcagatacat gtatctaaca 420
   gaagagattt tgaaagaaaa tcctagtctt tgtgaataca tggcaccttc attggatgct 480
   aggcaagaca tggtggtggt tgaggtacct agacttggga aggaggctgc agtgaaggct 540
   attaaagaat ggggtcaacc aaagtcaaag attactcact taatcttttg caccacaagt 600
   ggtgtagaca tgcctggtgc tgattaccaa ctcacaaaac tcttaggtct tcgcccatat 660
   gtgaagaggt acatgatgta ccaacaaggg tgctttgcag gtgggacggt gcttcgtttg 720
   gcaaaagatt tggccgagaa caacaaaggt gctcgtgtgt tggttgtttg ttctgaagta 780
   accgcagtca cattccgcgg ccccagtgac actcacttgg acagtcttgt tggacaagca 840
   ctatttggag atggagctgc tgcactcatt gttggctcag acccagtacc agaaattgag 900
   aaaccaatat ttgagatggt atggaccgca cagacaattg ctccagacag tgaaggtgcc 960
   attgatggtc accttcgtga agctggacta acatttcatc ttcttaaaga tgttcctggg 1020
   attgtatcaa agaacattaa taaagcattg gtcgaggctt tccaaccatt aggaatttct 1080
   gattacaact caatcttttg gattgcacac ccgggtggac ctgcaattct 1130
<210> 24
   <211> 309
   <212> PRT
   <213> Trifolium repens
<400> 24
   Met Val Ser Val Ala Glu Ile Arg Lys Ala Gln Arg Ala Glu Gly Pro
   1               5                   10                  15
   Ala Thr Ile Leu Ala Ile Gly Thr Ala Asn Pro Ala Asn Arg Val Glu
               20                  25                  30
   Gln Ser Thr Tyr Pro Asp Phe Tyr Phe Lys Ile Thr Asn Ser Glu His
           35                  40                  45
   Lys Thr Glu Leu Lys Glu Lys Phe Gln Arg Met Cys Asp Lys Ser Met
       50                  55                  60
   Ile Lys Ser Arg Tyr Met Tyr Leu Thr Glu Glu Ile Leu Lys Glu Asn
   65                  70                  75                  80
   Pro Ser Leu Cys Glu Tyr Met Ala Pro Ser Leu Asp Ala Arg Gln Asp
   85 90 95
   Met Val Val Val Glu Val Pro Arg Leu Gly Lys Glu Ala Ala Val Lys
   100 105 110
   Ala Ile Lys Glu Trp Gly Gln Pro Lys Ser Lys Ile Thr His Leu Ile
   115 120 125
   Phe Cys Thr Thr Ser Gly Val Asp Met Pro Gly Ala Asp Tyr Gln Leu
   130 135 140
   Thr Lys Leu Leu Gly Leu Arg Pro Tyr Val Lys Arg Tyr Met Met Tyr
   145 150 155 160
   Gln Gln Gly Cys Phe Ala Gly Gly Thr Val Leu Arg Leu Ala Lys Asp
   165 170 175
   Leu Ala Glu Asn Asn Lys Gly Ala Arg Val Leu Val Val Cys Ser Glu
   180 185 190
   Val Thr Ala Val Thr Phe Arg Gly Pro Ser Asp Thr His Leu Asp Ser
   195 200 205
   Leu Val Gly Gln Ala Leu Phe Gly Asp Gly Ala Ala Ala Leu Ile Val
   210 215 220
   Gly Ser Asp Pro Val Pro Glu Ile Glu Lys Pro Ile Phe Glu Met Val
   225 230 235 240
   Trp Thr Ala Gln Thr Ile Ala Pro Asp Ser Glu Gly Ala Ile Asp Gly
   245 250 255
   His Leu Arg Glu Ala Gly Leu Thr Phe His Leu Leu Lys Asp Val Pro
   260 265 270
   Gly Ile Val Ser Lys Asn Ile Asn Lys Ala Leu Val Glu Ala Phe Gln
   275 280 285
   Pro Leu Gly Ile Ser Asp Tyr Asn Ser Ile Phe Trp Ile Ala His Pro
   290 295 300
   Gly Gly Pro Ala Ile
   305
<210> 25
   <211> 663
   <212> DNA
   <213> Trifolium repens

<220>
   <221> misc_feature
   <222> (5) . . (5)
   <223> Any nucleotide
<220>
   <221> misc_feature
   <222> (7) . . (8)
   <223> Any nucleotides
<220>
   <221> misc_feature
   <222> (28) . . (28)
   <223> Any nucleotide
<220>
   <221> misc_feature
   <222> (38) . . (38)
   <223> Any nucleotide
<220>
   <221> misc_feature
   <222> (53) . . (53)
   <223> Any nucleotide
<220>
   <221> misc_feature
   <222> (61) . . (61)
   <223> Any nucleotide
<220>
   <221> misc_feature
   <222> (97) . . (97)
   <223> Any nucleotide
<220>
   <221> misc_feature
   <222> (128) . . (128)
   <223> Any nucleotide
<220>
   <221> misc_feature
   <222> (138) . . (139)
   <223> Any nucleotides
<220>
   <221> misc_feature
   <222> (148) .. (148)
   <223> Any nucleotide
<220>
   <221> misc_feature
   <222> (151) . . (151)
   <223> Any nucleotide
<220>
   <221> misc_feature
   <222> (163) .. (163)
   <223> Any nucleotide
<220>
   <221> misc_feature
   <222> (171) .. (171)
   <223> Any nucleotide
<400> 25
   tattntnnga aaccacttgt gttgaagncg tgaacttngc taccctccat atnatactat
   60
   nacctcttct gagacccttc atcatatata tataacncat ctcacctcat acatatttcc
   120
   acaacaanct ataacttnnc ctgttatnta naccaattga gtntcaaatt nacatacata
   180
   gcggaacata ctaaatacaa catggttagt gtttctgaaa ttcgcaaggc tcaaagggct
   240
   gaaggccctg caactatttt ggccattggt actgcaaatc cagcaaatcg tgttgaccag
   300
   agtacatatc ctgatttcta cttcaaaatc actaacagtg agcataaggt tgagcttaaa
   360
   gagaaatttc agcgcatgtg tgataaatct atgatcaaga gcagatacat gtatctaaca
   420
   gaagagattt tgaaagaaaa tcctagtctt tgtgaataca tggcaccttc attggatgct
   480
   aggcaagaca tggtggtggt tgaggtacct agacttggga aggaggctgc agtgaaagct
   540
   atcaaagaat ggggtcaacc aaagtcaaag attactcact taatcttttg caccacaagt
   600
   ggtgttgaca tgcctggtgc tgattaccaa ctcacaaaac tcttaggtct tcgcccatat
   660
   gtg
   663
<210> 26
   <211> 575
   <212> DNA
   <213> Trifolium repens
<220>
   <221> misc_feature
   <222> (2) . . (2)
   <223> Any nucleotide
<220>
   <221> misc_feature
   <222> (4) . . (5)
   <223> Any nucleotides
<220>
   <221> misc_feature
   <222> (18) . . (18)
   <223> Any nucleotide
<220>
   <221> misc_feature
   <222> (47) . . (47)
   <223> Any nucleotide
<220>
   <221> misc_feature
   <222> (65) . . (65)
   <223> Any nucleotide
<400> 26
   gnannacaac acacttcnac ctttgctttt ctacaacaac ttctatntaa accttttttt
   60
   agttnaaact tacactactg ctaaagatat atatcaacat ggttagtgtg tctgaaattc
   120
   gcaaggctca aagggctgaa ggccctgcaa ctattttggc cattggcact gcaaatccag
   180
   caaaccgtgt tgatcagagt acatatcctg atttctactt caaaatcact aacagtgagc
   240
   ataaagttga gctcaaagag aaattccagc gcatgtgtga taaatctatg atcaagagca
   300
   gatacatgta tctaacagaa gagattttga aagaaaatcc tagtctttgt gaatacatgg
   360
   caccttcatt ggatgctagg caagatatgg tggtggttga ggtacctaga cttggaaagg
   420
   aggctgcagt gaaggctatt aaagaatggg gtcaaccaaa atctaagatt acacatttga
   480
   tcttttgcac cacaagtggt gtagacatgc ctggtgctga ttaccagccc acaaaacttt
   540
   taggacttcg tccatatgtg aagaggtaca tgatg
   575
<210> 27
   <211> 607
   <212> DNA
   <213> Trifolium repens
<220>
   <221> misc_feature
   <222> (4) . . (4)
   <223> Any nucleotide
<220>
   <221> misc_feature
   <222> (10) . . (10)
   <223> Any nucleotide
<220>
   <221> misc_feature
   <222> (18) . . (18)
   <223> Any nucleotide
<220>
   <221> misc_feature
   <222> (26) . . (26)
   <223> Any nucleotide
<220>
   <221> misc_feature
   <222> (29) . . (30)
   <223> Any nucleotides
<220>
   <221> misc_feature
   <222> (55) . . (55)
   <223> Any nucleotide
<220>
   <221> misc_feature
   <222> (57) . . (57)
   <223> Any nucleotide
<220>
   <221> misc_feature
   <222> (75) . . (76)
   <223> Any nucleotides
<220>
   <221> misc_feature
   <222> (80) . . (81)
   <223> Any nucleotides
<220>
   <221> misc_feature
   <222> (92) . . (95)
   <223> Any nucleotides
<220>
   <221> misc_feature
   <222> (103) .. (104)
   <223> Any nucleotides
<400> 27
   cacnttagcn ctttgctntt tctacnacnn cttctgtcta aacctttttt gagtntnact
   60
   tgcatacata gaaannctan nctgctaaag annnntatca agnnatggtt agtgtgtctg
   120
   aaattcgcaa ggctcaaagg gcagaaggcc ctgcaacaat cttggccatt ggcactgcaa
   180
   atccagcaaa ccgtgttgat cagagcacat atcctgattt ctacttcaaa atcactaaca
   240
   gtgaacataa agttgagctc aaagagaaat tccagcgcat gtgtgataaa tctatgatca
   300
   agagcagata catgtatcta acagaagaga ttttgaaaga aaatcctagt ctttgtgagc
   360
   acatggcacc ttcattggat gctaggcaag acatggtggt ggttgaggta cctagacttg
   420
   ggaaggaggc tgcagtgaaa gctataaaag aatggggtca accaaagtca aagattactc
   480
   acttaatctt ttgcaccaca agtggtgttg acatgcctgg tgctgattac caactcacaa
   540
   aactcttagg tcttcgccca tatgtgaaga ggtacatgat gtaccaacaa gggtgctttg
   600
   caggtgg
   607
<210> 28
   <211> 606
   <212> DNA
   <213> Trifolium repens
<220>
   <221> misc_feature
   <222> (4) . . (4)
   <223> Any nucleotide
<220>
   <221> misc_feature
   <222> (40) . . (40)
   <223> Any nucleotide
<220>
   <221> misc_feature
   <222> (52) . . (52)
   <223> Any nucleotide
<400> 28
   cacnttagcc tttgcttttc tacactcttc tgtctaaacn ttttttgagt tnacttgcat
   60
   acatagaaac tactgctaaa gatatcaaga tggttagtgt gtctgaaatt cgcaaggctc
   120
   aaagggcaga aggccctgca acaatcttgg ccattggcac tgcaaatcca gcaaaccgtg
   180
   ttgatcagag cacatatcct gatttctact tcaaaatcac taacagtgaa cataaagttg
   240
   agctcaaaga gaaattccag cgcatgtgtg ataaatctat gatcaagagc agatacatgt
   300
   atctaacaga agagattttg aaagaaaatc ctagtctttg tgagcacatg gcaccttcat
   360
   tggatgctag gcaagacatg gtggtggttg aggtacctag acttgggaag gaggctgcag
   420
   tgaaagctat aaaagaatgg ggtcaaccaa agtcaaagat tactcactta atcttttgca
   480
   ccacaagtgg tgttgacatg cctggtgctg attaccaact cacaaaactc ttaggtcttc
   540
   gcccatatgt gaagaggtac atgatgtacc aacaagggtg ctttgcaggt gggacggttc
   600
   ttcgtt
   606
<210> 29
   <211> 594
   <212> DNA
   <213> Trifolium repens
<220>
   <221> misc_feature
   <222> (4) . . (4)
   <223> Any nucleotide
<220>
   <221> misc_feature
   <222> (26) . . (26)
   <223> Any nucleotide
<220>
   <221> misc_feature
   <222> (32) . . (33)
   <223> Any nucleotides
<220>
   <221> misc_feature
   <222> (43) . . (43)
   <223> Any nucleotide
<220>
   <221> misc_feature
   <222> (53) . . (53)
   <223> Any nucleotide
<220>
   <221> misc_feature
   <222> (62) . . (62)
   <223> Any nucleotide
<400> 29
   gcanacactc tatccctttc ttttcntata anntacagta ctngcaataa acnactgaat
   60
   tnaattacat aaaaatctta ctattaagat atggtgagtg tatctgaaat tcgtaaggct
   120
   caaagggcag aaggccctgc aaccatttta gccattggta ctgcaaatcc agcaaaccgt
   180
   gtagaccaga gcacatatcc tgatttctac ttcaaaatca ctaacagtga gcataaggtt
   240
   gagcttaaag agaaattcca gcgcatgtgt gataaatcta tgatcaagag cagatacatg
   300
   tatctaacag aagagatttt gaaagaaaat cctagtcttt gtgaatacat ggcaccttca
   360
   ttggatgcta ggcaagacat ggtggtggtt gaggtaccta gactaggaaa ggaggctgca
   420
   gtcaaggcca ttaaagaatg gggtcaacca aagtcaaaga ttactcactt aatcttttgc
   480
   accacaagtg gtgtagacat gcctggtgct gattaccaac tcacaaaact cttgggactt
   540
   cgtccatatg tgaagaggtt catgatgtac caacaaggtt gttttgcagg aggc
   594
<210> 30
   <211> 708
   <212> DNA
   <213> Trifolium repens
<220>
   <221> misc_feature
   <222> (3) . . (3)
   <223> Any nucleotide
<220>
   <221> misc_feature
   <222> (52) . . (52)
   <223> Any nucleotide
<220>
   <221> misc_feature
   <222> (706) . . (706)
   <223> Any nucleotide
<400> 30
   acnaaaacaa ctacgcatat tatatatata tatatagtct ataattgaaa gnaaactgct 60
   aaagatatta ttaagatatg gtgagtgtag ctgaaattcg caaggctcag agggctgaag 120
   gccctgcaac cattttggcc attggcactg caaatccacc aaaccgtgtt gagcagagca 180
   catatcctga tttctacttc aaaattacaa acagtgagca caagactgag ctcaaagaga 240
   agttccaacg catgtgtgac aaatccatga tcaagagcag atacatgtat ctaacagaag 300
   agattttgaa agaaaatcct agtctttgtg aatacatggc accttcattg gatgctaggc 360
   aagacatggt ggtggttgag gtacctagac ttgggaagga ggctgcagtc aaggccatta 420
   aagaatgggg tcaaccaaag tcaaagatta ctcacttaat cttttgcacc acaagtggtg 480
   ttgacatgcc tggtgctgat taccaactca caaaactctt aggtcttcgc ccatatgtga 540
   aaaggtatat gatgtaccaa caaggttgtt ttgcaggagg cacggtgctt cgtttggcaa 600
   aagatttggc cgagaacaac aaaggtgctc gtgtgctagt tgtttgttct gaagtcaccg 660
   cagtcacatt tcgcggcccc agtgatactc acttggacag tcttgntg 708
<210> 31
   <211> 407
   <212> DNA
   <213> Trifolium repens
<220>
   <221> misc_feature
   <222> (4) . . (4)
   <223> Any nucleotide
<220>
   <221> misc_feature
   <222> (20) . . (20)
   <223> Any nucleotide
<220>
   <221> misc_feature
   <222> (26) . . (26)
   <223> Any nucleotide
<220>
   <221> misc_feature
   <222> (28) . . (28)
   <223> Any nucleotide
<220>
   <221> misc_feature
   <222> (41) . . (42)
   <223> Any nucleotides
<220>
   <221> misc_feature
   <222> (73) . . (73)
   <223> Any nucleotide
<220>
   <221> misc_feature
   <222> (329) . . (329)
   <223> Any nucleotide
<220>
   <221> misc_feature
   <222> (345) .. (345)
   <223> Any nucleotide
<220>
   <221> misc_feature
   <222> (365) . . (365)
   <223> Any nucleotide
<220>
   <221> misc_feature
   <222> (378) . . (378)
   <223> Any nucleotide
<220>
   <221> misc_feature
   <222> (383) . . (384)
   <223> Any nucleotides
<220>
   <221> misc_feature
   <222> (386) .. (389)
   <223> Any nucleotides
<220>
   <221> misc_feature
   <222> (402) . . (402)
   <223> Any nucleotide
<220>
   <221> misc_feature
   <222> (407) . . (407)
   <223> Any nucleotide
<400> 31
   ccanggaaaa caacctccgn gcatantnta tatatatata nngtctataa ttgaaagaaa
   60
   ctactaaaga tanttattaa gatatggtga gtgtagctga aattcgcaag gctcagaggg
   120
   ctgaaggccc tgcaaccatt ttggccattg gcactgcaaa tccaccaaac cgtgttgagc
   180
   agagcacata tcctgatttc tacttcaaaa ttacaaacag tgagcacaag actgagctca
   240
   aagagaagtt ccaacgcatg tgtgacaaat ccatgatcaa gagcagatac atgtatctaa
   300
   cagaagagat tttgaaagaa aatcctagnc tttgtgaata catgncacct tcattggatg
   360
   ctagncaaga catggtgncg ccnnannnnc ccaccctccc cncgccn
   407
<210> 32
   <211> 483
   <212> DNA
   <213> Trifolium repens
<220>
   <221> misc_feature
   <222> (2) . . (2)
   <223> Any nucleotide
<220>
   <221> misc_feature
   <222> (21) . . (21)
   <223> Any nucleotide
<220>
   <221> misc_feature
   <222> (37) . . (37)
   <223> Any nucleotide
<220>
   <221> misc_feature
   <222> (388) .. (388)
   <223> Any nucleotide
<220>
   <221> misc_feature
   <222> (408) .. (409)
   <223> Any nucleotides
<220>
   <221> misc_feature
   <222> (444) .. (444)
   <223> Any nucleotide
<220>
   <221> misc_feature
   <222> (449) .. (449)
   <223> Any nucleotide
<220>
   <221> misc_feature
   <222> (453) .. (453)
   <223> Any nucleotide
<220>
   <221> misc_feature
   <222> (476) .. (476)
   <223> Any nucleotide
<220>
   <221> misc_feature
   <222> (479) .. (479)
   <223> Any nucleotide

<220>
   <221> misc_feature
   <222> (483) .. (483)
   <223> Any nucleotide
<400> 32
   gnacaaaaac actcgcatat ncatatatct ctctctngtc tataattgaa agaaactgct
   60
   aaagatatta ttaagatatg gtgagtgtag ctgaaattcg caaggctcag agggctgaag
   120
   gccctgcaac cattttggcc attggcactg caaatccacc aaaccgtgtt gagcagagca
   180
   catatcctga tttctacttc aaaattacaa acagtgagca caagactgag ctcaaagaga
   240
   agttccaacg catgtgtgac aaatccatga tcaagagcag atacatgtat ctaacagaag
   300
   agattttgaa agaaaatcct agtctttgtg aatacatggc accttcattg gatgctaggc
   360
   aagacatggt ggtggttgag gtacctanac ttgggaagga ggctgcannc aaggccatta
   420
   aagaatgggg tcaaccaaag tcanagatna ctnacttaat cttttgcacc acaagnggng
   480
   ccn
   483
<210> 33
   <211> 586
   <212> DNA
   <213> Trifolium repens
<220>
   <221> misc_feature
   <222> (14) . . (14)
   <223> Any nucleotide
<220>
   <221> misc_feature
   <222> (20) . . (20)
   <223> Any nucleotide
<220>
   <221> misc_feature
   <222> (26) . . (26)
   <223> Any nucleotide
<220>
   <221> misc_feature
   <222> (36) . . (36)
   <223> Any nucleotide
<220>
   <221> misc_feature
   <222> (42) . . (42)
   <223> Any nucleotide
<220>
   <221> misc_feature
   <222> (73) . . (73)
   <223> Any nucleotide
<400> 33
   gcacaaaaac actncgctan ttatantata tatatntata tnatatataa ttgaaagaaa
   60
   ctactaaaga tanttattaa gatatggtga gtgtagcaga aattcgcaag gctcagaggg
   120
   ctgaaggccc tgcaaccatt ttggccattg gcactgcaaa tccaccaaac cgtgttgagc
   180
   agagcacata tcctgatttc tacttcaaaa ttacaaacag tgagcacaag actgagctca
   240
   aagagaagtt ccaacgcatg tgtgacaaat ccatgatcaa gagcagatac atgtatctaa
   300
   cagaagagat tttgaaagaa aatcctagtc tttgtgaata catggcacct tcattggatg
   360
   ctagacaaga catggtggtg gttgaggtac ctagacttgg gaaggaggct gcagtcaagg
   420
   ctatcaaaga atggggtcaa ccaaaatcta agattacaca tttgatcttt tgcaccacaa
   480
   gtggtgtaga catgcctggt gctgattacc aactcacaaa actcttagga cttcgtccat
   540
   atgtgaagag gtacatgatg taccaacaag ggtgctttgc aggtgg
   586
<210> 34
   <211> 574
   <212> DNA
   <213> Trifolium repens
<220>
   <221> misc_feature
   <222> (3) . . (3)
   <223> Any nucleotide
<220>
   <221> misc_feature
   <222> (8) . . (8)
   <223> Any nucleotide
<220>
   <221> misc_feature
   <222> (11) . . (11)
   <223> Any nucleotide
<220>
   <221> misc_feature
   <222> (18) . . (19)
   <223> Any nucleotides
<220>
   <221> misc_feature
   <222> (28) . . (28)
   <223> Any nucleotide
<220>
   <221> misc_feature
   <222> (30) . . (31)
   <223> Any nucleotides
<220>
   <221> misc_feature
   <222> (34) . . (35)
   <223> Any nucleotides
<220>
   <221> misc_feature
   <222> (43) . . (43)
   <223> Any nucleotide
<220>
   <221> misc_feature
   <222> (45) . . (46)
   <223> Any nucleotides
<220>
   <221> misc_feature
   <222> (51) . . (51)
   <223> Any nucleotide
<400> 34
   gcnacaanct ntaacttnnc ctgttatntn nacnnattga gtntnnaatt nacatacata
   60
   gcggaacata ctaaatacaa catggttagt gtttctgaaa ttcgcaaggc tcaaagggct
   120
   gaaggccctg caactatttt ggccattggc actgcaaatc cagcaaaccg tgttgatcag
   180
   agcacatatc ctgattttta cttcaaaatc actaacagtg agcataaggt tgagcttaaa
   240
   gagaaatttc agcgcatgtg tgataaatct atgatcaaga gcagatacat gtatctaaca
   300
   gaagagattt tgaaagaaaa tcctagtctt tgtgaataca tggcaccttc attggatgct
   360
   aggcaagaca tggtggtggt tgaggtacct agacttggga aggaggctgc agtgaaagct
   420
   atcaaagaat ggggtcaacc aaagtcaaag attactcact taatcttttg caccacaagt
   480
   ggtgtagaca tgcctggagc tgattaccaa ctcacaaaac tcttaggtct tcgcccatat
   540
   gtgaagaggt acatgatgta ccaacaaggg tgct
   574
<210> 35
   <211> 604
   <212> DNA
   <213> Trifolium repens
<220>
   <221> misc_feature
   <222> (32) . . (32)
   <223> Any nucleotide
<400> 35
   aaaacaaccc cgcatattat atatatatat angtctataa ttgaaagaaa ctgctaaaga 60
   tattattaag atatggtgag tgtagctgaa attcgcaagg ctcagagggc tgaaggccct 120
   gcaaccattt tggccattgg tactgcaaat ccaccaaacc gtgttgagcg gagcacatat 180
   cctgatttct acttcaaaat tacaaacagt gagcacaaga ctgagctcaa agagaagttc 240
   caacgcatgt gtgacaaatc catgatcaag agcagataca tgtatctaac agaagagatt 300
   ttgaaagaaa atcctagtct ttgtgaatac atggcacctt cattggatgc taggcaagac 360
   atggtggtgg ttgaggtacc tagacttggg aaggaggctg cagtgaaagc tatcaaagaa 420
   tggggtcaac caaagtcaaa gattactcac ttaatctttt gcaccacaag tggtgtagac 480
   atgcctggag ctgattacca actcacaaaa ctcttaggtc ttcgcccata tgtgaaaagg 540
   tatatgatgt accaacaagg ttgttttgca ggaggcacgg tgcttcgttt ggcaaaagat 600
   ttgg 604
<210> 36
   <211> 570
   <212> DNA
   <213> Trifolium repens
<220>
   <221> misc_feature
   <222> (9) . . (9)
   <223> Any nucleotide
<220>
   <221> misc_feature
   <222> (37) . . (37)
   <223> Any nucleotide
<400> 36
   caacaaccnt aacttcctgt tattaaccat tgagttnaat tacatacata gcggaacata
   60
   ctaaatacaa catggttagt gtttctgaaa ttcgcaaggc tcaaagggct gaaggccctg
   120
   caactatttt ggccattggt actgcaaatc cagcaaatcg tgttgaccag agtacatatc
   180
   ctgatttcta cttcaaaatc actaacagtg agcataaggt tgagcttaaa gagaaatttc
   240
   agcgcatgtg tgataaatct atgatcaaga gcagatacat gtatctaaca gaagagattt
   300
   tgaaagaaaa tcctagtctt tgtgaataca tggcaccttc attggatgct aggcaagaca
   360
   tggtggtggt tgaggtacct agacttggga aggaggctgc agtgaaagct atcaaagaat
   420
   ggggtcaacc aaagtcaaag attactcact taatcttttg caccacaagt ggtgttgaca
   480
   tgcctggtgc cgattaccaa ctcacaaaac tcttaggtct tcgcccatat gtgaagaggt
   540
   acatgatgta ccaacaaggg tgctttgcag
   570
<210> 37
   <211> 598
   <212> DNA
   <213> Trifolium repens
<220>
   <221> misc_feature
   <222> (10) . . (10)
   <223> Any nucleotide
<220>
   <221> misc_feature
   <222> (17) . . (18)
   <223> Any nucleotides
<220>
   <221> misc_feature
   <222> (26) . . (27)
   <223> Any nucleotides
<220>
   <221> misc_feature
   <222> (29) . . (30)
   <223> Any nucleotides
<220>
   <221> misc_feature
   <222> (34) .. (34)
   <223> Any nucleotide
<220>
   <221> misc_feature
   <222> (42) . . (42)
   <223> Any nucleotide
<220>
   <221> misc_feature
   <222> (44) . . (45)
   <223> Any nucleotides
<220>
   <221> misc_feature
   <222> (50) . . (50)
   <223> Any nucleotide
<220>
   <221> misc_feature
   <222> (67) . . (67)
   <223> Any nucleotide
<400> 37
   caacaacctn taacttnncc tgttanntnn accnattgag tntnnaattn acatacatag
   60
   cggaacntac taaatacaac atggttagtg tttctgaaat tcgcaaggct caaagggctg
   120
   aaggccctgc aactattttg gccattggta ctgcaaatcc agcaaatcgt gttgaccaga
   180
   gtacatatcc tgatttctac ttcaaaatca ctaacagtga gcataaggtt gagcttaaag
   240
   agaaatttca gcgcatgtgt gataaatcta tgatcaagag cagatacatg tatctaacag
   300
   aagagatttt gaaagaaaat cctagtcttt gtgaatacat ggcaccttca ttggatgcta
   360
   ggcaagacat ggtggtggtt gaggtaccta gacttgggaa ggaggctgca gtgaaagcta
   420
   tcaaagaatg gggtcaacca aagtcaaaga ttactcactt aatcttttgc accacaagtg
   480
   gtgtagacat gcctggagct gattaccaac tcacaaaact cttaggtctt cgcccatatg
   540
   tgaagaggta catgatgtac caacaagggt gctttgcagg tgggacggtt cttcgttt
   598
<210> 38
   <211> 609
   <212> DNA
   <213> Trifolium repens
<220>
   <221> misc_feature
   <222> (3) . . (3)
   <223> Any nucleotide
<220>
   <221> misc_feature
   <222> (32) . . (32)
   <223> Any nucleotide
<220>
   <221> misc_feature
   <222> (34) . . (34)
   <223> Any nucleotide
<400> 38
   ganaaaaaca ctccgcatat tatatatata tntntatagt ctataattga aagaaactgc 60
   taaagatatt attaagatat ggtgagtgta gctgaaattc gcaaggctca gagggctgaa 120
   ggccctgcaa ccattttggc cattggcact gcaaatccac caaaccgtgt tgagcagagc 180
   acatatcctg atttctactt caaaattaca aacagtgagc acaagactga gctcaaagag 240
   aagttccaac gcatgtgtga caaatccatg atcaagagca gatacatgta tctaacagaa 300
   gagattttga aagaaaatcc tagtctttgt gaatacatgg caccttcatt ggatgctagg 360
   caagacatgg tggtggttga ggtacctaga cttgggaagg aggctgcagt caaggccatt 420
   aaagaatggg gtcaaccaaa gtcaaagatt actcacttaa tcttttgcac cacaagtggt 480
   gttgacatgc ctggtgctga ttaccaactc acaaaactct taggtcttcg cccatatgtg 540
   aaaaggtata tgatgtacca acaaggttgt tttgcaggag gcacggtgct tcgtttggca 600
   aaagatttg 609
<210> 39
   <211> 390
   <212> DNA
   <213> Trifolium repens
<220>
   <221> misc_feature
   <222> (8) . . (8)
   <223> Any nucleotide
<220>
   <221> misc_feature
   <222> (37) . . (37)
   <223> Any nucleotide
<220>
   <221> misc_feature
   <222> (97) . . (97)
   <223> Any nucleotide
<220>
   <221> misc_feature
   <222> (334) . . (334)
   <223> Any nucleotide
<220>
   <221> misc_feature
   <222> (345) . . (345)
   <223> Any nucleotide
<220>
   <221> misc_feature
   <222> (349) . . (349)
   <223> Any nucleotide
<220>
   <221> misc_feature
   <222> (353) . . (353)
   <223> Any nucleotide
<220>
   <221> misc_feature
   <222> (363) . . (363)
   <223> Any nucleotide
<220>
   <221> misc_feature
   <222> (366) .. (366)
   <223> Any nucleotide
<220>
   <221> misc_feature
   <222> (369) . . (369)
   <223> Any nucleotide
<220>
   <221> misc_feature
   <222> (371) .. (371)
   <223> Any nucleotide
<220>
   <221> misc_feature
   <222> (376) . . (376)
   <223> Any nucleotide
<220>
   <221> misc_feature
   <222> (380) .. (380)
   <223> Any nucleotide
<220>
   <221> misc_feature
   <222> (390) .. (390)
   <223> Any nucleotide
<400> 39
   aacaaccnat aacttcctgt tattaaccat tgagttnaat tacatacata gcggaacata 60
   ctaaatacaa catggttagt gtttctgaaa ttcgcanggc tcaaagggct gaaggccctg 120
   caactatttt ggccattggt actgcaaatc cagcaaatcg tgttgaccag agtacatatc 180
   ctgatttcta cttcaaaatc actaacagtg agcataaggt tgagcttaaa gagaaatttc 240
   agcgcatgtg tgataaatct atgatcaaga gcagatacat gtatctaaca gaagagattt 300
   tgaaagaaaa tcctagtctt tgtgaataca tggnaccttc attgnatgnt agncaagaca 360
   tgntgntgnc ngaggnaccn agacttgccn 390
<210> 40
   <211> 561
   <212> DNA
   <213> Trifolium repens
<220>
   <221> misc_feature
   <222> (15) . . (16)
   <223> Any nucleotide
<220>
   <221> misc_feature
   <222> (37) . . (38)
   <223> Any nucleotides
<220>
   <221> misc_feature
   <222> (69) . . (69)
   <223> Any nucleotide
<220>
   <221> misc_feature
   <222> (485) . . (485)
   <223> Any nucleotide
<220>
   <221> misc_feature
   <222> (507) .. (507)
   <223> Any nucleotide
<220>
   <221> misc_feature
   <222> (511) . . (511)
   <223> Any nucleotide
<220>
   <221> misc_feature
   <222> (520) .. (520)
   <223> Any nucleotide
<220>
   <221> misc_feature
   <222> (530) . . (530)
   <223> Any nucleotide
<220>
   <221> misc_feature
   <222> (543) . . (543)
   <223> Any nucleotide
<220>
   <221> misc_feature
   <222> (554) .. (554)
   <223> Any nucleotide
<220>
   <221> misc_feature
   <222> (557) . . (558)
   <223> Any nucleotides
<220>
   <221> misc_feature
   <222> (561) . . (561)
   <223> Any nucleotide
<400> 40
   aaaaaacaac ctacnngcat attatatata tatatanngt ctataattga aagaaactac
   60
   taaagatant tattaagata tggtgagtgt agctgaaatt cgcaaggctc agagggctga
   120
   aggccctgca accattttgg ccattggcac tgcaaatcca ccaaaccgtg ttgagcagag
   180
   cacatatcct gatttctact tcaaaattac aaacagtgag cacaagactg agctcaaaga
   240
   gaagttccaa cgcatgtgtg acaaatccat gatcaagagc agatacatgt atctaacaga
   300
   agagattttg aaagaaaatc ctagtctttg tgaatacatg gcaccttcat tggatgctag
   360
   acaagacatg gtggtggttg aggtacctag acttgggaag gaggctgcag tcaaggctat
   420
   caaagaatgg ggtcaaccaa aatctaagat tacacatttg atcttttgca ccacaagtgg
   480
   tgtanacatg cctggtgctg attaccnact ngcaaaactn ttaggacttn gcccatatgt
   540
   gangaggcgc gtgntgnncc n
   561

<210> 41
   <211> 543
   <212> DNA
   <213> Trifolium repens
<220>
   <221> misc_feature
   <222> (1) . . (2)
   <223> Any nucleotides
<220>
   <221> misc_feature
   <222> (14) . . (15)
   <223> Any nucleotides
<220>
   <221> misc_feature
   <222> (20) . . (20)
   <223> Any nucleotide
<220>
   <221> misc_feature
   <222> (25) . . (25)
   <223> Any nucleotide
<220>
   <221> misc_feature
   <222> (28) . . (28)
   <223> Any nucleotide
<220>
   <221> misc_feature
   <222> (31) . . (31)
   <223> Any nucleotide
<220>
   <221> misc_feature
   <222> (40) . . (40)
   <223> Any nucleotide
<220>
   <221> misc_feature
   <222> (42) . . (42)
   <223> Any nucleotide
<220>
   <221> misc_feature
   <222> (48) . . (48)
   <223> Any nucleotide
<400> 41
   nnaacctata actnnccctn ttatntanac ncattgagtn tnaaattnac atacatagcg 60
   gaacatacta aatacaacat ggttagtgtt tctgaaattc gcaaggctca aagggctgaa 120
   ggccctgcaa ctattttggc cattggtact gcaaatccag caaatcgtgt tgaccagagt 180
   acatatcctg atttctactt caaaatcact aacagtgagc ataaggttga gcttaaagag 240
   aaatttcagc gcatgtgtga taaatctatg atcaagagca gatacatgta tctaacagaa 300
   gagattttga aagaaaatcc tagtctttgt gaatacatgg caccttcatt ggatgctagg 360
   caagacatgg tggtggttga ggtacctaga cttgggaagg aggctgcagt gaaagctatc 420
   aaagaatggg gtcaaccaaa gtcaaagatt actcacttaa tcttttgcac cacaagtggt 480
   gttgacatgc ctggtgctga ttaccaactc acaaaactct taggtcttcg cccatatgtg 540
   aag 543
<210> 42
   <211> 437
   <212> DNA
   <213> Trifolium repens
<220>
   <221> misc_feature
   <222> (3) . . (3)
   <223> Any nucleotide
<220>
   <221> misc_feature
   <222> (13) . . (13)
   <223> Any nucleotide
<220>
   <221> misc_feature
   <222> (16) . . (16)
   <223> Any nucleotide
<220>
   <221> misc_feature
   <222> (21) . . (21)
   <223> Any nucleotide
<220>
   <221> misc_feature
   <222> (30) . . (31)
   <223> Any nucleotides
<220>
   <221> misc_feature
   <222> (35) . . (36)
   <223> Any nucleotides
<220>
   <221> misc_feature
   <222> (38) . . (38)
   <223> Any nucleotide
<220>
   <221> misc_feature
   <222> (41) . . (42)
   <223> Any nucleotides
<220>
   <221> misc_feature
   <222> (54) . . (54)
   <223> Any nucleotide
<220>
   <221> misc_feature
   <222> (59) . . (59)
   <223> Any nucleotide
<220>
   <221> misc_feature
   <222> (373) .. (373)
   <223> Any nucleotide
<220>
   <221> misc_feature
   <222> (388) .. (388)
   <223> Any nucleotide
<220>
   <221> misc_feature
   <222> (394) .. (394)
   <223> Any nucleotide
<220>
   <221> misc_feature
   <222> (407) . . (408)
   <223> Any nucleotides
<220>
   <221> misc_feature
   <222> (434) . . (434)
   <223> Any nucleotide
<220>
   <221> misc_feature
   <222> (437) . . (437)
   <223> Any nucleotide
<400> 42
   ggntaaccaa ccntanactt ncctgttatn ncttnnanat nntacataca tagncagcna 60
   aacaaagata tcaacatatg gttagtgttt ctgaaattcg taaggctcaa agggcagaag 120
   gccctgcaac tattttggcc attggtactg caaatccagc aaaccgtgtt gatcagagta 180
   catatcctga tttctacttc aaaatcacta acagtgagca taaagttgag ctcaaagaga 240
   aattccagcg catgtgtgat aaatctatga tcaagagcag atacatgtat ctaacagaag 300
   agattttgaa agaaaatcct agtctttgtg aatacatggc accttcattg gatgctaggc 360
   aagatatggt ggncgttgag gtacctanac ttgnaaagga ggctgcnntg aaggctatta 420
   aagaatgggg ccanccn 437
<210> 43
   <211> 607
   <212> DNA
   <213> Trifolium repens
<220>
   <221> misc_feature
   <222> (13) . . (13)
   <223> Any nucleotide
<220>
   <221> misc_feature
   <222> (18) . . (18)
   <223> Any nucleotide
<220>
   <221> misc_feature
   <222> (23) . . (23)
   <223> Any nucleotide
<220>
   <221> misc_feature
   <222> (29) . . (29)
   <223> Any nucleotide
<220>
   <221> misc_feature
   <222> (32) . . (32)
   <223> Any nucleotide
<220>
   <221> misc_feature
   <222> (35) .. (35)
   <223> Any nucleotide
<220>
   <221> misc_feature
   <222> (66) . . (66)
   <223> Any nucleotide
<400> 43
   caacaaaaac acntcgcnat atntatatna tnttngtcta taattgaaag aaactgctaa 60
   agatanttat taagatatgg tgagtgtagc tgaaattcgc aaggctcaga gggctgaagg 120
   ccctgcaacc attttggcca ttggcactgc aaatccacca aaccgtgttg agcagagcac 180
   atatcctgat ttctacttca aaattacaaa cagtgagcac aagactgagc tcaaagagaa 240
   gttccaacgc atgtgtgaca aatccatgat caagagcaga tacatgtatc taacagaaga 300
   gattttgaaa gaaaatccta gtctttgtga atacatggca ccttcattgg atgctaggca 360
   agacatggtg gtggttgagg tacctagact tgggaaggag gctgcagtga aagctatcaa 420
   agaatggggt caaccaaagt caaagattac tcacttaatc ttttgcacca caagtggtgt 480
   agacatgcct ggagctgatt accaactcac aaaactctta ggtcttcgcc catatgtgaa 540
   aaggtatatg atgtaccaac aaggttgttt tgcaggaggc acggtgcttc gtttggcaaa 600
   agatttg 607
<210> 44
   <211> 581
   <212> DNA
   <213> Trifolium repens
<220>
   <221> misc_feature
   <222> (7) . . (7)
   <223> Any nucleotide
<220>
   <221> misc_feature
   <222> (11) . . (11)
   <223> Any nucleotide
<220>
   <221> misc_feature
   <222> (17) . . (17)
   <223> Any nucleotide
<220>
   <221> misc_feature
   <222> (29) . . (29)
   <223> Any nucleotide
<220>
   <221> misc_feature
   <222> (33) . . (33)
   <223> Any nucleotide
<220>
   <221> misc_feature
   <222> (64) . . (64)
   <223> Any nucleotide
<400> 44
   aacactncgc natattntat atatatatnt atngtctata attgaaagaa actgctaaag
   60
   atanttatta agatatggtg agtgtagctg aaattcgcaa ggctcagagg gctgaaggcc
   120
   ctgcaaccat tttggccatt ggcactgcaa atccaccaaa ccgtgttgag cagagcacat
   180
   atcctgattt ctacttcaaa attacaaaca gtgagcacaa gactgagctc aaagagaagt
   240
   tccaacgcat gtgtgacaaa tccatgatca agagcagata catgtatcta acagaagaga
   300
   ttttgaaaga aaatcctagt ctttgtgaat acatggcacc ttcattggat gctaggcaag
   360
   acatggtggt ggttgaggta cctagacttg ggaaggaggc tgcagtcaag gccattaaag
   420
   aatggggtca accaaagtca aagattactc acttaatctt ttgcaccaca agtggtgttg
   480
   acatgcctgg tgctgattac caactcacaa aactcttagg tcttcgccca tatgtgaaaa
   540
   ggtatatgat gtaccaacaa ggttgttttg caggaggcac g
   581
<210> 45
   <211> 588
   <212> DNA
   <213> Trifolium repens
<220>
   <221> misc_feature
   <222> (9) . . (10)
   <223> Any nucleotides
<220>
   <221> misc_feature
   <222> (16) . . (16)
   <223> Any nucleotide
<220>
   <221> misc_feature
   <222> (20) . . (20)
   <223> Any nucleotide
<220>
   <221> misc_feature
   <222> (25) . . (28)
   <223> Any nucleotides
<220>
   <221> misc_feature
   <222> (32) . . (33)
   <223> Any nucleotides
<220>
   <221> misc_feature
   <222> (63) . . (63)
   <223> Any nucleotide
<400> 45
   aaacaaccnn ccgcantatn tatannnnta tnngtcataa ttgaaagaaa ctgctaaaga
   60
   tanttattaa gatatggtga gtgtagctga aattcgcaag gctcagaggg ctgaaggccc
   120
   tgcaaccatt ttggccattg gcactgcaaa tccaccaaac cgcgttgagc agagcacata
   180
   tcctgatttc tacttcaaaa ttacaaacag tgagcacaag actgagctca aagagaagtt
   240
   ccaacgcatg tgtgacaaat ccatgatcaa gagcagatac atgtatctaa cagaagagat
   300
   tttgaaagaa aatcctagtc tttgtgaata catggcacct tcattggatg ctaggcaaga
   360
   catggtggtg gttgaggtac ctagacttgg gaaggaggct gcagtcaagg ctatcaaaga
   420
   atggggtcaa ccaaaatcta agattacaca tttgatcttt tgcaccacaa gtggtgtaga
   480
   catgcctggt gctgattacc aactcacaaa actcttggga cttcgtccat atgtgaagag
   540
   attcatgatg taccaacaag gctgctttgc aggtgggacg gttcttcg
   588
<210> 46
   <211> 613
   <212> DNA
   <213> Trifolium repens
<230>
   <221> misc_feature
   <222> (5) . . (5)
   <223> Any nucleotide
<220>
   <221> misc_feature
   <222> (7) . . (7)
   <223> Any nucleotide
<220>
   <221> misc_feature
   <222> (12) . . (13)
   <223> Any nucleotides
<220>
   <221> misc_feature
   <222> (17) . . (18)
   <223> Any nucleotides
<220>
   <221> misc_feature
   <222> (20) . . (22)
   <223> Any nucleotides
<220>
   <221> misc_feature
   <222> (24) . . (24)
   <223> Any nucleotide
<220>
   <221> misc_feature
   <222> (26) . . (27)
   <223> Any nucleotides
<220>
   <221> misc_feature
   <222> (29) . . (29)
   <223> Any nucleotide
<220>
   <221> misc_feature
   <222> (50) . . (50)
   <223> Any nucleotide
<220>
   <221> misc_feature
   <222> (60) . . (60)
   <223> Any nucleotide
<400> 46
   caacncntcg cnntatnntn nntntnntng tctataattg aaagaaactn ctaaagatan
   60
   ttattaagat atggtgagtg tagctgaaat tcgcaaggct cagagggctg aaggccctgc
   120
   aaccattttg gccattggca ctgcaaatcc accaaaccgt gttgagcaga gcacatatcc
   180
   tgatttctac ttcaaaatta caaacagtga gcacaagact gagctcaaag agaagttcca
   240
   acgcatgtgt gacaaatcca tgatcaagag cagatacatg tatctaacag aagagatttt
   300
   gaaagaaaat cctagtcttt gtgaatacat ggcaccttca ttggatgcta ggcaagacat
   360
   ggtggtggtt gaggtaccta gacttgggaa ggaagctgca gtcaaggcca ttaaagaatg
   420
   gggtcagcca aagtcaaaga ttactcactt aatcttttgc accacaagtg gtgttgacat
   480
   gcctggagct gattaccaac tcacaaaact cttaggtctt cgcccatatg tgaaaaggta
   540
   tatgatgtac caacaaggtt gttttgcagg aggcacggtg cttcgtttgg caaaagattt
   600
   ggccgagaac aac
   613
<210> 47
   <211> 544
   <212> DNA
   <213> Trifolium repens
<220>
   <221> misc_feature
   <222> (3) . . (3)
   <223> Any nucleotide
<220>
   <221> misc_feature
   <222> (6) . . (6)
   <223> Any nucleotide
<220>
   <221> misc_feature
   <222> (20) . . (21)
   <223> Any nucleotides
<400> 47
   ccnatnacct tcctgttatn ncttacatat actacataca tagcaggagc aaagatatca
   60
   acatatggtt actgtttctg aaattcgcaa ggctcaaagg gctgaaggcc ctgcaaccat
   120
   tttggccatt ggtactgcaa atccagcaaa ccgtgttgat cagagtacat atcctgattt
   180
   ctacttcaaa atcactaaca gtgagcataa ggttgagctc aaggagaaat tccagcgcat
   240
   gtgtgacaaa tctatgatca agagcagata catgtatcta acagaagaga ttttgaaaga
   300
   aaatcctagt ctttgtgaat acatggcacc ttcattggat gctaggcaag atatggtggt
   360
   cgttgaggta cctagactgg gaaaggaagc tgcagtgaag gctattaaag aatggggtca
   420
   accaaagtca aagattactc acttaatctt ttgcactaca agtggtgtag acatgcctgg
   480
   tgctgattac cagctcacaa aactcttagg tcttcgccca tatgtgaaga ggtatatgat
   540
   gtat
   544
<210> 48
   <211> 555
   <212> DNA
   <213> Trifolium repens
<220>
   <221> misc_feature
   <222> (4) . . (5)
   <223> Any nucleotides
<220>
   <221> misc_feature
   <222> (8) . . (8)
   <223> Any nucleotide
<220>
   <221> misc_feature
   <222> (11) . . (11)
   <223> Any nucleotide
<220>
   <221> misc_feature
   <222> (21) . . (22)
   <223> Any nucleotides
<220>
   <221> misc_feature
   <222> (513) . . (513)
   <223> Any nucleotide
<400> 48
   accnnatnac ntactgttat nncttacata tactacatag caggactgca aaattcaagt 60
   atcaacatat ggttactgtt tctgaaattc gcaaggctca aagggctgaa ggccctgcaa 120
   ccattttggc cattggtact gcaaatccag caaaccgtgt tgatcagagt acatatcctg 180
   atttctactt caaaatcact aacagtgagc ataaggttga gctcaaggag aaattccagc 240
   gcatgtgtga taaatctatg atcaagagca gatacatgta tctaacagaa gagattttga 300
   aagaaaatcc tagtctgtgt gagtacatgg caccttcatt ggatgctagg caagacatgg 360
   ttgtggttga ggtacctaga cttggaaagg aggctgcagt caaggccatt aaagaatggg 420
   gtcaaccaaa gtcaaagatt actcacttaa tcttttgcac cacaagtggt gtagacatgc 480
   ctggtgctga ttaccaactc acaaaactct tangtcttcg tccatacgtg aagagggaca 540
   tgatgtacca acaag 555

<210> 49
   <211> 570
   <212> DNA
   <213> Trifolium repens
<220>
   <221> misc_feature
   <222> (3) . . (3)
   <223> Any nucleotide
<220>
   <221> misc_feature
   <222> (5) . . (5)
   <223> Any nucleotide
<220>
   <221> misc_feature
   <222> (13) . . (13)
   <223> Any nucleotide
<220>
   <221> misc_feature
   <222> (25) . . (25)
   <223> Any nucleotide
<220>
   <221> misc_feature
   <222> (58) . . (58)
   <223> Any nucleotide
<400> 49
   acncntcgct atntatttat atatntagtc tataattgaa agaaactgct aaagatantt 60
   attaagatat ggtgagtgta gctgaaattc gcaaggctca gagggctgaa ggccctgcaa 120
   ccattttggc cattggcact gcaaatccac caaaccgtgt tgagcagagc acatatcctg 180
   atttctactt caaaattaca aacagtgagc acaagactga gctcaaagag aagttccaac 240
   gcatgtgtga caaatccatg atcaagagca gatacatgta tctaacagaa gagattttga 300
   aagaaaatcc tagtctttgt gaatacatgg caccttcatt ggatgctagg caagacatgg 360
   tggtggttga ggtacctaga cttgggaagg aggctgcagt caaggccatt aaagaatggg 420
   gtcaaccaaa gtcaaagatt actcacttaa tcttttgcac cacaagtggt gttgacatgc 480
   ctggtgctga ttaccaactc acaaaactct taggtcttcg cccatatgtg aaaaggtata 540
   tgatgtacca acaaggttgt tttgcaggag 570
<210> 50
   <211> 546
   <212> DNA
   <213> Trifolium repens
<220>
   <221> misc_feature
   <222> (6) . . (6)
   <223> Any nucleotide
<220>
   <221> misc_feature
   <222> (25) . . (25)
   <223> Any nucleotide
<220>
   <221> misc_feature
   <222> (35) . . (35)
   <223> Any nucleotide
<220>
   <221> misc_feature
   <222> (49) . . (49)
   <223> Any nucleotide
<400> 50
   actatnacta ctgtttactt acatntactc atagnggact gcaagtatna gtataacata
   60
   tggttactgt ttctgaaatt cgcaaggctc aaagggctga aggccctgca accattttgg
   120
   ccattggtac tgcaaatcca gcaaaccgtg ttgatcagag tacatatcct gatttctact
   180
   tcaaaatcac taacagtgag cataaggttg agctcaagga gaaattccag cgcatgtgtg
   240
   ataaatctat gatcaagagc agatacatgt atctaacaga agagattttg aaagaaaatc
   300
   ctagtctgtg tgagtacatg gcaccttcat tggatgctag gcaagacatg gttgtggttg
   360
   aggtacctag acttggaaag gaggctgcag tcaaggccat taaagaatgg ggtcaaccaa
   420
   agtcaaagat tactcactta atcttttgca ccacaagtgg tgtagacatg cctggtgctg
   480
   attaccaact cacaaaactc ttaggtcttc gtccatacgt gaagaggtac atgatgtacc
   540
   aacaag
   546
<210> 51
   <211> 582
   <212> DNA
   <213> Trifolium repens
<220>
   <221> misc_feature
   <222> (18) . . (18)
   <223> Any nucleotide
<220>
   <221> misc_feature
   <222> (22) . . (22)
   <223> Any nucleotide
<220>
   <221> misc_feature
   <222> (34) . . (34)
   <223> Any nucleotide
<400> 51
   acctaccttc ctgttatnct tnaattcata catngcagca aacaaagata taacatatgg
   60
   ttagtgtatc tgaaattcgc aaggctcaaa gggcagaagg ccctgcaact attttggcca
   120
   ttggtactgc aaatccagca aaccgtgttg atcaaagtac atatcctgat ttctacttca
   180
   aaatcactaa cagtgagcat aaggttgagc tcaaagagaa attccagcgc atgtgtgata
   240
   aatctatgat caagagcaga tacatgtatc caacagaaga gattttgaaa gaaaatccta
   300
   gtctttgtga atacatggca ccttcattgg atgctagaca agacatggtg gtggttgagg
   360
   tacctagact tggaaaggag gctgcagtga aggccattaa agaatggggt caaccaaaat
   420
   ctaagattac acatttgatc ttttgcacca caagtggtgt agacatgcct ggtgctgatt
   480
   accagctcac aaaactctta ggtcttcgtc catatgtgaa aaggtatatg atgtaccaac
   540
   aagggtgctt tgcaggtggg acggtgcttc gtttggccaa gg
   582
<210> 52
   <211> 649
   <212> DNA
   <213> Trifolium repens
<220>
   <221> misc_feature
   <222> (3) .. (3)
   <223> Any nucleotide
<220>
   <221> misc_feature
   <222> (21) .. (21)
   <223> Any nucleotide
<220>
   <221> misc_feature
   <222> (24) .. (24)
   <223> Any nucleotide
<220>
   <221> misc_feature
   <222> (26) .. (26)
   <223> Any nucleotide
<220>
   <221> misc_feature
   <222> (40) .. (40)
   <223> Any nucleotide
<220>
   <221> misc_feature
   <222> (45) .. (45)
   <223> Any nucleotide
<220>
   <221> misc_feature
   <222> (80) .. (81)
   <223> Any nucleotides
<220>
   <221> misc_feature
   <222> (238) .. (239)
   <223> Any nucleotides
<220>
   <221> misc_feature
   <222> (469) .. (469)
   <223> Any nucleotide
<220>
   <221> misc_feature
   <222> (578) .. (578)
   <223> Any nucleotide
<220>
   <221> misc_feature
   <222> (588) .. (588)
   <223> Any nucleotide
<220>
   <221> misc_feature
   <222> (600) .. (600)
   <223> Any nucleotide
<220>
   <221> misc_feature
   <222> (610) .. (610)
   <223> Any nucleotide
<220>
   <221> misc_feature
   <222> (612) .. (612)
   <223> Any nucleotide
<220>
   <221> misc_feature
   <222> (618) .. (618)
   <223> Any nucleotide
<220>
   <221> misc_feature
   <222> (620) .. (620)
   <223> Any nucleotide
<220>
   <221> misc_feature
   <222> (626) .. (626)
   <223> Any nucleotide
<220>
   <221> misc_feature
   <222> (637) .. (637)
   <223> Any nucleotide
<220>
   <221> misc_feature
   <222> (640) .. (640)
   <223> Any nucleotide
<220>
   <221> misc_feature
   <222> (649) .. (649)
   <223> Any nucleotide
<400> 52
   acnacttcct gttatcactt ncananatta catacatagn cagcnaaaca aagatatcaa
   60
   catatggtta gtgtatctgn nattcgcaag gctcaaaggg cagaaggccc tgcaactatt
   120
   ttggccattg gtactgcaaa tccagcaaac cgtgttgatc aaagtacata tcctgatttc
   180
   tacttcaaaa tcactaacag tgagcataag gttgagctca aagagaaatt ccagcgcnng
   240
   tgtgataaat ctatgatcaa gagcagatac atgtatctaa cagaagagat tttgaaagaa
   300
   aatcctagtc tttgtgaata catggcacct tcattggatg ctagacaaga catggtggtg
   360
   gttgaggtac ctagacttgg aaaggaggct gcagtgaagg ccattaaaga atggggtcaa
   420
   ccaaaatcta agattacaca tttgatcttt tgcaccacaa gtggtgtana catgcctggt
   480
   gctgattacc agctcacaaa actcttaggt cttcgtccat atgtgaaaag ggatatgatg
   540
   taccaacaag ggtgctttgc aggtgggacc gtgcttcntt tggccaanga tttggcccan
   600
   aacaacaaan gngctcgngn gttggntggt tggtctnaan tcaccgcan
   649
<210> 53
   <211> 521
   <212> DNA
   <213> Trifolium repens
<220>
   <221> misc_feature
   <222> (2) .. (2)
   <223> Any nucleotide
<220>
   <221> misc_feature
   <222> (4) .. (4)
   <223> Any nucleotide
<220>
   <221> misc_feature
   <222> (8) .. (8)
   <223> Any nucleotide
<400> 53
   tntngtcnta ttgaagaaac tgctaagatt tttaagatat ggtgagtgta gctgaaattc 60
   gcaaggctca gagggctgaa ggccctgcaa ccattttggc cattggcact gcaaatccac 120
   caaaccgtgt tgagcagagc acatatcctg atttctactt caaaattaca aacagtgagc 180
   acaagactga gctcaaagag aagttccaac gcatgtgtga caaatccatg atcaagagca 240
   gatacatgta tctaacagaa gagattttga aagaaaatcc tagtctttgt gaatacatgg 300
   caccttcatt ggatgctagg caagacatgg tggtggttga ggtacctaga cttgggaagg 360
   aggctgcagt gaaagctatc aaagaatggg gtcaaccaaa gtcaaagatt actcacttaa 420
   tcttttgcac cacaagtggt gtagacatgc ctggagctga ttaccaactc acaaaactct 480
   taggtcttcg cccatatgtg aaaaggtata tgatgtacca a 521
<210> 54
   <211> 506
   <212> DNA
   <213> Trifolium repens
<220>
   <221> misc_feature
   <222> (2) .. (2)
   <223> Any nucleotide
<220>
   <221> misc_feature
   <222> (354) .. (354)
   <223> Any nucleotide
<220>
   <221> misc_feature
   <222> (356) .. (356)
   <223> Any nucleotide
<220>
   <221> misc_feature
   <222> (368) .. (368)
   <223> Any nucleotide
<220>
   <221> misc_feature
   <222> (376) .. (376)
   <223> Any nucleotide
<220>
   <221> misc_feature
   <222> (378) .. (378)
   <223> Any nucleotide
<220>
   <221> misc_feature
   <222> (386) .. (386)
   <223> Any nucleotide
<220>
   <221> misc_feature
   <222> (434) .. (434)
   <223> Any nucleotide
<220>
   <221> misc_feature
   <222> (469) .. (469)
   <223> Any nucleotide
<220>
   <221> misc_feature
   <222> (484) .. (484)
   <223> Any nucleotide
<220>
   <221> misc_feature
   <222> (491) .. (491)
   <223> Any nucleotide
<220>
   <221> misc_feature
   <222> (503) .. (503)
   <223> Any nucleotide
<220>
   <221> misc_feature
   <222> (506) .. (506)
   <223> Any nucleotide
<400> 54
   gngctaaaga tattattaag atatggtgag tgtagctgaa attcgcaagg ctcagagggc 60
   tgaaggccct gcaaccattt tggccattgg cactgcaaat ccaccaaacc gtgttgagca 120
   gagcacatat cctgatttct acttcaaaat tacaaacagt gagcacaaga ctgagctcaa 180
   agagaagttc caacgcatgt gtgacaaatc catgatcaag agcagataca tgtatctaac 240
   agaagagatt ttgaaagaaa atcctagtct ttgtgaatac atggcacctt cattggatgc 300
   taggcaagac atggtggtgg ttgaggtacc tagacttggg aaggaggctg cagncnaggc 360
   cattaaanaa tggggncnac caaagncaaa gattactcac ttaatctttt gcaccacaag 420
   tggtgctgac atgnctggtg ctgattacca actcacaaaa ctcttaggnc ttcccccata 480
   tgtnaaaagg natatgatgc ccncan 506
<210> 55
   <211> 504
   <212> DNA
   <213> Trifolium repens
<220>
   <221> misc_feature
   <222> (3) .. (3)
   <223> Any nucleotide
<220>
   <221> misc_feature
   <222> (9) .. (11)
   <223> Any nucleotides
<220>
   <221> misc_feature
   <222> (14) .. (14)
   <223> Any nucleotide
<220>
   <221> misc_feature
   <222> (20) .. (20)
   <223> Any nucleotide
<220>
   <221> misc_feature
   <222> (53) .. (53)
   <223> Any nucleotide
<220>
   <221> misc_feature
   <222> (374) .. (374)
   <223> Any nucleotide
<220>
   <221> misc_feature
   <222> (395) .. (395)
   <223> Any nucleotide
<220>
   <221> misc_feature
   <222> (431) .. (431)
   <223> Any nucleotide
<220>
   <221> misc_feature
   <222> (466) .. (467)
   <223> Any nucleotides
<220>
   <221> misc_feature
   <222> (473) .. (473)
   <223> Any nucleotide
<220>
   <221> misc_feature
   <222> (504) .. (504)
   <223> Any nucleotide
<400> 55
   gtnctaaann nttnttaagn aatggtgagt gtagctgaat tcgcaggctc agnaggctga
   60
   aggccctgca accattttgg ccattggcac tgcaaatcca ccaaaccgtg ttgagcagag
   120
   cacatatcct gatttctact tcaaaattac aaacagtgag cacaagactg agctcaaaga
   180
   gaagttccaa cgcatgtgtg acaaatccat gatcaagagc agatacatgt atctaacaga
   240
   agagattttg aaagaaaatc ctagtctttg tgaatacatg gcaccttcat tggatgctag
   300
   gcaagacatg gtggtggttg aggtacctag acttgggaag gaggctgcag tcaaggccat
   360
   taaagaatgg ggtnaaccaa agtgaaagat tactnactta atcttttgca ccacaagtgg
   420
   tgttgacatg nctggtgctg attaccaact cacaaaactc ttaggnnttg gcncatatgt
   480
   gaaaaggtct atgatgcacc aacn
   504
<210> 56
   <211> 782
   <212> DNA
   <213> Trifolium repens
<400> 56
   gataagatat tattaagata tggtgagtgt agctgaaatt cgcaaggctc agagggctga 60
   aggccctgca accattttgg ccattggcac tgcaaatcca ccaaaccgtg ttgagcagag 120
   cacatatcct gatttctact tcaaaattac aaacagtgag cacaagactg agctcaaaga 180
   gaagttccaa cgcatgtgtg acaaatccat gatcaagagc agatacatgt atctaacaga 240
   agagattttg aaagaaaatc ctagtctttg tgaatacatg gcaccttcat tggatgctag 300
   gcaagacatg gtggtggttg aggtacctag acttgggaag gaggctgcag tgaaagctat 360
   caaagaatgg ggtcaaccaa agtcaaagat tactcactta atcttttgca ccacaagtgg 420
   tgtagacatg cctggagctg attaccaact cacaaaactc ttaggtcttc gcccatatgt 480
   gaaaaggtat atgatgtacc aacaaggttg ttttgcagga ggcacggtgc ttcgtttggc 540
   aaaagatttg gccgagaaca acaaaggtgc tcgtgtgttg gttgtttgtt ctgaagtcac 600
   tgcagttaca ttccgtggcc cgagtgacac tcacttggac agtcttgttg gacaagcatt 660
   gtttggagat ggagcagctg cacttatcgt tggttctgat ccagtgccag aaattgagaa 720
   accaatattt gagatggttt ggactgcaca aacaattgct ccagacagtg aaggtgccat 780
   tg 782

<210> 57
   <211> 597
   <212> DNA
   <213> Trifolium repens
<220>
   <221> misc_feature
   <222> (2) .. (2)
   <223> Any nucleotide
<220>
   <221> misc_feature
   <222> (9) .. (10)
   <223> Any nucleotides
<220>
   <221> misc_feature
   <222> (12) .. (13)
   <223> Any nucleotides
<220>
   <221> misc_feature
   <222> (15) .. (15)
   <223> Any nucleotide
<220>
   <221> misc_feature
   <222> (19) .. (20)
   <223> Any nucleotides
<400> 57
   tnaagatann tnntnagann tggtgagtgt agcagaaatt cgcaaggctc agagggctga
   60
   aggccctgca accattttgg ccattggcac tgcaaatcca ccaaaccgtg ttgagcagag
   120
   cacatatcct gatttctact tcaaaattac aaacagtgag cacaagactg agctcaaaga
   180
   gaagttccaa cgcatgtgtg acaaatccat gatcaagagc agatacatgt atctaacaga
   240
   agagattttg aaagaaaatc ctagtctttg tgaatacatg gcaccttcat tggatgctag
   300
   acaagacatg gtggtggttg aggtacctag acttgggaag gaggctgcag tcaaggctat
   360
   caaagaatgg ggtcaaccaa aatctaagat tacacatttg atcttttgca ccacaagtgg
   420
   tgtagacatg cctggtgctg attaccaact cacaaaactc ttaggacttc gtccatatgt
   480
   gaagaggtac atgatgtacc aacaagggtg ctttgcaggt ggggcggttc ttcgtttggc
   540
   taaagatttg gccgagaaca acaaaggtgc tcgtgtgttg gttgtttgtt ctgaagt
   597
<210> 58
   <211> 590
   <212> DNA
   <213> Trifolium repens
<220>
   <221> misc_feature
   <222> (3) .. (3)
   <223> Any nucleotide
<220>
   <221> misc_feature
   <222> (7) .. (7)
   <223> Any nucleotide
<400> 58
   ggntcanagg gctgaaggcc ctgcaacctt ttggccgttg gcactgcaaa tccgcaaacc
   60
   gtgttgagca gagcacatat cctgatttct acttcaaaat tacaaacagt gagcacaaga
   120
   ctgagctcaa agagaagttc caacgcatgt gtgacaaatc catgatcaag agcagataca
   180
   tgtatctaac agaagagatt ttgaaagaaa atcctagtct ttgtgaatac atggcacctt
   240
   cattggatgc tagacaagac atggtggtgg ttgaggtacc tagacttggg aaggaggccg
   300
   cagtcaaggc tatcaaagaa tggggtcaac caaaatctaa gattacacat ttgatctttt
   360
   gcaccacaag tggtgtagac atgcctggtg ctgattacca actcacaaaa ctcttaggac
   420
   ttcgtccata tgtgaagagg tacatgatgt accaacaagg gtgctttgca ggtgggacgg
   480
   ttcttcgttt ggctaaagat ttggccgaga acaacaaagg tgctcgtgtg ttggttgttt
   540
   gttctgaagt aactgcagtc acattccgtg gcccgagtga cactcacttg
   590
<210> 59
<211> 618
<212> DNA
   <213> Trifolium repens
<220>
   <221> misc_feature
   <222> (13) .. (13)
   <223> Any nucleotide
<220>
   <221> misc_feature
   <222> (15) .. (15)
   <223> Any nucleotide
<220>
   <221> misc_feature
   <222> (41) .. (41)
   <223> Any nucleotide
<220>
   <221> misc_feature
   <222> (58) .. (58)
   <223> Any nucleotide
<400> 59
   aaggccctgc aancnttttt ggccttggta ctgcaaatcc ngcaaatcgt gttgaccnga 60
   gtacatatcc tgatttctac ttcaaaatca ctaacagtga gcataagatt gagcttaaag 120
   agaaatttca gcgcatgtgt gataaatcta tgatcaagag cagatacatg tatctaacag 180
   aagagatttt gaaagaaaat cctagtcttt gtgaatacat ggcaccttca ttggatgcta 240
   ggcaagacat ggtggtggtt gaggtaccta gacttgggaa ggaggctgca gtgaaagcta 300
   tcaaagaatg ggtcaaccca aagtcaaaga ttactcactt aatcttttgc accacaagtg 360
   gtgttgacat gcctggtgct gattaccaac tcacaaaact cttaggtctt cgcccatatg 420
   tgaagaggta catgatgtac caacaagggt gctttgcagg tgggacggtt cttcgtttgg 480
   ccaaggattt ggccgagaac aacaaaggtg ctcgtgtgtt ggttgtttgc tctgaagtaa 540
   ccgcagtcac attccgcggc cccagtgaca ctcatttgga cagccttgtt ggacaagcac 600
   tatttggaga tggagctg 618
<210> 60
   <211> 619
   <212> DNA
   <213> Trifolium repens
<220>
   <221> misc_feature
   <222> (13) .. (14)
   <223> Any nucleotides
<220>
   <221> misc_feature
   <222> (39) .. (39)
   <223> Any nucleotide
<400> 60
   ggccctgcaa ccnnttttgg ccttggtact gcaaatccng caaatcgtgt tgaccagagt 60
   acatatcctg atttctactt caaaatcact aacagtgagc ataaggttga gcttaaagag 120
   aaatttcagc gcatgtgtga taaatctatg atcaagagca gatacatgta tctaacagaa 180
   gagattttga aagaaaatcc tagtctttgt gaatacatgg caccttcatt ggatgctagg 240
   caagacatgg tggtggttga ggtacctaga cttgggaagg aggctgcagt gaaagctatc 300
   aaagaatggg gtcaaccaaa gtcaaagatt actcacttaa tcttttgcac cacaagtggt 360
   gttgacatgc ctggtgctga ttaccaactc acaaaactct taggtcttcg cccatatgtg 420
   aagaggtaca tgatgtacca acaagggtgc tttgcaggtg ggacggttct tcgtttggcc 480
   aaggatttgg ccgagaacaa caaaggtgct cgtgtgttgg ttgtttgctc tgaagtaacc 540
   gcagtcacat tccgcggccc cagtgacact catttggaca gccttgttgg acaagcacta 600
   tttggagatg gagctgctg 619
<210> 61
   <211> 559
   <212> DNA
   <213> Trifolium repens
<220>
   <221> misc_feature
   <222> (27) .. (28)
   <223> Any nucleotides
<220>
   <221> misc_feature
   <222> (559) .. (559)
   <223> Any nucleotide
<400> 61
   ataaggctga gcttaaagag aaatttnngc gcatgtgtga taaatctatg atcaagagca
   60
   gatacatgta tctaacagaa gagattttga aagaaaatcc tagtctttgt gaatacatgg
   120
   caccttcatt ggatgctagg caagacatgg tggtggttga ggtacctaga cttgggaagg
   180
   aggctgcagt gaaagctatc aaagaatggg gtcaaccaaa gtcaaagatt actcacttaa
   240
   tcttttgcac cacaagtggt gtagacatgc ctggagctga ttaccaactc acaaaactct
   300
   taggtcttcg cccatatgtg aagaggtaca tgatgtacca acaagggtgc tttgcaggtg
   360
   ggacggttct tcgtttggcc aaggatttgg ccgagaacaa caaaggtgct cgtgtgttgg
   420
   ttgtttgctc tgaagtaacc gcagtcacat tccgcggccc cagtgacact catttggaca
   480
   gtcttgttgg acaagcacta ttcggagatg gagctgctgc actcattgtt ggctcagacc
   540
   cagtaccaga aattgagan
   559
<210> 62
   <211> 553
   <212> DNA
   <213> Trifolium repens
<400> 62
   atcaagagca gatacatgta tctaacagaa gagattttga aagaaaatcc tagtctgtgt
   60
   gagtacatgg caccttcatt ggatgctagg caagacatgg ttgtggttga ggtacctaga
   120
   cttggaaagg aggctgcagt caaggccatt aaagaatggg gtcaaccaaa gtcaaagatt
   180
   actcacttaa tcttttgcac cacaagtggt gtagacatgc ctggtgctga ttaccaactc
   240
   acaaaactct taggtcttcg tccatacgtg aagaggtaca tgatgtacca acaagggtgc
   300
   tttgcaggtg ggacggtgct tcgtttggcc aaggatttgg ccgagaacaa caaaggtgct
   360
   cgtgtgttgg ttgtttgttc tgaagtcacc gcagtcacat tccgtggccc tagtgacact
   420
   catttggaca gtcttgttgg acaagcacta tttggagatg gagctgctgc tctcattgtt
   480
   ggttctgatc cagtaccaga aattgagaag ccaatatttg agatggtatg gaccgcacag
   540
   acaattgctc cag
   553
<210> 63
   <211> 591
   <212> DNA
   <213> Trifolium repens
<220>
   <221> misc_feature
   <222> (5) .. (5)
   <223> Any nucleotide
<220>
   <221> misc_feature
   <222> (18) .. (18)
   <223> Any nucleotide
<220>
   <221> misc_feature
   <222> (22) .. (22)
   <223> Any nucleotide
<220>
   <221> misc_feature
   <222> (33) .. (33)
   <223> Any nucleotide
<220>
   <221> misc_feature
   <222> (35) .. (35)
   <223> Any nucleotide
<400> 63
   gcttnaagaa tggggacnac cnaaatctaa gantncacat ttgatctttt gcaccacaag 60
   tggtgtagac atgcctggtg ctgattacca gctcacaaaa ctcttaggtc ttcgcccata 120
   tgtgaagagg tatatgatgt atcaacaagg ttgctttgca ggaggcacgg tgcttcgttt 180
   ggctaaagat ttggccgaga acaacaaagg tgctcgtgtg ctagttgttt gttctgaagt 240
   aacagcagtc acattccgcg gtccaagtga cactcacttg gacagtcttg ttggacaagc 300
   actattcgga gatggagctg ctgctctcat tgttggctca gaccctgtac cagaaatcga 360
   gaaacctata tttgagatgg tatggaccgc acagacaatt gctccggaca gtgaaggtgc 420
   cattgatggt caccttcgtg aagctggact aacatttcat cttcttaaag atgttcctgg 480
   gattgtatca aagaacatta ataaagcatt ggtcgaggct ttccaaccat taggaatttc 540
   tgattacaac tcaatctttt ggattgcaca cccgggtgga cctgcaattc t 591
<210> 64
   <211> 634
   <212> DNA
   <213> Trifolium repens
<220>
   <221> misc_feature
   <222> (7) .. (7)
   <223> Any nucleotide
<220>
   <221> misc_feature
   <222> (9) .. (9)
   <223> Any nucleotide
<220>
   <221> misc_feature
   <222> (18) .. (18)
   <223> Any nucleotide
<220>
   <221> misc_feature
   <222> (26) .. (26)
   <223> Any nucleotide
<400> 64
   tcttcgncna gctggacnaa catttntgct tcttaaagat gttcctgaga ttgtctcaaa 60
   gaacattgat aaggcattgg ttgaggcatt ccaaccatta aacatctctg attacaattc 120
   aatcttttgg attgctcatc caggtggtcc tgcaattcta gaccaagttg agataaagtt 180
   gggcttaaaa cctgaaaaaa tgaaggccac cagagatgta cttagtgaat atggtaacat 240
   gtcaagtgca tgtgtattgt tcatcttaga tgagatgcaa aagaaatcgg ctgaaaatgg 300
   actgaaaacc acaggagaag gacttgactg gggtgtgttg tttggatttg gaccaggact 360
   taccattgaa actgttgttc tacatagtgt ggctatatga gaatgcgaga cttgattgtt 420
   ttgtattgta ttgtattgta ttgtattact tttaatcttg cttgaatttc catttaacaa 480
   taaatatgga gttcaataag taccatcagt gttaaaataa tatatcgtta atagctatta 540
   ttttagtgtc tgtttctttt tactaaacta tattttattt tagtatttgc tattgatttg 600
   aaataaatat tgtcctctta actgaaaaaa aaaa 634
<210> 65
   <211> 132
   <212> PRT
   <213> Trifolium repens
<220>
   <221> MISC_FEATURE
   <222> (3) .. (3)
   <223> Any amino acid
<220>
   <221> MISC_FEATURE
   <222> (6) .. (6)
   <223> Any amino acid
<220>
   <221> MISC_FEATURE
   <222> (9) .. (9)
   <223> Any amino acid
<400> 65
   Leu Arg Xaa Ala Gly Xaa Thr Phe Xaa Leu Leu Lys Asp Val Pro Glu
   1 5 10 15
   Ile Val Ser Lys Asn Ile Asp Lys Ala Leu Val Glu Ala Phe Gln Pro
   20 25 30
   Leu Asn Ile Ser Asp Tyr Asn Ser Ile Phe Trp Ile Ala His Pro Gly
   35 40 45
   Gly Pro Ala Ile Leu Asp Gln Val Glu Ile Lys Leu Gly Leu Lys Pro
   50 55 60
   Glu Lys Met Lys Ala Thr Arg Asp Val Leu Ser Glu Tyr Gly Asn Met
   65 70 75 80
   Ser Ser Ala Cys Val Leu Phe Ile Leu Asp Glu Met Gln Lys Lys Ser
   85 90 95
   Ala Glu Asn Gly Leu Lys Thr Thr Gly Glu Gly Leu Asp Trp Gly Val
   100 105 110
   Leu Phe Gly Phe Gly Pro Gly Leu Thr Ile Glu Thr Val Val Leu His
   115 120 125
   Ser Val Ala Ile
   130
<210> 66
   <211> 491
   <212> DNA
   <213> Trifolium repens
<220>
   <221> misc_feature
   <222> (7) .. (7)
   <223> Any nucleotide
<220>
   <221> misc_feature
   <222> (9) .. (9)
   <223> Any nucleotide
<220>
   <221> misc_feature
   <222> (18) .. (18)
   <223> Any nucleotide
<220>
   <221> misc_feature
   <222> (26) .. (26)
   <223> Any nucleotide
<220>
   <221> misc_feature
   <222> (429) .. (429)
   <223> Any nucleotide
<220>
   <221> misc_feature
   <222> (452) .. (452)
   <223> Any nucleotide
<220>
   <221> misc_feature
   <222> (468) .. (468)
   <223> Any nucleotide
<220>
   <221> misc_feature
   <222> (480) .. (480)
   <223> Any nucleotide
<220>
   <221> misc_feature
   <222> (482) .. (482)
   <223> Any nucleotide
<220>
   <221> misc_feature
   <222> (486) .. (486)
   <223> Any nucleotide
<220>
   <221> misc_feature
   <222> (488) .. (488)
   <223> Any nucleotide
<220>
   <221> misc_feature
   <222> (491) .. (491)
   <223> Any nucleotide
<400> 66
   tcttcgncna gctggacnaa catttntgct tcttaaagat gttcctgaga ttgtctcaaa 60
   gaacattgat aaggcattgg ttgaggcatt ccaaccatta aacatctctg attacaattc 120
   aatcttttgg attgctcatc caggtggtcc tgcaattcta gaccaagttg agataaagtt 180
   gggcttaaaa cctgaaaaaa tgaaggccac cagagatgta cttagtgaat atggtaacat 240
   gtcaagtgca tgtgtattgt tcatcttaga tgagatgaga aagaaatcgg ctgaaaatgg 300
   acttaaaacc acaggagaag gacttgactg gggtgtgttg tttggatttg ggcccggact 360
   taccattgaa actgttgttc tacatagtgt ggctatatga gaatgagaga cttgatttgt 420
   ttttattgna ttgtattact ttaaatcttg gntgaacttc catttaanaa taagtatggn 480
   gntcantntg n 491
<210> 67
   <211> 363
   <212> DNA
   <213> Trifolium repens
<220>
   <221> misc_feature
   <222> (22) .. (22)
   <223> Any nucleotide
<400> 67
   gagatgcaca agaaatcggc tnaaaatgga ctgaaaacca caggagaagg acttgactgg 60
   ggtgtgttgt ttggatttgg accaggactt accattgaaa ctgttgttct acatagtgtg 120
   gctatatgag aatgcgagac ttgattgttt tgtattgtat tgtattgtat tgtattactt 180
   ttaatcttgc ttgaatttcc atttaacaat aaatatggag ttcaataagt accatcagtg 240
   ttaaaataat atatcgttaa tagctattat tttagtgtct gtttcttttt actaaactat 300
   attttatttt agtatttgct attgatttga aataaatatt gtcctcttaa ctgaaaaaaa 360
   aaa
   363

<210> 68
   <211> 363
   <212> DNA
   <213> Trifolium repens
<400> 68
   gagatgccta agaaatcggc tcaaaatgga ctgaaaacca caggagaagg acttgactgg 60
   ggtgtgttgt ttggatttgg accaggactt accattgaaa ctgttgttct acatagtgtg 120
   gctatatgag aatgcgagac ttgattgttt tgtattgtat tgtattgtat tgtattactt 180
   ttaatcttgc ttgaatttcc atttaacaat aaatatggag ttcaataagt accatcagtg 240
   ttaaaataat atatcgttaa tagctattat tttagtgtct gtttcttttt actaaactat 300
   attttatttt agtatttgct attgatttga aataaatatt gtcctcttaa ctgaaaaaaa 360
   aaa 363
<210> 69
   <211> 897
   <212> DNA
   <213> Trifolium repens
<220>
   <221> misc_feature
   <222> (2) .. (2)
   <223> Any nucleotide
<220>
   <221> misc_feature
   <222> (26) .. (26)
   <223> Any nucleotide
<220>
   <221> misc_feature
   <222> (33) .. (33)
   <223> Any nucleotide
<400> 69
   gnttcaatct gttgtgcata aaattncttt gcnatagaaa accatacaca tttgatcttg
   60
   caaagaagaa atatgggaga cgaaggtata gtgagaggtg tcacaaagca gacaacccct
   120
   gggaaggcta ctatattggc tcttggcaag gcattccctc accaacttgt gatgcaagag
   180
   tatttagttg atggttattt tagggacact aattgtgaca atcctgaact taagcagaaa
   240
   cttgctagac tttgtaagac aaccacggta aaaacaaggt atgttgttat gaatgaggag
   300
   atactaaaga aatatccaga acttgttgtc gaaggcgcct caactgtaaa acaacgttta
   360
   gagatatgta atgaggcagt aacacaaatg gcaattgaag cttcccaagt ttgcctaaag
   420
   aattggggta gatccttatc ggacataact catgtggttt atgtttcatc tagtgaagct
   480
   agattacccg gtggtgacct atacttgtca aaaggactag gactaaaccc taaaattcaa
   540
   agaaccatgc tctatttctc tggatgctcg ggaggcgtag ccggccttcg cgttgcgaaa
   600
   gacatagctg agaacaaccc tggaagtaga gttttgcttg ctacttctga aactacaatt
   660
   attggattca agccaccaag tgttgataga ccttatgatc ttgttggtgt ggcactcttt
   720
   ggagatggtg ctggtgctat gataattggc tcagacccaa tacttgaaac tgagactcca
   780
   ttgtttgagc ttcatacttc agctcaggag tttataccag acacagagaa gaaaatagat
   840
   gggcggctga cggaggaggg cataagtttc acgctagcga gggaactgcc gcagata
   897
<210> 70
   <211> 275
   <212> PRT
   <213> Trifolium repens
<400> 70
   Met Gly Asp Glu Gly Ile Val Arg Gly Val Thr Lys Gln Thr Thr Pro
   1               5                   10                  15
   Gly Lys Ala Thr Ile Leu Ala Leu Gly Lys Ala Phe Pro His Gln Leu
               20                  25                  30
   Val Met Gln Glu Tyr Leu Val Asp Gly Tyr Phe Arg Asp Thr Asn Cys
           35                  40                  45
   Asp Asn Pro Glu Leu Lys Gln Lys Leu Ala Arg Leu Cys Lys Thr Thr
       50                  55                  60
   Thr Val Lys Thr Arg Tyr Val Val Met Asn Glu Glu Ile Leu Lys Lys
   65                  70                  75                  80
   Tyr Pro Glu Leu Val Val Glu Gly Ala Ser Thr Val Lys Gln Arg Leu
                   85                  90                  95
   Glu Ile Cys Asn Glu Ala Val Thr Gln Met Ala Ile Glu Ala Ser Gln
   100 105 110
   Val Cys Leu Lys Asn Trp Gly Arg Ser Leu Ser Asp Ile Thr His Val
   115 120 125
   Val Tyr Val Ser Ser Ser Glu Ala Arg Leu Pro Gly Gly Asp Leu Tyr
   130 135 140
   Leu Ser Lys Gly Leu Gly Leu Asn Pro Lys Ile Gln Arg Thr Met Leu
   145 150 155 160
   Tyr Phe Ser Gly Cys Ser Gly Gly Val Ala Gly Leu Arg Val Ala Lys
   165 170 175
   Asp Ile Ala Glu Asn Asn Pro Gly Ser Arg Val Leu Leu Ala Thr Ser
   180 185 190
   Glu Thr Thr Ile Ile Gly Phe Lys Pro Pro Ser Val Asp Arg Pro Tyr
   195 200 205
   Asp Leu Val Gly Val Ala Leu Phe Gly Asp Gly Ala Gly Ala Met Ile
   210 215 220
   Ile Gly Ser Asp Pro Ile Leu Glu Thr Glu Thr Pro Leu Phe Glu Leu
   225 230 235 240
   His Thr Ser Ala Gln Glu Phe Ile Pro Asp Thr Glu Lys Lys Ile Asp
   245 250 255
   Gly Arg Leu Thr Glu Glu Gly Ile Ser Phe Thr Leu Ala Arg Glu Leu
   260 265 270
   Pro Gln Ile
   275
<210> 71
   <211> 577
   <212> DNA
   <213> Trifolium repens
<220>
   <221> misc_feature
   <222> (2) .. (2)
   <223> Any nucleotide
<220>
   <221> misc_feature
   <222> (26) .. (26)
   <223> Any nucleotide
<220>
   <221> misc_feature
   <222> (33) .. (33)
   <223> Any nucleotide
<220>
   <221> misc_feature
   <222> (43) .. (43)
   <223> Any nucleotide
<400> 71
   gnttcaatct gttgtgcata aaattncttt gcnatagaaa acnctacaca tttgatcttg
   60
   caaagaagaa atatgggaga cgaaggtata gtgagaggtg tcacaaagca gacaacccct
   120
   gggaaggcta ctatattggc tcttggcaag gcattccctc accaacttgt gatgcaagag
   180
   tgtttagttg atggttattt tagggacact aattgtgaca atcctgaact taagcagaaa
   240
   cttgctagac tttgtaagac aaccacggta aaaacaaggt atgttgttat gaatgaggag
   300
   atactaaaga aatatccaga acttgttgtc gaaggcgcct caactgtaaa acaacgttta
   360
   gagatatgta atgaggcagt aacacaaatg gcaattgaag cttcccaagt ttgcctaaag
   420
   aattggggta gatccttatc ggacataact catgtggttt atgtttcatc tagtgaagct
   480
   agattacccg gtggtgacct atacttgtca aaaggactag gactaaaccc taaaattcaa
   540
   agaaccatgc tctatttctc tggatgctcg ggaggcg
   577
<210> 72
   <211> 599
   <212> DNA
   <213> Trifolium repens
<220>
   <221> misc_feature
   <222> (12) .. (12)
   <223> Any nucleotide
<220>
   <221> misc_feature
   <222> (20) .. (20)
   <223> Any nucleotide
<220>
   <221> misc_feature
   <222> (23) .. (23)
   <223> Any nucleotide
<220>
   <221> misc_feature
   <222> (30) .. (30)
   <223> Any nucleotide
<400> 72
   tcaatctgtt gngcattttn ttnctttgcn atagaaaacc atacacattt gatcttgctt
   60
   agaagaaata tgggagacga aggtatagtg agaggtgtca caaagcagac aacccctggg
   120
   aaggctacta tattggctct tggcaaggca ttccctcacc aacttgtgat gcaagagtgt
   180
   ttagttgatg gttattttag ggacactaat tgtgacaatc ctgaacttaa gcagaaactt
   240
   gctagacttt gtaagacaac cacggtaaaa acaaggtatg ttgttatgaa tgaggagata
   300
   ctaaagaaat atccagaact tgttgtcgaa ggcgcctcaa ctgtaaaaca acgtttagag
   360
   atatgtaatg aggcagtaac acaaatggca attgaagctt cccaagtttg cctaaagaat
   420
   tggggtagat ccttatcgga cataactcat gtggtttatg tttcatctag tgaagctaga
   480
   ttacccggtg gtgacctata cttgtcaaaa ggactaggac taaaccctaa aattcaaaga
   540
   accatgctct atttctctgg atgctcggga ggcgtagccg gccttcgcgt tgcgaaaga
   599
<210> 73
   <211> 581
   <212> DNA
   <213> Trifolium repens
<220>
   <221> misc_feature
   <222> (10) .. (11)
   <223> Any nucleotides
<220>
   <221> misc_feature
   <222> (19) .. (19)
   <223> Any nucleotide
<220>
   <221> misc_feature
   <222> (26) .. (26)
   <223> Any nucleotide
<400> 73 <210> 74
   <211> 588
   <212> DNA
   <213> Trifolium repens
<220>
   <221> misc_feature
   <222> (11) .. (11)
   <223> Any nucleotide
<220>
   <221> misc_feature
   <222> (13) .. (13)
   <223> Any nucleotide
<220>
   <221> misc_feature
   <222> (25) .. (27)
   <223> Any nucleotides
<220>
   <221> misc_feature
   <222> (36) .. (36)
   <223> Any nucleotide
<400> 74
<210> 75
   <211> 563
   <212> DNA
   <213> Trifolium repens
<400> 75
   ttaacatttt tttattgtag aaaatataca tttgatctag caaagaagaa atatgggaga
   60
   cgaaggtata gtgagaggtg tcacaaggca gccaacccct gggaaggcta ctatattggc
   120
   tcttggcaag gcattccctc accaacttgt gatgcaagag tatttagttg atggttattt
   180
   tagggacact aattgtgaca atcctaagct taagcagaaa cttgctagac tttgcaagac
   240
   aaccacagtg aaaacaaggt atgttgttat gaatgaagag atactaaaga aatatccaga
   300
   acttactatc ggaggcacct cgacggtaaa acaacattta gagatatgta atgaggcagt
   360
   aacacaaatg gcaattgaag cttcccaagt ttgcctaaag aattggggta gacccatatc
   420
   agacataact catgtggttt atgtttcatc tagtgaagct agattacctg gtggtgacct
   480
   atacttgtca aaaggactag gactaaaccc taaaattcaa agaaccatgc tctatttctc
   540
   gggatgctcg ggaggcgtag ccg
   563
<210> 76
   <211> 603
   <212> DNA
   <213> Trifolium repens
<220>
   <221> misc_feature
   <222> (4) .. (4)
   <223> Any nucleotide
<400> 76
   gcantagaaa accctacaca tttgatcttg caaagaagaa atatgggaga cgaatgtata 60
   gtgagaggtg tcacaaagca gacaacccct gggaaggcta ctatattggc tcttggcaag 120
   gcattccctc accaacttgt gatgcaagag tatttagttg atggttattt tagggacact 180
   aattgtgaca atcctgacct taagcagaaa cttgctagac tttgtaagac aaccacggta 240
   aaaacaaggt atgttgttat gaatgaggag atactaaaga aatatccaga acttgttgtc 300
   gaaggcgcct caactgtaaa acaacgttta gagatatgta atgaggcagt aacacaaatg 360
   gcaattgaag cttcccaagt ttgcctaaag aattggggta gatccttatc ggacataact 420
   catgtggttt atgtttcatc tagtgaagct agattacccg gtggtgacct atacttgtca 480
   aaaggactag gactaaaccc taaaattcaa agaaccatgc tctatttctc tggatgctcg 540
   ggaggcgtag ccggccttcg cgttgcgaaa gacatagctg agaacaaccc tggaagtaga 600
   gtt 603
<210> 77
   <211> 584
   <212> DNA
   <213> Trifolium repens
<220>
   <221> misc_feature
   <222> (1) .. (1)
   <223> Any nucleotide
<220>
   <221> misc_feature
   <222> (5) .. (5)
   <223> Any nucleotide
<220>
   <221> misc_feature
   <222> (40) .. (40)
   <223> Any nucleotide
<400> 77
   ntccngaact tactgtggaa ggcgcctcga ctgtaaaacn acgtttagag atatgtaatg 60
   aggcagtaac acaaatggca attgaagctt cccaagtttg cctaaagaat tggggtagac 120
   ccttatcaga cataactcat gtggtttatg tttcctctag tgaagctaga ttacccggtg 180
   gtgacctata tttgtcaaaa ggactaggac taaatcctaa aattcaaaga accatgctct 240
   atttctctgg atgctcagga ggcgtagccg gccttcgcgt tgcgaaagac atagctgaga 300
   acaaccctgg aagtagagtt ttgcttgcta cttctgaaac tacaattatt ggattcaagc 360
   caccaagtgt tgatagacct tatgatcttg ttggtgtggc actctttgga gatggtgctg 420
   gtgctatgat aattggctca gacccaatac ttgaaactga gactccattg tttgagcttc 480
   atacttcagc tcaggagttt ataccagaca cagagaagaa aatagatggg cggctgacgg 540
   aggagggcat aagtttcacg ctagcgaggg aactgccgca gata 584
<210> 78
   <211> 735
   <212> DNA
   <213> Trifolium repens
<220>
   <221> misc_feature
   <222> (47) .. (47)
   <223> Any nucleotide
<400> 78
   gtagcaacac acactttgat ttctttttga gtccttgcta cgtggcntta ccaaaaaacg 60
   ttgctaagtc atcaaccatt ccaattcctt aatataacct atcagtactc accatctttt 120
   cttcctccct gctaacttta tacttagaga agatggtgaa agttaatgag atccgccagg 180
   cacagagagc tgaaggccct gccactgtgt tggcaatcgg cactgcaact cctccaaact 240
   gtgttgatca gagtacatac cccgactact acttccgcat cacaaacagt gagcacaaga 300
   cagagctcaa agaaaaattc cagcgcatgt gtgacaaatc tatgattaag aagagataca 360
   tgcatttgac agaagagatt ttgaaggaga atccaagttt atgtgagtac atggcacctt 420
   cattggatgc aagacaagac atggtggttg tggaagtacc aaggctagga aaagaggcag 480
   caacaaaggc aatcaaggaa tggggtcaac ctaagtccaa gattacccac ctcatctttt 540
   gcaccacaag tggtgtggac atgcccggtg ccgactatca gcttacaaag cttttaggcc 600
   ttcgtccgca tgtgaagcgt tacatgatgt accaacaagg ttgttttgct ggtggcacgg 660
   tgcttcgttt ggctaaagac ttggctgaaa acaacaaagg tgcccgtgta ttggtggttt 720
   gttcagagat aactg 735
<210> 79
   <211> 194
   <212> PRT
   <213> Trifolium repens
<400> 79
   Met Val Lys Val Asn Glu Ile Arg Gln Ala Gln Arg Ala Glu Gly Pro
   1               5                   10                  15
   Ala Thr Val Leu Ala Ile Gly Thr Ala Thr Pro Pro Asn Cys Val Asp
               20                  25                  30
   Gln Ser Thr Tyr Pro Asp Tyr Tyr Phe Arg Ile Thr Asn Ser Glu His
           35                  40                  45
   Lys Thr Glu Leu Lys Glu Lys Phe Gln Arg Met Cys Asp Lys Ser Met
       50                  55                  60
   Ile Lys Lys Arg Tyr Met His Leu Thr Glu Glu Ile Leu Lys Glu Asn
   65                  70                  75                  80
   Pro Ser Leu Cys Glu Tyr Met Ala Pro Ser Leu Asp Ala Arg Gln Asp
                   85                  90                  95
   Met Val Val Val Glu Val Pro Arg Leu Gly Lys Glu Ala Ala Thr Lys
               100                 105                 110
   Ala Ile Lys Glu Trp Gly Gln Pro Lys Ser Lys Ile Thr His Leu Ile
           115                 120                 125
   Phe Cys Thr Thr Ser Gly Val Asp Met Pro Gly Ala Asp Tyr Gln Leu
       130                 135                 140
   Thr Lys Leu Leu Gly Leu Arg Pro His Val Lys Arg Tyr Met Met Tyr
   145                 150                 155                 160
   Gln Gln Gly Cys Phe Ala Gly Gly Thr Val Leu Arg Leu Ala Lys Asp
                   165                 170                 175
   Leu Ala Glu Asn Asn Lys Gly Ala Arg Val Leu Val Val Cys Ser Glu
               180                 185                 190
   Ile Thr

<210> 80
   <211> 574
   <212> DNA
   <213> Trifolium repens
<220>
   <221> misc_feature
   <222> (47) .. (47)
   <223> Any nucleotide
<400> 80
   gtagcaacac acactttgat ttctttttga gtccttgcta cgtggcntta ccaaaaaacg 60
   ttgctcactc atcaaccatt ccaattcctt aatataacct atcagtactt accatctttt 120
   cttcctccct gctaacttta gaatcagaga agatggtgaa tgttaatgag atccgccagg 180
   cacagagagc tgaaggccct gccactgtgt tggcaatcgg cactgcaact cctccaaact 240
   gtgtcgatca gagtacatac ccggactact acttccgcat cacaaacagt gagcacaaga 300
   cagagctcaa agaaaaattc cagcgcatgt gtgacaaatc tatgattaag aagagataca 360
   tgcatttgac agaagagatt ttgaaggaga atccaagttt atgtgagtac atggcacctt 420
   cattggatgc aagacaagac atggtggttg tggaagtacc aaggctagga aaagaggcag 480
   caacaaaggc aattaaggaa tggggtcaac ctaagtccaa gattacccac ctcatctttt 540
   gcaccaccag tggtgtcgac atgcccggtg ccgc 574
<210> 81
   <211> 597
   <212> DNA
   <213> Trifolium repens
<400> 81
   gtagcaacac acactttgat ttctttttga gtccttgcta cgtggcttta ccaaaaaacg 60
   ttgctaagtc atcaaccatt ccaattcctt aatataacct atcagtactc accatctttt 120
   cttcctccct gctaacttta gactcagaga agatggtgaa tgttaatgag atccgccagg 180
   cacagagagc tgaaggccct gccaccgtgt tggcaatcgg cactgcaact cctccaaact
   240
   gtgtcgatca gagtacatac ccagactact acttccgcat cacaaacagt gagcacaaga
   300
   cagagctcaa agaaaaattc cagcgcatgt gtgacaaatc tatgattaag aagagataca
   360
   tgcatttgac agaagagatt ttgaaggaga atccaagttt atgtgagtac atggcacctt
   420
   cattggatgc aagacaagac atggtggttg tggaagtacc aaggctagga aaagaggctg
   480
   caacaaaggc tatcaaggaa tggggtcaac ctaagtccaa gattactcac ctcatctttt
   540
   gcaccacaag tggtgtggac atgcctggcg ccgactatca gcttacaaag cttttag
   597
<210> 82
   <211> 616
   <212> DNA
   <213> Trifolium repens
<220>
   <221> misc_feature
   <222> (70) .. (70)
   <223> Any nucleotide
<220>
   <221> misc_feature
   <222> (616) .. (616)
   <223> Any nucleotide
<400> 82
   gtagcaacac acactttgat ttctttttga gtccttgcta cgtggcttta ccaaaaaacg
   60
   ttgctaagtn atcaaccatt ccaattcctt aatataacct atcagtactc accatctttt
   120
   cttcctccct gctaacttta gactcagaga agatggtgaa tgttaatgag atccgccagg
   180
   cacagagagc tgaaggccct gccaccgtgt tggcaatcgg cactgcaact cctccaaact
   240
   gtgtcgatca gagtacatac ccagactact acttccgcat cacaaacagt gagcacaaga
   300
   cagagctcaa agaaaaattc cagcgcatgt gtgacaaatc tatgattaag aagagataca
   360
   tgcatttgac agaagagatt ttgaaggaga atccaagttt atgtgagtac atggcacctt
   420
   cattggatgc aagacaagac atggtggttg tggaagtacc aaggctagga aaagaggctg
   480
   caacaaaggc tatcaaggaa tggggtcaac ctaagtccaa gattactcac ctcatctttt
   540
   gcaccacaag tggtgtggac atgcctggcg ccgactatca gcttacaaag cttttaggcc
   600
   ttcgtccgca tgtgan
   616
<210> 83
   <211> 585
   <212> DNA
   <213> Trifolium repens
<220>
   <221> misc_feature
   <222> (1) .. (3)
   <223> Any nucleotides
<220>
   <221> misc_feature
   <222> (7) .. (7)
   <223> Any nucleotide
<220>
   <221> misc_feature
   <222> (20) .. (20)
   <223> Any nucleotide
<220>
   <221> misc_feature
   <222> (37) .. (37)
   <223> Any nucleotide
<220>
   <221> misc_feature
   <222> (61) .. (61)
   <223> Any nucleotide
<220>
   <221> misc_feature
   <222> (451) .. (451)
   <223> Any nucleotide
<220>
   <221> misc_feature
   <222> (507) .. (507)
   <223> Any nucleotide
<400> 83
   nnncacncac acttttttgn atccctgcta cgtggcntta ccaaaaaacg ttgctaagtc
   60
   ntcaaccatt ccaattcctt aatataacct atcagtactc accatctttt cttcctccct
   120
   gctaacttta gactcagaga agatggtgaa tgttaatgag atccgccagg cacagagagc
   180
   tgaaggccct gccaccgtgt tggcaatcgg cactgcaact cctccaaact gtgttgatca
   240
   gagtacatac ccggactact acttccgcat cacaaacagt gagcacaaga cagagctcaa
   300
   agaaaaattc cagcgcatgt gtgacaaatc tatgattaag aagagataca tgcatttaac
   360
   agaagagatt ttgaaggaga atccaagttt atgtgagtac atggcacctt cattggatgc
   420
   aagacaagac atggtggttg tggaagtacc naggctagga aaagaggcag taacaaaggc
   480
   aattagggaa tggggtcaac ctaagtncaa gattacccac ctcatctttt gcaccaccag
   540
   tggtgtggac atgcccggtg ccgactatca gctcacaaag ctctt
   585
<210> 84
   <211> 596
   <212> DNA
   <213> Trifolium repens
<220>
   <221> misc_feature
   <222> (3) .. (3)
   <223> Any nculeotide
<220>
   <221> misc_feature
   <222> (14) .. (14)
   <223> Any nculeotide
<220>
   <221> misc_feature
   <222> (37) .. (37)
   <223> Any nculeotide
<400> 84
   ttnatcccct aatntagttt tcttctttgt tacttanaga atatggttac agttaatgag
   60
   atccgccagg cacagagagc tgaaggccct gcaactgtgt ttgcaatcgg cactgcaact
   120
   cctaaaaact gtgttgatca gagtacttac cccgactact atttccgaat cacaaacagc
   180
   gaacacaaga ctgaactcaa agaaaaattc cagcgcatgt gtgacaaatc tatgattaag
   240
   aagagataca tgcatttgac agaagagatt ttgaaggaga attcaagttt atgtgagtac
   300
   atggcacctt cattggatgc aagacaagac atggtggttg tggaagtacc aaggctagga
   360
   aaagaggctg caacaaaggc tatcaaggaa tggggtcaac ctaagtccaa gattactcac
   420
   ctcatctttt gcaccacaag tggtgtggac atgcctggtg ccgactatca gcttacaaag
   480
   cttttaggcc ttcgtccgca tgtgaagcgt tatatgatgt accaacaagg ttgttttgct
   540
   ggtggcacgg tgcttcgttt ggctaaagac ttggctgaaa acaacaaagg tgcccg
   596
<210> 85
   <211> 618
   <212> DNA
   <213> Trifolium repens
<220>
   <221> misc_feature
   <222> (3) .. (3)
   <223> Any nucleotide
<220>
   <221> misc_feature
   <222> (6) .. (6)
   <223> Any nucleotide
<220>
   <221> misc_feature
   <222> (9) .. (9)
   <223> Any nucleotide
<220>
   <221> misc_feature
   <222> (14) .. (14)
   <223> Any nucleotide
<220>
   <221> misc_feature
   <222> (37) .. (37)
   <223> Any nucleotide
<400> 85
   ttnatnccnt aatntagttt tcttctttgt tacttanaga atatggttac agttaatgag
   60
   atccgccagg cacagagagc tgaaggccct gcaactgtgt ttgcaatcgg cactgcaact
   120
   cctaaaaact gtgttgatca gagtacttac cccgactact atttccgaat cacaaacagc
   180
   gaacacaaga ctgaactcaa agaaaaattc cagcgcatgt gtgacaaatc tatgattaag
   240
   aagagataca tgcatttgac agaagagatt ttgaaggaga attcaagttt atgtgagtac
   300
   atggcacctt cattggatgc aagacaagac atggtggttg tggaagtacc aaggctagga
   360
   aaagaggctg caacaaaggc tatcaaggaa tggggtcaac ctaagtccaa gattactcac
   420
   ctcatctttt gcaccacaag tggtgtggac atgcctggtg ccgactatca gcttacaaag
   480
   cttttaggcc ttcgtccgca tgtgaagcgt tatatgatgt accaacaagg ttgttttgct
   540
   ggtggcacgg tgcttcgttt ggctaaagac ttggctgaaa acaacaaagg tgcccgtgta
   600
   ttggtggttt gttcagag
   618
<210> 86
   <211> 609
   <212> DNA
   <213> Trifolium repens
<220>
   <221> misc_feature
   <222> (4) .. (4)
   <223> Any nucleotide
<220>
   <221> misc_feature
   <222> (10) .. (10)
   <223> Any nucleotide
<220>
   <221> misc_feature
   <222> (32) .. (33)
   <223> Any nucleotides
<400> 86
   tccnataatn tagttttctt ctttgttact tnnagaatat ggttacagtt aatgagatcc
   60
   gccaggcaca gagagctgaa ggccctgcaa ctgtgtttgc aatcggcact gcaactccta
   120
   aaaactgtgt tgatcagagt acttaccccg actactattt ccgaatcaca aacagcgaac
   180
   acaagactga actcaaagaa aaattccagc gcatgtgtga caaatctatg attaagaaga
   240
   gatacatgca tttgacagaa gagattttga aggagaattc aagtttatgt gagtacatgg
   300
   caccttcatt ggatgcaaga caagacatgg tggttgtgga agtaccaagg ctaggaaaag
   360
   aggctgcaac aaaggctatc aaggaatggg gtcaacctaa gtccaagatt actcacctca
   420
   tcttttgcac cacaagtggt gtggacatgc ctggtgccga ctatcagctt acaaagcttt
   480
   taggccttcg tccgcatgtg aagcgttata tgatgtacca acaaggttgt tttgctggtg
   540
   gcacggtgct tcgtttggct aaagacttgg ctgaaaacaa caaaggtgcc cgtgtattgg
   600
   tggtttgtt
   609
<210> 87
   <211> 571
   <212> DNA
   <213> Trifolium repens
<220>
   <221> misc_feature
   <222> (3) .. (4)
   <223> Any nucleotides
<220>
   <221> misc_feature
   <222> (14) .. (14)
   <223> Any nucleotide
<220>
   <221> misc_feature
   <222> (18) .. (19)
   <223> Any nucleotides
<220>
   <221> misc_feature
   <222> (22) .. (22)
   <223> Any nucleotide
<220>
   <221> misc_feature
   <222> (60) .. (60)
   <223> Any nucleotide
<220>
   <221> misc_feature
   <222> (87) .. (87)
   <223> Any nucleotide
<220>
   <221> misc_feature
   <222> (89) .. (89)
   <223> Any nucleotide
<220>
   <221> misc_feature
   <222> (91) .. (91)
   <223> Any nucleotide
<220>
   <221> misc_feature
   <222> (247) .. (247)
   <223> Any nucleotide
<220>
   <221> misc_feature
   <222> (571) .. (571)
   <223> Any nucleotide
<400> 87
   gcnntaacct ttanaatnng anaagatggt gaaagttaat gagatccgcc aggcacagan
   60
   agctgaaggc cctgccactg tgttggnant nggcactgca actcctccaa actgtgtcga
   120
   tcagagtaca tacccggact actacttccg catcacaaac agtgagcaca agacagagct
   180
   caaagaaaaa ttccagcgca tgtgtgacaa atctatgatt aagaagagat acatgcattt
   240
   gacagangag attttgaagg agaatccaag tttatgtgag tacatggcac cttcattgga
   300
   tgcaagacaa gacatggtgg ttgtggaagt accaaggcta ggaaaagagg cagcaacaaa
   360
   ggcaattaag gaatggggcc aacctaagtc caagattacc cacctcatct tttgcaccac
   420
   cagtggtgtc gacatgcccg gtgccgacta tcagctcaca aagctcttag gcctgcgtcc
   480
   atatgtgaag cgttacatga tgtatcaaca aggttgtttt gctggtggca cggtgcttcg
   540
   tttggctaaa gacttggctg aaaacaacaa n
   571
<210> 88
   <211> 603
   <212> DNA
   <213> Trifolium repens
<220>
   <221> misc_feature
   <222> (2) .. (4)
   <223> Any nucleotides
<220>
   <221> misc_feature
   <222> (9) .. (9)
   <223> Any nucleotide
<220>
   <221> misc_feature
   <222> (13) .. (14)
   <223> Any nucleotides
<220>
   <221> misc_feature
   <222> (32) .. (32)
   <223> Any nucleotide
<220>
   <221> misc_feature
   <222> (567) .. (567)
   <223> Any nucleotide
<220>
   <221> misc_feature
   <222> (594) .. (594)
   <223> Any nucleotide
<400> 88
   tnnntttana atnngagaag atggtgaaag tnaatgagat ccgccaggca cagagagctg 60
   aaggccctgc cactgtgttg gcaatcggca ctgcaactcc tccaaactgt gtcgatcaga 120
   gtacataccc ggactactac ttccgcatca caaacagtga gcacaagaca gagctcaaag 180
   aaaaattcca gcgcatgtgt gacaaatcta tgattaagaa gagatacatg catttgacag 240
   aagagatttt gaaggagaat ccaagtttat gtgagtacat ggcaccttca ttggatgcaa 300
   gacaagacat ggtggttgtg gaagtaccaa ggctaggaaa agaggcagca acaaaggcaa 360
   ttaaggaatg gggccaacct aagtccaaga ttacccacct catcttttgc accaccagtg 420
   gtgtcgacat gcccggtgcc gactatcagc tcacaaagct cttaggcctg cgtccatatg 480
   tgaagcgtta catgatgtat caacaaggtt gttttgctgg tggcacggtg cttcgtttgg 540
   ctaaagactt ggctgaaaac aacaaangtg cccgtgtgtt ggtgggttgt tcanagataa 600
   ctg 603
<210> 89
   <211> 588
   <212> DNA
   <213> Trifolium repens
<220>
   <221> misc_feature
   <222> (7) .. (7)
   <223> Any nucleotide
<220>
   <221> misc_feature
   <222> (12) .. (12)
   <223> Any nucleotide
<220>
   <221> misc_feature
   <222> (43) .. (43)
   <223> Any nucleotide
<220>
   <221> misc_feature
   <222> (68) .. (68)
   <223> Any nucleotide
<400> 89
   tttgttnatt gnagaatatg gttacagtta atgagatccg ccnggcacaa agagccgaag 60
   gccctgcncc tgtgtttgca atcggcactg caactcctaa aaactgtgtt gatcagagta 120
   cttaccccga ctactatttc cgaatcacaa acagcgaaca caagactgag ctcaaagaaa 180
   aattccagcg catgtgtgac aaatctatga ttaagaagag atacatgcat ttgacagaag 240
   agattttgaa ggagaatcca agtttatgtg agtacatggc accttcattg gatgcaagac 300
   aagacatggt ggtagtggaa gtaccaaggc taggaaaaga ggcagcaaca aaggcaatta 360
   aagaatgggg tcaacctaag tccaagatta cccacctcat cttttgcacc accagtggtg 420
   tggacatgcc cggtgccgac tatcagctca caaagctctt aggcctgcgt ccatatgtga 480
   agcgttacat gatgtatcaa caaggttgtt ttgctggtgg cacggtactt cgtttggcta 540
   aagacttggc tgaaaacaac aaaggtgccc gtgtgttggt ggtttgtt 588
<210> 90
   <211> 250
   <212> DNA
   <213> Trifolium repens
<220>
   <221> misc_feature
   <222> (17) .. (17)
   <223> Any nucleotide
<220>
   <221> misc_feature
   <222> (35) .. (35)
   <223> Any nucleotide
<220>
   <221> misc_feature
   <222> (48) .. (49)
   <223> Any nucleotides
<220>
   <221> misc_feature
   <222> (165) .. (165)
   <223> Any nucleotide

<220>
   <221> misc_feature
   <222> (188) .. (188)
   <223> Any nucleotide
<220>
   <221> misc_feature
   <222> (212) .. (212)
   <223> Any nucleotide
<220>
   <221> misc_feature
   <222> (224) .. (225)
   <223> Any nucleotides
<220>
   <221> misc_feature
   <222> (228) .. (228)
   <223> Any nucleotide
<220>
   <221> misc_feature
   <222> (232) .. (232)
   <223> Any nucleotide
<220>
   <221> misc_feature
   <222> (235) .. (235)
   <223> Any nucleotide
<220>
   <221> misc_feature
   <222> (240) .. (240)
   <223> Any nucleotide
<220>
   <221> misc_feature
   <222> (247) .. (247)
   <223> Any nucleotide
<220>
   <221> misc_feature
   <222> (250) .. (250)
   <223> Any nucleotide
<400> 90
   taaaactgtg ttgatcngag tacttacccc gactnctatt tccgaatnnc aaacagcgaa 60
   cacaagactg agctcaaaga aaaattccag cgcatgtgtg acaaatctat gattaagaag 120
   agatacatgc atttgacaga agagattttg aaggagaatc caagnttatg tgagtacatg
   180
   gcaccttnat tggatgcaag acaagacatg gnggccgccc accnnccntc cnccncaccn
   240
   caacccnccn
   250
<210> 91
   <211> 583
   <212> DNA
   <213> Trifolium repens
<220>
   <221> misc_feature
   <222> (2) .. (2)
   <223> Any nucleotide
<220>
   <221> misc_feature
   <222> (47) .. (47)
   <223> Any nucleotide
<220>
   <221> misc_feature
   <222> (552) .. (552)
   <223> Any nucleotide
<400> 91
   gnagcaacac acactttgat ttctttttga atccctgcta cgtggcncac caaaaaacgt
   60
   tgctaagtca tcaaccattc caattcctta atataaccta tcagtactca ccatcttttc
   120
   ttcctccctg ctaactttag actcagtaga agatggtgaa tgttaatgag atccgccagg
   180
   cacagagagc tgaaggccct gccaccgtgt tggcaatcgg cactgcaact cctccaaact
   240
   gtgttgatca gagtacatac ccggactact acttccgcat cacaaacagt gagcacaaga
   300
   cagagctcaa agaaaaattc cagcgcacgt gtaagatatt tatcttatac tccatgcatg
   360
   tctttttctg ctgactgccg tgtttatata ttgttttgtt ttgttcctta aatttgttat
   420
   gtcactctca catgtacaaa acacttaaga ctaaactgca tatcattttt ttcagggaca
   480
   aatctatgat taagaagaga tacatgcatt tgacagaaga gattttgaag gagaatccaa
   540
   gtttatgtga gnacatggca ccttcttggg atgcaagaca agt
   583
<210> 92
   <211> 95
   <212> PRT
   <213> Trifolium repens
<220>
   <221> MISC_FEATURE
   <222> (86) .. (86)
   <223> Any amino acid
<400> 92
   Met Val Asn Val Asn Glu Ile Arg Gln Ala Gln Arg Ala Glu Gly Pro
   1               5                   10                  15
   Ala Thr Val Leu Ala Ile Gly Thr Ala Thr Pro Pro Asn Cys Val Asp
               20                  25                  30
   Gln Ser Thr Tyr Pro Asp Tyr Tyr Phe Arg Ile Thr Asn Ser Glu His
           35                  40                  45
   Lys Thr Glu Leu Lys Glu Lys Phe Gln Arg Thr Arg Asp Lys Ser Met
       50                  55                  60
   Ile Lys Lys Arg Tyr Met His Leu Thr Glu Glu Ile Leu Lys Glu Asn
   65                  70                  75                  80
   Pro Ser Leu Cys Glu Xaa Met Ala Pro Ser Trp Asp Ala Arg Gln
                   85                  90                  95
<210> 93
   <211> 582
   <212> DNA
   <213> Trifolium repens
<220>
   <221> misc_feature
   <222> (2) .. (2)
   <223> Any nucleotide
<220>
   <221> misc_feature
   <222> (551) .. (551)
   <223> Any nucleotide
<400> 93
   gnagcaacac acactttgat ttctttttga atccctgcta cgtggcttac caaaaaacgt 60
   tgctaagtca tcaaccattc caattcctta atataaccta tcagtactca ccattttttc 120
   ttcctccctg ctaactttag actcagagaa gatggtgaat gttaatgaga tccgccaggc 180
   acagagagct gaaggccctg ccaccgtgtt ggcaatcggc actgcaactc ctccaaactg 240
   tgttgatcag agtacatacc cggactacta cttccgcatc acaaacagtg agcacaagac
   300
   agagctcaaa gaaaaattcc agcgcacgtg taagatattt atcttatact ccatgcatgt 360
   ctttttctgc tgactgccgt gtttatatat tgttttgttt tgttccttaa atttgttatg 420
   tcactctcac atgtacaaaa cacttaagac taaactgcat atcatttttt tcagggacaa 480
   atctatgatt aagaagagat acatgcattt gacagaagag attttgaagg agaatccaag 540
   tttatgtgag nacatggcac cttcttggga tgcaagacaa gt 582
<210> 94
   <211> 167
   <212> DNA
   <213> Trifolium repens
<220>
   <221> misc_feature
   <222> (17) .. (17)
   <223> Any nucleotide
<220>
   <221> misc_feature
   <222> (34) .. (34)
   <223> Any nucleotide
<220>
   <221> misc_feature
   <222> (38) .. (38)
   <223> Any nucleotide
<400> 94
   gtccctgctg cgtggcncac caaaaacgtt gctnagtntg aaccttccat tccttaatat 60
   aacctatcag tactcaccat cttttcttcc tccctgctaa ctttagactc agtagaagat 120
   ggtgaatgtt aatgagatcc gccaggcaca gagagctgaa ggccctg 167
<210> 95
   <211> 613
   <212> DNA
   <213> Trifolium repens
<220>
   <221> misc_feature
   <222> (3) .. (3)
   <223> Any nucleotide
<220>
   <221> misc_feature
   <222> (16) .. (16)
   <223> Any nucleotide
<400> 95
   gcntaagcct tgattnttgt ttgtttccta acacaagaac tagtgtttgc ttgaatctta 60
   agaaaaaatg cctcaaggtg atttgaatgg aagttcctcg gtgaatggag cacgtgctag 120
   acgtgctcct actcagggaa aggcaacgat acttgcatta ggaaaggctt tccccgccca 180
   agtcctccct caagagtgct tggtggaagg attcattcgc gacactaagt gtgacgatac 240
   ttatattaag gagaaattgg agcgtctttg caaaaacaca actgtaaaaa caagatacac 300
   agtaatgtca aaggagatct tagacaacta tccagagcta gccatagatg gaacaccaac 360
   aataaggcaa aagcttgaaa tagcaaatcc agcagtagtt gaaatggcaa caagagcaag 420
   caaagattgc atcaaagaat ggggaaggtc acctcaagat atcacacaca tagtctatgt 480
   ttcctcgagc gaaattcgtc tacccggtgg tgacctttat cttgcaaatg aactcggctt 540
   aaacagcgat gttaatcgcg taatgctcta tttcctcggt tgctacggcg gtgtcactgg 600
   cttacgtgtc gcc 613
<210> 96
   <211> 182
   <212> PRT
   <213> Trifolium repens
<400> 96 <210> 97
   <211> 613
   <212> DNA
   <213> Trifolium repens
<220>
   <221> misc_feature
   <222> (3) .. (3)
   <223> Any nucleotide
<220>
   <221> misc_feature
   <222> (16) .. (16)
   <223> Any nucleotide
<400> 97
<210> 98
   <211> 570
   <212> DNA
   <213> Trifolium repens
<220>
   <221> misc_feature
   <222> (13) .. (13)
   <223> Any nucleotide
<400> 98
   taagccttga ttnttgtttg tttcctaaca caagaactag tgtttgcttg aatcttaaga 60
   aaaaatgcct caaggtgatt tgaatggaag ttcctcggtg aatggagcac gtgctagacg 120
   tgctcctact cagggaaagg caacgatact tgcattagga aaggctttcc ccgcccaggt 180
   cctccctcaa gagtgcttgg tggaaggatt cattcgcgac actaagtgtg acgatactta 240
   tattaaggag aaattggagc gtctttgcaa aaacacaact gtgaaaacaa gatacacagt 300
   aatgtcaaag gagatcttag acaactatcc agagctagcc atagatggaa caccaacaat 360
   aaggcaaaag cttgaaatag caaatccagc agtagttgaa atggcaacaa gagcaagcaa 420
   agattgcatc aaagaatggg gaaggtcacc tcaagatatc acacacatag tctatgtttc 480
   ctcgagcgaa attcgtctac ccggtggtga cctttatctt gcaaatgaac tcggcttaaa 540
   cagcgatgtt aatcgcgtaa tgctctattt 570
<210> 99
   <211> 575
   <212> DNA
   <213> Trifolium repens
<220>
   <221> misc_feature
   <222> (33) .. (33)
   <223> Any nucleotide
<400> 99
   ttgattcttg tttgtttcct aacacaagaa ctngtgtttg cttgaatctt aagaaaaaat 60
   gcctcaaggt gatttgaatg gaagttcctc ggtgaatgga gcacgtgcta gacgtgctcc 120
   tactcaggga aaggcaacga tacttgcatt aggaaaggct ttccccgccc aggtcctccc 180
   tcaagagtgc ttggtggaag gattcattcg cgacactaag tgtgacgata cttatattaa 240
   ggagaaattg gagcgtcttt gcaaaaacac aactgtgaaa acaagataca cagtaatgtc 300
   aaaggagatc ttagacaact atccagagct agccatagat ggaacaccaa caataaggca 360
   aaagcttgaa atagcaaatc cagcagtagt tgaaatggca acaagagcaa gcaaagattg 420
   catcaaagaa tggggaaggt cacctcaaga tatcacacac atagtctatg tttcctcgag 480
   cgaaattcgt ctacccggtg gtgaccttta tcttgcaaat gaactcggct taaacagcga 540
   tgttaatcgc gtaatgctct atttcctcgg ttgct 575
<210> 100
   <211> 573
   <212> DNA
   <213> Trifolium repens
<400> 100
   gtttgtttcc taacacaaga actagtgttt gcttgaatct taagaaaaaa tgcctcaagg 60
   tgatttgaat ggaagttcct cggtgaatgg agcacgtgct agacgtgctc ctactcaggg 120
   aaaggcaacg atacttgcat taggaaaggc tttccccgcc caagtcctcc ctcaagagtg 180
   cttggtggaa ggattcattc gcgacactaa gtgtgacgat acttatatta aggagaaatt 240
   ggagcgtctt tgcaaaaaca caactgtaaa aacaagatac acagtaatgt caaaggagat 300
   cttagacaac tatccagagc tagccataga tggaacacca acaataaggc aaaagcttga 360
   aatagcaaat ccagcagtag ttgaaatggc aacaagagca agcaaagatt gcatcaaaga 420
   atggggaagg tcacctcaag atatcacaca catagtctat gtttcctcga gcgaaattcg 480
   tctacccggt ggtgaccttt atcttgcaaa tgaactcggc ttaaacagcg atgttaatcg 540
   cgtaatgctc tatttcctcg gttgctacgg cgg 573
<210> 101
   <211> 607
   <212> DNA
   <213> Trifolium repens
<400> 101
   gtataccaag gttgttttgc tggtggcacg gtacttcgtt tggctaaaga cttggctgaa 60
   aacaacaaag gtgcccgtgt gttggtggtt tgttcagaga taactgcagt tactttccgt 120
   ggacccagtg acactcacct tgatagcctt gtggggcaag cattgtttgg agatggtgca 180
   gcagctgtga ttgttggttc agaccctttg ccagaagttg agaagccttt gtttgaattg 240
   gtatggaccg cacaaacaat cgctccagat agtgaaggag ccattgatgg tcaccttcgc 300
   gaagcagggc tgacattcca tctcctcaag gatgttccta gccttgtctc aaataacatt 360
   gagaaagcgc ttgttgatgc ctttcaacct ttgaatattt ctgactacaa ttccatcttt 420
   tggattgcac acccaggcgg accagcaatt cttgaccaag ttgaagctaa gttaggctta 480
   aagccagaga aaatgcaagc cactcgacat gtacttagcg aatatggtaa catgtcaagt 540
   gcgtgtgtgt tatttatctt ggatgagatg aggaggaagt caaaagaaga cggacttgcc 600
   acaacag 607
<210> 102
   <211> 202
   <212> PRT
   <213> Trifolium repens
<400> 102
   Val Tyr Gln Gly Cys Phe Ala Gly Gly Thr Val Leu Arg Leu Ala Lys
   1               5                   10                  15
   Asp Leu Ala Glu Asn Asn Lys Gly Ala Arg Val Leu Val Val Cys Ser
               20                  25                  30
   Glu Ile Thr Ala Val Thr Phe Arg Gly Pro Ser Asp Thr His Leu Asp
           35                  40                  45
   Ser Leu Val Gly Gln Ala Leu Phe Gly Asp Gly Ala Ala Ala Val Ile
       50                  55                  60
   Val Gly Ser Asp Pro Leu Pro Glu Val Glu Lys Pro Leu Phe Glu Leu
   65                  70                  75                  80
   Val Trp Thr Ala Gln Thr Ile Ala Pro Asp Ser Glu Gly Ala Ile Asp
                   85                  90                  95
   Gly His Leu Arg Glu Ala Gly Leu Thr Phe His Leu Leu Lys Asp Val
               100                 105                 110
   Pro Ser Leu Val Ser Asn Asn Ile Glu Lys Ala Leu Val Asp Ala Phe
           115                 120                 125
   Gln Pro Leu Asn Ile Ser Asp Tyr Asn Ser Ile Phe Trp Ile Ala His
       130                 135                 140
   Pro Gly Gly Pro Ala Ile Leu Asp Gln Val Glu Ala Lys Leu Gly Leu
   145                 150                 155                 160
   Lys Pro Glu Lys Met Gln Ala Thr Arg His Val Leu Ser Glu Tyr Gly
                   165                 170                 175
   Asn Met Ser Ser Ala Cys Val Leu Phe Ile Leu Asp Glu Met Arg Arg
               180                 185                 190
   Lys Ser Lys Glu Asp Gly Leu Ala Thr Thr
           195                 200
<210> 103
   <211> 607
   <212> DNA
   <213> Trifolium repens
<220>
   <221> misc_feature
   <222> (12) .. (12)
   <223> Any nucleotide
<220>
   <221> misc_feature
   <222> (46) .. (46)
   <223> Any nucleotide
<400> 103
   gtataccaag gntgttttgc tggtggcacg gtacttcgtt tggctnaaga cttggctgaa 60
   aacaacaaag gtgcccgtgt gttggtggtt tgttcagaga taactgcagt tactttccgt 120
   ggacccagtg acactcatct tgatagcctt gtggggcaag cattgtttgg agatggtgca 180
   gcagctgtga ttgttggttc agaccctttg ccagaagttg agaagccttt gtttgaattg 240
   gtatggaccg cacaaacaat cgctccagat agtgaaggag ccattgatgg tcaccttcgc 300
   gaagcagggc tgacattcca tctcctcaag gatgttccta gccttgtctc aaataacatt 360
   gagaaagctc ttgttgatgc ctttcaacct ttgaatatct ccgattacaa ttccatcttt 420
   tggattgcac acccaggcgg accagcaatt cttgaccaag ttgaagctaa gttaggctta 480
   aagccagaga aaatgcaatc cactcgacat gtacttagcg aatatggtaa catgtcgagt 540
   gcgtgtgtgt tatttatctt ggatgagatg aggaggaagt caaaagaaga cggacttgcc 600
   acaacag 607

<210> 104
   <211> 591
   <212> DNA
   <213> Trifolium repens
<220>
   <221> misc_feature
   <222> (562) .. (562)
   <223> Any nucleotide
<220>
   <221> misc_feature
   <222> (587) .. (587)
   <223> Any nucleotide
<220>
   <221> misc_feature
   <222> (589) .. (589)
   <223> Any nucleotide
<400> 104
   ccaaggttgt tttgctggtg gcacggtact tcgtttggct aaagacttgg ctgaaaacaa
   60
   caaaggtgcc cgtgtgttgg tggtttgttc agagataact gcagttactt tccgtggacc
   120
   cagtgacact caccttgata gccttgtggg gcaagcattg tttggagatg gtgcagcagc
   180
   tgtgattgtt ggttcagacc ctttgccaga agttgagaag cctttgtttg aattggtatg
   240
   gaccgcacaa acaatcgctc cagatagtga aggagccatt gatggtcacc ttcgcgaagc
   300
   agggctgaca ttccatctcc tcaaggatgt tcctagcctt gtctcaaata acattgagaa
   360
   agcgcttgtt gatgcctttc aacctttgaa tatttctgac tacaattcca tcttttggat
   420
   tgcacaccca ggcggaccag caattcttga ccaagttgaa gctaagttag gcttaaagcc
   480
   agagaaaatg caagccactc gacatgtact tagcgaatat ggtaacatgt caagtgcgtg
   540
   tgtgttattt atcttggatg anatgaggag gaagtcaaaa gaacacngnc t
   591
<210> 105
   <211> 590
   <212> DNA
   <213> Trifolium repens
<220>
   <221> misc_feature
   <222> (55) .. (55)
   <223> Any nucleotide
<400> 105
   ggttgttttg ctggtggcac ggtacttcgt ttggctaaag acttggctga aaacnacaaa 60
   ggtgcccgtg tgttggtggt ttgttcagag ataactgcag ttactttccg tggacccagt 120
   gacactcacc ttgatagcct tgtggggcaa gcattgtttg gagatggtgc agcagctgtg 180
   attgttggtt cagacccttt gccagaagtt gagaagcctt tgtttgaatt ggtatggacc 240
   gcacaaacaa tcgctccaga tagtgaagga gccattgatg gtcaccttcg cgaagcaggg 300
   ctgacattcc atctcctcaa ggatgttcct agccttgtct caaataacat tgagaaagcg 360
   cttgttgatg cctttcaacc tttgaatatt tctgactaca attccatctt ttggattgca 420
   cacccaggcg gaccagcaat tcttgaccaa gttgaagcta agttaggctt aaagccagag 480
   aaaatgcaag ccactcgaca tgtacttagc gaatatggta acatgtcaag tgcgtgtgtg 540
   ttatttatct tggatgagat gaggaggaag tcaaaagaag acggacttgc 590
<210> 106
   <211> 510
   <212> DNA
   <213> Trifolium repens
<220>
   <221> misc_feature
   <222> (4) .. (4)
   <223> Any nucleotide
<220>
   <221> misc_feature
   <222> (10) .. (10)
   <223> Any nucleotide
<220>
   <221> misc_feature
   <222> (12) .. (12)
   <223> Any nucleotide
<220>
   <221> misc_feature
   <222> (14) .. (14)
   <223> Any nucleotide
<220>
   <221> misc_feature
   <222> (397) .. (397)
   <223> Any nucleotide
<220>
   <221> misc_feature
   <222> (417) .. (417)
   <223> Any nucleotide
<220>
   <221> misc_feature
   <222> (437) .. (437)
   <223> Any nucleotide
<220>
   <221> misc_feature
   <222> (458) .. (458)
   <223> Any nucleotide
<220>
   <221> misc_feature
   <222> (496) .. (496)
   <223> Any nucleotide
<400> 106
   aatnacaccn tnanaccttc caattctcgt acctcaccaa tctcattttt attatatatc
   60
   ttggtacatc ttttgttacc tccaacaaaa aaatggtgac cgtagaagag attcgtaacg
   120
   cccaacgttc aaatggccct gccactatct tagcttttgg cacagccact ccttctaact
   180
   gtgtcactca agctgattat cctgattact actttcgtat caccaacagc gaacatatga
   240
   ctgatcttaa ggaaaaattc aagcggatgt gtgatagatc aatgataaag aaacgttaca
   300
   tgcacctaac agaagacttt ctgaaggaga atccaaatat gtgtgaatac atggcaccat
   360
   cactagatgt aagacgagac atagtggttg ttgaagnacc aaagctaggt aaagaancac
   420
   caaaaaaagc catatgngaa tggggacaac caaaatcnaa aatcacacat gcttggtttc
   480
   tgaccacttc cggtgntgac atgcccgggg
   510
<210> 107
   <211> 137
   <212> PRT
   <213> Trifolium repens
<220>
   <221> MISC_FEATURE
   <222> (102) .. (102)
   <223> Any amino acid
<220>
   <221> MISC_FEATURE
   <222> (114) .. (114)
   <223> Any amino acid
<220>
   <221> MISC_FEATURE
   <222> (121) .. (121)
   <223> Any amino acid
<400> 107
   Met Val Thr Val Glu Glu Ile Arg Asn Ala Gln Arg Ser Asn Gly Pro
   1               5                   10                  15
   Ala Thr Ile Leu Ala Phe Gly Thr Ala Thr Pro Ser Asn Cys Val Thr
               20                  25                  30
   Gln Ala Asp Tyr Pro Asp Tyr Tyr Phe Arg Ile Thr Asn Ser Glu His
           35                  40                  45
   Met Thr Asp Leu Lys Glu Lys Phe Lys Arg Met Cys Asp Arg Ser Met
       50                  55                  60
   Ile Lys Lys Arg Tyr Met His Leu Thr Glu Asp Phe Leu Lys Glu Asn
   65                  70                  75                  80
   Pro Asn Met Cys Glu Tyr Met Ala Pro Ser Leu Asp Val Arg Arg Asp
                   85                  90                  95
   Ile Val Val Val Glu Xaa Pro Lys Leu Gly Lys Glu Pro Lys Lys Ala
               100                 105                 110
   Ile Xaa Glu Trp Gly Gln Pro Lys Xaa Lys Ile Thr His Ala Trp Phe
           115                 120                 125
   Leu Thr Thr Ser Gly Asp Met Pro Gly
       130                 135
<210> 108
   <211> 240
   <212> DNA
   <213> Trifolium repens
<220>
   <221> misc_feature
   <222> (10) .. (10)
   <223> Any nucleotide
<220>
   <221> misc_feature
   <222> (129) .. (129)
   <223> Any nucleotide
<220>
   <221> misc_feature
   <222> (162) .. (162)
   <223> Any nucleotide
<220>
   <221> misc_feature
   <222> (184) .. (184)
   <223> Any nucleotide
<220>
   <221> misc_feature
   <222> (194) .. (194)
   <223> Any nucleotide
<220>
   <221> misc_feature
   <222> (217) .. (218)
   <223> Any nucleotides
<220>
   <221> misc_feature
   <222> (231) .. (231)
   <223> Any nucleotide
<400> 108
   gacaaatgcn tgtggttgga atgggatccg cacctgattt tacatgtaag aaagacacaa
   60
   aagatgcaat cgttgaagcc atcaaacaag gttatagaca ctttgatact gctgctgctt
   120
   atggctcana acaagctctt ggtgaaggtt tgaaagaagc anttgaactt ggtcttgtca
   180
   ctanagaaga gctntttgtt acttctaaac tttgggnnac tgaaaatcat nctaaccttg
   240
<210> 109
   <211> 79
   <212> PRT
   <213> Trifolium repens
<220>
   <221> MISC_FEATURE
   <222> (3) .. (3)
   <223> Any amino acid
<220>
   <221> MISC_FEATURE
   <222> (43) .. (43)
   <223> Any amino acid
<220>
   <221> MISC_FEATURE
   <222> (54) .. (54)
   <223> Any amino acid
<220>
   <221> MISC_FEATURE
   <222> (61) .. (61)
   <223> Any amino acid
<220>
   <221> MISC_FEATURE
   <222> (64) .. (64)
   <223> Any amino acid
<220>
   <221> MISC_FEATURE
   <222> (72) .. (72)
   <223> Any amino acid
<220>
   <221> MISC_FEATURE
   <222> (77) .. (77)
   <223> Any amino acid
<400> 109
   Gln Met Xaa Val Val Gly Met Gly Ser Ala Pro Asp Phe Thr Cys Lys
   1               5                   10                  15
   Lys Asp Thr Lys Asp Ala Ile Val Glu Ala Ile Lys Gln Gly Tyr Arg
               20                  25                  30
   His Phe Asp Thr Ala Ala Ala Tyr Gly Ser Xaa Gln Ala Leu Gly Glu
           35                  40                  45
   Gly Leu Lys Glu Ala Xaa Glu Leu Gly Leu Val Thr Xaa Glu Glu Xaa
       50                  55                  60
   Phe Val Thr Ser Lys Leu Trp Xaa Thr Glu Asn His Xaa Asn Leu
   65                  70                  75
<210> 110
   <211> 604
   <212> DNA
   <213> Trifolium repens
<220>
   <221> misc_feature
   <222> (13) .. (13)
   <223> Any nucleotide
<400> 110
   gtgtagcaga gtnagaaaaa gagagaaaaa aaaacatggc aggaaagaaa atcccagaag
   60
   tgttattgaa ttcaggacac aaaatgccag tcataggaat gggaacatca gtagacaatc
   120
   gtccatcaaa tgatgttctt gcttcaatct ttgttgatgc aattgaagtt ggttatcgcc
   180
   atttcgattc tgcttctgtg tatggaacag aggaagccat aggaattgct ttagcaaaag
   240
   ctttagaaaa agggcttatt aagagtagag atgaagtttt catcacttca aagccatgga
   300
   atactgatgc agattatgaa cttattgttc cagctctcaa gaccacattg aaaaagctgg
   360
   ggacggagta tgtggatctt tatctgatcc attggccagt gagacttaga catgatcttg
   420
   aaaaccctgt tgttttcacc aaagaagatt tacttccctt tgatatagaa gggacatgga
   480
   aagctatgga agaatgttat aagttaggct tagcaaagtc tattggtata tgcaattatg
   540
   gtaccaaaaa actcaccaaa ctcttggaaa cagccaccat tacccctgca gtcaatcagg
   600
   tgga
   604
<210> 111
   <211> 189
   <212> PRT
   <213> Trifolium repens
<400> 111
   Met Ala Gly Lys Lys Ile Pro Glu Val Leu Leu Asn Ser Gly His Lys
   1 5 10 15
   Met Pro Val Ile Gly Met Gly Thr Ser Val Asp Asn Arg Pro Ser Asn
   20 25 30
   Asp Val Leu Ala Ser Ile Phe Val Asp Ala Ile Glu Val Gly Tyr Arg
   35 40 45
   His Phe Asp Ser Ala Ser Val Tyr Gly Thr Glu Glu Ala Ile Gly Ile
   50 55 60
   Ala Leu Ala Lys Ala Leu Glu Lys Gly Leu Ile Lys Ser Arg Asp Glu
   65 70 75 80
   Val Phe Ile Thr Ser Lys Pro Trp Asn Thr Asp Ala Asp Tyr Glu Leu
   85 90 95
   Ile Val Pro Ala Leu Lys Thr Thr Leu Lys Lys Leu Gly Thr Glu Tyr
   100 105 110
   Val Asp Leu Tyr Leu Ile His Trp Pro Val Arg Leu Arg His Asp Leu
   115 120 125
   Glu Asn Pro Val Val Phe Thr Lys Glu Asp Leu Leu Pro Phe Asp Ile
   130 135 140
   Glu Gly Thr Trp Lys Ala Met Glu Glu Cys Tyr Lys Leu Gly Leu Ala
   145 150 155 160
   Lys Ser Ile Gly Ile Cys Asn Tyr Gly Thr Lys Lys Leu Thr Lys Leu
   165 170 175
   Leu Glu Thr Ala Thr Ile Thr Pro Ala Val Asn Gln Val
   180 185
<210> 112
   <211> 334
   <212> DNA
   <213> Trifolium repens
<220>
   <221> misc_feature
   <222> (10) .. (10)
   <223> Any nucleotide
<220>
   <221> misc_feature
   <222> (17) .. (17)
   <223> Any nucleotide
<220>
   <221> misc_feature
   <222> (21) .. (21)
   <223> Any nucleotide
<220>
   <221> misc_feature
   <222> (246) .. (246)
   <223> Any nucleotide
<220>
   <221> misc_feature
   <222> (268) .. (268)
   <223> Any nucleotide
<220>
   <221> misc_feature
   <222> (277) .. (277)
   <223> Any nucleotide
<220>
   <221> misc_feature
   <222> (281) .. (281)
   <223> Any nucleotide
<220>
   <221> misc_feature
   <222> (294) .. (294)
   <223> Any nucleotide
<220>
   <221> misc_feature
   <222> (328) .. (328)
   <223> Any nucleotide
<220>
   <221> misc_feature
   <222> (330) .. (330)
   <223> Any nucleotide
<220>
   <221> misc_feature
   <222> (334) .. (334)
   <223> Any nucleotide
<400> 112
   gtgtagcagn attaganaaa natataaaaa aaaacatggc aggaaagaaa atcccagaag
   60
   tgttattgaa ttcaggacac aaaatgccag tcataggaat gggaacatca gtagacaatc
   120
   gtccatcaaa tgatgttctt gcttcaatct ttgttgatgc aattgaagtt ggttatcgcc
   180
   atttcgattc tgcttctgtg tatggaacag aggaagccat aggaattgct ttagcaaaag
   240
   ctttanaaaa agggcttatt aagagtanag atgaagnttt natcacttgc aagncatgga
   300
   atactgatgc acattatgaa cttattgntn caan
   334
<210> 113
   <211> 602
   <212> DNA
   <213> Trifolium repens
<220>
   <221> misc_feature
   <222> (8) .. (8)
   <223> Any nucleotide
<220>
   <221> misc_feature
   <222> (14) .. (14)
   <223> Any nucleotide
<220>
   <221> misc_feature
   <222> (16) .. (16)
   <223> Any nucleotide
<220>
   <221> misc_feature
   <222> (20) .. (20)
   <223> Any nucleotide
<220>
   <221> misc_feature
   <222> (22) .. (22)
   <223> Any nucleotide
<400> 113
   gtagcagngt tagnanaagn gngaaaaaaa aacatggcag gaaagaaaat cccagaagtg 60
   ttattgaatt caggacacaa aatgccagtc ataggaatgg gaacatcagt agacaatcgt 120
   ccatcaaatg atgttcttgc ttcaatcttt gttgatgcaa ttgaagttgg ttatcgccat 180
   ttcgattctg cttctgtgta tggaacagag gaagccatag gaattgcttt agcaaaagct 240
   ttagaaaaag ggcttattaa gagtagagat gaagttttca tcacttcaaa gccatggaat 300
   actgatgcag attatgaact tattgttcca gctctcaaga ccacattgaa aaagctgggg 360
   acggagtatg tggatcttta tctgatccat tggccagtga gacttagaca tgatcttgaa 420
   aaccctgttg ttttcaccaa agaagattta cttccctttg atatagaagg gacatggaaa 480
   gctatggaag aatgttataa gttaggctta gcaaagtcta ttggtatatg caattatggt 540
   accaaaaaac tcaccaaact cttggaaaca gccaccatta cccctgcagt caatcaggtg 600
   ga 602

<210> 114
   <211> 584
   <212> DNA
   <213> Trifolium repens
<220>
   <221> misc-feature
   <222> (4) .. (5)
   <223> Any nucleotides
<220>
   <221> misc_feature
   <222> (27) .. (27)
   <223> Any nucleotide
<400> 114
   agtnngaaaa agagagaaaa aaaaacntgg caggaaagaa aatcccagaa gtgttattga 60
   attcaggaca caaaatgcca gtcataggaa tgggaacatc agtagacaat cgtccatcaa 120
   atgatgttct tgcttcaatc tttgttgatg caattgaagt tggttatcgc catttcgatt 180
   ctgcttctgt gtatggaaca gaggaagcca taggaattgc tttagcaaaa gctttagaaa 240
   aagggcttat taagagtaga gatgaagttt tcatcacttc aaagccatgg aatactgatg 300
   cagattatga acttattgtt ccagctctca agaccacatt gaaaaagctg gggacggagt 360
   atgtggatct ttatctgatc cattggccag tgagacttag acatgatctt gaaaaccctg 420
   ttgttttcac caaagaagat ttacttccct ttgatataga agggacatgg aaagctatgg 480
   aagaatgtta taagttaggc ttagcaaagt ctattggtat atgcaattat ggtaccaaaa 540
   aactcaccaa actcttggaa acagccacca ttacccctgc agtc 584
<210> 115
   <211> 547
   <212> DNA
   <213> Trifolium repens
<220>
   <221> misc_feature
   <222> (4) . . (5)
   <223> Any nucleotides
<220>
   <221> misc_feature
   <222> (27) .. (27)
   <223> Any nucleotide
<400> 115
   agtnngaaaa agagagaaaa aaaaacntgg caggaaagaa aatcccagaa gtgttattga 60
   attcaggaca caaaatgcca gtcataggaa tgggaacatc agtagacaat cgtccatcaa 120
   atgatgttct tgcttcaatc tttgttgatg caattgaagt tggttatcgc catttcgatt 180
   ctgcttctgt gtatggaaca gaggaagcca taggaattgc tttagcaaaa gctttagaaa 240
   aagggcttat taagagtaga gatgaagttt tcatcacttc aaagccatgg aatactgatg 300
   cagattatga acttattgtt ccagctctca agaccacatt gaaaaagctg gggacggagt 360
   atgtggatct ttatctgatc cattggccag tgagacttag acatgatctt gaaaaccctg 420
   ttgttttcac caaagaagat ttacttccct ttgatataga agggacatgg aaagctatgg 480
   aagaatgtta taagttaggc ttagcaaagt ctattggtat atgcaattat ggtaccaaaa 540
   aactcac 547
<210> 116
   <211> 334
   <212> DNA
   <213> Trifolium repens
<220>
   <221> misc_feature
   <222> (295) .. (295)
   <223> Any nucleotide
<400> 116
   acatggcagg aaagaaaatc ccagaagtgt tattgaattc aggacacaaa atgccagtca 60
   taggaatggg aacatcagta gacaatcgtc catcaaatga tgttcttgct tcaatctttg 120
   ttgatgcaat tgaagttggt tatcgtcatt tcgattctgc ttctgtttat ggaacagagg 180
   aagctatagg aattgcttta gcaaaagctt tagaaaaagg gcttattaag agtagagatg 240
   aagttttcat cacttcaaag ccatggaata ctgatgcaga ttatgatctt attgntccag 300
   ctctcaagac cacattgaaa aagctgggga caga 334
<210> 117
   <211> 694
   <212> DNA
   <213> Trifolium repens
<220>
   <221> misc_feature
   <222> (11) .. (11)
   <223> Any nucleotide
<220>
   <221> misc_feature
   <222> (23) .. (23)
   <223> Any nucleotide
<220>
   <221> misc_feature
   <222> (25) .. (25)
   <223> Any nucleotide
<220>
   <221> misc_feature
   <222> (32) .. (33)
   <223> Any nucleotides
<220>
   <221> misc_feature
   <222> (36) .. (36)
   <223> Any nucleotide
<220>
   <221> misc_feature
   <222> (43) .. (43)
   <223> Any nucleotide
<220>
   <221> misc_feature
   <222> (48) .. (49)
   <223> Any nucleotides
<220>
   <221> misc_feature
   <222> (62) .. (62)
   <223> Any nucleotide
<220>
   <221> misc_feature
   <222> (94) .. (96)
   <223> Any nucleotides
<220>
   <221> misc_feature
   <222> (101) .. (101)
   <223> Any nucleotide
<220>
   <221> misc_feature
   <222> (106) .. (106)
   <223> Any nucleotide
<400> 117
   taagaatgaa ncaattttat ctnanaaaag gnncangcaa gtnagttnna ttcaaacata 60
   gncttaaagt gtgtaacata ttcttaactt aaannntttt nacccnacaa aaaaaaacaa 120
   agacaataac atgggtagtg ttgaaattcc aacaaaggtt cttactaaca cttctagtca 180
   agtgaaaatg cctgtggttg gaatgggatc agctcctgat ttcacatgta agaaagatac 240
   aaaagatgca atcattgaag ccatcaaaca aggttataga cactttgata ctgctgctgc 300
   ttatggctca gaacaagctc ttggtgaagg tttgaaagaa gcaattgaac ttggccttgt 360
   cactagagaa gagctttttg ttacttctaa actttgggtc actgaaaatc atcctcacct 420
   tgttgttcct gctcttcaaa aatctctcaa gactcttcaa ttggagtact tggacttgta 480
   tttgatccat tggccactta gttctcagcc tggaaagttt tcatttccaa ttgatgtggc 540
   agatctcttg ccatttgatg tgaagggtgt ttgggaatcc atggaagaag gcttgaaact 600
   tggactcact aaagctattg gtgttagtaa cttctctgtc aagaaacttc aaaatcttgt 660
   ctcagttgcc accgttcttc ctgcggtcaa tcag 694
<210> 118
   <211> 188
   <212> PRT
   <213> Trifolium repens
<400> 118
   Met Gly Ser Val Glu Ile Pro Thr Lys Val Leu Thr Asn Thr Ser Ser
   1 5 10 15
   Gln Val Lys Met Pro Val Val Gly Met Gly Ser Ala Pro Asp Phe Thr
   20 25 30
   Cys Lys Lys Asp Thr Lys Asp Ala Ile Ile Glu Ala Ile Lys Gln Gly
   35 40 45
   Tyr Arg His Phe Asp Thr Ala Ala Ala Tyr Gly Ser Glu Gln Ala Leu
   50 55 60
   Gly Glu Gly Leu Lys Glu Ala Ile Glu Leu Gly Leu Val Thr Arg Glu
   65 70 75 80
   Glu Leu Phe Val Thr Ser Lys Leu Trp Val Thr Glu Asn His Pro His
   85 90 95
   Leu Val Val Pro Ala Leu Gln Lys Ser Leu Lys Thr Leu Gln Leu Glu
   100 105 110
   Tyr Leu Asp Leu Tyr Leu Ile His Trp Pro Leu Ser Ser Gln Pro Gly
   115 120 125
   Lys Phe Ser Phe Pro Ile Asp Val Ala Asp Leu Leu Pro Phe Asp Val
   130 135 140
   Lys Gly Val Trp Glu Ser Met Glu Glu Gly Leu Lys Leu Gly Leu Thr
   145 150 155 160
   Lys Ala Ile Gly Val Ser Asn Phe Ser Val Lys Lys Leu Gln Asn Leu
   165 170 175
   Val Ser Val Ala Thr Val Leu Pro Ala Val Asn Gln
   180 185
<210> 119
   <211> 576
   <212> DNA
   <213> Trifolium repens
<220>
   <221> misc_feature
   <222> (11) .. (11)
   <223> Any nucleotide
<220>
   <221> misc_feature
   <222> (33) .. (33)
   <223> Any nucleotide
<220>
   <221> misc_feature
   <222> (36) .. (36)
   <223> Any nucleotide
<220>
   <221> misc_feature
   <222> (48) .. (48)
   <223> Any nucleotide
<220>
   <221> misc_feature
   <222> (62) .. (62)
   <223> Any nucleotide
<220>
   <221> misc_feature
   <222> (65) .. (65)
   <223> Any nucleotide
<220>
   <221> misc_feature
   <222> (95) .. (95)
   <223> Any nucleotide
<400> 119
   taagaatgaa ncaattttat cttataaaag gtncangcaa gttagttnct attaaacata
   60
   gnctnaaagt gtgtaacata ttcttaactt aaacnatttt cacccaacaa aaaaaaacaa
   120
   agacaataac atgggtagtg ttgaaattcc aacaaaggtt cttactaaca cttctagtca
   180
   agtgaaaatg cctgtggttg gaatgggatc agctcctgat ttcacatgta agaaagatac
   240
   aaaagatgca atcattgaag ccatcaaaca aggttataga cactttgata ctgctgctgc
   300
   ttatggctca gaacaagctc ttggtgaagg tttgaaagaa gcaattgaac ttggtcttgt
   360
   cactagagaa gagctttttg ttgcttctaa actttgggtc actgaaaatc atcctcatct
   420
   tgttgttcct gctcttcaaa aatctctcaa gactcttcaa ttggagtact tggacttgta
   480
   tttgattcac tggccactta gttctcagcc cggaaagttt tcatttccaa ttgaggtggc
   540
   agatctcttg ccatttgatg tgaagggtgt ttgggg
   576
<210> 120
   <211> 524
   <212> DNA
   <213> Trifolium repens
<220>
   <221> misc_feature
   <222> (4) .. (6)
   <223> Any nucleotides
<220>
   <221> misc_feature
   <222> (13) .. (14)
   <223> Any nucleotides
<220>
   <221> misc_feature
   <222> (17) .. (17)
   <223> Any nucleotide
<220>
   <221> misc_feature
   <222> (24) .. (24)
   <223> Any nucleotide
<220>
   <221> misc_feature
   <222> (29) .. (30)
   <223> Any nucleotides
<220>
   <221> misc_feature
   <222> (33) .. (33)
   <223> Any nucleotide
<220>
   <221> misc_feature
   <222> (36) .. (36)
   <223> Any nucleotide
<220>
   <221> misc_feature
   <222> (43) .. (43)
   <223> Any nucleotide
<220>
   <221> misc_feature
   <222> (46) .. (46)
   <223> Any nucleotide
<220>
   <221> misc_feature
   <222> (55) .. (55)
   <223> Any nucleotide
<220>
   <221> misc_feature
   <222> (76) .. (76)
   <223> Any nucleotide
<220>
   <221> misc_feature
   <222> (524) .. (524)
   <223> Any nucleotide
<400> 120
   tctnnnaaaa ggnncangca agtnagttnn tanttnaact agnctnaaag tgtgnaacat 60
   attcttaact taaacnattt tcacccaaca aaaaaaaaca aagacaataa catgggtagt 120
   gttgaaattc caacaaaggt tcttactaac acttctagtc aagtgaaaat gcctgtggtt 180
   ggaatgggat cagctcctga tttcacatgt aagaaagata caaaagatgc aatcattgaa 240
   gccatcaaac aaggttatag acactttgat actgctgctg cttatggctc agaacaagct 300
   cttggtgaag gtttgaaaga agcaattgaa cttggtcttg tcactagaga agagcttttt 360
   gttgcttcta aactttgggt cactgaaaat catcctcatc ttgttgttcc tgctcttcaa 420
   aaatctctca agactcttca attggagtac ttggacttgt atttgattca ctggccactt 480
   agttctcagc ccggaaagtt ttcatttcca attgaggtgg cagn 524
<210> 121
   <211> 577
   <212> DNA
   <213> Trifolium repens
<220>
   <221> misc_feature
   <222> (2) .. (2)
   <223> Any nucleotide
<220>
   <221> misc_feature
   <222> (15) .. (15)
   <223> Any nucleotide
<220>
   <221> misc_feature
   <222> (18) .. (18)
   <223> Any nucleotide
<220>
   <221> misc_feature
   <222> (54) .. (54)
   <223> Any nucleotide
<400> 121
   gnattcaaac atagnctnaa agtgtgtaac atattcttaa cttaaaacat tttnacccaa 60
   caaaaaaaaa caaagacaac aacatgggta gtgttgaaat tccaacaaag gttcttacta 120
   acacttctag tcaagtgaaa atgcctgtgg ttggaatggg atcagctcct gatttcacat 180
   gtaagaaaga tacaaaagat gcaatcattg aagccatcaa acaaggttat agacactttg 240
   atattgctgc tgcttatggc tcagaacaag ctcttggtga aggtttgaaa gaagcaattg 300
   aacttggtct tgtcactaga gaagaccttt ttgttacttc taaactttgg gtcactgaaa 360
   atcatcctca ccttgttatt cctgctcttc aaaaatctct caagactctt caattggagt 420
   acttggactt gtatttgatc cactggccac ttagttctca gcccggaaag ttttcatttc 480
   caattgaggt ggcagatctc ttgccatttg atgtgagggg tgtttggcaa tccatggaag 540
   aaggcttgaa acttggactc actaaagcta ttggtgt 577
<210> 122
   <211> 597
   <212> DNA
   <213> Trifolium repens
<220>
   <221> misc_feature
   <222> (13) .. (13)
   <223> Any nucleotide
<220>
   <221> misc_feature
   <222> (16) .. (16)
   <223> Any nucleotide
<220>
   <221> misc_feature
   <222> (29) .. (29)
   <223> Any nucleotide
<220>
   <221> misc_feature
   <222> (47) .. (47)
   <223> Any nucleotide
<220>
   <221> misc_feature
   <222> (52) .. (52)
   <223> Any nucleotide
<220>
   <221> misc_feature
   <222> (57) .. (57)
   <223> Any nucleotide
<220>
   <221> misc_feature
   <222> (597) .. (597)
   <223> Any nucleotide
<400> 122
   attcaaacat agnctnaaag tgtgtaacnt attcttaact taaaacnttt tnacccnaca 60
   aaaaaaaaca aagacaacaa catgggtagt gttgaaattc caacaaaggt tcttactaac 120
   acttctagtc aagtgaaaat gcctgtggtt ggaatgggat cagctcctga tttcacatgt 180
   aagaaagata caaaagatgc aatcattgaa gccatcaaac aaggttatag acactttgat 240
   attgctgctg cttatggctc agaacaagct cttggtgaag gtttgaaaga agcaattgaa 300
   cttggtcttg tcactagaga agaccttttt gttacttcta aactttgggt cactgaaaat 360
   catcctcacc ttgttattcc tgctcttcaa aaatctctca agactcttca attggagtac 420
   ttggacttgt atttgatcca ctggccactt agttctcagc ccggaaagtt ttcatttcca 480
   attgaggtgg cagatctctt gccatttgat gtgaggggtg tttggcaatc catggaagaa 540
   ggcttgaaac ttggactcac taaagctatt ggtgtcagta acttctctgt caagaan 597
<210> 123
   <211> 293
   <212> DNA
   <213> Trifolium repens
<220>
   <221> misc_feature
   <222> (17) .. (17)
   <223> Any nucleotide
<220>
   <221> misc_feature
   <222> (51) .. (51)
   <223> Any nucleotide
<220>
   <221> misc_feature
   <222> (59) .. (59)
   <223> Any nucleotide
<220>
   <221> misc_feature
   <222> (224) .. (224)
   <223> Any nucleotide
<220>
   <221> misc_feature
   <222> (250) .. (250)
   <223> Any nucleotide
<220>
   <221> misc_feature
   <222> (257) .. (257)
   <223> Any nucleotide

<220>
   <221> misc_feature
   <222> (274) .. (277)
   <223> Any nucleotides
<220>
   <221> misc_feature
   <222> (279) .. (280)
   <223> Any nucleotides
<220>
   <221> misc feature
   <222> (285) .. (285)
   <223> Any nucleotide
<220>
   <221> misc_feature
   <222> (293) .. (293)
   <223> Any nucleotide
<400> 123
   ttcaaacata gcctccnagt gtgtaacata ttcttaactt aaaacatttt nacccaacna
   60
   taaaaaacaa agacaacaac atgggtagtg ttgaaattcc aacaaaggtt cttactaaca
   120
   gttctagtca agtgaaaatg cctgtggttg gaatgggatc agctcctgac ttcacatgta
   180
   agaaagacac aaaagatgca atcattgaag ccatcaaaca aggntataga cactttgaaa
   240
   ctgctgctgn ttatgcntca caacaagctc ttgnnnnann cttcnatgcc ccn
   293
<210> 124
   <211> 636
   <212> DNA
   <213> Trifolium repens
<220>
   <221> misc_feature
   <222> (4) .. (4)
   <223> Any nucleotide
<220>
   <221> misc_feature
   <222> (12) .. (12)
   <223> Any nucleotide
<220>
   <221> misc_feature
   <222> (86) .. (86)
   <223> Any nucleotide
<220>
   <221> misc_feature
   <222> (244) .. (244)
   <223> Any nucleotide
<220>
   <221> misc_feature
   <222> (261) .. (261)
   <223> Any nucleotide
<220>
   <221> misc_feature
   <222> (458) .. (458)
   <223> Any nucleotide
<220>
   <221> misc_feature
   <222> (485) .. (485)
   <223> Any nucleotide
<220>
   <221> misc_feature
   <222> (507) .. (507)
   <223> Any nucleotide
<220>
   <221> misc_feature
   <222> (515) .. (515)
   <223> Any nucleotide
<220>
   <221> misc_feature
   <222> (540) .. (540)
   <223> Any nucleotide
<220>
   <221> misc_feature
   <222> (545) .. (545)
   <223> Any nucleotide
<220>
   <221> misc_feature
   <222> (574) .. (574)
   <223> Any nucleotide
<220>
   <221> misc_feature
   <222> (577) .. (578)
   <223> Any nucleotides
<220>
   <221> misc_feature
   <222> (588) .. (588)
   <223> Any nucleotide
<220>
   <221> misc_feature
   <222> (593) .. (593)
   <223> Any nucleotide
<220>
   <221> misc_feature
   <222> (609) .. (609)
   <223> Any nucleotide
<220>
   <221> misc_feature
   <222> (630) .. (630)
   <223> Any nucleotide
<400> 124
   ttcnaacata gnctcaaagt gtgtaacata ttcttaactt aaaacatttt cacccaacaa 60
   aaaaaaacaa agacaacaac atgggnaggg ttgaaattcc aacaaaggtt cttactaaca 120
   cttctagtca agtgaaaatg cctgtggttg gaatgggatc agctcctgat tttacatgta 180
   aaaaagacac aaaagatgca atcattgaag ccatcaaaca aggttataga cactttgata 240
   ctgntgctgc ttatggctca naacaagctc ttggtgaagg tttgaaagaa gcaattgaac 300
   ttggccttgt cactagaaaa gagctttttg ttacttctaa actttgggtc actgaaaatc 360
   atcctcacct tgttgttcct gctcttcaaa aatctctcaa gactcttcaa ttggagtact 420
   tggacttgta tttgatccat tggccactta attctcancc tggaaagttt tcatttccca 480
   ttgangtggc aaatctcttg ccatttnatg tgaanggtgt ttgggaatcc atggaaaaan 540
   gcttnaaact tggactcact aaagctattg gtgncannaa cttctctntc aanaaacttc 600
   aaaatcttnt ctcaattgcc accgttcttn ctgcgg 636
<210> 125
   <211> 570
   <212> DNA
   <213> Trifolium repens
<220>
   <221> misc_feature
   <222> (8) .. (8)
   <223> Any nucleotide
<220>
   <221> misc_feature
   <222> (11) .. (11)
   <223> Any nucleotide
<220>
   <221> misc_feature
   <222> (21) .. (21)
   <223> Any nucleotide
<220>
   <221> misc_feature
   <222> (23) .. (23)
   <223> Any nucleotide
<220>
   <221> misc_feature
   <222> (25) .. (25)
   <223> Any nucleotide
<220>
   <221> misc_feature
   <222> (41) .. (42)
   <223> Any nucleotides
<220>
   <221> misc_feature
   <222> (55) .. (55)
   <223> Any nucleotide
<400> 125
   aacatagnct naaagtgtgt nananattct taacttaaaa nnttttcacc caacnaaaaa
   60
   aaacaaagac aaaaacatgg gtagtgttga aattccaaca aaggttctta ctaacacttc
   120
   tagtcaagtg aaaatgcctg tggttggaat gggatcagca cctgatttca catgtaagaa
   180
   agacacaaaa gatgcaatca ttgaagccat caaacaaggt tatagacact ttgatactgc
   240
   tgctgcttat ggctcagaac aagctcttgg tggaggtttg aaagaagcaa ttgaacttgg
   300
   ccttgtcact agagaagagc tttttgttac ttctaaactt tgggtcactg aaaatcatcc
   360
   tcaccttgtt gttcctgctc ttcaaaaatc tctcaagact cttcaattgg agtacttgga
   420
   cttgtatttg atccattggc cacttagttc tcagcctgga aagttttcat ttccaattga
   480
   tgtggcagat ctcttgccat ttgatgtgaa gggtgtttgg caatccatgg aagaaggctt
   540
   gaaacttgga ctcactaaag ctattggtgt
   570
<210> 126
   <211> 621
   <212> DNA
   <213> Trifolium repens
<220>
   <221> misc_feature
   <222> (5) .. (5)
   <223> Any nucleotide
<220>
   <221> misc_feature
   <222> (9) .. (9)
   <223> Any nucleotide
<220>
   <221> misc_feature
   <222> (17) .. (18)
   <223> Any nucleotides
<220>
   <221> misc_feature
   <222> (21) .. (22)
   <223> Any nucleotides
<220>
   <221> misc_feature
   <222> (27) .. (27)
   <223> Any nucleotide
<220>
   <221> misc_feature
   <222> (32) .. (32)
   <223> Any nucleotide
<220>
   <221> misc_feature
   <222> (38) .. (38)
   <223> Any nucleotide
<220>
   <221> misc_feature
   <222> (44) .. (44)
   <223> Any nucleotide
<220>
   <221> misc_feature
   <222> (49) .. (51)
   <223> Any nucleotides
<220>
   <221> misc_feature
   <222> (62) .. (62)
   <223> Any nucleotide
<400> 126
   acatngctna agtgtgnnaa nnattcntaa cntaaacnat tttnacccnn nacaaaaaaa
   60
   cnaagacaat aacatgggta gtgttgaaat tccaacaaag gttcttacta acacttctag 120
   tcaagtgaaa atgcctgtgg ttggaatggg atcagctcct gatttcacat gtaagaaaga 180
   tacaaaagat gcaatcattg aagccatcaa acaaggttat agacactttg atactgctgc 240
   tgcttatggc tcagaacaag ctcttggtga aggtttgaaa gaagcaattg aacttggcct 300
   tgtcactaga gaagagcttt ttgttacttc taaactttgg gtcactgaaa atcatcctca 360
   tcttgttgtt cctgctcttc aaaaatctct caagactctt caattggagt acttggactt 420
   gtatttgatc cattggccac ttagttctca gcctggaaag ttttcatttc caattgatgt 480
   ggcagatctc ttgccatttg atgtgaaggg tgtttgggaa tccatggaag aaggcttgaa 540
   acttggactc actaaagcta ttggtgttag taacttctct gtcaagaaac ttcaaaatct 600
   tgtctcagtt gccaccgttc t 621
<210> 127
   <211> 573
   <212> DNA
   <213> Trifolium repens
<220>
   <221> misc_feature
   <222> (8) .. (8)
   <223> Any nucleotide
<220>
   <221> misc_feature
   <222> (16) .. (16)
   <223> Any nucleotide
<220>
   <221> misc_feature
   <222> (19) .. (19)
   <223> Any nucleotide
<220>
   <221> misc_feature
   <222> (36) .. (38)
   <223> Any nucleotides
<220>
   <221> misc_feature
   <222> (43) .. (43)
   <223> Any nucleotide
<220>
   <221> misc_feature
   <222> (48) .. (48)
   <223> Any nucleotide
<220>
   <221> misc_feature
   <222> (61) .. (61)
   <223> Any nucleotide
<220>
   <221> misc_feature
   <222> (573) .. (573)
   <223> Any nucleotide
<400> 127
   catagctnaa gtgtgnaana atttcttaac taaaannntt ttnacccnac aaaaaaaaac
   60
   naagacaaaa acatgggtag tgttgaaatt ccaacaaagg ttcttactaa cagttctagt
   120
   caagtgaaaa tgcctgtggt tggaatggga tcagcacctg atttcacatg taagaaagac
   180
   acaaaagatg caatcattga agccatcaaa caaggttata gacactttga tactgctgct
   240
   gcttatggct cagaacaagc tcttggtgaa ggtttgaaag aagcaattga acttggcctt
   300
   gtcactagag aagagctttt tgttacttct aaactttggg tcactgaaaa tcatcctcac
   360
   cttgttgttc ctgctcttca aaaatctctc aagactcttc aattggagta cttggacttg
   420
   tatttgatcc attggccact tagttctcag cctggaaagt tttcatttcc aattgatgtg
   480
   gcagatctct tgccatttga tgtgaagggt gtttgggaat ccatggaaga aggcttgaaa
   540
   cttggactca ctaaagctat tggtgttagt aan
   573
<210> 128
   <211> 583
   <212> DNA
   <213> Trifolium repens
<220>
   <221> misc_feature
   <222> (4) .. (4)
   <223> Any nucleotide
<220>
   <221> misc_feature
   <222> (16) .. (17)
   <223> Any nucleotides
<220>
   <221> misc_feature
   <222> (19) .. (20)
   <223> Any nucleotides
<220>
   <221> misc_feature
   <222> (26) .. (26)
   <223> Any nucleotide
<220>
   <221> misc_feature
   <222> (31) .. (31)
   <223> Any nucleotide
<220>
   <221> misc_feature
   <222> (36) .. (38)
   <223> Any nucleotides
<220>
   <221> misc_feature
   <222> (43) .. (43)
   <223> Any nucleotide
<220>
   <221> misc_feature
   <222> (48) .. (48)
   <223> Any nucleotide
<220>
   <221> misc_feature
   <222> (61) .. (61)
   <223> Any nucleotide
<220>
   <221> misc_feature
   <222> (79) .. (79)
   <223> Any nucleotide
<220>
   <221> misc_feature
   <222> (81) .. (81)
   <223> Any nucleotide
<220>
   <221> misc_feature
   <222> (83) .. (84)
   <223> Any nucleotides
<220>
   <221> misc_feature
   <222> (239) .. (239)
   <223> Any nucleotides
<400> 128
   catngctaaa gtgtgnnann tattcntaac ntaaannntt ttnacccnac aaaaaaaaac 60
   naagacaata acatgggtng ngnngaaatt ccaacaaagg ttcttactaa cacttctagt 120
   caagtgaaaa tgcctgtggt tggaatggga tcagctcctg atttcacatg taagaaagat 180
   acaaaagatg caatcattga agccatcaaa caaggttata gacactttga tactgctgnt 240
   gcttatggct cagaacaagc tcttggtgaa ggtttgaaag aagcaattga acttggcctt 300
   gtcactagag aagagctttt tgttacttct aaactttggg tcactgaaaa tcatcctcac 360
   cttgttattc ctgctcttca aaaatctctc aagactcttc aattggagta cttggacttg 420
   tatttgatcc attggccact tagttctcag cctggaaagt tttcatttcc aattgatgtg 480
   gcagatctct tgccatttga tgtgaagggt gtttgggaat ccatggaaga aggcttgaaa 540
   cttggactca ctaaagctat tggtgttagt aacttctctg tcc 583
<210> 129
   <211> 597
   <212> DNA
   <213> Trifolium repens
<220>
   <221> misc_feature
   <222> (4) .. (4)
   <223> Any nucleotide
<220>
   <221> misc_feature
   <222> (8) .. (8)
   <223> Any nucleotide
<220>
   <221> misc_feature
   <222> (16) .. (16)
   <223> Any nucleotide
<220>
   <221> misc_feature
   <222> (18) .. (18)
   <223> Any nucleotide
<220>
   <221> misc_feature
   <222> (21) .. (21)
   <223> Any nucleotide
<220>
   <221> misc_feature
   <222> (36) .. (38)
   <223> Any nucleotides
<220>
   <221> misc_feature
   <222> (48) .. (48)
   <223> Any nucleotides
<220>
   <221> misc_feature
   <222> (61) .. (61)
   <223> Any nucleotides
<220>
   <221> misc_feature
   <222> (67) .. (67)
   <223> Any nucleotides
<400> 129
   catngctnaa gtgtgnanca natttctaac taaaannntt ttcacccnac aaaaaaaaac
   60
   naagacnaaa acatgggtag tgttgaaatt ccaacaaagg ttcttactaa cagttctagt
   120
   caagtgaaaa tgcctgtggt tggaatggga tcagcacctg atttcacatg taagaaagac
   180
   acaaaagatg caatcattga agccatcaaa cagggttata gacactttga tactgctgct
   240
   gcttatggct cagaacaagc tcttggtgaa ggtttgaaag aagcaattga acttggtctt
   300
   gtcactagag aagacctttt tgttacttct aaactttggg tcactgaaaa tcatcctcat
   360
   cttgttgttc ctgctcttca aaaatctctc aagactcttc aattggagta cttggacttg
   420
   tatttgatcc attggccact tagttctcag cctggaaagt tttcatttcc aattgatgtg
   480
   gcagatctct tgccatttga tgtgaagggt gtttgggaat ccatggaaga aggcttgaaa
   540
   cttggactca ctaaagctat tggtgttagt aacttctctg tcaagaaact tcaaaat
   597
<210> 130
   <211> 597
   <212> DNA
   <213> Trifolium repens
<220>
   <221> misc_feature
   <222> (16) .. (17)
   <223> Any nucleotides

<220>
   <221> misc_feature
   <222> (19) .. (19)
   <223> Any nucleotide
<220>
   <221> misc_feature
   <222> (28) .. (28)
   <223> Any nucleotide
<220>
   <221> misc_feature
   <222> (36) .. (38)
   <223> Any nucleotides
<220>
   <221> misc_feature
   <222> (48) .. (48)
   <223> Any nucleotide
<220>
   <221> misc_feature
   <222> (55) .. (55)
   <223> Any nucleotide
<220>
   <221> misc_feature
   <222> (61) .. (61)
   <223> Any nucleotide
<400> 130
   catagctaaa gtgtgnnana atttcttnac taaaannntt ttcacccnac aaaanaaaac 60
   naagacaaaa acatgggtag tgttgaaatt ccaacaaagg ttcttactaa cacttctagt 120
   caagtgaaaa tgcctgtggt tggaatggga tcagcacctg atttcacatg taagaaagac 180
   acaaaagatg caatcattga agccatcaaa caaggttata gacactttga tactgctgct 240
   gcttatggct cagaacaagc tcttggtgga ggtttgaaag aagcaattga acttggcctt 300
   gtcactagag aagagctttt tgttacttct aaactttggg tcactgaaaa tcatcctcac 360
   cttgttgttc ctgctcttca aaaatctctc aagactcttc aattggagta cttggacttg 420
   tatttgatcc attggccact tagttctcag cctggaaagt tttcatttcc aattgatgtg 480
   gcagatctct tgccatttga tgtgaagggt gtttggcaat ccatggaaga aggcttgaaa 540
   cttggactca ctaaagctat tggtgttagt aacttctctg tcaagaaact tcaaaat 597
<210> 131
   <211> 574
   <212> DNA
   <213> Trifolium repens
<220>
   <221> misc_feature
   <222> (2) .. (2)
   <223> Any nucleotide
<220>
   <221> misc_feature
   <222> (26) .. (28)
   <223> Any nucleotides
<220>
   <221> misc_feature
   <222> (33) .. (33)
   <223> Any nucleotide
<220>
   <221> misc_feature
   <222> (38) .. (38)
   <223> Any nucleotide
<220>
   <221> misc_feature
   <222> (51) .. (51)
   <223> Any nucleotide
<220>
   <221> misc_feature
   <222> (57) .. (57)
   <223> Any nucleotide
<220>
   <221> misc_feature
   <222> (83) .. (83)
   <223> Any nucleotide
<220>
   <221> misc_feature
   <222> (519) .. (519)
   <223> Any nucleotide
<220>
   <221> misc_feature
   <222> (540) .. (540)
   <223> Any nucleotide
<220>
   <221> misc_feature
   <222> (559) .. (561)
   <223> Any nucleotides
<220>
   <221> misc_feature
   <222> (568) .. (568)
   <223> Any nucleotide
<220>
   <221> misc_feature
   <222> (572) .. (572)
   <223> Any nucleotide
<220>
   <221> misc_feature
   <222> (574) .. (574)
   <223> Any nucleotide
<400> 131
   gngtgtaaca tattcttaac ttaaannntt ttnacccnac aaaaaaaaac naagacnata 60
   acatgggtag tgttgaaatt ccnacaaagg ttcttactaa cacttctagt caagtgaaaa 120
   tgcctgtggt tggaatggga tcagctcctg atttcacatg taagaaagat acaaaagatg 180
   caatcattga agccatcaaa caaggttata gacactttga tactgctgct gcttatggct 240
   cagaacaagc tcttggtgaa ggtttgaaag aagcaattga acttggcctt gtcactagag 300
   aagagctttt tgttacttct aaactttggg tcactgaaaa tcatcctcac cttgttgttc 360
   ctgctcttca aaaatctctc aagactcttc aattggagta cttggacttg tatttgatcc 420
   attggccact tagttctcag cctggaaagt tttcatttcc aattgatgtg gcagatctct 480
   tgccatttga tgtgaagggt gtttgggaat ccatggaana aggcttgaaa cttggactcn 540
   ctaaagctat tggtgttann nacttctntg tnan 574
<210> 132
   <211> 578
   <212> DNA
   <213> Trifolium repens
<220>
   <221> misc_feature
   <222> (2) .. (2)
   <223> Any nucleotide
<220>
   <221> misc_feature
   <222> (14) .. (14)
   <223> Any nucleotide
<220>
   <221> misc_feature
   <222> (26) .. (27)
   <223> Any nucleotides
<220>
   <221> misc_feature
   <222> (33) .. (33)
   <223> Any nucleotide
<220>
   <221> misc_feature
   <222> (37) .. (37)
   <223> Any nucleotide
<220>
   <221> misc_feature
   <222> (578) .. (578)
   <223> Any nucleotide
<400> 132
   gngtgtaaca tatncttaac ttaaannatt ttnaccnaac aaaaaaaaac aaagacaata
   60
   acatgggtag tgttgaaatt ccaacaaagg ttcttactaa cacttctagt caagtgaaaa
   120
   tgcctgtggt tggaatggga tcagctcctg atttcacatg taagaaagat acaaaagatg
   180
   caatcattga agccatcaaa caaggttata gacactttga tactgctgct gcttatggct
   240
   cagaacaagc tcttggtgaa ggtttgaaag aagcaattga acttggcctt gtcactagag
   300
   aagagctttt tgttacttct aaactttggg tcactgaaaa tcatcctcac cttgttgttc
   360
   ctgctcttca aaaatctctc aagactcttc aattggagta cttggacttg tatttgatcc
   420
   attggccact tagttctcag cctggaaagt tttcatttcc aattgatgtg gcagatctct
   480
   tgccatttga tgtgaagggt gtttgggaat ccatggaaga aggcttgaaa cttggactca
   540
   ctaaagctat tggtgttagt aacttctctg tcaagaan
   578
<210> 133
   <211> 600
   <212> DNA
   <213> Trifolium repens
<220>
   <221> misc_feature
   <222> (6) .. (6)
   <223> Any nucleotide
<220>
   <221> misc_feature
   <222> (8) .. (12)
   <223> Any nucleotides
<220>
   <221> misc_feature
   <222> (26) .. (28)
   <223> Any nucleotides
<220>
   <221> misc_feature
   <222> (33) .. (33)
   <223> Any nucleotide
<220>
   <221> misc_feature
   <222> (37) .. (38)
   <223> Any nucleotides
<220>
   <221> misc_feature
   <222> (48) .. (48)
   <223> Any nucleotide
<220>
   <221> misc_feature
   <222> (51) .. (51)
   <223> Any nucleotide
<220>
   <221> misc_feature
   <222> (57) .. (59)
   <223> Any nucleotides
<400> 133
   gtgtgnannn nntttctaac taaaannntt ttnaccnnac aaaaaatnaa ngacacnnna 60
   acatgggtag tgttgaaatt ccaacaaagg ttcttactaa cagttctagt caagtgaaaa 120
   tgcctgtggt tggaatggga tcagcacctg atttcacatg taagaaagac acaaaagatg 180
   caatcattga agccatcaaa caaggttata gacactttga tactgctgct gcttatggct 240
   cagaacaagc tcttggtgaa ggtttgaaag aagcaattga acttggcctt gtcactagag 300
   aagagctttt tgttacttct aaactttggg tcactgaaaa tcatcctcac cttgttgttc 360
   ctgctcttca aaaatctctc aagactcttc aattggagta cttggacttg tatttgatcc 420
   attggccact tagttctcag cccgaaaagt tttcatttcc aattgatgtg gcagatctct 480
   tgccatttga tgtgaagggt gtttgggaat ccatggaaga aggcttgaaa cttggactca 540
   ctaaagctat tggtgtcagt aacttctctg tcaagaaact tcaaaatctt gtctcagttg 600
<210> 134
   <211> 612
   <212> DNA
   <213> Trifolium repens
<220>
   <221> misc_feature
   <222> (1) .. (1)
   <223> Any nucleotide
<220>
   <221> misc_feature
   <222> (6) .. (6)
   <223> Any nucleotide
<220>
   <221> misc_feature
   <222> (13) .. (14)
   <223> Any nucleotides
<220>
   <221> misc_feature
   <222> (19) .. (19)
   <223> Any nucleotide
<220>
   <221> misc_feature
   <222> (23) .. (24)
   <223> Any nucleotides
<220>
   <221> misc_feature
   <222> (27) .. (27)
   <223> Any nucleotide
<220>
   <221> misc_feature
   <222> (37) .. (37)
   <223> Any nucleotide
<220>
   <221> misc_feature
   <222> (43) .. (43)
   <223> Any nucleotide
<220>
   <221> misc_feature
   <222> (46) .. (46)
   <223> Any nucleotide
<220>
   <221> misc_feature
   <222> (69) .. (69)
   <223> Any nucleotide
<400> 134
   nttctnacta aannttttna ccnnacnaaa aaaaacnaag acnatnacat gggtagtgtt
   60
   gaaattccna caaaggttct tactaacact tctagtcaag tgaaaatgcc tgtggttgga
   120
   atgggatcag ctcctgattt cacatgtaag aaagatacaa aagatgcaat cattgaagcc
   180
   atcaaacaag gttatagaca ctttgatact gctgctgctt atggctcaga acaagctctt
   240
   ggtgaaggtt tgaaagaagc aattgaactt ggccttgtca ctagagaaga gctttttgtt
   300
   acttctaaac tttgggtcac tgaaaatcat cctcaccttg ttgttcctgc tcttcaaaaa
   360
   tctctcaaga ctcttcaatt ggagtacttg gacttgtatt tgatccattg gccacttagt
   420
   tctcagcctg gaaagttttc atttccaatt gatgtggcag atctcttgcc atttgatgtg
   480
   aagggtgttt gggaatccat ggaagaaggc ttgaaacttg gactcactaa agctattggt
   540
   gttagtaact tctctgtcaa gaaacttcaa aatcttgtct cagttgccac cgttcttcct
   600
   gcggtcaatc ag
   612
<210> 135
   <211> 610
   <212> DNA
   <213> Trifolium repens
<220>
   <221> misc_feature
   <222> (7) .. (7)
   <223> Any nucleotide
<220>
   <221> misc_feature
   <222> (30) .. (30)
   <223> Any nucleotide
<220>
   <221> misc_feature
   <222> (41) .. (41)
   <223> Any nucleotide
<220>
   <221> misc_feature
   <222> (67) .. (67)
   <223> Any nucleotide
<400> 135
   gcacacnttc ttgacttacc aattgtaatn cataataatt ntaaacatgt caaagacagt
   60
   ttgtgtnacc ggagccagcg gagccatcgg ttcatgggtg gttcgcctcc tcctcgaacg
   120
   cggctacatt gtccacgcca ccatccaaga tctcgaggat gagaacgaga caaaacattt
   180
   ggaagcaatg gaaggagcaa agggtcatct caaatttttc gaaatggatc ttcttaacag
   240
   cgactctatt gcggccgccg tgaaaggttg tgccggagtt atacatcttg catgtcctaa
   300
   catcattggt gaagtcaaag accccgagaa gcaaattttg gaaccggcaa ttcaaggaac
   360
   ggttaatgtg ttgaaggtgg caaaggaagc aggggtggag cgtgtggtgg cgacatcgtc
   420
   gatctccgcc atcataccga gtcctaattg gccagctgat aagattaagg gagaagattg
   480
   ttggacagac cttgattatt gcaaggaaaa gaagttatac taccccattg caaagacact
   540
   agcagaaaaa gctggttggg aatttgctaa agagaccggt tttgatgttg ttatgattaa
   600
   ccctggtact
   610
<210> 136
   <211> 188
   <212> PRT
   <213> Trifolium repens
<220>
   <221> MISC_FEATURE
   <222> (7) .. (7)
   <223> Any amino acid
<400> 136
   Met Ser Lys Thr Val Cys Xaa Thr Gly Ala Ser Gly Ala Ile Gly Ser
   1 5 10 15
   Trp Val Val Arg Leu Leu Leu Glu Arg Gly Tyr Ile Val His Ala Thr
   20 25 30
   Ile Gln Asp Leu Glu Asp Glu Asn Glu Thr Lys His Leu Glu Ala Met
   35 40 45
   Glu Gly Ala Lys Gly His Leu Lys Phe Phe Glu Met Asp Leu Leu Asn
   50 55 60
   Ser Asp Ser Ile Ala Ala Ala Val Lys Gly Cys Ala Gly Val Ile His
   65 70 75 80
   Leu Ala Cys Pro Asn Ile Ile Gly Glu Val Lys Asp Pro Glu Lys Gln
   85 90 95
   Ile Leu Glu Pro Ala Ile Gln Gly Thr Val Asn Val Leu Lys Val Ala
   100 105 110
   Lys Glu Ala Gly Val Glu Arg Val Val Ala Thr Ser Ser Ile Ser Ala
   115 120 125
   Ile Ile Pro Ser Pro Asn Trp Pro Ala Asp Lys Ile Lys Gly Glu Asp
   130 135 140
   Cys Trp Thr Asp Leu Asp Tyr Cys Lys Glu Lys Lys Leu Tyr Tyr Pro
   145 150 155 160
   Ile Ala Lys Thr Leu Ala Glu Lys Ala Gly Trp Glu Phe Ala Lys Glu
   165 170 175
   Thr Gly Phe Asp Val Val Met Ile Asn Pro Gly Thr
   180 185
<210> 137
   <211> 327
   <212> DNA
   <213> Trifolium repens
<220>
   <221> misc_feature
   <222> (30) .. (30)
   <223> Any nucleotide
<220>
   <221> misc_feature
   <222> (41) .. (41)
   <223> Any nucleotide
<220>
   <221> misc_feature
   <222> (67) .. (67)
   <223> Any nucleotide
<220>
   <221> misc_feature
   <222> (255) .. (255)
   <223> Any nucleotide
<220>
   <221> misc_feature
   <222> (271) .. (271)
   <223> Any nucleotide
<220>
   <221> misc_feature
   <222> (287) .. (287)
   <223> Any nucleotide
<220>
   <221> misc_feature
   <222> (291) .. (291)
   <223> Any nucleotide
<220>
   <221> misc_feature
   <222> (313) .. (313)
   <223> Any nucleotide
<220>
   <221> misc_feature
   <222> (317) .. (319)
   <223> Any nucleotides
<220>
   <221> misc_feature
   <222> (326) .. (327)
   <223> Any nucleotides
<400> 137
   gcacaccttc ttgacttacc aattgtaatn cataataatt ntaaacatgt caaagacagt
   60
   ttgtgtnacc ggagccagcg gagccatcgg ttcatgggtg gttcgcctcc tcctcgaacg
   120
   cggctacatt gtccacgcca ccatccaaga tctcgaggat gagaacgaga caaaacattt
   180
   ggaagcaatg gaaggagcaa agggtcatct caaatttttc gaaatggatc ttcttaacag
   240
   cgactctatt gcggncgccg tgaaaggttg ngccggagtt atacatnttg nctgtcctac
   300
   cccccttggg gangagnnng cacccnn
   327
<210> 138
   <211> 349
   <212> DNA
   <213> Trifolium repens

<220>
   <221> misc_feature
   <222> (3) .. (3)
   <223> Any nucleotide
<220>
   <221> misc_feature
   <222> (7) .. (7)
   <223> Any nucleotide
<220>
   <221> misc_feature
   <222> (30) .. (30)
   <223> Any nucleotide
<220>
   <221> misc_feature
   <222> (67) .. (67)
   <223> Any nucleotide
<220>
   <221> misc_feature
   <222> (287) .. (287)
   <223> Any nucleotide
<220>
   <221> misc_feature
   <222> (314) .. (314)
   <223> Any nucleotide
<220>
   <221> misc_feature
   <222> (316) .. (316)
   <223> Any nucleotide
<220>
   <221> misc_feature
   <222> (320) .. (320)
   <223> Any nucleotide
<220>
   <221> misc_feature
   <222> (324) .. (324)
   <223> Any nucleotide
<220>
   <221> misc_feature
   <222> (328) .. (328)
   <223> Any nucleotide
<220>
   <221> misc_feature
   <222> (333) .. (333)
   <223> Any nucleotide
<220>
   <221> misc_feature
   <222> (341) .. (341)
   <223> Any nucleotide
<220>
   <221> misc_feature
   <222> (346) .. (346)
   <223> Any nucleotide
<220>
   <221> misc_feature
   <222> (348) .. (349)
   <223> Any nucleotides
<400> 138
   gcncacnttc ttgacttacc aattgtaatn cataatattt ataaacatgt caaagacagt
   60
   ttgtgtnacc ggagccagcg gagccatcgg ttcatgggtg gttcgcctcc tcctggaacg
   120
   cggctacatc gtccacgcca ccatccaaga tctcgaggat gagaacgaga caaaacattt
   180
   ggaagcaatg gaaggggcaa agggtcatct taaatttttc gaaatggatc ttcttaacag
   240
   tgactctatt gcggccgccg tgaaaggttg tgccggagtt atacatnttg catgtgctaa
   300
   catcattgct gaangnaaan accncganaa cgngattttg naaccngnn
   349
<210> 139
   <211> 592
   <212> DNA
   <213> Trifolium repens
<220>
   <221> misc_feature
   <222> (7) .. (7)
   <223> Any nucleotide
<220>
   <221> misc_feature
   <222> (20) .. (20)
   <223> Any nucleotide
<220>
   <221> misc_feature
   <222> (30) . .(30)
   <223> Any nucleotide
<220>
   <221> misc_feature
   <222> (41) .. (41)
   <223> Any nucleotide
<220>
   <221> misc_feature
   <222> (481) .. (481)
   <223> Any nucleotide
<220>
   <221> misc_feature
   <222> (505) .. (505)
   <223> Any nucleotide
<220>
   <221> misc_feature
   <222> (542) .. (542)
   <223> Any nucleotide
<400> 139
   gcacacnttc ttgacttacn aattgtaatn cataataatt ntaaacatgt caaaggcagt
   60
   ttgtgtcacc ggagccagcg gagccattgg ttcatgggtg gttcgcctcc tcctccaacg
   120
   cggctacatt gtccacgcca ccatccaaga tctcgaggat gagaacgaga caaaacattt
   180
   ggaagcaatg gaaggagcaa agggtcatct caaatttttt gaaatggatc ttcttaacag
   240
   cgactctatt gcggccgccg tgaaaggttg tgccggagtt atacatcttg catgtcctaa
   300
   catcattggt gaagtcaaag accccgagaa gcaaattttg gaaccggcaa ttcaaggaac
   360
   ggttaatgtg ttgaaggtgg caaaggaagc aggggtggag cgtgtggtgg cgacatcgtc
   420
   gatctccgcc atcataccga gtcctaattg gccagctgat aagattaagg gagaagattg
   480
   ntggacagac cttgattatt gcaangaaaa gaagttatac taccccattg caaagacatt
   540
   ancagaaaaa gctggttggg aatttgctaa agagaccggt tttgatgttg tt
   592
<210> 140
   <211> 594
   <212> DNA
   <213> Trifolium repens
<220>
   <221> misc_feature
   <222> (7) .. (7)
   <223> Any nucleotide
<220>
   <221> misc_feature
   <222> (30) .. (30)
   <223> Any nucleotide
<220>
   <221> misc_feature
   <222> (41) .. (41)
   <223> Any nucleotide
<400> 140
   gcacacnttc ttgacttacc aattgtaatn cataataatt ntaaacatgt caaagacagt 60
   ttgtgtcacc ggagccagcg gagccatcgg ttcatgggtg gttcgcctcc tcctcgaacg 120
   cggctacatt gtccacgcca ccatccaaga tctcgaggat gagaacgaga caaaacattt 180
   ggaagcaatg gaaggagcaa agggtcatct caaatttttc gaaatggatc ttctcaacag 240
   cgactctatt gcggccgccg tgaaaggttg tgccggagtt atacatcttg catgtcctag 300
   catcattggt gaagtcaaag accccgagaa gcaaattttg gaaccggcaa ttcaaggaac 360
   ggttaatgtg ttgaaggtgg caaaggaagc aggggtggag cgtgtggtgg cgacatcgtc 420
   gatctccgcc atcataccga gtcctaattg gccagctgat aagattaagg gagaagattg 480
   ttggacagac cttgattatt gcaaggaaaa gaagttatac taccccattg caaagacact 540
   agcagaaaaa gctggttggg aatttgctaa agagaccggt tttgatgttg ttat 594
<210> 141
   <211> 583
   <212> DNA
   <213> Trifolium repens
<220>
   <221> misc_feature
   <222> (4) .. (4)
   <223> Any nucleotide
<220>
   <221> misc_feature
   <222> (14) .. (14)
   <223> Any nucleotide
<220>
   <221> misc_feature
   <222> (18) .. (18)
   <223> Any nucleotide
<220>
   <221> misc_feature
   <222> (28).. (28)
   <223> Any nucleotide
<220>
   <221> misc_feature
   <222> (49) .. (50)
   <223> Any nucleotides
<400> 141
   gccncttctt gacncacnaa ttgtaatnca taataattat aaacatgtnn aagacagttt
   60
   gtgtcactgg agccagcgga gccatcggtt catgggtggt tcgcctcctc ctccaacgcg
   120
   gctacattgt ccacgccacc atccaagatc tcgaggatga gaacgagaca aaacatttgg
   180
   aagcaatgga aggagcaaag ggtcatctca aatttttcga aatggatctt cttaacagcg
   240
   actctattgc ggccgccgtg aaaggttgtg ccggagttat acatcttgca tgtcctaaca
   300
   tcattggtga agtcaaagac cccgagaagc aaattttgga accggcaatt caaggaacgg
   360
   ttaatgtgtt gaaggtggca aaggaagcag gggtggagcg tgtggtggcg acatcgtcga
   420
   tctctgccat cataccgagt cctaattggc cagctgataa gattaaggga gaagattgtt
   480
   ggacagacct tgattattgc aaggaaaaga agttatacta ccccattgca aagacactag
   540
   cagaaaaagc tggttgggaa tttgctagag agaccggttt tgt
   583
<210> 142
   <211> 608
   <212> DNA
   <213> Trifolium repens
<220>
   <221> misc_feature
   <222> (4) .. (4)
   <223> Any nucleotide
<220>
   <221> misc_feature
   <222> (18) .. (18)
   <223> Any nucleotide
<220>
   <221> misc_feature
   <222> (28) .. (28)
   <223> Any nucleotide
<220>
   <221> misc_feature
   <222> (39) .. (39)
   <223> Any nucleotide
<220>
   <221> misc_feature
   <222> (49) .. (49)
   <223> Any nucleotide
<220>
   <221> misc_feature
   <222> (65) .. (65)
   <223> Any nucleotide
<400> 142
   gctncttctt gacttacnaa ttgtaatnca taataattnt aaacatgtna aagacagttt
   60
   gtgtnaccgg agccagcgga gccatcggtt catgggtggt tcgcctcctc ctcgaacgcg
   120
   gctacattgt ccacgccacc atccaagatc tcgaggatga gaacgagaca aaacatttgg
   180
   aagcaatgga aggagcaaag ggtcatctca aatttttcga aatggatctt ctcaacagcg
   240
   actctattgc ggccgccgtg aaaggttgtg ccggagttat acatcttgca tgtcctagca
   300
   tcattggtga agtcaaagac cccgagaagc aaattttgga accggcaatt caaggaacgg
   360
   ttaatgtgtt gaaggtggca aaggaagcag gggtggagcg tgtggtggcg acatcgtcga
   420
   tctccgccat cataccgagt cctaattggc cagctgataa gattaaggga gaagattgtt
   480
   ggacagacct tgattattgc aaggaaaaga agttatacta ccccattgca aagacactag
   540
   cagaaaaagc tggttgggaa tttgctaaag agaccggttt tgatgttgtt atgattaacc
   600
   ctggtact
   608
<210> 143
   <211> 571
   <212> DNA
   <213> Trifolium repens
<220>
   <221> misc_feature
   <222> (2) .. (2)
   <223> Any nucleotide
<220>
   <221> misc_feature
   <222> (11) .. (11)
   <223> Any nucleotide
<220>
   <221> misc_feature
   <222> (25) .. (25)
   <223> Any nucleotide
<220>
   <221> misc_feature
   <222> (36) .. (36)
   <223> Any nucleotide
<220>
   <221> misc_feature
   <222> (47) .. (47)
   <223> Any nucleotide
<220>
   <221> misc_feature
   <222> (62) .. (63)
   <223> Any nucleotides
<220>
   <221> misc_feature
   <222> (72) .. (72)
   <223> Any nucleotide
<220>
   <221> misc_feature
   <222> (537) .. (537)
   <223> Any nucleotide
<400> 143
   cnttcttgac ntaccaattg taatncataa taattntaaa catgtcnaag gcagtttgtg 60
   tnnccggagc cngcggagcc attggttcat gggtggttcg cctcctcctc caacgcggct 120
   acattgtcca cgccaccatc caagatctcg aggatgagaa cgagacaaaa catttggaag 180
   caatggaagg agcaaagggt catctcaaat tttttgaaat ggatcttctt aacagcgact 240
   ctattgcggc cgccgtgaaa ggttgtgccg gagttataca tcttgcatgt cctaacatca 300
   ttggtgaagt caaagacccc gagaagcaaa ttttggaacc ggcaattcaa ggaacggtta 360
   atgtgttgaa ggtggcaaag gaagcagggg tggagcgtgt ggtggcgaca tcgtcgatct 420
   ccgccatcat accgagtcct aattggccag ctgataagat taagggagaa gattgttgga 480
   cagaccttga ttattgcaag gaaaagaagt tatactaccc cattgcaaag acattancag 540
   aaaaagctgg ttgggaattt gctaaagaga c 571
<210> 144
   <211> 586
   <212> DNA
   <213> Trifolium repens
<220>
   <221> misc_feature
   <222> (2) .. (2)
   <223> Any nucleotide
<220>
   <221> misc_feature
   <222> (15) .. (15)
   <223> Any nucleotide
<220>
   <221> misc_feature
   <222> (25) .. (25)
   <223> Any nucleotide
<400> 144
   cnttcttgac ttacnaattg taatncataa tatttataaa catgtcaaag acagtttgtg 60
   tcaccggagc cagcggagcc atcggttcat gggtggttcg tctcctcctg gaacgcggct 120
   acatcgtcca cgccaccatc caagatctcg aggatgagaa cgagacaaaa catttggaag 180
   caatggaagg ggcaaagggt tatcttaaat ttttcgaaat ggatcttctt aacagtgact 240
   ctattgcggc cgccgtgaaa ggttgtgccg gagttataca tcttgcatgt cctaacatca 300
   ttggtgaagt caaagacccc gagaagcaaa ttttggaacc ggcgattcaa ggaacggtta 360
   acgtgttgaa ggtggcaaag gaagcagggg tggagcgtgt ggtggcgacg tcatcgatct 420
   ccgccatcat accgagtcct aattggccag ctgataagat taagggagaa gattgttgga 480
   cggaccttga ttattgcaag gaaaagaagt tacactaccc catcgcaaag acactagcag 540
   aaaaagctgg ttgggaattt gctaaagaga ccggttttga tgttgt 586
<210> 145
   <211> 328
   <212> DNA
   <213> Trifolium repens
<220>
   <221> misc_feature
   <222> (10) .. (10)
   <223> Any nucleotide
<220>
   <221> misc_feature
   <222> (19) .. (19)
   <223> Any nucleotide
<220>
   <221> misc_feature
   <222> (24) .. (24)
   <223> Any nucleotide
<220>
   <221> misc_feature
   <222> (31) .. (31)
   <223> Any nucleotide
<220>
   <221> misc_feature
   <222> (35) .. (37)
   <223> Any nucleotides
<220>
   <221> misc_feature
   <222> (41) .. (41)
   <223> Any nucleotide
<220>
   <221> misc_feature
   <222> (45) .. (45)
   <223> Any nucleotide
<220>
   <221> misc_feature
   <222> (61) .. (61)
   <223> Any nucleotide
<220>
   <221> misc_feature
   <222> (71) .. (71)
   <223> Any nucleotide
<220>
   <221> misc_feature
   <222> (249) .. (249)
   <223> Any nucleotide
<220>
   <221> misc_feature
   <222> (289) .. (289)
   <223> Any nucleotide
<220>
   <221> misc_feature
   <222> (310) .. (310)
   <223> Any nucleotide
<220>
   <221> misc_feature
   <222> (315) .. (315)
   <223> Any nucleotide
<220>
   <221> misc_feature
   <222> (320) .. (320)
   <223> Any nucleotide
<220>
   <221> misc_feature
   <222> (326) .. (326)
   <223> Any nucleotide
<220>
   <221> misc feature
   <222> (328) .. (328)
   <223> Any nucleotide
<400> 145
   tttcttgacn tacccattnt tatncataat nattnnnaac ntgtnaaaga cagtttgtgt 60
   naccggagcc ngcggagcca tcggttcatg ggtggttcgc ctcctcctcg aacgcggcta 120
   cattgtccac gccaccatcc aagatctcga ggatgagaac gagacaaaac atttggaagc 180
   aatggaagga gcaaagggtc atctcaaatt ttttgaaatg gatcttctta acagcgactc 240
   tattgcggnc gccgtgaaag gttgtgccgg agttatacat cttgcatgng ctaacatcat 300
   tggggaagcn aaagnactcn ataagnan 328
<210> 146
   <211> 232
   <212> DNA
   <213> Trifolium repens
<220>
   <221> misc_feature
   <222> (1). (1)
   <223> Any nucleotide
<220>
   <221> misc_feature
   <222> (3) .. (3)
   <223> Any nucleotide

<220>
   <221> misc_feature
   <222> (12) .. (12)
   <223> Any nucleotide
<220>
   <221> misc_feature
   <222> (14) .. (14)
   <223> Any nucleotide
<220>
   <221> misc_feature
   <222> (21) .. (22)
   <223> Any nucleotides
<220>
   <221> misc_feature
   <222> (39) .. (39)
   <223> Any nucleotide
<220>
   <221> misc_feature
   <222> (60) .. (60)
   <223> Any nucleotide
<220>
   <221> misc_feature
   <222> (69) .. (69)
   <223> Any nucleotide
<220>
   <221> misc_feature
   <222> (154) .. (154)
   <223> Any nucleotide
<220>
   <221> misc_feature
   <222> (156) .. (156)
   <223> Any nucleotide
<220>
   <221> misc_feature
   <222> (178) .. (178)
   <223> Any nucleotide
<220>
   <221> misc_feature
   <222> (186) .. (186)
   <223> Any nucleotide
<220>
   <221> misc_feature
   <222> (232) .. (232)
   <223> Any nucleotide
<400> 146
   ngngtttgtg tnanctgagc nngcggagcc ctcggttant gggtggttcg cctcctcctn
   60
   ccacgcggnt acattgtcca cgccaccatc caagatctcg aggatgagaa cgagacaaaa
   120
   catttggaag caatggaagg agcaaagggt catntnaaat ttttcgaaat ggatcttntt
   180
   aacagngact ctattgcggc cgccgtgaaa ggttgtgccg gagttataca tn
   232
<210> 147
   <211> 623
   <212> DNA
   <213> Trifolium repens
<220>
   <221> misc_feature
   <222> (11) .. (11)
   <223> Any nucleotide
<220>
   <221> misc_feature
   <222> (14) .. (14)
   <223> Any nucleotide
<400> 147
   ataaaaccaa nctncaaaac tgattggaac tcagaaaaat agaagaaaag agatgcctga 60
   gttttgtgtt acagggggca ctggcttcat agcagcctac ctagtgaaag ccttattaga 120
   aaagggtcac acagtaagga ctactgtaag aaacccagat gatttggaga aggttggtta 180
   tctaactgaa ctaagtgaag acaaagagag attgaagatt ttaaaagcag atctattggt 240
   ggaagggagt tttgatgagg cagtgagtgg tgttgatggt gtgtttcata cagcttcccc 300
   tgttcttgtt ccacatgatg acaacattca ggttactttg attgatccat gtataaaagg 360
   aacacaaaat gtgcttaact catgcatcaa agcaaaggtg aaacgtgtgg tgttaacatc 420
   ttcatgctct tccataagat accgtgacga tgtgcaacaa atttctcctc ttaatgaatc 480
   tcattggagt gattctgaat actgcaaacg ctataacctg tggtatgcat atgcaaagac 540
   tttaggagaa aaagaagcat ggaggattgc aaaggaaagt ggaattgatc tagttgtagt 600
   taacccctct tttgttggtg gtc 623
<210> 148
   <211> 190
   <212> PRT
   <213> Trifolium repens
<400> 148
   Met Pro Glu Phe Cys Val Thr Gly Gly Thr Gly Phe Ile Ala Ala Tyr
   1               5                   10                  15
   Leu Val Lys Ala Leu Leu Glu Lys Gly His Thr Val Arg Thr Thr Val
               20                  25                  30
   Arg Asn Pro Asp Asp Leu Glu Lys Val Gly Tyr Leu Thr Glu Leu Ser
           35                  40                  45
   Glu Asp Lys Glu Arg Leu Lys Ile Leu Lys Ala Asp Leu Leu Val Glu
       50                  55                  60
   Gly Ser Phe Asp Glu Ala Val Ser Gly Val Asp Gly Val Phe His Thr
   65                  70                  75                  80
   Ala Ser Pro Val Leu Val Pro His Asp Asp Asn Ile Gln Val Thr Leu
                   85                  90                  95
   Ile Asp Pro Cys Ile Lys Gly Thr Gln Asn Val Leu Asn Ser Cys Ile
               100                 105                 110
   Lys Ala Lys Val Lys Arg Val Val Leu Thr Ser Ser Cys Ser Ser Ile
           115                 120                 125
   Arg Tyr Arg Asp Asp Val Gln Gln Ile Ser Pro Leu Asn Glu Ser His
       130                 135                 140
   Trp Ser Asp Ser Glu Tyr Cys Lys Arg Tyr Asn Leu Trp Tyr Ala Tyr
   145                 150                 155                 160
   Ala Lys Thr Leu Gly Glu Lys Glu Ala Trp Arg Ile Ala Lys Glu Ser
                   165                 170                 175
   Gly Ile Asp Leu Val Val Val Asn Pro Ser Phe Val Gly Gly
               180                 185                 190
<210> 149
   <211> 570
   <212> DNA
   <213> Trifolium repens
<220>
   <221> misc_feature
   <222> (11) .. (11)
   <223> Any nucleotide
<220>
   <221> misc_feature
   <222> (14) .. (14)
   <223> Any nucleotide
<220>
   <221> misc_feature
   <222> (32) .. (32)
   <223> Any nucleotide
<400> 149
   ataaaaccaa nctncaaaac tgattggaac tnagaaaaat agaagaaaag agatgcctga 60
   gttttgtgtt acagggggca ctgggttcat agcagcctac ctagtgaaag ccttattaga 120
   aaagggtcac acagtaagga ctactgtaag aaacccagat gatttggaga aggttggtta 180
   tctaactgaa ctaagtgaag acaaagagag attgaagatt ttaaaagcag atctattggt 240
   ggaagggagt tttgatgagg cagtgagtgg tgttgatggt gtgtttcata cagcttcccc 300
   tgttcttgtt ccacatgatg acaatattca ggttactttg attgatccat gtataaaagg 360
   aacacaaaat gtgcttagct catgcatcaa agcaaaggtg aaacgtgtgg tgttaacatc 420
   ttcatgctct tccataagat accgtgacga tgtgcaacaa atttcaccac ttaatgaatc 480
   tcattggagt gattctgaat actgcaaacg ctataacctg tggtatgcat atgcaaagac 540
   attaggagaa aaagaagcat ggaggattgc 570
<210> 150
   <211> 572
   <212> DNA
   <213> Trifolium repens
<220>
   <221> misc_feature
   <222> (2) .. (2)
   <223> Any nucleotide
<220>
   <221> misc_feature
   <222> (8) .. (9)
   <223> Any nucleotides
<400> 150
   gnacctcnna aaaatagaag aaaagagatg cctgagtttt gtgttacagg gggcactggg 60
   ttcatagcag cctacctagt gaaagcccta ttagaaaagg gtcacacagt aaggactact
   120
   gtaagaaacc cagatgattt ggagaaggtt ggttatctaa ctgaactaag tgaagacaaa
   180
   gagagattga agattttaaa agcagatcta ttggtggaag ggagttttga tgaggcagtg
   240
   agtggtgttg atggtgtgtt tcatacagct tcccctgttc ttgttccaca tgatgacaat
   300
   attcaggtta ctttgattga tccatgtata aaaggaacac agaatgtgct taactcatgc
   360
   atcaaagcaa aggtgaaacg tgtggtgtta acatcctcat gctcttccat aagataccgt
   420
   gacgatgtgc aacaaatttc tcctcttaat gaatctcatt ggagtgattc tgattactgc
   480
   aaacgctata acctgtggta tgcatatgca aagactttag gagaaaaaga agcatggagg
   540
   attgcaaagg aaagtgggat taatctagtt gt
   572
<210> 151
   <211> 572
   <212> DNA
   <213> Trifolium repens
<400> 151
   ggaaagagat gcctgagttt tgtgttacag ggggcactgg cttcatagca gcttacctag
   60
   tgaaagcctt attagaaaag ggtcacacag taaggactac tgtaagaaac ccagatgatt
   120
   tggagaaggt tggttatcta actgaactta gtgaagacaa agagagattg aagattttaa
   180
   aagcagatct attggtggaa gggagttttg atgaggcagt gagtggtgtt gatggtgtgt
   240
   ttcatacagc ttcccctgtt cttgttccac atgatgacaa cattcaggtt actttgattg
   300
   atccatgtat aaaaggaaca caaaatgtgc ttaactcatg catcaaagca aaggtgaaac
   360
   gtgtggtgtt aacatcttca tgctcttcca taagataccg tgacgatgtg caacaaattt
   420
   ctcctcttaa tgaatctcat tggagtgatt ctgaatactg caaacgctat aacctgtggt
   480
   atgcatatgc aaagacttta ggagaaaaag aagcatggag gattgcaaag gaaagtggaa
   540
   ttgatctagt tgtagttaac ccctcttttg tt
   572
<210> 152
   <211> 574
   <212> DNA
   <213> Trifolium repens
<220>
   <221> misc_feature
   <222> (40) .. (40)
   <223> Any nucleotide
<400> 152
   gagatgcctg agttttgtgt tacagggggc actggcttcn tagcagctta cctagtgaaa 60
   gccttattag aaaagggtca cacagtaagg actactgtaa gaaacccaga tgctttggag 120
   aaggttggtt atctaactga acttagtgaa gacaaagaga gattgaagat tttaaaagca 180
   gatctattgg tggaagggag ttttgatgag gcagtgagtg gtgttgatgg tgtgtttcat 240
   acagcttccc ctgttcttgt tccacatgat gacaacattc aggttacttt gattgatcca 300
   tgtataaaag gaacacaaaa tgtgcttaac tcatgcatca tagcaaaggt gaaacgtgtg 360
   gtgttaacat cttcatgctc ttccataaga taccgtgacg atgtgcaaca aatttctcct 420
   cttaatgaat ctcattggag tgattctgaa tactgcaaac gctataacct gtggtatgca 480
   tatgcaaaga ctttaggaga aaaagaagca tggaggattg caaaggaaag tggaattgat 540
   ctagttgtag ttaacccctc ttttgttggt ggtc 574
<210> 153
   <211> 641
   <212> DNA
   <213> Trifolium repens
<220>
   <221> misc_feature
   <222> (2) .. (2)
   <223> Any nucleotide
<220>
   <221> misc_feature
   <222> (8) .. (8)
   <223> Any nucleotide
<220>
   <221> misc_feature
   <222> (28) .. (28)
   <223> Any nucleotide
<220>
   <221> misc_feature
   <222> (505) .. (505)
   <223> Any nucleotide
<220>
   <221> misc_feature
   <222> (565) .. (565)
   <223> Any nucleotide
<220>
   <221> misc_feature
   <222> (608) .. (608)
   <223> Any nucleotide
<220>
   <221> misc_feature
   <222> (615) .. (616)
   <223> Any nucleotides
<220>
   <221> misc_feature
   <222> (626) .. (628)
   <223> Any nucleotides
<220>
   <221> misc_feature
   <222> (632) .. (632)
   <223> Any nucleotide
<220>
   <221> misc_feature
   <222> (641) .. (641)
   <223> Any nucleotide
<400> 153
   gngaaganct agtttgcgta acccgganca acggtttcat cggaacatgg ctagttaaaa 60
   ccctactcca aaaacactac aaaattcacg ccacaatctt ccccaattcc aacgcatctc 120
   atctcttcac actccacccg gaagctcaat cccggatcac aattttccct gtcgatatcc 180
   tcgactccac cgccgtcttc tccgctatca ataactgctc aggtgtcttt catgccgctt 240
   ctccatgtac cctcgaagat ccaactgatc cgcaaaaaga gcttctagaa cctgctgtac 300
   aaggaaccct aaatgttcta gaagcatcca gcgcgcaggt accaaaccct aattggccgg 360
   agaaaaaggc gatcgatgag gcgtcgtgga cggatgttga gtactgtaaa ttgagaggga 420
   agtggtatct ggtgtcgaaa acggaggcgg agaaggcggc ttgggatttt cgagagaaaa 480
   atggtggtgt tgatgtgggg gcggntcatc cggggacttg tttgggagag ttgatacaga 540
   aggagttgaa tgcgagttca gcggntttac agaggttgat gatggggagt gaggatactc 600
   aagagtgnta ttggnngggg ggctgnnnat gntaaagatg n 641
<210> 154
   <211> 206
   <212> PRT
   <213> Trifolium repens
<220>
   <221> MISC_FEATURE
   <222> (2) .. (2)
   <223> Any amino acid
<220>
   <221> MISC_FEATURE
   <222> (9) .. (9)
   <223> Any amino acid
<220>
   <221> MISC_FEATURE
   <222> (168) .. (168)
   <223> Any amino acid
<220>
   <221> MISC_FEATURE
   <222> (201) .. (201)
   <223> Any amino acid
<220>
   <221> MISC_FEATURE
   <222> (204) .. (204)
   <223> Any amino acid
<400> 154
   Glu Xaa Leu Val Cys Val Thr Arg Xaa Asn Gly Phe Ile Gly Thr Trp
   1               5                   10                  15
   Leu Val Lys Thr Leu Leu Gln Lys His Tyr Lys Ile His Ala Thr Ile
               20                  25                  30
   Phe Pro Asn Ser Asn Ala Ser His Leu Phe Thr Leu His Pro Glu Ala
           35                  40                  45
   Gln Ser Arg Ile Thr Ile Phe Pro Val Asp Ile Leu Asp Ser Thr Ala
       50                  55                  60
   Val Phe Ser Ala Ile Asn Asn Cys Ser Gly Val Phe His Ala Ala Ser
   65                  70                  75                  80
   Pro Cys Thr Leu Glu Asp Pro Thr Asp Pro Gln Lys Glu Leu Leu Glu
                   85                  90                  95
   Pro Ala Val Gln Gly Thr Leu Asn Val Leu Glu Ala Ser Ser Ala Gln
               100                 105                 110
   Val Pro Asn Pro Asn Trp Pro Glu Lys Lys Ala Ile Asp Glu Ala Ser
           115                 120                 125
   Trp Thr Asp Val Glu Tyr Cys Lys Leu Arg Gly Lys Trp Tyr Leu Val
       130                 135                 140
   Ser Lys Thr Glu Ala Glu Lys Ala Ala Trp Asp Phe Arg Glu Lys Asn
   145                 150                 155                 160
   Gly Gly Val Asp Val Gly Ala Xaa His Pro Gly Thr Cys Leu Gly Glu
                   165                 170                 175
   Leu Ile Gln Lys Glu Leu Asn Ala Ser Ser Ala Leu Gln Arg Leu Met
               180                 185                 190
   Met Gly Ser Glu Asp Thr Gln Glu Xaa Tyr Trp Xaa Gly Gly
           195                 200                 205
<210> 155
   <211> 822
   <212> DNA
   <213> Trifolium repens
<220>
   <221> misc_feature
   <222> (3) .. (3)
   <223> Any nucleotide
<220>
   <221> misc_feature
   <222> (17) .. (17)
   <223> Any nucleotide
<220>
   <221> misc_feature
   <222> (26) .. (26)
   <223> Any nucleotide
<220>
   <221> misc_feature
   <222> (574) .. (574)
   <223> Any nucleotide
<220>
   <221> misc_feature
   <222> (601) .. (601)
   <223> Any nucleotide
<220>
   <221> misc_feature
   <222> (614) .. (614)
   <223> Any nucleotide
<220>
   <221> misc_feature
   <222> (619) .. (619)
   <223> Any nucleotide
<220>
   <221> misc_feature
   <222> (692) .. (693)
   <223> Any nucleotides
<220>
   <221> misc_feature
   <222> (729) .. (730)
   <223> Any nucleotides
<220>
   <221> misc_feature
   <222> (743) .. (743)
   <223> Any nucleotide
<220>
   <221> misc_feature
   <222> (752) .. (752)
   <223> Any nucleotide
<220>
   <221> misc_feature
   <222> (756) .. (757)
   <223> Any nucleotides
<220>
   <221> misc_feature
   <222> (777) .. (777)
   <223> Any nucleotide
<220>
   <221> misc_feature
   <222> (779) .. (780)
   <223> Any nucleotides
<220>
   <221> misc_feature
   <222> (789) .. (789)
   <223> Any nucleotide
<220>
   <221> misc_feature
   <222> (794) .. (794)

   <223> Any nucleotide

<220>
   <221> misc_feature

   <222> (797) .. (797)
   <223> Any nucleotide
<220>
   <221> misc_feature
   <222> (812) .. (813)
   <223> Any nucleotides
<400> 155
   gcnttgatca gcgtatnaac acacangtct tcccttgagc tctgtttctc cacatgtcga 60
   agctagtttg cgtcaccggc ggcagcggat gcatcggttc atggctagtc catctccttc 120
   tcctccgcgg ctacactgtt cacgccaccg tccaaaatct caatgatgag aacgaaacga 180
   agcatctaga agctctcgaa ggagcacaaa ctaatctccg tctcttccag atcgatctcc 240
   ttaactacga cacaatcctc gctgctgtcc gcggttgcgt cggaattttc cacctcgctt 300
   caccttgcac tgtagacaaa gttcatgatc ctcagaagga gcttttggat cctgcaatta 360
   aagggacttt gaatgtgctt actgcagcta aggaagtagg ggtgaagcgt gtggttgtta 420
   cctcgtctgt ctcggcgatt actcctagtc ctgattggcc ttctgatgtt gttaaaagag 480
   aggattgttg gactgatgtt gaatattgca agaaaaaaga gttggggtat ccgttgtcca 540
   aaacattggc tgaaaaagct gcgtgggatt tttncaaaga aaatggtttg gatgttgttg 600
   nggtgaatcc cggnactgng atgggtcctg tttttccacc acggcataat gcaagcatgc 660
   tcatgccttg ggaaactttt ttgaaggctg gnnctgaaac atttgaagac tattttatgg 720
   gattggccnn ctttaaagat gtngcattgg cncatnnttt ggggtatgag aacaaanann 780
   ctttgggana catnggnggg ttgaaactat cnntccttac gg 822
<210> 156
   <211> 256
   <212> PRT
   <213> Trifolium repens
<220>
   <221> MISC_FEATURE
   <222> (174) .. (174)
   <223> Any amino acid
<220>
   <221> MISC_FEATURE
   <222> (183) .. (183)
   <223> Any amino acid
<220>
   <221> MISC_FEATURE
   <222> (187) .. (187)
   <223> Any amino acid
<220>
   <221> MISC_FEATURE
   <222> (189) .. (189)
   <223> Any amino acid
<220>
   <221> MISC_FEATURE
   <222> (213) .. (214)
   <223> Any amino acids
<220>
   <221> MISC_FEATURE
   <222> (226) .. (226)
   <223> Any amino acid
<220>
   <221> MISC_FEATURE
   <222> (230) .. (230)
   <223> Any amino acid
<220>
   <221> MISC_FEATURE
   <222> (233) .. (233)
   <223> Any amino acid
<220>
   <221> MISC_FEATURE
   <222> (235) .. (235)
   <223> Any amino acid
<220>
   <221> MISC_FEATURE
   <222> (242) .. (243)
   <223> Any amino acids
<220>
   <221> MISC_FEATURE
   <222> (246) .. (248)
   <223> Any amino acids
<220>
   <221> MISC_FEATURE
   <222> (253) .. (254)
   <223> Any amino acids
<400> 156
   Met Ser Lys Leu Val Cys Val Thr Gly Gly Ser Gly Cys Ile Gly Ser
   1 5 10 15
   Trp Leu Val His Leu Leu Leu Leu Arg Gly Tyr Thr Val His Ala Thr
   20 25 30
   Val Gln Asn Leu Asn Asp Glu Asn Glu Thr Lys His Leu Glu Ala Leu
   35 40 45
   Glu Gly Ala Gln Thr Asn Leu Arg Leu Phe Gln Ile Asp Leu Leu Asn
   50 55 60
   Tyr Asp Thr Ile Leu Ala Ala Val Arg Gly Cys Val Gly Ile Phe His
   65 70 75 80
   Leu Ala Ser Pro Cys Thr Val Asp Lys Val His Asp Pro Gln Lys Glu
   85 90 95
   Leu Leu Asp Pro Ala Ile Lys Gly Thr Leu Asn Val Leu Thr Ala Ala
   100 105 110
   Lys Glu Val Gly Val Lys Arg Val Val Val Thr Ser Ser Val Ser Ala
   115 120 125
   Ile Thr Pro Ser Pro Asp Trp Pro Ser Asp Val Val Lys Arg Glu Asp
   130 135 140
   Cys Trp Thr Asp Val Glu Tyr Cys Lys Lys Lys Glu Leu Gly Tyr Pro
   145 150 155 160
   Leu Ser Lys Thr Leu Ala Glu Lys Ala Ala Trp Asp Phe Xaa Lys Glu
   165 170 175
   Asn Gly Leu Asp Val Val Xaa Val Asn Pro Xaa Thr Xaa Met Gly Pro
   180 185 190
   Val Phe Pro Pro Arg His Asn Ala Ser Met Leu Met Pro Trp Glu Thr
   195 200 205
   Phe Leu Lys Ala Xaa Xaa Glu Thr Phe Glu Asp Tyr Phe Met Gly Leu
   210 215 220
   Ala Xaa Phe Lys Asp Xaa Ala Leu Xaa His Xaa Leu Gly Tyr Glu Asn
   225 230 235 240
   Lys Xaa Xaa Leu Gly Xaa Xaa Xaa Gly Leu Lys Leu Xaa Xaa Leu Thr
   245 250 255
<210> 157
   <211> 535
   <212> DNA
   <213> Trifolium repens
<220>
   <221> misc_feature
   <222> (3) .. (3)
   <223> Any nucleotide
<220>
   <221> misc_feature
   <222> (17) .. (17)
   <223> Any nucleotide
<220>
   <221> misc_feature
   <222> (26) .. (26)
   <223> Any nucleotide
<220>
   <221> misc_feature
   <222> (52) .. (52)
   <223> Any nucleotide
<400> 157
   gcnttgatca gcgtatnaac acacangtct tcccttgagc tctgtttctc cncatgtcga 60
   agctagtttg cgtcaccggc ggcagcggat gcatcggttc atggctagtc catctccttc 120
   tcctccgcgg ctacactgtt cacgccaccg tccaaaatct caatgatgag aacgaaacga 180
   agcatctaga agctctcgaa ggagcacaaa ctaatctccg tctcttccag atcgatctcc 240
   ttaactacga cacaatcctc gctgctgtcc gcggttgcgt cggaattttc cacctcgctt 300
   caccttgcac tgtagacaaa gttcatgatc ctcagaagga gcttttggat cctgcaatta 360
   aagggacttt gaatgtgctt actgcagcta aggaagtagg ggtgaagcgt gtggttgtta 420
   cctcgtctgt ctcggcgatt actcctagtc ctgattggcc ttctgatgtt gttaaaagag 480
   aggattgttg gactgatgtt gaatattgca agaaaaaaga gttgtggtat ccgtt 535
<210> 158
   <211> 801
   <212> DNA
   <213> Trifolium repens
<220>
   <221> misc_feature
   <222> (1) .. (1)
   <223> Any nucleotide
<220>
   <221> misc_feature
   <222> (5) .. (6)
   <223> Any nucleotides
<220>
   <221> misc_feature
   <222> (62) .. (62)
   <223> Any nucleotide
<220>
   <221> misc_feature
   <222> (553) .. (553)
   <223> Any nucleotide
<220>
   <221> misc_feature
   <222> (580) .. (580)
   <223> Any nucleotide
<220>
   <221> misc_feature
   <222> (593) .. (593)
   <223> Any nucleotide
<220>
   <221> misc_feature
   <222> (598) .. (598)
   <223> Any nucleotide
<220>
   <221> misc_feature
   <222> (671) .. (672)
   <223> Any nucleotides
<220>
   <221> misc_feature
   <222> (708) .. (709)
   <223> Any nucleotides
<220>
   <221> misc_feature
   <222> (722) .. (722)
   <223> Any nucleotide
<220>
   <221> misc_feature
   <222> (731) .. (731)
   <223> Any nucleotide
<220>
   <221> misc_feature
   <222> (735) .. (736)
   <223> Any nucleotides
<220>
   <221> misc_feature
   <222> (756) .. (756)
   <223> Any nucleotide
<220>
   <221> misc_feature
   <222> (758) .. (759)
   <223> Any nucleotides
<220>
   <221> misc_feature
   <222> (768) .. (768)
   <223> Any nucleotide
<220>
   <221> misc_feature
   <222> (773) .. (773)
   <223> Any nucleotide
<220>
   <221> misc_feature
   <222> (776) .. (776)
   <223> Any nucleotide
<220>
   <221> misc_feature
   <222> (791) .. (792)
   <223> Any nucleotides
<400> 158
   nacanntctt cccttgagct ctgtttctcc acatgtcgaa gctagtttgc gtcaccggcg 60
   gnggcggatg catcggttca tggctagtcc atctccttct cctccgcggc tacactgttc 120
   acgccaccgt ccaaaatctc aatgatgaga acgaaacgaa gcatctagaa gctctcgaag 180
   gagcacaaac taatctccgt ctcttccaga tcgatctcct taactacgac acaatcctcg 240
   ctgctgtccg cggttgcgtc ggaattttcc acctcgcttc accttgcact gtagacaaag 300
   ttcatgatcc tcagaaggag cttttggatc ctgcaattac agggactttg aatgtgctta 360
   ctgcagctaa ggaagtaggg gtgaagcgtg tggttgttac ctcgtctgtc tcggcgatta 420
   ctcctagtcc tgattggcct tctgatgttg ttaaaagaga ggattgttgg actgatgttg 480
   aatattgcaa gaaaaaagag ttggggtatc cgttgtccaa aacattggct gaaaaagctg 540
   cgtgggattt ttncaaagaa aatggtttgg atgttgttgn ggtgaatccc ggnactgnga 600
   tgggtcctgt ttttccacca cggcataatg caagcatgct catgccttgg gaaacttttt 660
   tgaaggctgg nnctgaaaca tttgaagact attttatggg attggccnnc tttaaagatg 720
   tngcattggc ncatnntttg gggtatgaga acaaanannc tttggganac atnggngggt 780
   tgaaactatc nntccttacg g 801
<210> 159
   <211> 582
   <212> DNA
   <213> Trifolium repens
<400> 159
   gtcacttatg aaatggaaca caaaggtgga gacaaagtat gtgtgacagg ggcatcaggc 60
   tttttagcat cttggcttat taagaaactt cttttgtctg gctatcaagt cattggaaca 120
   gttagagatt tagggaagaa gaagaaagtt gaacatttat ggaaattgga aggagcaaca 180
   gaaagactag aactaatcca agctgattta atggaagaaa atagtttcga caaagcgatc 240
   atgggatgca aaggtgtctt ccacattgcc tctccagtac tcaatcatat atcagataat 300
   cctaaggcgg aaatcttgga accggcagtc caaggtacgc taaatgtgtt gcgttcttgt 360
   aagaggaacc ccgatcttgt tcgagtggtg ctagcctcat catcttcggc tgttagagta 420
   agagctgatt ttgatccaag cataccaatt gatgaatcat cttggagctc cttggaattg 480
   tgcgagaaac tcaaggcatg gtacccaatg tcaaagacaa tggcagaaaa agcagcttgg 540
   gaatatagca aagagaatgg aatagactta gtgactattt tc 582
<210> 160
   <211> 190
   <212> PRT
   <213> Trifolium repens
<400> 160
   Met Glu His Lys Gly Gly Asp Lys Val Cys Val Thr Gly Ala Ser Gly
   1               5                   10                  15
   Phe Leu Ala Ser Trp Leu Ile Lys Lys Leu Leu Leu Ser Gly Tyr Gln
               20                  25                  30
   Val Ile Gly Thr Val Arg Asp Leu Gly Lys Lys Lys Lys Val Glu His
           35                  40                  45
   Leu Trp Lys Leu Glu Gly Ala Thr Glu Arg Leu Glu Leu Ile Gln Ala
       50                  55                  60
   Asp Leu Met Glu Glu Asn Ser Phe Asp Lys Ala Ile Met Gly Cys Lys
   65                  70                  75                  80
   Gly Val Phe His Ile Ala Ser Pro Val Leu Asn His Ile Ser Asp Asn
                   85                  90                  95
   Pro Lys Ala Glu Ile Leu Glu Pro Ala Val Gln Gly Thr Leu Asn Val
               100                 105                 110
   Leu Arg Ser Cys Lys Arg Asn Pro Asp Leu Val Arg Val Val Leu Ala
           115                 120                 125
   Ser Ser Ser Ser Ala Val Arg Val Arg Ala Asp Phe Asp Pro Ser Ile
       130                 135                 140
   Pro Ile Asp Glu Ser Ser Trp Ser Ser Leu Glu Leu Cys Glu Lys Leu
   145                 150                 155                 160
   Lys Ala Trp Tyr Pro Met Ser Lys Thr Met Ala Glu Lys Ala Ala Trp
                   165                 170                 175
   Glu Tyr Ser Lys Glu Asn Gly Ile Asp Leu Val Thr Ile Phe
               180                 185                 190
<210> 161
   <211> 572
   <212> DNA
   <213> Trifolium repens
<220>
   <221> misc_feature
   <222> (2) .. (2)
   <223> Any nucleotide
<220>
   <221> misc_feature
   <222> (4) .. (5)
   <223> Any nucleotides
<220>
   <221> misc_feature
   <222> (9) .. (9)
   <223> Any nucleotide
<220>
   <221> misc_feature
   <222> (11) .. (11)
   <223> Any nucleotide
<220>
   <221> misc_feature
   <222> (13) .. (13)
   <223> Any nucleotide
<220>
   <221> misc_feature
   <222> (24) .. (24)
   <223> Any nucleotide
<220>
   <221> misc_feature
   <222> (39) .. (39)
   <223> Any nucleotide
<220>
   <221> misc_feature
   <222> (47) .. (47)
   <223> Any nucleotide
<400> 161
   tncnngctnc ntncgggcag agantttccc tgacctatnt gttactnaag aatatttcta 60
   tatatatatt tgtgtttcaa gaacccaaaa aatagaatag tgatggaaag gagttgcaag 120
   gtttgtgtca ccggtggtgc tggttatatt ggttctcttt tagtcaaaaa gcttttggaa 180
   aagggttaca ccgttcatgc tactcttaga aacttgaagg acgaatccaa agtagatttt 240
   ttgagaggct ttccacatgc agatactaga cttatgttat ttgaagctga tatatacaaa 300
   tcagatgaat tttggcccgc aattcaaggt tgtgagtttg tttttcacct tgctactcct 360
   tttcaacatc aaactgattc tcagtttaag agcatagagg aagctgcaat agcaggggta 420
   aaaagcatag ctgaaaattg cataaaatca ggaacagtga gaaaattgat atacactgga 480
   actgtaattg cttcttcttc tctgaaagat gatggaagtg gctacaaaga cttcattgat 540
   gaaacttgtt ggacacctct ccatcttcct ct 572
<210> 162
   <211> 156
   <212> PRT
   <213> Trifolium repens
<400> 162
   Met Glu Arg Ser Cys Lys Val Cys Val Thr Gly Gly Ala Gly Tyr Ile
   1               5                   10                  15
   Gly Ser Leu Leu Val Lys Lys Leu Leu Glu Lys Gly Tyr Thr Val His
               20                  25                  30
   Ala Thr Leu Arg Asn Leu Lys Asp Glu Ser Lys Val Asp Phe Leu Arg
           35                  40                  45
   Gly Phe Pro His Ala Asp Thr Arg Leu Met Leu Phe Glu Ala Asp Ile
       50                  55                  60
   Tyr Lys Ser Asp Glu Phe Trp Pro Ala Ile Gln Gly Cys Glu Phe Val
   65                  70                  75                  80
   Phe His Leu Ala Thr Pro Phe Gln His Gln Thr Asp Ser Gln Phe Lys
                   85                  90                  95
   Ser Ile Glu Glu Ala Ala Ile Ala Gly Val Lys Ser Ile Ala Glu Asn
               100                 105                 110
   Cys Ile Lys Ser Gly Thr Val Arg Lys Leu Ile Tyr Thr Gly Thr Val
           115                 120                 125
   Ile Ala Ser Ser Ser Leu Lys Asp Asp Gly Ser Gly Tyr Lys Asp Phe
       130                 135                 140
   Ile Asp Glu Thr Cys Trp Thr Pro Leu His Leu Pro
   145                 150                 155
<210> 163
   <211> 714
   <212> DNA
   <213> Trifolium repens
<220>
   <221> misc_feature
   <222> (4) .. (4)
   <223> Any nucleotide
<220>
   <221> misc_feature
   <222> (131) .. (131)
   <223> Any nucleotide
<220>
   <221> misc_feature
   <222> (147) .. (147)
   <223> Any nucleotide

<400> 163
   gccnttgcct actactaaac tatatattat tattatatta tatgatgata catagtgaca 60
   ttaataattg gaagggagaa taaatagttg aaaaacacac agttggagtg tttttgttgt 120
   taaagaagct ngaaaatgga ggaagcnaca aagatggtga aaaagagtgg acaaattgtt 180
   cctactgcca aatactgtgt tacaggagca acaggctata ttggttcatg gcttgttgaa 240
   gctcttcttc aaagaggttg cactgttcat gctactgtta gagatcctga aaaatcgtta 300
   cacctcctgt cgttgtggaa aggtagtgac caattgagaa ttttccgtgc ggatttgcaa 360
   gaagaaggaa gtttcgatga tgccgtaaaa ggatgtattg gtgtgttcca tgttgcagct 420
   tcaatgcaat tcaatattag tgacaaagaa aacactgagg actttgttga agcaaatata 480
   attgaccctg caatcaaagg aaccataaat cttctcaaat catgcttgaa atcaaattca 540
   gtgaaaaggg ttgttttcac atcttccata agtactatta ctgctaaaga caacgacgga 600
   aaatggaaac ctattgttga tgaatcttgc caaacaaaaa ctgagattct gtggaataca 660
   caaccaagtg gatgggttta tgcactttca aagcttcatg cagaagaagc ggct 714
<210> 164
   <211> 187
   <212> PRT
   <213> Trifolium repens
<400> 164
   Met Val Lys Lys Ser Gly Gln Ile Val Pro Thr Ala Lys Tyr Cys Val
   1               5                   10                  15
   Thr Gly Ala Thr Gly Tyr Ile Gly Ser Trp Leu Val Glu Ala Leu Leu
               20                  25                  30
   Gln Arg Gly Cys Thr Val His Ala Thr Val Arg Asp Pro Glu Lys Ser
           35                  40                  45
   Leu His Leu Leu Ser Leu Trp Lys Gly Ser Asp Gln Leu Arg Ile Phe
       50                  55                  60
   Arg Ala Asp Leu Gln Glu Glu Gly Ser Phe Asp Asp Ala Val Lys Gly
   65                  70                  75                  80
   Cys Ile Gly Val Phe His Val Ala Ala Ser Met Gln Phe Asn Ile Ser
                   85                  90                  95
   Asp Lys Glu Asn Thr Glu Asp Phe Val Glu Ala Asn Ile Ile Asp Pro
               100                 105                 110
   Ala Ile Lys Gly Thr Ile Asn Leu Leu Lys Ser Cys Leu Lys Ser Asn
           115                 120                 125
   Ser Val Lys Arg Val Val Phe Thr Ser Ser Ile Ser Thr Ile Thr Ala
       130                 135                 140
   Lys Asp Asn Asp Gly Lys Trp Lys Pro Ile Val Asp Glu Ser Cys Gln
   145                 150                 155                 160
   Thr Lys Thr Glu Ile Leu Trp Asn Thr Gln Pro Ser Gly Trp Val Tyr
                   165                 170                 175
   Ala Leu Ser Lys Leu His Ala Glu Glu Ala Ala
               180                 185
<210> 165
   <211> 289
   <212> DNA
   <213> Trifolium repens
<220>
   <221> misc_feature
   <222> (4) .. (4)
   <223> Any nucleotide
<400> 165
   gccnttgcct actactaaac tatatattat tattatatta tatgatgata catagtgaca 60
   ttaataattg gaagggagaa taaatagttg aaaaacacac agttggagtg tttttgttgt 120
   taaagaagct ataaaatgga ggaaacaaca aagatggtga aaaatagtgg acaaattgtt 180
   cctatagcca aatactgtgt cacaggagcc acaggctata ttggttcatg gcttgttgaa 240
   gctcttcttc aaagaggttg cactgttcat gctactgtta gagatcctg 289
<210> 166
   <211> 591
   <212> DNA
   <213> Trifolium repens
<220>
   <221> misc_feature
   <222> (8) .. (8)
   <223> Any nucleotide
<220>
   <221> misc_feature
   <222> (23) .. (24)
   <223> Any nucleotides
<400> 166
   agaagctnga aaatggagga agnnacaaag atggtgaaaa agagtggaca aattgttcct 60
   actgccaaat actgtgttac aggagcaaca ggctatattg gttcatggct tgttgaagct 120
   cttcttcaaa gaggttgcac tgttcatgct actgttagag atcctgaaaa atcgttacac 180
   ctcctgtcgt tgtggaaagg tagtgaccaa ttgagaattt tccgtgcgga tttgcaagaa 240
   gaaggaagtt tcgatgatgc cgtaaaagga tgtattggtg tgttccatgt tgcagcttca 300
   atgcaattca atattagtga caaagaaaac actgaggact ttgttgaagc aaatataatt 360
   gaccctgcaa tcaaaggaac cataaatctt ctcaaatcat gcttgaaatc aaattcagtg 420
   aaaagggttg ttttcacatc ttccataagt actattactg ctaaagacaa cgacggaaaa 480
   tggaaaccta ttgttgatga atcttgccaa acaaaaactg agattctgtg gaatacacaa 540
   ccaagtggat gggtttatgc actttcaaag cttcatgcag aagaagcggc t 591
<210> 167
   <211> 572
   <212> DNA
   <213> Trifolium repens
<220>
   <221> misc_feature
   <222> (3) .. (3)
   <223> Any nucleotide
<220>
   <221> misc_feature
   <222> (19) .. (19)
   <223> Any nucleotide
<400> 167
   ctngaaaatg gaggaagcna caaagatggt gaaaaagagt ggacaaattg ttcctactgc 60
   caaatactgt gttacaggag caacaggcta tattggttca tggcttgttg aagctcttct 120
   tcaaagaggt tgcactgttc atgctactgt tagagatcct gaaaaatcgt tacacctcct 180
   gtcgttgtgg aaaggtagtg accaattgag aattttccgt gcggatttgc aagaagaagg 240
   aagtttcgat gatgccgtaa aaggatgtat tggtgtgttc catgttgcag cttcaatgca 300
   attcaatatt agtgacaaag aaaacactga ggactttgtt gaagcaaata taattgaccc 360
   tgcaatcaaa ggaaccataa atcttctcaa atcatgcttg aaatcaaatt cagtgaaaag 420
   ggttgttttc acatcttcca taagtactat tactgctaaa gacaacgacg gaaaatggaa 480
   acctattgtt gatgaatctt gccaaacaaa aactgagatt ctgtggaata cacaaccaag 540
   tggatgggtt tatgcacttt caaagcttca tg 572
<210> 168
   <211> 976
   <212> DNA
   <213> Trifolium repens
<220>
   <221> misc_feature
   <222> (2) .. (3)
   <223> Any nucleotides
<220>
   <221> misc_feature
   <222> (5) .. (5)
   <223> Any nucleotide
<220>
   <221> misc_feature
   <222> (16) .. (16)
   <223> Any nucleotide
<220>
   <221> misc_feature
   <222> (22) .. (22)
   <223> Any nucleotide
<220>
   <221> misc_feature
   <222> (25) .. (25)
   <223> Any nucleotide
<400> 168
   gnngngtctt ccgttnaatt tnagnctata ttgaaaagga aaaaaagagt agagaagtga 60
   agtgaagtga aaacatacat gggttccgaa tcagaaatag tttgtgttac cggagcttca 120
   ggtttcatcg gatcgtggct tgttatgaga cttatagagc gtggctacac ggttcgagcc 180
   accgttcgcg acccagataa catgaagaag gtgaagcatt tggtggaact gccgggtgca 240
   aaaagcaaat tgtctctttg gaaggctgat cttgataaag aggggagttt tgatgaagca 300
   attaaagggt gcacaggagt ttttcatgtt gctacaccaa tggattttga atccaaggac 360
   cctgagaatg aagtgataaa gcctacaata aatggattaa tagacatact gaaagcatgc 420
   gaaaaggcaa aaacagttag aaaattggtt ttcacatcat cggctggaac tgtggacgtt 480
   actgaacatc caaagtctat tattgatgaa acatgctgga gtgacgttga cttttgccgt 540
   agagtcaaga tgaccggttg gatgtatttt gtttcaaaga ccctagcaga gcaagaagct 600
   tggaagtatt cgaaagagca caacatagac tttgtctcca tcattccacc tcttgttgtt 660
   ggcccctttc ttatggcctc aatgccacct agtctaatca ctgctctttc tcttatcaca 720
   ggaaatgagg cccattactc aatcataaag caagggcaat acgtccattt agatgacctt 780
   tgtcttgctc atatatttct gtatgagaat ccaaaagctc aagggagata catttgctgt 840
   tcacatgaag caaccattca tcaagttgca aaacttatta aagaaaaata cccagagttc 900
   aatgtcccaa caaaattcaa tgatatccca gatgaattgg aaattattaa attttctaaa 960
   aagaagatca cagact 976
<210> 169
   <211> 299
   <212> PRT
   <213> Trifolium repens
<400> 169
   Met Gly Ser Glu Ser Glu Ile Val Cys Val Thr Gly Ala Ser Gly Phe
   1               5                   10                  15
   Ile Gly Ser Trp Leu Val Met Arg Leu Ile Glu Arg Gly Tyr Thr Val
               20                  25                  30
   Arg Ala Thr Val Arg Asp Pro Asp Asn Met Lys Lys Val Lys His Leu
           35                  40                  45
   Val Glu Leu Pro Gly Ala Lys Ser Lys Leu Ser Leu Trp Lys Ala Asp
       50                  55                  60
   Leu Asp Lys Glu Gly Ser Phe Asp Glu Ala Ile Lys Gly Cys Thr Gly
   65 70 75 80
   Val Phe His Val Ala Thr Pro Met Asp Phe Glu Ser Lys Asp Pro Glu
   85 90 95
   Asn Glu Val Ile Lys Pro Thr Ile Asn Gly Leu Ile Asp Ile Leu Lys
   100 105 110
   Ala Cys Glu Lys Ala Lys Thr Val Arg Lys Leu Val Phe Thr Ser Ser
   115 120 125
   Ala Gly Thr Val Asp Val Thr Glu His Pro Lys Ser Ile Ile Asp Glu
   130 135 140
   Thr Cys Trp Ser Asp Val Asp Phe Cys Arg Arg Val Lys Met Thr Gly
   145 150 155 160
   Trp Met Tyr Phe Val Ser Lys Thr Leu Ala Glu Gln Glu Ala Trp Lys
   165 170 175
   Tyr Ser Lys Glu His Asn Ile Asp Phe Val Ser Ile Ile Pro Pro Leu
   180 185 190
   Val Val Gly Pro Phe Leu Met Ala Ser Met Pro Pro Ser Leu Ile Thr
   195 200 205
   Ala Leu Ser Leu Ile Thr Gly Asn Glu Ala His Tyr Ser Ile Ile Lys
   210 215 220
   Gln Gly Gln Tyr Val His Leu Asp Asp Leu Cys Leu Ala His Ile Phe
   225 230 235 240
   Leu Tyr Glu Asn Pro Lys Ala Gln Gly Arg Tyr Ile Cys Cys Ser His
   245 250 255
   Glu Ala Thr Ile His Gln Val Ala Lys Leu Ile Lys Glu Lys Tyr Pro
   260 265 270
   Glu Phe Asn Val Pro Thr Lys Phe Asn Asp Ile Pro Asp Glu Leu Glu
   275 280 285
   Ile Ile Lys Phe Ser Lys Lys Lys Ile Thr Asp
   290 295
<210> 170
   <211> 586
   <212> DNA
   <213> Trifolium repens
<220>
   <221> misc_feature
   <222> (2) .. (3)
   <223> Any nucleotides
<220>
   <221> misc_feature
   <222> (16) .. (16)
   <223> Any nucleotide
<220>
   <221> misc_feature
   <222> (22) .. (22)
   <223> Any nucleotide
<220>
   <221> misc_feature
   <222> (25) .. (25)
   <223> Any nucleotide
<220>
   <221> misc_feature
   <222> (53) .. (55)
   <223> Any nucleotides
<220>
   <221> misc_feature
   <222> (57) .. (58)
   <223> Any nucleotides
<400> 170
   gnnttgtctt ccgttnaatt tnagnctata ttgaaaaaga aaaaaagagt agnnnannga 60
   agtgaagtga aaacatacat gggttccgaa tcagaaatag tttgtgttac cggagcttca 120
   tgtttcatcg gatcgtggct tgttatgaga cttatagagc gtggctacac ggttcgagcc 180
   accgttcgcg acccagataa catgaagaag gtgaagcatt tggtggaact gccgggtgca 240
   aaaagcaaat tgtctctttg gaaggctgat cttgataaag aggggagttt tgatgaagca 300
   attaaagggt gcacaggagt ttttcatgtt gctacaccaa tggattttga atccaaggac 360
   cctgagaatg aagtgataaa gcctacaata aatggattaa tagacatact gaaagcatgc 420
   gaaaaggcaa aaacagttaa aaaattggtt ttcacatcat cggctggaac tgtggacgtt 480
   actgaacatc caaagtctat tattgatgaa acatgctgga gtgacgttga cttttgccgt 540
   agagtcaaga tgaccggttg gatgtatttt gtttcaaaga ccctag
   586
<210> 171
   <211> 569
   <212> DNA
   <213> Trifolium repens
<220>
   <221> misc_feature
   <222> (24) .. (24)
   <223> Any nucleotide
<220>
   <221> misc_feature
   <222> (209) .. (209)
   <223> Any nucleotide
<400> 171
   ttttgtctta aatccaattt cagnctaaat tgaaaaggaa aaaaagagga gagaagtcaa
   60
   ctggagtgaa aatatacatg ggttctgaat cggaaatagt ttgtgttacc ggagcttcag
   120
   gtttcatcgg gtcgtggctt gttatgagac ttatggagca tggctacact gttcgagcca
   180
   ccgttcgtga cccagataac atgaagaang tgaagcattt gctggaactg ccaggtgcaa
   240
   aaagcaaatt gtctctttgg aaggctgatc ttgataaaga ggggagtttt gatgaagcaa
   300
   ttaaagggtg cacaggagtt tttcatgttg ctacaccaat ggattttgag tccaaggacc
   360
   ctgagaatga agtgataaag cctacaataa acggattaat agacatactg aaagcatgcg
   420
   aaaaggcaaa aacagttaga aaattggttt tcacatcatc ggctggaact gtggacgtta
   480
   ctgaacatcc aaagtctatt attgatgaaa catgctggag tgacgttgac ttttgccgta
   540
   gagtcaaaat gaccggttgg atgtatttt
   569
<210> 172
   <211> 493
   <212> DNA
   <213> Trifolium repens
<220>
   <221> misc_feature
   <222> (2) .. (2)
   <223> Any nucleotide
<220>
   <221> misc_feature
   <222> (13) .. (13)
   <223> Any nucleotide
<220>
   <221> misc_feature
   <222> (19) .. (19)
   <223> Any nucleotide
<220>
   <221> misc_feature
   <222> (22) .. (22)
   <223> Any nucleotide
<220>
   <221> misc_feature
   <222> (50) .. (52)
   <223> Any nucleotides
<220>
   <221> misc_feature
   <222> (54) .. (55)
   <223> Any nucleotides
<220>
   <221> misc_feature
   <222> (69) .. (69)
   <223> Any nucleotide
<220>
   <221> misc_feature
   <222> (102) .. (102)
   <223> Any nucleotide
<220>
   <221> misc_feature
   <222> (123) .. (123)
   <223> Any nucleotide
<220>
   <221> misc_feature
   <222> (154) .. (154)
   <223> Any nucleotide
<220>
   <221> misc_feature
   <222> (161) .. (161)
   <223> Any nucleotide
<220>
   <221> misc_feature
   <222> (322) .. (322)
   <223> Any nucleotide
<220>
   <221> misc_feature
   <222> (418) .. (418)
   <223> Any nucleotide
<220>
   <221> misc_feature
   <222> (464) .. (464)
   <223> Any nucleotide
<220>
   <221> misc_feature
   <222> (493) .. (493)
   <223> Any nucleotide
<400> 172
   gngtcttccg ttnaatttna gnctatattg aaaaagaaaa aaagagtagn nnanngaagt 60
   gaagtgaana catacatggg ttccgaatca gaaatagttt gngttaccgg agcttcatgt 120
   ttnatcggat cgtggcttgt tatgagactt atanagcgtg nctacacggc tcgagccacc 180
   gttcgcgacc cagataacat gaagaaggtg aagcatttgg tggaactgcc gggtgcaaaa 240
   agcaaattgt ctctttggaa ggctgatctt gataaagagg ggagttttga tgaagcaatt 300
   aaagggtgca caggagtttt tnatgttgct acaccaatgg attttgaatc caaggaccct 360
   gagaatgaag tgataaagcc tacaataaat ggattaatag acatactgaa agcatgcnaa 420
   aaggcaaaaa cagttaaaaa attggttttc acatcatcgg ctgnaactgt ggacgttact 480
   gaacatccaa agn 493
<210> 173
   <211> 580
   <212> DNA
   <213> Trifolium repens
<220>
   <221> misc_feature
   <222> (2) .. (2)
   <223> Any nucleotide
<220>
   <221> misc_feature
   <222> (13) .. (13)
   <223> Any nucleotide
<220>
   <221> misc_feature
   <222> (19) .. (19)
   <223> Any nucleotide

<220>
   <221> misc_feature
   <222> (22) .. (22)
   <223> Any nucleotide
<220>
   <221> misc_feature
   <222> (54) .. (55)
   <223> Any nucleotides
<220>
   <221> misc_feature
   <222> (57) .. (59)
   <223> Any nucleotides
<220>
   <221> misc_feature
   <222> (539) .. (539)
   <223> Any nucleotide
<220>
   <221> misc_feature
   <222> (546) .. (546)
   <223> Any nucleotide
<220>
   <221> misc_feature
   <222> (576) .. (576)
   <223> Any nucleotide
<400> 173
   gngtcttccg ttnaatttna gnctatattg aaaaagaaaa aaagagtaga gaanngnnnt
   60
   gaagtgaaaa catacatggg ttccgaatca gaaatagttt gtgttaccgg agcttcatgt
   120
   ttcatcggat cgtggcttgt tatgagactt atagagcgtg gctacacggt tcgagccacc
   180
   gttcgcgacc cagataacat gaagaaggtg aagcatttgg tggaactgcc gggtgcaaaa
   240
   agcaaattgt ctctttggaa ggctgatctt gataaagagg ggagttttga tgaagcaatt
   300
   aaagggtgca caggagtttt tcatgttgct acaccaatgg attttgaatc caaggaccct
   360
   gagaatgaag tgataaagcc tacaataaat ggattaatag acatactgaa agcatgcgaa
   420
   aaggcaaaaa cagttaaaaa attggttttc acatcatcgg ctggaactgt ggacgttact
   480
   gaacatccaa agtctattat tgatgaaaca tgctggagtg acgttgactt ttgccgtana
   540
   gtcaanatga ccggctggat gtattttgtt tcaaanaccc
   580
<210> 174
   <211> 581
   <212> DNA
   <213> Trifolium repens
<220>
   <221> misc_feature
   <222> (2) .. (2)
   <223> Any nucleotide
<220>
   <221> misc_feature
   <222> (13) .. (13)
   <223> Any nucleotide
<220>
   <221> misc_feature
   <222> (22) .. (22)
   <223> Any nucleotide
<220>
   <221> misc_feature
   <222> (36) .. (36)
   <223> Any nucleotide
<220>
   <221> misc_feature
   <222> (50) .. (52)
   <223> Any nucleotides
<220>
   <221> misc_feature
   <222> (54) .. (55)
   <223> Any nucleotides
<400> 174
   gngtcttccg ttnaatttaa gnctatattg aaaagnaaaa aaagagtagn nnanngaagt 60
   gaagtgaaaa catacatggg ttccgaatca gaaatagttt gtgttaccgg agcttcaggt 120
   ttcatcggat cgtggcttgt tatgagactt atagagcgtg gctacacggt tcgagccacc 180
   gttcgcgacc cagataacat gaagaaggtg aagcatttgg tggaactgcc gggtgcaaaa 240
   agcaaattgt ctctttggaa ggctgatctt gataaagagg ggagttttga tgaagcaatt 300
   aaagggtgca caggagtttt tcatgttgct acaccaatgg attttgaatc caaggaccct 360
   gagaatgaag tgataaagcc tacaataaat ggattaatag acatactgaa agcatgcgaa 420
   aaggcaaaaa cagttagaaa attggttttc acatcatcgg ctggaactgt ggacgttact 480
   gaacatccaa agtctattat tgatgaaaca tgctggagtg acgttgactt ttgccgtaga 540
   gtcaagatga ccggttggat gtattttgtt tcaaagaccc t 581
<210> 175
   <211> 592
   <212> DNA
   <213> Trifolium repens
<220>
   <221> misc_feature
   <222> (2) .. (2)
   <223> Any nucleotide
<220>
   <221> misc_feature
   <222> (13) .. (13)
   <223> Any nucleotide
<220>
   <221> misc_feature
   <222> (16) .. (16)
   <223> Any nucleotide
<220>
   <221> misc_feature
   <222> (22) .. (22)
   <223> Any nucleotide
<220>
   <221> misc_feature
   <222> (36) .. (36)
   <223> Any nucleotide
<220>
   <221> misc_feature
   <222> (50) .. (52)
   <223> Any nucleotides
<220>
   <221> misc_feature
   <222> (54) .. (55)
   <223> Any nucleotides
<400> 175
   gngtcttccg ttnaanttaa gnctatattg aaaagnaaaa aaagagtagn nnanngaagt 60
   gaagtgaaaa catacatggg ttccgaatca gaaatagttt gtgttaccgg agcttcaggt 120
   ttcatcggat cgtggcttgt tatgagactt atagagcgtg gctacacggt tcgagccacc 180
   gttcgcgacc cagataacat gaagaaggtg aagcatttgg tggaactgcc gggtgcaaaa 240
   agcaaattgt ctctttggaa ggctgatctt gataaagagg ggagttttga tgaagcaatt 300
   aaagggtgca caggagtttt tcatgttgct acaccaatgg attttgaatc caaggaccct 360
   gagaatgaag tgataaagcc tacaataaat ggattaatag acatactgaa agcatgcgaa 420
   aaggcaaaaa cagttagaaa attggttttc acatcatcgg ctggaactgt ggacgttact 480
   gaacatccaa agtctattat tgatgaaaca tgctggagtg acgttgactt ttgccgtaga 540
   gtcaagatga ccggttggat gtattttgtt tcaaagaccc tagcagagca ag 592
<210> 176
   <211> 598
   <212> DNA
   <213> Trifolium repens
<220>
   <221> misc_feature
   <222> (2) .. (2)
   <223> Any nucleotide
<220>
   <221> misc_feature
   <222> (13) .. (13)
   <223> Any nucleotide
<220>
   <221> misc_feature
   <222> (19) .. (19)
   <223> Any nucleotide
<220>
   <221> misc_feature
   <222> (22) .. (22)
   <223> Any nucleotide
<400> 176
   gngtcttccg ttnaatttna gnctatattg aaaaagaaaa aaagagtaga gaagtgaagt 60
   gaagtgaaaa catacatggg ttctgaatca gaaatagttt gtgttaccgg agcatcaggt 120
   ttcatcggat cgtggcttgt tatgagactt atagagcgtg gctacacggt tcgagccact 180
   gttcgcgacc cagataacat gaagaaggtg aagcatttgg tggaactgcc gggtgcaaaa 240
   agcaaattgt ctctttggaa ggctgatctt gataaagagg ggagttttga tgaagcaatt 300
   aaagggtgca caggagtttt tcatgttgct acaccaatgg attttgaatc caaggaccct 360
   gagaatgaag tgataaagcc tacaataaat ggattaatag acatactgaa agcatgcgaa 420
   aaggcaaaaa cagttaaaaa attggttttc acatcatcgg ctggaactgt ggacgttact 480
   gaacatccaa agtctattat tgatgaaaca tgctggagtg acgttgactt ttgccgtaga 540
   gtcaagatga ccggttggat gtattttgtt tcaaagaccc tagcagagca agaagctt 598
<210> 177
   <211> 576
   <212> DNA
   <213> Trifolium repens
<220>
   <221> misc_feature
   <222> (9) .. (9)
   <223> Any nucleotide
<220>
   <221> misc_feature
   <222> (12) .. (12)
   <223> Any nucleotide
<220>
   <221> misc_feature
   <222> (18) .. (18)
   <223> Any nucleotide
<220>
   <221> misc_feature
   <222> (21) .. (21)
   <223> Any nucleotide
<400> 177
   ggtcttccnt tnaattcnag ncgaaattga aaaggaaaaa aagaggagag aagtgaactg 60
   aagtgaaaat atacatgggt tccgaatcgg aaatagtttg tgttaccgga gcttcaggtt 120
   tcatcgggtc gtggcttgtt atgagactta tggagcgcgg ctacacggtt cgagccactg 180
   ttcgcgaccc agataacatg aagaaggtga agcatttgat ggaactgccg ggtgcaaaaa 240
   gcaaattgtc tctttggaag gctgatcttg ataaagaggg gagttttgat gaagcaatta 300
   aagggtgcac aggagttttt catgttgcta caccaatgga ttttgaatcc aaggaccctg 360
   agaacgaagt gataaagcct acaataaatg gactaataga catactgaga gcatgtgaaa 420
   aggcaaaaac aattagaaga ttggttttca catcatcagc tggaactgtg gacgtaactg 480
   aacactcaaa atcaattgtt gatgaaacat gttggagtga cgttgacttt tgccgtagag 540
   tcaaaatgac cggttggatg tattttgttt caaaga 576
<210> 178
   <211> 587
   <212> DNA
   <213> Trifolium repens
<220>
   <221> misc_feature
   <222> (12) .. (12)
   <223> Any nucleotide
<220>
   <221> misc_feature
   <222> (21) .. (21)
   <223> Any nucleotide
<400> 178
   ggtcttccgt tnaatttaag nctatattga aaaagaaaaa aagagtagag aagtgaagtg 60
   aagtgaaaac atacatgggt tctgaatcag aaatagtttg tgttaccgga gcatcaggtt 120
   tcatcggatc gtggcttgtt atgagactta tagagcgtgg ctacacggtt cgagccactg 180
   ttcgcgaccc agataacatg aagaaggtga agcatttggt ggaactgccg ggtgcaaaaa 240
   gcaaattgtc tctttggaag gctgatcttg ataaagaggg gagttttgat gaagcaatta 300
   aagggtgcac aggagttttt catgttgcta caccaatgga ttttgaatcc aaggaccctg 360
   agaatgaagt gataaagcct acaataaatg gattaataga catactgaaa gcatgcgaaa 420
   aggcaaaaac agttaaaaaa ttggttttca catcatcggc tggaactgtg gacgttactg 480
   aacatccaaa gtctattatt gatgaaacat gctggagtga cgttgacttt tgccgtagag 540
   tcaagatgac cggttggatg tattttgttt caaagaccct agcagag 587
<210> 179
   <211> 630
   <212> DNA
   <213> Trifolium repens
<220>
   <221> misc_feature
   <222> (12) .. (13)
   <223> Any nucleotides
<220>
   <221> misc_feature
   <222> (21) .. (21)
   <223> Any nucleotide
<400> 179
   ggtcttccgt tnnatttaag nctatattga aaaagaaaaa aagagtagag aagtgaagtg 60
   aagtgaaaac atacatgggt tctgaatcag aaatagtttg tgttaccgga gcatcaggtt 120
   tcatcggatc gtggcttgtt atgagactta tagagcgtgg ctacacggtt cgagccactg 180
   ttcgcgaccc agataacatg aagaaggtga agcatttggt ggaactgccg ggtgcaaaaa 240
   gcaaattgtc tctttggaag gctgatcttg ataaagaggg gagttttgat gaagcaatta 300
   aagggtgcac aggagttttt catgttgcta caccaatgga ttttgaatcc aaggaccctg 360
   agaatgaagt gataaagcct acaataaatg gattaataga catactgaaa gcatgcgaaa 420
   aggcaaaaac agttaaaaaa ttggttttca catcatcggc tggaactgtg gacgttactg 480
   aacatccaaa gtctattatt gatgaaacat gctggagtga cgttgacttt tgccgtagag 540
   tcaagatgac cggttggatg tattttgttt caaagaccct agcagagcaa gaagcttgga 600
   agtattctaa agagcacaac atagattttg 630
<210> 180
   <211> 579
   <212> DNA
   <213> Trifolium repens
<220>
   <221> misc_feature
   <222> (7) .. (8)
   <223> Any nucleotides
<220>
   <221> misc_feature
   <222> (11) .. (11)
   <223> Any nucleotide
<220>
   <221> misc_feature
   <222> (17) .. (17)
   <223> Any nucleotide
<220>
   <221> misc_feature
   <222> (20) .. (20)
   <223> Any nucleotide
<400> 180
   gtcttcnntt naattcnagn cgaaattgaa aaggaaaaaa agaggagaga agtgaactga 60
   agtgaaaata tacatgggtt ccgaatcgga aatagtttgt gttaccggag cttcaggttt 120
   catcgggtcg tggcttgtta tgagacttat ggagcgcggc tacacggttc gagccactgt 180
   tcgcgaccca gataacatga agaaggtgaa gcatttgatg gaactgccgg gtgcaaaaag 240
   caaattgtct ctttggaagg ctgatcttga taaagagggg agttttgatg aagcaattaa 300
   agggtgcaca ggagtttttc atgttgctac accaatggat tttgaatcca aggaccctga 360
   gaacgaagtg ataaagccta caataaatgg actaatagac atactgagag catgtgaaaa 420
   ggcaaaaaca attagaagat tggttttcac atcatcagct ggaactgtgg acgtaactga 480
   acactcaaaa tcaattgttg atgaaacatg ttggagtgac gttgactttt gccgtagagt 540
   caaaatgacc ggttggatgt attttgtttc aaagaccct 579
<210> 181
   <211> 604
   <212> DNA
   <213> Trifolium repens
<220>
   <221> misc_feature
   <222> (12) .. (12)
   <223> Any nucleotide
<220>
   <221> misc_feature
   <222> (17) .. (17)
   <223> Any nucleotide
<220>
   <221> misc_feature
   <222> (20) .. (20)
   <223> Any nucleotide
<220>
   <221> misc_feature
   <222> (34) .. (34)
   <223> Any nucleotide
<220>
   <221> misc_feature
   <222> (52) .. (53)
   <223> Any nucleotides
<220>
   <221> misc_feature
   <222> (55) .. (57)
   <223> Any nucleotides
<400> 181
   gtcttccgtt cnatttnagn ctatattgaa aagnaaaaaa agagtagaga anngnnntga
   60
   agtgaaaaca tacatgggtt ccgaatcaga aatagtttgt gttaccggag cttcaggttt
   120
   catcggatcg tggcttgtta tgagacttat agagcgtggc tacacggttc gagccaccgt
   180
   tcgcgaccca gataacatga agaaggtgaa gcatttggtg gaactgccgg gtgcaaaaag
   240
   caaattgtct ctttggaagg ctgatcttga taaagagggg agttttgatg aagcaattaa
   300
   agggtgcaca ggagtttttc atgttgctac accaatggat tttgaatcca aggaccctga
   360
   gaatgaagtg ataaagccta caataaatgg attaatagac atactgaaag catgcgaaaa
   420
   ggcaaaaaca gttagaaaat tggttttcac atcatcggct ggaactgtgg acgttactga
   480
   acatccaaag tctattattg atgaaacatg ctggagtgac gttgactttt gccgtagagt
   540
   caagatgacc ggttggatgt attttgtttc aaagacccta gcagagcaag aagcttggaa
   600
   gtat
   604
<210> 182
   <211> 586
   <212> DNA
   <213> Trifolium repens
<220>
   <221> misc_feature
   <222> (10) .. (10)
   <223> Any nucleotide
<400> 182
   ggtcttcttn aatccagcta aattgaaaag gaaaaaaaga ggagagaagt gaactggagt
   60
   gaaaatatac atgggttctg aatcggaaat agtttgtgtt accggagctt caggtttcat 120
   cgggtcgtgg cttgttatga gacttatgga gcgtggctac actgttcgag ccaccgttcg 180
   tgacccagat aacatgaaga aggtgaagca tttgctggaa ctgccgggtg caaaaagcaa 240
   attgtctctt tggaaggctg atcttgataa agaggggagt tttgatgaag caattaaagg 300
   gtgcacagga gtttttcatg ttgctacacc aatggatttt gaatccaagg accctgagaa 360
   tgaagtgata aagcctacaa taaacggatt aatagacata ctgaaagcat gcgaaaaggc 420
   aaaaacagtt agaaaattgg ttttcacatc atcggctgga actgtggacg ttactgaaca 480
   tccaaagtct attattgatg aaacatgctg gagtgacgtt gacttttgcc gtagagtcaa 540
   aatgaccggt tggatgtatt ttgtttcaaa gaccctagca gagcag 586
<210> 183
   <211> 586
   <212> DNA
   <213> Trifolium repens
<220>
   <221> misc_feature
   <222> (2) .. (2)
   <223> Any nucleotide
<220>
   <221> misc_feature
   <222> (11) .. (11)
   <223> Any nucleotide
<400> 183
   gnagagaagt nacctggagt gaaaatatac atgggttctg aatcggaaat agtttgtgtt 60
   accggagctt caggtttcat cgggtcgtgg cttgttatga gacttatgga gcgtggctac 120
   actgttcgag ccaccgttcg tgacccagat aacatgaaga aggtgaagca tttgctggaa 180
   ctgccgggtg caaaaagcaa attgtctctt tggaaggctg atcttgataa agaggggagt 240
   tttgatgaag caattaaagg gtgcacagga gtttttcatg ttgctacacc aatggatttt 300
   gagtccaagg accctgagaa tgaagtgata aagcctacaa taaacggatt aatagacata 360
   ctgaaagcat gcgaaaaggc aaaaacagtt agaaaattgg ttttcacatc atcggctgga 420
   actgtggacg ttactgaaca tccaaagtct attattgatg aaacatgctg gagtgacgtt 480
   gacttttgcc gtagagtcaa aatgaccggt tggatgtatt ttgtttcaaa gaccctagca 540
   gagcaagaag cttggaagta ttcgaaagag cacaacatag actttg 586

<210> 184
   <211> 570
   <212> DNA
   <213> Trifolium repens
<220>
   <221> misc_feature
   <222> (10) .. (11)
   <223> Any nucleotides
<220>
   <221> misc_feature
   <222> (23) .. (23)
   <223> Any nucleotide
<400> 184
   tactgaaagn ntgcgaaaag gcnaaaacag ttagaaaatt ggttttcaca tcatcggctg
   60
   gaactgtgga cgttactgaa catccaaagt ctattattga tgaaacatgc tggagtgacg
   120
   ttgacttttg ccgtagagtc aaaatgaccg gttggatgta ttttgtttca aagaccctag
   180
   cagagcaaga agcttggaag tattcgaaag agcacaacat agactttgtc tccatcattc
   240
   cacctcttgt tgttggcccc tttcttatgg cctcaatgcc acctagtcta atcactgctc
   300
   tttctcttat cacaggaaat gaggcccatt actcaatcat aaagcaaggg caatacgtcc
   360
   atttagatga cctttgtctt gctcatatat ttctgtatga gaatccaaaa gctcaaggga
   420
   gatacatttg ctgttcacat gaagcaacca ttcatcaagt tgcaaaactt attaaagaaa
   480
   aatacccaga gttcaatgtc ccaacaaaat tcaatgatat cccagatgaa ttggaaatta
   540
   ttaaattttc taaaaagaag atcacagact
   570
<210> 185
   <211> 833
   <212> DNA
   <213> Trifolium repens
<220>
   <221> misc_feature
   <222> (3) .. (3)
   <223> Any nucleotide
<220>
   <221> misc_feature
   <222> (34) .. (34)
   <223> Any nucleotide
<400> 185
   ggncataaaa actgcactag tgtgtataag tttnttagtg aaaaaagagt gtgtaaatta 60
   acatcatggc tagtatcaaa caaattggaa acaagaaagc atgtgtgatt ggtggcactg 120
   gttttgttgc atctatgttg atcaaacagt tacttgaaaa gggttatgct gttaatacta 180
   ctgttagaga cccagatagt cctaagaaaa tatctcacct agtggcactg caaagtttgg 240
   gggaactgaa tctatttaga gcagacttaa cagttgaaga agattttgat gctcctatag 300
   caggatgtga gcttgttttt caacttgcta cacctgtgaa ctttgcttct caagatcctg 360
   agaatgacat gataaagcca gcaatcaaag gtgtgttgaa tgtgttgaaa gcaagtgcaa 420
   gagcaaaaga agtcaaaaga gttatcttaa catcttcggc agccgcggtg actataaatg 480
   aactcaaagg gacaggtcat gttatggatg aaaccaactg gtcagatgtt gaatttctga 540
   acactgcaaa gccacccact tggggttatc ctgcttcaaa aatgctagct gaaaaggctg 600
   catggaaatt tgctgaagaa aatgacattg atctaatcac tgtgatacct agtttaacaa 660
   ctggtccttc tctcacacca gatatcccat ctagtgttgg cttggcaatg tctctaataa 720
   caggcaatga tttcctcata aatgctctga aaggaatgca atttctgtcg ggttcgttat 780
   ccatcactca tgttgaggat atttgccgag ctcatatatt tctggcagag aag 833
<210> 186
   <211> 256
   <212> PRT
   <213> Trifolium repens
<400> 186
   Met Ala Ser Ile Lys Gln Ile Gly Asn Lys Lys Ala Cys Val Ile Gly
   1 5 10 15
   Gly Thr Gly Phe Val Ala Ser Met Leu Ile Lys Gln Leu Leu Glu Lys
               20                  25                  30
   Gly Tyr Ala Val Asn Thr Thr Val Arg Asp Pro Asp Ser Pro Lys Lys
           35                  40                  45
   Ile Ser His Leu Val Ala Leu Gln Ser Leu Gly Glu Leu Asn Leu Phe
       50                  55                  60
   Arg Ala Asp Leu Thr Val Glu Glu Asp Phe Asp Ala Pro Ile Ala Gly
   65                  70                  75                  80
   Cys Glu Leu Val Phe Gln Leu Ala Thr Pro Val Asn Phe Ala Ser Gln
                   85                  90                  95
   Asp Pro Glu Asn Asp Met Ile Lys Pro Ala Ile Lys Gly Val Leu Asn
               100                 105                 110
   Val Leu Lys Ala Ser Ala Arg Ala Lys Glu Val Lys Arg Val Ile Leu
           115                 120                 125
   Thr Ser Ser Ala Ala Ala Val Thr Ile Asn Glu Leu Lys Gly Thr Gly
       130                 135                 140
   His Val Met Asp Glu Thr Asn Trp Ser Asp Val Glu Phe Leu Asn Thr
   145                 150                 155                 160
   Ala Lys Pro Pro Thr Trp Gly Tyr Pro Ala Ser Lys Met Leu Ala Glu
                   165                 170                 175
   Lys Ala Ala Trp Lys Phe Ala Glu Glu Asn Asp Ile Asp Leu Ile Thr
               180                 185                 190
   Val Ile Pro Ser Leu Thr Thr Gly Pro Ser Leu Thr Pro Asp Ile Pro
           195                 200                 205
   Ser Ser Val Gly Leu Ala Met Ser Leu Ile Thr Gly Asn Asp Phe Leu
       210                 215                 220
   Ile Asn Ala Leu Lys Gly Met Gln Phe Leu Ser Gly Ser Leu Ser Ile
   225                 230                 235                 240
   Thr His Val Glu Asp Ile Cys Arg Ala His Ile Phe Leu Ala Glu Lys
                   245                 250                 255
<210> 187
   <211> 576
   <212> DNA
   <213> Trifolium repens
<220>
   <221> misc_feature
   <222> (3) .. (3)
   <223> Any nucleotide
<220>
   <221> misc_feature
   <222> (34) .. (34)
   <223> Any nucleotide
<400> 187
   ggncataaaa actgcactag tgtgtataag tttnatagtg aaaaaagagt gtgtaaatta
   60
   acatcatggc tagtatcaaa caaattggaa acaagaaagc atgtgtgatt ggtggcactg
   120
   gttttgttgc atctatgttg atcaaacagt tacttgaaaa gggttatgct gttaatacta
   180
   ctgttagaga cccagatagt cctaagaaaa tatctcacct agtggcactg caaagtttgg
   240
   gggaactgaa tctatttaga gcagacttaa cagttgaaga agattttgat gctcctatag
   300
   caggatgtga gcttgttttt caacttgcta cacctgtgaa ctttgcttct caagatcctg
   360
   agaatgacat gataaagcca gcaatcaaag gtgtgttgaa tgtgttgaaa gcaagtgcaa
   420
   gagcaaaaga agtcaaaaga gttatcttaa catcttcggc agccgcggtg actataaatg
   480
   aactcaaagg gacaggtcat gttatggatg aaaccaactg gtcagatgtt gaatttctga
   540
   acactgcaaa gccacccact tggggttatc ctgctt
   576
<210> 188
   <211> 580
   <212> DNA
   <213> Trifolium repens
<220>
   <221> misc_feature
   <222> (3) .. (3)
   <223> Any nucleotide
<220>
   <221> misc_feature
   <222> (5) .. (5)
   <223> Any nucleotide
<220>
   <221> misc_feature
   <222> (34) .. (35)
   <223> Any nucleotides
<220>
   <221> misc_feature
   <222> (580) .. (580)
   <223> Any nucleotide
<400> 188
   ggncntaaaa actgcactag tgtgtataag tttnntagtg aaaaaagagt gtgtaaatta 60
   acatcatggc tagtatcaaa caaattggaa acaagaaagc atgtgtgatt ggtggcactg 120
   gttttgttgc atctatgttg atcaaacagt tacttgaaaa gggttatgct gttaatacta 180
   ctgttagaga cccagatagt cctaagaaaa tatctcacct agtggcactg caaagtttgg 240
   gggaactgaa tctatttaga gcagacttaa cagttgaaga agattttgat gctcctatag 300
   caggatgtga gcttgttttt caacttgcta cacctgtgaa ctttgcttct caagatcctg 360
   agaatgacat gataaagcca gcaatcaaag gtgtgttgaa tgtgttgaaa gcaagtgcaa 420
   gagcaaaaga agtcaaaaga gttatcttaa catcttcggc agccgcggtg actataaatg 480
   aactcaaagg gacaggtcat gttatggatg aaaccaactg gtcagatgtt gaatttctga 540
   acactgcaaa gccacccact tggggttatc ctgcttcaan 580
<210> 189
   <211> 578
   <212> DNA
   <213> Trifolium repens
<220>
   <221> misc_feature
   <222> (14) .. (14)
   <223> Any nucleotide
<220>
   <221> misc_feature
   <222> (29) .. (30)
   <223> Any nucleotides
<400> 189
   taaaaactgt actngtgtgt ataagtttnn tagtgaaaaa agagtgtgta aattaacatc 60
   atggctagta tcaaacaaat tggaaacaag aaagcatgtg tgattggtgg cactggtttt 120
   gttgcatcta tgttgatcaa acagttactt gaaaagggtt atgctgttaa tactactgtt 180
   agagacccag atagtcctaa gaaaatatct cacctagtgg cactgcaaag tttgggggaa 240
   ctgaatctat ttagagcaga cttaacagtt gaagaagatt ttgatgctcc tatagcagga 300
   tgtgagcttg tttttcaact tgctacacct gtgaactttg cttctcaaga tcctgagaat 360
   gacatgataa agccagcaat caaaggtgtg ttgaatgtgt tgaaagcaag tgcaagagca 420
   aaagaagtca aaagagttat cttaacatct tcggcagccg cggtgactat aaatgaactc 480
   aaagggacag gtcatgttat ggatgaaacc aactggtcag atgttgaatt tctgaacact 540
   gcaaagccac ccacttgggg ttatcctgct tcaaaaat 578
<210> 190
   <211> 619
   <212> DNA
   <213> Trifolium repens
<400> 190
   taaaaactgc actagtgtgt ataagtttct tggtgaaaaa agagtttgta aattaacatc 60
   atggctagta tcaaacaaat tggaaacaag aaagcatgtg tgattggtgg cactggtttt 120
   gttgcatcta tgttgatcaa gcagttactt gaaaagggtt atgctgttaa tactaccgtt 180
   agagacccag atagccctaa gaaaatatct cacctagtgg cactgcaaag tttgggggaa 240
   ctgaatctat ttagagcaga cttaacagtt gaagaagatt ttgatgctcc tatagcagga 300
   tgtgaacttg tttttcaact tgctacacct gtgaactttg cttctcaaga tcctgagaat 360
   gacatgataa agccagcaat caaaggtgtg ttgaatgtgt tgaaagcaat tgcaagagca 420
   aaagaagtta aaagagttat cttaacatct tcggcagccg cggtgactat aaatgaactc 480
   aaagggacag gtcatgttat ggatgaaacc aactggtctg atgttgaatt tctcaacact 540
   gcaaaaccac ccacttgggg ttatcctgcc tcaaaaatgc tagctgaaaa ggctgcatgg 600
   aaatttgctg aagaaaatg 619
<210> 191
   <211> 619
   <212> DNA
   <213> Trifolium repens
<220>
   <221> misc_feature
   <222> (12) .. (12)
   <223> Any nucleotide
<220>
   <221> misc_feature
   <222> (14) .. (14)
   <223> Any nucleotide
<220>
   <221> misc_feature
   <222> (45) .. (45)
   <223> Any nucleotide
<220>
   <221> misc_feature
   <222> (53) .. (53)
   <223> Any nucleotide
<220>
   <221> misc_feature
   <222> (59) .. (59)
   <223> Any nucleotide
<220>
   <221> misc_feature
   <222> (614) .. (614)
   <223> Any nucleotide
<400> 191
   gacctcgtgt gnantagttt cttggtgaaa aaagagtttg taaantaaca tcntggctng
   60
   tatcaaacaa attggaaaca agaaagcatg tgtgattggt ggcactggtt ttgttgcatc
   120
   tatgttgatc aagcagttac ttgaaaaggg ttatgctgtt aatactaccg ttagagaccc
   180
   agatagccct aagaaaatat ctcacctagt ggcactgcaa agtttggggg aactgaatct
   240
   atttagagca gacttaacag ttgaagaaga ttttgatgct cctatagcag gatgtgaact
   300
   tgtttttcaa cttgctacac ctgtgaactt tgcttctcaa gatcctgaga atgacatgat
   360
   aaagccagca atcaaaggtg tgttgaatgt gttgaaagca attgcaagag caaaagaagt
   420
   taaaagagtt atcttaacat cttcggcagc cgcggtgact ataaatgaac tcaaagggac
   480
   aggtcatgtt atggatgaaa ccaactggtc tgatgttgaa tttctcaaca ctgcaaaacc
   540
   acccacttgg ggttatcctg cctcaaaaat gctagctgaa aaggctgcat ggaaatttgc
   600
   tgaagaaaat gacnttgat
   619
<210> 192
   <211> 586
   <212> DNA
   <213> Trifolium repens
<220>
   <221> misc_feature
   <222> (2) .. (2)
   <223> Any nucleotide
<220>
   <221> misc_feature
   <222> (4) .. (4)
   <223> Any nucleotide
<220>
   <221> misc_feature
   <222> (23) .. (23)
   <223> Any nucleotide
<400> 192
   gngnacctga atctatttag agnagactta acagttgaag aagattttga tgctcctata 60
   gcaggatgtg agcttgtttt tcaacttgct acacctgtga cctttgcttc tcaagatcct 120
   gagaatgaca tgataaagcc agcaatcaaa ggtgtgttga atgtgttgaa agcaagtgca 180
   agagcaaaag aagtcaaaag agttatctta acatcttcgg cagccgcggt gactataaat 240
   gaactcaaag ggacaggtca tgttatggat gaaaccaact ggtcaggtgt tgaatttctg 300
   aacactgcaa agccacccac ttggggttat cctgcttcaa aaatgctagc tgaaaaggct 360
   gcatggaaat ttgctgaaga aaatgacatt gatctaatca ctgtgatacc tagtttaaca 420
   actggtcctt ctctcacacc agatatccca tctagtgttg gcttggcaat gtctctaata 480
   acaggcaatg atttcctcat aaatgctctg aaaggaatgc aatttctgtc gggttcgtta 540
   tccatcactc atgttgagga tatttgccga gctcatatat ttctgg 586
<210> 193
   <211> 567
   <212> DNA
   <213> Trifolium repens
<220>
   <221> misc_feature
   <222> (55) .. (55)
   <223> Any nucleotide
<400> 193
   ttaacagttg aagaagattt tgatgctcct atagcaggat gtgagcttgt ttttnaactt 60
   gctacacctg tgacctttgc ttctcaagat cctgagaatg acatgataaa gccagcaatc 120
   aaaggtgtgt tgaatgtgtt gaaagcaagt gcaagagcaa aagaagtcaa aagagttatc 180
   ttaacatctt cggcagccgc ggtgactata aatgaactca aagggacagg tcatgttatg 240
   gatgaaacca actggtcaga tgttgaattt ctgaacactg caaagccacc cacttggggt 300
   tatcctgctt caaaaatgct agctgaaaag gctgcatgga aatttgctga agaaaatgac 360
   attgatctaa tcactgtgat acctagttta acaactggtc cttctctcac accagatatc 420
   ccatctagtg ttggcttggc aatgtctcta ataacaggca atgatttcct cataaatgct 480
   ctgaaaggaa tgcaatttct gtcgggttcg ttatccatca ctcatgttga ggatatttgc 540
   cgagctcata tatttctggc agagaag 567
<210> 194
   <211> 597
   <212> DNA
   <213> Trifolium repens
<400> 194
   ggaaccaatt tgtcggactt ttttcccggg ttggcccgat tcgatttgca gggtgtggtg 60
   aaagagatgg atgtcttggt tccacgtttt gatagcatat ttgaaaaaat gattggtgaa 120
   cgtaagaaga aggaagtgga ggggaaagaa aatgaaagta aggattttct gcagtttttg 180
   ttgaatttga aggatgaggg tgattctaag actccattca caattaccca tgttaaggct 240
   ctactcatgg acatggttgt gggtggatca gacacatcct ccaacacaat tgagtttgca 300
   ttggcagaaa tgatgaacaa cccagaagta atgaggaagg ttcaagagga attagaagat 360
   gtagttggga aagataactt agtagaagag tctcacattc ataagctacc ctacttgcat 420
   gcagtgatga aagaaacact tcgtttacac ccagcacttc cacttttagt ccctcactgt 480
   ccaagtgaaa ccaccaatgt tggaggctac acaattccaa agggatctcg tgtgtttgtg 540
   aacgtttggg ctattcatag agacccttcc atttgggaga aaccactaga atttgat 597
<210> 195
   <211> 199
   <212> PRT
   <213> Trifolium repens
<400> 195
   Gly Thr Asn Leu Ser Asp Phe Phe Pro Gly Leu Ala Arg Phe Asp Leu
   1 5 10 15
   Gln Gly Val Val Lys Glu Met Asp Val Leu Val Pro Arg Phe Asp Ser
   20 25 30
   Ile Phe Glu Lys Met Ile Gly Glu Arg Lys Lys Lys Glu Val Glu Gly
   35 40 45
   Lys Glu Asn Glu Ser Lys Asp Phe Leu Gln Phe Leu Leu Asn Leu Lys
   50 55 60
   Asp Glu Gly Asp Ser Lys Thr Pro Phe Thr Ile Thr His Val Lys Ala
   65 70 75 80
   Leu Leu Met Asp Met Val Val Gly Gly Ser Asp Thr Ser Ser Asn Thr
   85 90 95
   Ile Glu Phe Ala Leu Ala Glu Met Met Asn Asn Pro Glu Val Met Arg
   100 105 110
   Lys Val Gln Glu Glu Leu Glu Asp Val Val Gly Lys Asp Asn Leu Val
   115 120 125
   Glu Glu Ser His Ile His Lys Leu Pro Tyr Leu His Ala Val Met Lys
   130 135 140
   Glu Thr Leu Arg Leu His Pro Ala Leu Pro Leu Leu Val Pro His Cys
   145 150 155 160
   Pro Ser Glu Thr Thr Asn Val Gly Gly Tyr Thr Ile Pro Lys Gly Ser
   165 170 175
   Arg Val Phe Val Asn Val Trp Ala Ile His Arg Asp Pro Ser Ile Trp
   180 185 190
   Glu Lys Pro Leu Glu Phe Asp
   195

<210> 196
   <211> 700
   <212> DNA
   <213> Trifolium repens
<220>
   <221> misc_feature
   <222> (2) .. (2)
   <223> Any nucleotide
<220>
   <221> misc_feature
   <222> (10) .. (10)
   <223> Any nucleotide
<220>
   <221> misc_feature
   <222> (22) .. (22)
   <223> Any nucleotide
<220>
   <221> misc_feature
   <222> (698) .. (698)
   <223> Any nucleotide
<400> 196
   gnaatccacn aatctcttga antaatacca tttctttaca agaacttaac catggtgatg
   60
   atcactcaat accaaacctt cctttacaaa gaactttcta tatccttttt cattttcttg
   120
   ataacccatt tcatcataag ttttctcttc aaaaaaaatc tcaaaaaact tccaccaggc
   180
   ccaaaaggtt ttccagttgt tggtgcactc ccactaatgg gatccatgcc tcatgttacc
   240
   ctattcaaaa tgtcacaaaa atatggtccc ataatgtacc taaaaatggg atcaaataac
   300
   atggttgtag catcaactcc ttcttcagcc aaagcatttc tcaaaacact tgacctaaat
   360
   ttctccaata gaccgccgaa cgctggcgca actcacctag cttatgattc acaagacttg
   420
   gttttcgccg actatggatc taggtggaaa ttacttagga aactaagtaa cttgcacatg
   480
   ctcggcggaa aagccctcga aaattggtcg aaagttcgtg agattgaaat gggtcacatg
   540
   attcgtacaa tgtacgattg tagcaagaaa gacgaatccg ttgttgtggc cgaaatgttg
   600
   acatatgcta tggccaatat gataggtcaa gttatattga gtcgtcgcgt gttcgagaca
   660
   aaaggtagtg actcaaatga atttaaggat atggttgntg
   700
<210> 197
   <211> 216
   <212> PRT
   <213> Trifolium repens
<220>
   <221> MISC_FEATURE
   <222> (216) .. (216)
   <223> Any amino acid
<400> 197
   Met Val Met Ile Thr Gln Tyr Gln Thr Phe Leu Tyr Lys Glu Leu Ser
   1               5                   10                  15
   Ile Ser Phe Phe Ile Phe Leu Ile Thr His Phe Ile Ile Ser Phe Leu
               20                  25                  30
   Phe Lys Lys Asn Leu Lys Lys Leu Pro Pro Gly Pro Lys Gly Phe Pro
           35                  40                  45
   Val Val Gly Ala Leu Pro Leu Met Gly Ser Met Pro His Val Thr Leu
       50                  55                  60
   Phe Lys Met Ser Gln Lys Tyr Gly Pro Ile Met Tyr Leu Lys Met Gly
   65                  70                  75                  80
   Ser Asn Asn Met Val Val Ala Ser Thr Pro Ser Ser Ala Lys Ala Phe
                   85                  90                  95
   Leu Lys Thr Leu Asp Leu Asn Phe Ser Asn Arg Pro Pro Asn Ala Gly
               100                 105                 110
   Ala Thr His Leu Ala Tyr Asp Ser Gln Asp Leu Val Phe Ala Asp Tyr
           115                 120                 125
   Gly Ser Arg Trp Lys Leu Leu Arg Lys Leu Ser Asn Leu His Met Leu
       130                 135                 140
   Gly Gly Lys Ala Leu Glu Asn Trp Ser Lys Val Arg Glu Ile Glu Met
   145                 150                 155                 160
   Gly His Met Ile Arg Thr Met Tyr Asp Cys Ser Lys Lys Asp Glu Ser
                   165                 170                 175
   Val Val Val Ala Glu Met Leu Thr Tyr Ala Met Ala Asn Met Ile Gly
               180                 185                 190
   Gln Val Ile Leu Ser Arg Arg Val Phe Glu Thr Lys Gly Ser Asp Ser
   195 200 205
   Asn Glu Phe Lys Asp Met Val Xaa
   210 215
<210> 198
   <211> 584
   <212> DNA
   <213> Trifolium repens
<220>
   <221> misc_feature
   <222> (2) .. (2)
   <223> Any nucleotide
<220>
   <221> misc_feature
   <222> (10) .. (10)
   <223> Any nucleotide
<220>
   <221> misc_feature
   <222> (27) .. (27)
   <223> Any nucleotide
<400> 198
   gnaatccacn aatctcttga attaatncca tttctttaca agaacttaac catggtgatg
   60
   atcactcaat accaaacctt ccttttcaaa gaactttcta tctccttttt cattttcttg
   120
   ataacccgtt tcatcataag ttttctcttc aaaaaaaatc tcaaaaaact tccaccaggc
   180
   ccaaagggtt ttccagttgt tggtgcactc ccactaatgg gatccatgcc tcatgttacc
   240
   ctattcaaaa tgtcacaaaa atatggtccc ataatgtacc taaaaatggg atcaaatagc
   300
   atggttgtag catcaactcc ttcttcagcc aaagcatttc tcaaaacact tgacctaaat
   360
   ttctccaata gaccgccgaa cgctggcgcg actcacctag cttatgattc acaagacttg
   420
   gttttcgccg actatggatc taggtggaaa ttacttagga aactaagtaa cttgcacatg
   480
   ctcggcggaa aagccctcga agattggtcg aaagttcgtg agattgaaat gggtcacatg
   540
   attcgtacaa tgtatgattg tagcaagaaa gacgaatccg ttgt
   584
<210> 199
   <211> 694
   <212> DNA
   <213> Trifolium repens
<220>
   <221> misc_feature
   <222> (2) .. (2)
   <223> Any nucleotide
<220>
   <221> misc_feature
   <222> (16) .. (16)
   <223> Any nucleotide
<220>
   <221> misc_feature
   <222> (20) .. (21)
   <223> Any nucleotides
<220>
   <221> misc_feature
   <222> (692) .. (692)
   <223> Any nucleotide
<400> 199
   gncaaatctc ttgcantaan nccatttctt tacaagaact taaccatggt gatgatcact 60
   caacaccaaa ccttccttta caaagaactt tctatatcct ttttcatttt cttgataacc 120
   catttcatca ttagttttct cttcaaaaaa aatctcaaaa aacttccacc aggcccaaaa 180
   ggttttccag ttgttggtgc actcccacta atgggatcca tgcctcatgt taccctattc 240
   aaaatgtcac aaaaatatgg tcctataatg tacctaaaaa tgggatcaaa taacatggtt 300
   gtagcatcaa ctccttcttc agccaaagca tttctcaaaa cacttgacct aaatttctcc 360
   aataggccgg cgaacgctgg cgcaactcac ctagcttatg atccacaaga cttggttttc 420
   gccgactatg gatctaggtg gaaattactt aggaaactaa gtaacttgca catgctcggc 480
   ggaaaagccc ttgaaaattg gtcgaaagtt cgtgagattg aaatgggtca catgattcgt 540
   acaatgtacg attgtagcaa gaaagacgaa tccgttgttg tggccgaaat gttgacatat 600
   gctatggcca atatgatagg tcaagttata ttgagtcgtc gcgtgttcga gacaaaaggt 660
   agtgactcaa atgaatttaa ggatatggtt gntg 694
<210> 200
   <211> 580
   <212> DNA
   <213> Trifolium repens
<220>
   <221> misc_feature
   <222> (2) .. (2)
   <223> Any nucleotide
<220>
   <221> misc_feature
   <222> (14) .. (14)
   <223> Any nucleotide
<220>
   <221> misc_feature
   <222> (16) .. (16)
   <223> Any nucleotide
<220>
   <221> misc_feature
   <222> (44) .. (44)
   <223> Any nucleotide
<220>
   <221> misc_feature
   <222> (55) .. (56)
   <223> Any nucleotides
<400> 200
   cnaatctctt gaantnatac catttcttta caagaactta accntggtga tgatnnctca 60
   ataccaaacc ttccttttca aagaactttc tatctccttt ttcattttct tgataacccg 120
   tttcatcata agtcttctct tcaaaaaaaa tctcaaaaaa cttccaccag gcccaaaggg 180
   ttttccagtt gttggtgcac tcccactaat gggatccatg cctcatgtta ccctattcaa 240
   aatgtcacaa aaatatggtc ccataatgta cctaaaaatg ggatcaaata acatggttgt 300
   agcatcaact ccttcttcag ccaaagcatt tctcaaaaca cttgacctaa atttctccaa 360
   tagaccgccg aacgctggcg cgactcacct agcttatgat tcacaagact tggttttcgc 420
   cgactatgga tctaggtgga aattgcttag gaaactaagt aacttgcaca tgctcggcgg 480
   aaaagccctc gaagattggt cgaaagttcg cgagattgag atgggtcaca tgattcgtac 540
   aatgtacgat tgtagcaaga aagacgaata tgttgttgtg 580
<210> 201
   <211> 574
   <212> DNA
   <213> Trifolium repens
<220>
   <221> misc_feature
   <222> (8) .. (8)
   <223> Any nucleotide
<220>
   <221> misc_feature
   <222> (12) .. (12)
   <223> Any nucleotide
<220>
   <221> misc_feature
   <222> (40) .. (40)
   <223> Any nucleotide
<220>
   <221> misc_feature
   <222> (51) .. (52)
   <223> Any nucleotides
<220>
   <221> misc_feature
   <222> (56) .. (56)
   <223> Any nucleotide
<220>
   <221> misc_feature
   <222> (569) .. (569)
   <223> Any nucleotide
<220>
   <221> misc_feature
   <222> (572) .. (572)
   <223> Any nucleotide
<400> 201
   tctcttgnaa tnataccatt tctttacaag aacttaaccn tggtgatgat nnctcnatac 60
   caaaccttcc tttacaaaga actttctata tcctttttca ttttcttgat aacccatttc 120
   atcattagtt ttctcttcaa aaaaaatctc aaaaaacttc caccaggccc aaaaggtttt 180
   ccagttgttg gtgcactccc actaatggga tccatgcctc atgttaccct attcaaaatg 240
   tcacaaaaat atggtcctat aatgtaccta aaaatgggat caaataacat ggttgtagca 300
   tcaactcctt cttcagccaa agcatttctc aaaacacttg acctaaattt ctccaatagg 360
   ccggcgaacg ctggcgcaac tcacctagct tatgattcac aagacttggt tttcgccgac 420
   tatggatcta ggtggaaatt acttaggaaa ctaagtaact tgcacatgct cggcggaaaa 480
   gcccttgaaa attggtcgaa agttcgtgag attgaaatgg gtcacatgat tcgtacaatg 540
   tacgattgta gcaagaaaga cgaatccgnt gntg 574
<210> 202
   <211> 1261
   <212> DNA
   <213> Trifolium repens
<220>
   <221> misc_feature
   <222> (7) .. (7)
   <223> Any nucleotide
<220>
   <221> misc_feature
   <222> (31) .. (31)
   <223> Any nucleotide
<220>
   <221> misc_feature
   <222> (43) .. (43)
   <223> Any nucleotide
<220>
   <221> misc_feature
   <222> (92) .. (93)
   <223> Any nucleotides
<220>
   <221> misc_feature
   <222> (96) .. (96)
   <223> Any nucleotide
<220>
   <221> misc_feature
   <222> (957) .. (957)
   <223> Any nucleotide
<400> 202
   gcacacntct atttatttct acttaaacct nacaaaaaat aanacccaca aaacacaaac
   60
   accacaaaca ccaaaaccga gtccgtttcc tnntcnaaca tggcaccaag ccaaactcta
   120
   agttatctct cacaacaaaa cactcttgag tcaagtttcg ttagggaaga agatgagcgt
   180
   ccaaaagttg cctacaataa cttcagcaac gagattccaa tcatttctct tgctggaatt
   240
   gatgaggttg atggtcgtag aacagagata tgtaacaaga ttgttgaagc ttgtgagaat
   300
   tggggtattt ttcaggttgt tgatcatggt gttgatacaa aacttgtttc tgagatgacc
   360
   cgttttgcta gagagttttt tgctttgcca ccggaagaga agctccggtt tgacatgtcc
   420
   ggtggtaaaa agggtggttt cattgtctct agtcatctcc aaggagaagc agtgaaggat
   480
   tggagagagc tagtgacata tttttcatac ccaattaaac aaagagatta ttcaaggtgg
   540
   ccagacaagc cagaaggatg gaaagaggta acagaaaaat acagtgaaaa cctaatgaat
   600
   ttagcttgca aactattgga agttttatca gaagcaatgg gtttagaaaa agaagctcta
   660
   acaaaagcat gtgttgatat ggatcaaaaa gttgttataa attattaccc aaaatgccct
   720
   gaacctgacc tcacacttgg ccttaaacgt cacactgacc ctggcacaat tactcttttg
   780
   cttcaagatc aagttggtgg ccttcaagct accaaagata atggtaagac gtggattaca
   840
   gttcaaccag ttgaaggtgc ttttgttgtt aatcttggag accatggtca ctatctaagt
   900
   aatggacggt tcaaaaatgc tgaccaccaa gcagtggtga attcgaacta cagccgntta
   960
   tcaatagcaa catttcaaaa tccagctccc gatgcaactg tatacccttt gaagattaga
   1020
   gagggtgaaa aatctgtgtt ggaagaacca atcacttttg ctgaaatgta tagaaggaag
   1080
   atgaccaaag accttgaaat tgctaggatg aagaagttgg ctaaggaaca acaacttagg
   1140
   gacttggagg agaacaagac taaatatgag gccaaacctt tgaatgagat ctttgcttaa
   1200
   ttaattagtc ttaatttaaa taattaataa attttagact taatttacat ataataattt
   1260
   t
   1261
<210> 203
   <211> 366
   <212> PRT
   <213> Trifolium repens
<220>
   <221> MISC_FEATURE
   <222> (286) .. (286)
   <223> Any amino acid
<400> 203
   Met Ala Pro Ser Gln Thr Leu Ser Tyr Leu Ser Gln Gln Asn Thr Leu
   1               5                   10                  15
   Glu Ser Ser Phe Val Arg Glu Glu Asp Glu Arg Pro Lys Val Ala Tyr
               20                  25                  30
   Asn Asn Phe Ser Asn Glu Ile Pro Ile Ile Ser Leu Ala Gly Ile Asp
           35                  40                  45
   Glu Val Asp Gly Arg Arg Thr Glu Ile Cys Asn Lys Ile Val Glu Ala
       50                  55                  60
   Cys Glu Asn Trp Gly Ile Phe Gln Val Val Asp His Gly Val Asp Thr
   65                  70                  75                  80
   Lys Leu Val Ser Glu Met Thr Arg Phe Ala Arg Glu Phe Phe Ala Leu
                   85                  90                  95
   Pro Pro Glu Glu Lys Leu Arg Phe Asp Met Ser Gly Gly Lys Lys Gly
               100                 105                 110
   Gly Phe Ile Val Ser Ser His Leu Gln Gly Glu Ala Val Lys Asp Trp
           115                 120                 125
   Arg Glu Leu Val Thr Tyr Phe Ser Tyr Pro Ile Lys Gln Arg Asp Tyr
       130                 135                 140
   Ser Arg Trp Pro Asp Lys Pro Glu Gly Trp Lys Glu Val Thr Glu Lys
   145                 150                 155                 160
   Tyr Ser Glu Asn Leu Met Asn Leu Ala Cys Lys Leu Leu Glu Val Leu
                   165                 170                 175
   Ser Glu Ala Met Gly Leu Glu Lys Glu Ala Leu Thr Lys Ala Cys Val
               180                 185                 190
   Asp Met Asp Gln Lys Val Val Ile Asn Tyr Tyr Pro Lys Cys Pro Glu
           195                 200                 205
   Pro Asp Leu Thr Leu Gly Leu Lys Arg His Thr Asp Pro Gly Thr Ile
       210                 215                 220
   Thr Leu Leu Leu Gln Asp Gln Val Gly Gly Leu Gln Ala Thr Lys Asp
   225                 230                 235                 240
   Asn Gly Lys Thr Trp Ile Thr Val Gln Pro Val Glu Gly Ala Phe Val
                   245                 250                 255
   Val Asn Leu Gly Asp His Gly His Tyr Leu Ser Asn Gly Arg Phe Lys
           260                 265                 270
   Asn Ala Asp His Gln Ala Val Val Asn Ser Asn Tyr Ser Xaa Leu Ser
           275                 280                 285
   Ile Ala Thr Phe Gln Asn Pro Ala Pro Asp Ala Thr Val Tyr Pro Leu
       290                 295                 300
   Lys Ile Arg Glu Gly Glu Lys Ser Val Leu Glu Glu Pro Ile Thr Phe
   305                 310                 315                 320
   Ala Glu Met Tyr Arg Arg Lys Met Thr Lys Asp Leu Glu Ile Ala Arg
                   325                 330                 335
   Met Lys Lys Leu Ala Lys Glu Gln Gln Leu Arg Asp Leu Glu Glu Asn
               340                 345                 350
   Lys Thr Lys Tyr Glu Ala Lys Pro Leu Asn Glu Ile Phe Ala
           355                 360                 365
<210> 204
   <211> 586
   <212> DNA
   <213> Trifolium repens
<220>
   <221> misc_feature
   <222> (7) .. (7)
   <223> Any nucleotide
<400> 204
   gcacacntct atttatttct acttaaacct cacaaaaaat aaacccacaa cacacaaaca 60
   ccaaaaacag agcaccgttt ccatcatcaa acatggcacc aagccaaact ctaagttatc 120
   tctcacaaca aaacactctc gagtcaagtt tcgttaggga agaagatgag cgtccaaaag 180
   ttgcctacaa taacttcagc aacgagattc caatcatttc tcttgctgga attgatgagg 240
   ttgatggtcg tagaacagag atatgtaaca agattgttga agcttgtgag aattggggta 300
   tttttcaggt tgttgatcat ggtgttgata caaaacttgt ttctgagatg acccgttttg 360
   ctagagagtt ttttgctttg ccaccggaag agaagctccg gtttgacatg tccggtggta 420
   aaaagggtgg tttcattgtc tctagtcatc ttcaaggaga agcagtgaag gattggagag 480
   agctagtgac atatttttca tacccaatta aacaaagaga ttattcaagg tggccagaca 540
   agccagaagg atggaaagag gtaacagaaa aatacagtga aaacct 586

<210> 205
   <211> 597
   <212> DNA
   <213> Trifolium repens
<220>
   <221> misc_feature
   <222> (2) .. (2)
   <223> Any nucleotide
<220>
   <221> misc_feature
   <222> (5) .. (5)
   <223> Any nucleotide
<220>
   <221> misc_feature
   <222> (7) .. (7)
   <223> Any nucleotide
<220>
   <221> misc_feature
   <222> (31) .. (31)
   <223> Any nucleotide
<400> 205
   gcacngntct atttatttct acttaaacct naaaaataaa cccacaacac aaaccaaaaa 60
   cagagcaccg tttccatcat caaacatggc accaagccaa accctaagtt atctctcaca 120
   acaaaagact cttgagtcaa gtttcgttag ggaagaagat gagcgtccaa aagttgccta 180
   caataacttc agcaacgaga ttccaatcat ttctcttgct ggaattgatg aggttgatgg 240
   tcgcagaaca gagatatgta acaagattgt tgaagcttgt gagaattggg gtatttttca 300
   ggttgttgat catggtgttg atacaaagct tgtttctgag atgactcgtt ttgctagaga 360
   gttttttgct ttgccgccgg aagagaagct ccggtttgac atgtccggtg gtaaaaaggg 420
   tggtttcatt gtctctagtc atctccaagg agaagcagtg aaagattgga gagagctagt 480
   gacatatttt tcatacccaa ttaaacaaag agattattca aggtggccag acaagccaga 540
   agaatggaaa gaagtaacag aaaaatacag tgaaaaccta atgaatttag cttgcag 597
<210> 206
   <211> 605
   <212> DNA
   <213> Trifolium repens
<220>
   <221> misc_feature
   <222> (6) .. (6)
   <223> Any nucleotide
<220>
   <221> misc_feature
   <222> (30) .. (30)
   <223> Any nucleotide
<220>
   <221> misc_feature
   <222> (42) .. (42)
   <223> Any nucleotide
<220>
   <221> misc_feature
   <222> (71) .. (71)
   <223> Any nucleotide
<220>
   <221> misc_feature
   <222> (90) .. (92)
   <223> Any nucleotides
<220>
   <221> misc_feature
   <222> (94) .. (95)
   <223> Any nucleotides
<400> 206
   cacacntcta tttatttcta cttaaacctn acaaaaaata anacccacaa cacacaaaca
   60
   ccaaaaacag nagcaccgtt tccatcatcn nnannaacat ggcaccaagc caaactctaa
   120
   gttatctctc acaacaaaac actctcgagt caagtttcgt tagggaagaa gatgagcgtc
   180
   caaaagttgc ctacaataac ttcagcaacg agattccaat catttctctt gctggaattg
   240
   atgaggttga tggtcgtaga acagagatat gtaacaagat tgttgaagct tgtgagaatt
   300
   ggggtatttt tcaggttgtt gatcatggtg ttgatacaaa acttgtttct gagatgaccc
   360
   gttttgctag agagtttttt gctttgccac cggaagagaa gctccggttt gacatgtccg
   420
   gtggtaaaaa gggtggtttc attgtctcta gtcatcttca aggagaagca gtgaaggatt
   480
   ggagagagct agtgacatat ttttcatacc caattaaaca aagagattat tcaaggtggc
   540
   cagacaagcc agaaggatgg aaagaggtaa cagaaaaata cagtgaaaac ctaatgaatt
   600
   tagct
   605
<210> 207
   <211> 492
   <212> DNA
   <213> Trifolium repens
<220>
   <221> misc_feature
   <222> (10) .. (10)
   <223> Any nucleotide
<220>
   <221> misc_feature
   <222> (19) .. (19)
   <223> Any nucleotide
<220>
   <221> misc_feature
   <222> (29) .. (30)
   <223> Any nucleotides
<220>
   <321> misc_feature
   <222> (38) .. (38)
   <223> Any nucleotide
<220>
   <221> misc_feature
   <222> (40) .. (41)
   <223> Any nucleotides
<220>
   <221> misc_feature
   <222> (55) .. (55)
   <223> Any nucleotide
<220>
   <221> misc_feature
   <222> (58) .. (58)
   <223> Any nucleotide
<220>
   <221> misc_feature
   <222> (60) .. (60)
   <223> Any nucleotide
<220>
   <221> misc_feature
   <222> (68) .. (68)
   <223> Any nucleotide
<220>
   <221> misc_feature
   <222> (70) .. (70)
   <223> Any nucleotide
<220>
   <221> misc_feature
   <222> (75) .. (75)
   <223> Any nucleotide
<220>
   <221> misc_feature
   <222> (436) .. (436)
   <223> Any nucleotide
<220>
   <221> misc_feature
   <222> (439) .. (439)
   <223> Any nucleotide
<220>
   <221> misc_feature
   <222> (450) .. (450)
   <223> Any nucleotide
<220>
   <221> misc_feature
   <222> (452) .. (452)
   <223> Any nucleotide
<220>
   <221> misc_feature
   <222> (455).. (455)
   <223> Any nucleotide
<220>
   <221> misc_feature
   <222> (459) .. (459)
   <223> Any nucleotide
<220>
   <221> misc_feature
   <222> (469).. (469)
   <223> Any nucleotide
<220>
   <221> misc_feature
   <222> (471) .. (471)
   <223> Any nucleotide
<220>
   <221> misc_feature
   <222> (473) .. (473)
   <223> Any nucleotide
<220>
   <221> misc_feature
   <222> (490).. (492)
   <223> Any nucleotides
<400> 207
   tcccctctan ttatttctnc ttaaacctnn caaaaatnan nacccacaac acacnaanan 60
   caccaaanan acagnagtac cgtttccatc atcaaacatg gcaccaagcc aaactctaag 120
   ttatctctca caacaaaaca ctctcgagtc aagtttcgtt agggaagaag atgagcgtcc 180
   aaaagttgcc tacaataact tcagcaacga gattccaatc atttctcttg ctggaattga 240
   tgaggttgat ggtcgtagaa cagagatatg taacaagatt gttgaagctt gtgagaattg 300
   gggtattttt caggttgttg atcatggtgt tgatacaaaa cttgtttctg agatgacccg 360
   ttttgctaga gagttttttg ctttgccacc ggaagagaag ctccggtttg acatgtccgg 420
   tggtaaaaag ggtggnttna ttgtctgtan tngtntccna ggacaaccng ngnaggattg 480
   cagagagctn nn 492
<210> 208
   <211> 690
   <212> DNA
   <213> Trifolium repens
<220>
   <221> misc_feature
   <222> (11) .. (11)
   <223> Any nucleotide
<220>
   <221> misc_feature
   <222> (34) .. (34)
   <223> Any nucleotide
<220>
   <221> misc_feature
   <222> (36) .. (36)
   <223> Any nucleotide
<220>
   <221> misc_feature
   <222> (49) .. (49)
   <223> Any nucleotide
<400> 208
   gttaacacac nccaacacaa acaccaaaaa cagnancacc gtttccatnc atcaaacatg 60
   gcaccaagcc aaactctaag ttatctctca caacaaaaca ctctcgagtc aagtttcgtt 120
   agggaagaag atgagcgtcc aaaagttgcc tacaataact tcagcaacga gattccaatc 180
   atttctcttg ctggaattga tgaggttgat ggtcgtagaa cagagatatg taacaagatt 240
   gttgaagctt gtgagaattg gggtattttt caggttgttg atcatggtgt tgatacaaaa 300
   cttgtttctg agatgacccg ttttgctaga gagttttttg ctttgccacc ggaagagaag 360
   ctccggtttg acatgtccgg tggtaaaaag ggtggtttca ttgtctctag tcatctccaa 420
   ggagaagcag tgaaggattg gagagagcta gtgacatatt tttcataccc aattaaacaa 480
   agagattatt caaggtggcc agacaagcca gaaggatgga aagaggtaac agaaaaatac 540
   agtgaaaacc taatgaattt agcttgcaaa ctattggaag ttttatcaga agcaatgggt 600
   ttagaaaaag aagctctaac aaaagcatgt gttgatatgg atcaaaaagt tgttataaat 660
   tattacccaa aatgccctga acctgacctt 690
<210> 209
   <211> 573
   <212> DNA
   <213> Trifolium repens
<220>
   <221> misc_feature
   <222> (2) .. (2)
   <223> Any nucleotide
<220>
   <221> misc_feature
   <222> (33) .. (33)
   <223> Any nucleotide
<220>
   <221> misc_feature
   <222> (41) .. (41)
   <223> Any nucleotide
<220>
   <221> misc_feature
   <222> (43) .. (43)
   <223> Any nucleotide
<400> 209
   gnaacccaca acacacaaac accaaaacag agnccgtttc ntnatcaaac atggcaccaa 60
   gccaaactct aagttatctc tcacaacaaa acactctcga gtcaagtttc gttagggaag 120
   aagatgagcg tccaaaagtt gcctacaata acttcagcaa cgagattcca atcatttctc 180
   ttgctggaat tgatgaggtt gatggtcgta gaacagagat atgtaacaag attgttgaag 240
   cttgtgagaa ttggggtatt tttcaggttg ttgatcatgg tgttgataca aaacttgttt 300
   ctgagatgac ccgttttgct agagagtttt ttgctttgcc accggaagag aagctccggt 360
   ttgacatgtc cggtggtaaa aagggtggtt tcattgtctc tagtcatctc caaggagaag 420
   cagtgaagga ttggagagag ctagtgacat atttttcata cccaattaaa caaagagatt 480
   attcaaggtg gccagacaag ccagaaggat ggaaagaggt aacagaaaaa tacagtgaaa 540
   acctaatgaa tttagcttgc aagctattgg aag 573
<210> 210
   <211> 716
   <212> DNA
   <213> Trifolium repens
<220>
   <221> misc_feature
   <222> (6) .. (6)
   <223> Any nucleotide
<220>
   <221> misc_feature
   <222> (10) .. (10)
   <223> Any nucleotide
<220>
   <221> misc_feature
   <222> (28) .. (28)
   <223> Any nucleotide
<220>
   <221> misc_feature
   <222> (30) .(30)
   <223> Any nucleotide
<220>
   <221> misc_feature
   <222> (43)). (43)
   <223> Any nucleotide
<220>
   <221> misc_feature
   <222> (47).. (47)
   <223> Any nucleotide
<220>
   <221> misc_feature
   <222> (576) .. (576)
   <223> Any nucleotide
<220>
   <221> misc_feature
   <222> (619) .. (619)
   <223> Any nucleotide
<220>
   <221> misc_feature
   <222> (702) .. (702)
   <223> Any nucleotide
<220>
   <221> misc_feature
   <222> (714) .. (714)
   <223> Any nucleotide
<400> 210
   cacacnaacn ccaaacacca aaaacagnan caccgtttcc atncatncaa acatggcacc 60
   aagccaaact ctaagttatc tctcacaaca aaacactctt gagtcaagtt tcgttaggga 120
   agaagatgag cgtccaaaag ttgcctacaa taacttcagc aacgagattc caatcatttc 180
   tcttgctgga attgatgagg ttgatggtcg tagaacagag atatgtaaca agattgttga 240
   agcttgtgag aattggggta tttttcaggt tgttgatcat ggtgttgata caaaacttgt 300
   ttctgagatg acccgttttg ctagagagtt ttttgctttg ccaccggaag agaagctccg 360
   gtttgacatg tccggtggta aaaagggtgg tttcattgtc tctagtcatc tccaaggaga 420
   agcagtgaag gattggagag agctagtgac atatttttca tacccaatta aacaaagaga 480
   ttattcaagg tggccagaca agccagaagg atggaaagag gtaacagaaa aatacagtga 540
   aaacctaatg aatttagctt gcaaactatt ggaagnttta tcagaagcaa tgggtttaga 600
   aaaagaagct ctaacaaang catgtgttga tatggatcaa aaagttgtta taaattatta 660
   cccaaaatgc cctgaacctg acctcccact tgccctaaac gncacactga cccnga 716
<210> 211
   <211> 721
   <212> DNA
   <213> Trifolium repens
<220>
   <221> misc_feature
   <222> (4) .. (4)
   <223> Any nucleotide
<220>
   <221> misc_feature
   <222> (7) .. (7)
   <223> Any nucleotide
<220>
   <221> misc_feature
   <222> (28) .. (28)
   <223> Any nucleotide
<220>
   <221> misc_feature
   <222> (30) .. (30)
   <223> Any nucleotide
<220>
   <221> misc_feature
   <222> (43) . (43)
   <223> Any nucleotide
<220>
   <221> misc_feature
   <222> (721) .. (721)
   <223> Any nucleotide
<400> 211
   cacnacnacc acaaacacca aaaacagnan caccgtttcc atncatcaaa catggcacca 60
   agccaaactc taagttatct ctcacaacaa aacactcttg agtcaagttt cgttagggaa 120
   gaagatgagc gtccaaaagt tgcctacaat aacttcagca acgagattcc aatcatttct 180
   cttgctggaa ttgatgaggt tgatggtcgt agaacagaga tatgtaacaa gattgttgaa 240
   gcttgtgaga attggggtat ttttcaggtt gttgatcatg gtgttgatac aaaacttgtt 300
   tctgagatga cccgttttgc tagagagttt tttgctttgc caccggaaga gaagctccgg 360
   tttgacatgt ccggtggtaa aaagggtggt ttcattgtct ctagtcatct ccaaggagaa 420
   gcagtgaagg attggagaga gctagtgaca tatttttcat acccaattaa acaaagagat 480
   tattcaaggt ggccagacaa gccagaagga tggaaagagg taacagaaaa atacagtgaa 540
   aacctaatga atttagcttg caaactattg gaagttttat cagaagcaat gggtttagaa 600
   aaagaagctc taacaaaagc atgtgttgat atggatcaaa aagttgttat aaattattac 660
   ccaaaatgcc ctgaacctga cctcacactt ggccttaaac gtcacactga ccctggcaca 720
   n 721
<210> 212
   <211> 453
   <212> DNA
   <213> Trifolium repens
<220>
   <221> misc_feature
   <222> (6) .. (6)
   <223> Any nucleotide
<220>
   <221> misc_feature
   <222> (79) .. (79)
   <223> Any nucleotide
<220>
   <221> misc_feature
   <222> (173) .. (173)
   <223> Any nucleotide
<220>
   <221> misc_feature
   <222> (215) .. (215)
   <223> Any nucleotide
<220>
   <221> misc_feature
   <222> (220) .. (220)
   <223> Any nucleotide
<220>
   <221> misc_feature
   <222> (235) .. (235)
   <223> Any nucleotide
<220>
   <221> misc_feature
   <222> (245) .. (245)
   <223> Any nucleotide

<220>
   <221> misc feature
   <222> (257) .. (257)
   <223> Any nucleotide
<220>
   <221> misc_feature
   <222> (283) .. (284)
   <223> Any nucleotides
<220>
   <221> misc_feature
   <222> (303) .. (303)
   <223> Any nucleotide
<220>
   <221> misc_feature
   <222> (307) .. (307)
   <223> Any nucleotide
<220>
   <221> misc feature
   <222> (311) .. (311)
   <223> Any nucleotide
<220>
   <221> misc_feature
   <222> (317) .. (317)
   <223> Any nucleotide
<220>
   <221> misc_feature
   <222> (322) .. (322)
   <223> Any nucleotide
<220>
   <221> misc_feature
   <222> (336) .. (336)
   <223> Any nucleotide
<220>
   <221> misc_feature
   <222> (346) .. (346)
   <223> Any nucleotide
<220>
   <221> misc_feature
   <222> (361) .. (361)
   <223> Any nucleotide
<220>
   <221> misc_feature
   <222> (366) .. (367)
   <223> Any nucleotides
<220>
   <221> misc_feature
   <222> (375) .. (375)
   <223> Any nucleotide
<220>
   <221> misc_feature
   <222> (393) .. (393)
   <223> Any nucleotide
<220>
   <221> misc_feature
   <222> (397) .. (397)
   <223> Any nucleotide
<220>
   <221> misc_feature
   <222> (399) .. (399)
   <223> Any nucleotide
<220>
   <221> misc_feature
   <222> (401) .. (401)
   <223> Any nucleotide
<220>
   <221> misc_feature
   <222> (407) .. (407)
   <223> Any nucleotide
<220>
   <221> misc_feature
   <222> (411) .. (411)
   <223> Any nucleotide
<220>
   <221> misc_feature
   <222> (422) .. (422)
   <223> Any nucleotide
<220>
   <221> misc_feature
   <222> (425) .. (425)
   <223> Any nucleotide
<220>
   <221> misc_feature
   <222> (430) .. (432)
   <223> Any nucleotides
<220>
   <221> misc_feature
   <222> (437) .. (437)
   <223> Any nucleotide
<220>
   <221> misc_feature
   <222> (445).. (445)
   <223> Any nucleotide
<220>
   <221> misc_feature
   <222> (447) .. (448)
   <223> Any nucleotides
<220>
   <221> misc_feature
   <222> (450) .. (453)
   <223> Any nucleotides
<400> 212
   ccacancaca caaacaccaa aaacagaagc accgtttcca tcatcaaaca tggcaccaag 60
   ccaaactcta agttatctng gacaacaaaa cactctcgag tcaagtttcg ttagggaaga 120
   agatgaccgt ccaaaagttg cctacaatac cttcagcacc gagattccaa tcntttctct 180
   tgctggaatt gatgaggttg atggccgaaa aacanaaatn tttaccaaga ttggnggggc 240
   ttgtnaaaat tggggtnttt ttcaggttgg tgatcatggg gtnnaacaaa acttgtttcc 300
   canaaanccc nttttgntaa anagtttttt gctttncccc cggaanaaaa cctccggttt 360
   nacttnnccg ggggnaaaaa aggggggggt ttnattngnc nttaagnccc nccccaaggg 420
   anaancccan nnaaggnttt ggaananncn nnn 453
<210> 213
   <211> 267
   <212> DNA
   <213> Trifolium repens
<220>
   <221> misc_feature
   <222> (4) .. (4)
   <223> Any nucleotide
<220>
   <221> misc_feature
   <222> (10) .. (10)
   <223> Any nucleotide
<220>
   <221> misc_feature
   <222> (15) ..(15)
   <223> Any nucleotide
<220>
   <221> misc_feature
   <222> (23) .. (23)
   <223> Any nucleotide
<220>
   <221> misc_feature
   <222> (30) .. (30)
   <223> Any nucleotide
<220>
   <221> misc_feature
   <222> (45) .. (46)
   <223> Any nucleotides
<220>
   <221> misc_feature
   <222> (48) .. (49)
   <223> Any nucleotides
<220>
   <221> misc_feature
   <222> (208) .. (208)
   <223> Any nucleotide
<220>
   <221> misc_feature
   <222> (210) .. (210)
   <223> Any nucleotide
<220>
   <221> misc_feature
   <222> (215) .. (215)
   <223> Any nucleotide
<220>
   <221> misc_feature
   <222> (226) .. (227)
   <223> Any nucleotides
<220>
   <221> misc_feature
   <222> (231) .. (231)
   <223> Any nucleotide
<220>
   <221> misc_feature
   <222> (245) .. (245)
   <223> Any nucleotide
<220>
   <221> misc_feature
   <222> (250) .. (250)
   <223> Any nucleotide
<220>
   <221> misc_feature
   <222> (253) .. (253)
   <223> Any nucleotide
<220>
   <221> misc_feature
   <222> (260) .. (260)
   <223> Any nucleotide
<220>
   <221> misc_feature
   <222> (265) .. (265)
   <223> Any nucleotide
<220>
   <221> misc_feature
   <222> (267) .. (267)
   <223> Any nucleotide
<400> 213
   gaancccacn aacancaaac acncaaaaan agagaccgtt tcctnntnna acatggcacc 60
   aagccaaact ctaagttatc tctcacaaca aaacactctt gagtcaagtt tcgttaggga 120
   agaagatgag cgtccaaaag ttgcctacaa taacttcagc aacgagattc caatcatttc 180
   tcttgctgga attgatgagg ttgatggncn cacancacac atctgnncca nattgctgga 240
   acctngcgan agncgccctn cctcncn 267
<210> 214
   <211> 580
   <212> DNA
   <213> Trifolium repens
<220>
   <221> misc_feature
   <222> (2) .. (2)
   <223> Any nucleotide
<220>
   <221> misc_feature
   <222> (38) .. (38)
   <223> Any nucleotide
<400> 214
   gnaccacaca aacacccaaa acagagcacc gtttcctnat caacaatggc accaagccaa 60
   actctaagtt atctctcaca acaaaacact ctcgagtcaa gtttcgttag ggaagaagat 120
   gagcgtccaa aagttgccta caataacttc agcaacgaga ttccaatcat ttctcttgct 180
   ggaattgatg aggttgatgg tcgtagaaca gagatatgta acaagattgt tgaagcttgt 240
   gagaattggg gtatttttca ggttgttgat catggtgttg atacaaaact tgtttctgag 300
   atgacccgtt ttgctagaga gttttttgct ttgccaccgg aagagaagct ccggtttgac 360
   atgtccggtg gtaaaaaggg tggtttcatt gtctctagtc atcttcaagg agaagcagtg 420
   aaggattgga gagagctagt gacatatttt tcatacccaa ttaaacaaag agattattca 480
   aggtggccag acaagccaga aggatggaaa gaggtaacag aaaaatacag tgaaaaccta 540
   atgaatttag cttgcaagct attggaagtt ttatcagaag 580
<210> 215
   <211> 586
   <212> DNA
   <213> Trifolium repens
<220>
   <221> misc_feature
   <222> (31) .. (31)
   <223> Any nucleotide
<220>
   <221> misc_feature
   <222> (33) .. (33)
   <223> Any nucleotide
<220>
   <221> misc_feature
   <222> (586) .. (586)
   <223> Any nucleotide
<400> 215
   ggcacacaaa ccaaaacgag cccgtttcct ntnaacatgg caccaagcca aaccctaagt 60
   tatctctcca acaaaagact cttgagtcaa gtttcgttag ggaagaagat gagcgtccaa 120
   aagttgccta caataacttc agcaacgaga ttccaatcat ttctcttgct ggaattgatg 180
   aggttgatgg tcgcagaaca gagatatgta acaagattgt tgaagcttgt gagaattggg 240
   gtatttttca ggttgttgat catggtgttg atacaaagct tgtttctgag atgactcgtt 300
   ttgctagaga gttttttgct ttgccgccgg aagagaagct ccggtttgac atgtccggtg
   360
   gtaaaaaggg tggtttcatt gtctctagtc atctccaagg agaagcagtg aaagattgga 420
   gggagctagt gacatatttt tcatacccaa ttaaacaaag agattattca aggtggccag 480
   acaagccaga aggatggaaa gaagtaacag aaaaatacag tgaaaaccta atgaatttag 540
   cttgcaagct attggaagtt ttatcagaag caatgggatt agaaan 586
<210> 216
   <211> 450
   <212> DNA
   <213> Trifolium repens
<220>
   <221> misc_feature
   <222> (26) .. (26)
   <223> Any nucleotide
<220>
   <221> misc_feature
   <222> (37) .. (37)
   <223> Any nucleotide
<220>
   <221> misc_feature
   <222> (96) .. (96)
   <223> Any nucleotide
<220>
   <221> misc_feature
   <222> (363) .. (363)
   <223> Any nucleotide
<220>
   <221> misc_feature
   <222> (390) .. (390)
   <223> Any nucleotide
<220>
   <221> misc_feature
   <222> (403) .. (403)
   <223> Any nucleotide
<220>
   <221> misc_feature
   <222> (410) .. (410)
   <223> Any nucleotide
<220>
   <221> misc_feature
   <222> (416) .. (417)
   <223> Any nucleotides
<220>
   <221> misc_feature
   <222> (421).. (421)
   <223> Any nucleotide
<220>
   <221> misc_feature
   <222> (436).. (436)
   <223> Any nucleotide
<220>
   <221> misc_feature
   <222> (438) .. (438)
   <223> Any nucleotide
<220>
   <221> misc_feature
   <222> (450) .. (450)
   <223> Any nucleotide
<400> 216
   tctaacacaa acacccaaaa cagagnaccg tttcctntcc aacatggcac caagccaaac
   60
   tctaagttat ctctcaccac aaaacactct tgagtnaagt ttcgttaggg aagaagatga
   120
   gcgtccaaaa gttgcctaca ataacttcag caacgagatt ccaatcattt ctcttgctgg
   180
   aattgatgag gttgatggtc gtagaacaga gatatgtaac aagattgttg aagcttgtga
   240
   gaattggggt atttttcagg ttgttgatca tggtgttgat acaaaacttg tttctgagat
   300
   gacccgtttt gctagagagt tttttgcttt gccaccggaa gagaagctcc ggtttgacat
   360
   gtncggtggt aaaaagggtg gtttcattgn ctctagtcat ctncaaggan aagcanngaa
   420
   ngattggaga gagctngnga catatttttn
   450
<210> 217
   <211> 681
   <212> DNA
   <213> Trifolium repens
<220>
   <221> misc_feature
   <222> (2) .. (2)
   <223> Any nucleotide
<220>
   <221> misc_feature
   <222> (24) .. (24)
   <223> Any nucleotide
<220>
   <221> misc_feature
   <222> (26) .. (26)
   <223> Any nucleotide
<220>
   <221> misc_feature
   <222> (39) .. (39)
   <223> Any nucleotide
<220>
   <221> misc_feature
   <222> (43) .. (43)
   <223> Any nucleotide
<220>
   <221> misc_feature
   <222> (644) .. (644)
   <223> Any nucleotide
<400> 217
   cncaacacaa acaccaaaaa cagnancacc gtttccatnc atncaaacat ggcaccaagc 60
   caaactctaa gttatctctc acaacaaaac actcttgagt caagtttcgt tagggaagaa 120
   gatgagcgtc caaaagttgc ctacaataac ttcagcaacg agattccaat catttctctt 180
   gctggaattg atgaggttga tggtcgtaga acagagatat gtaacaagat tgttgaagct 240
   tgtgagaatt ggggtatttt tcaggttgtt gatcatggtg ttgatacaaa acttgtttct 300
   gagatgaccc gttttgctag agagtttttt gctttgccac cggaagagaa gctccggttt 360
   gacatgtccg gtggtaaaaa gggtggtttc attgtctcta gtcatctcca aggagaagca 420
   gtgaaggatt ggagagagct agtgacatat ttttcatacc caattaaaca aagagattat 480
   tcaaggtggc cagacaagcc agaaggatgg aaagaggtaa cagaaaaata cagtgaaaac 540
   ctaatgaatt tagcttgcaa actattggaa gttttatcag aagcaatggg tttagaaaaa 600
   gaagctctaa caaaagcatg tgttgatatg gatcaaaaag ttgntataaa ttattaccca 660
   aaatgccctg aacctgacct c 681
<210> 218
   <211> 582
   <212> DNA
   <213> Trifolium repens
<220>
   <221> misc_feature
   <222> (2) .. (3)
   <223> Any nucleotide
<220>
   <221> misc_feature
   <222> (15) .. (15)
   <223> Any nucleotide
<220>
   <221> misc_feature
   <222> (21) .. (21)
   <223> Any nucleotide
<220>
   <221> misc_feature
   <222> (25) .. (25)
   <223> Any nucleotide
<220>
   <221> misc_feature
   <222> (43) .. (43)
   <223> Any nucleotide
<220>
   <221> misc_feature
   <222> (47) .. (47)
   <223> Any nucleotide
<400> 218
   gnnaatccac aacancaaac nccanaaacg agcccgtttc ctnctcnaac atggcaccaa 60
   gccaaactct aagttatctc tcacaacaaa acactctcga gtcaagtttc gttagggaag 120
   aagatgagcg tccaaaagtt gcctacaata acttcagcaa cgagattcca atcatttctc 180
   ttgctggaat tgatgaggtt gatggtcgaa gaacagagat atgtaacaag attgttgaag 240
   cttgtgagaa ttggggtatt tttcaggttg ttgatcatgg tgttgataca aaacttgttt 300
   ctgagatgac ccgttttgct agagagtttt ttgctttgcc accggaagag aagctccggt 360
   ttgacatgtc cggtggtaaa aagggtggtt tcattgtctc tagtcatctc caaggagaag 420
   cagtgaagga ttggagagag ctagtgacat atttttcata cccaattaaa caaagagatt 480
   attcaaggtg gccagacaag ccagaaggat ggaaagaggt aacagaaaaa tacagtgaaa 540
   acctaatgaa tttagcttgc aaactattgg aagttttatc ag 582

<210> 219
   <211> 610
   <212> DNA
   <213> Trifolium repens
<220>
   <221> misc_feature
   <222> (31) .. (31)
   <223> Any nucleotide
<220>
   <221> misc_feature
   <222> (41) .. (41)
   <223> Any nucleotide
<400> 219
   gaaccccaca acacccaaac ccaaaacgag nccgtttcct ntcaacatgg caccaagcca
   60
   aactctaagt tatctctcac aacaaaacac tctcgagtca agtttcgtta gggaagaaga
   120
   tgagcgtcca aaagttgcct acaataactt cagcaacgag attccaatca tttctcttgc
   180
   tggaattgat gaggttgatg gtcgtagaac agagatatgt aacaagattg ttgaagcttg
   240
   tgagaattgg ggtatttttc aggttgttga tcatggtgtt gatacaaaac ttgtttctga
   300
   gatgacccgt tttgctagag agttttttgc tttgccaccg gaagagaagc tccggtttga
   360
   catgtccggt ggtaaaaagg gtggtttcat tgtctctagt catctccaag gagaagcagt
   420
   gaaggattgg agagagctag tgacatattt ttcataccca attaaacaaa gagattattc
   480
   aaggtggcca gacaagccag aaggatggaa agaggtaaca gaaaaataca gtgaaaacct
   540
   aatgaattta gcttgcaagc tattggaagt tttatcagaa gcaatgggtt tagaaaaaga
   600
   agctctaaca
   610
<210> 220
   <211> 593
   <212> DNA
   <213> Trifolium repens
<220>
   <221> misc_feature
   <222> (16) .. (16)
   <223> Any nucleotide
<220>
   <221> misc_feature
   <222> (23) .. (23)
   <223> Any nucleotide
<220>
   <221> misc_feature
   <222> (41) .. (42)
   <223> Any nucleotides
<220>
   <221> misc_feature
   <222> (45) .. (45)
   <223> Any nucleotide
<220>
   <221> misc_feature
   <222> (593) .. (593)
   <223> Any nucleotide
<400> 220
   aaccccacga caccanaacc canaaacgag cccgtttcct nntcnaacat ggcaccaagc 60
   caaactctaa gttatctctc acaacaaaac actctcgagt caagtttcgt tagggaagaa 120
   gatgagcgtc caaaagttgc ctacaataac ttcagcaacg agattccaat catttctctt 180
   gctggaattg atgaggttga tggtcgtaga acagagatat gtaacaagat tgttgaagct 240
   tgtgagaatt ggggtatttt tcaggttgtt gatcatggtg ttgatacaaa acttgtttct 300
   gagatgaccc gttttgctag agagtttttt gctttgccac cggaagagaa gctccggttt 360
   gacatgtccg gtggtaaaaa gggtggtttc attgtctcta gtcatcttca aggagaagca 420
   gtgaaggatt ggagagagct agtgacatat ttttcatacc caattaaaca aagagattat 480
   tcaaggtggc cagacaagcc agaaggatgg aaagaggtaa cagaaaaata cagtgaaaac 540
   ctaatgaatt tagcttgcaa gctattggaa gttttatcag aagcaatggg ttn 593
<210> 221
   <211> 236
   <212> DNA
   <213> Trifolium repens
<220>
   <221> misc_feature
   <222> (5) .. (5)
   <223> Any nucleotide
<220>
   <221> misc_feature
   <222> (10) .. (10)
   <223> Any nucleotide
<220>
   <221> misc_feature
   <222> (18) .. (18)
   <223> Any nucleotide
<220>
   <221> misc_feature
   <222> (26) .. (26)
   <223> Any nucleotide
<220>
   <221> misc_feature
   <222> (41) .. (41)
   <223> Any nucleotide
<220>
   <221> misc_feature
   <222> (43) .. (43)
   <223> Any nucleotide
<220>
   <221> misc_feature
   <222> (203) .. (206)
   <223> Any nucleotides
<220>
   <221> misc_feature
   <222> (209) .. (209)
   <223> Any nucleotide
<220>
   <221> misc_feature
   <222> (213) .. (213)
   <223> Any nucleotide
<220>
   <221> misc_feature
   <222> (216) .. (217)
   <223> Any nucleotides
<220>
   <221> misc_feature
   <222> (224).. (225)
   <223> Any nucleotides
<220>
   <221> misc_feature
   <222> (234) .. (236)
   <223> Any nucleotides
<400> 221
   gcacnaacan cacaaacncc caaaangaga ccgtttcctc ntnaaacatg gcaccaagcc
   60
   aaactctaag ttatctctca caacaaaaca ctctcgagtc aagtttcgtt agggaagaag
   120
   atgagcgtcc aaaagttgcc tacaataact tcagcaacga gattccaatc atttctcttg
   180
   ctggaattga tgaggttgat ggnnnnggna ccnggnnatg tggnnagagg acgnnn
   236
<210> 222
   <211> 273
   <212> DNA
   <213> Trifolium repens
<220>
   <221> misc_feature
   <222> (4) .. (4)
   <223> Any nucleotide
<220>
   <221> misc_feature
   <222> (9) ..(10)
   <223> Any nucleotides
<220>
   <221> misc_feature
   <222> (13) .. (13)
   <223> Any nucleotide
<220>
   <221> misc_feature
   <222> (34) .. (34)
   <223> Any nucleotide
<220>
   <221> misc_feature
   <222> (36) .. (36)
   <223> Any nucleotide
<220>
   <221> misc_feature
   <222> (151) .. (151)
   <223> Any nucleotide
<220>
   <221> misc_feature
   <222> (198) .. (198)
   <223> Any nucleotide
<220>
   <221> misc_feature
   <222> (205) .. (205)
   <223> Any nucleotide
<220>
   <221> misc_feature
   <222> (255) .. (255)
   <223> Any nucleotide
<220>
   <221> misc_feature
   <222> (270) .. (270)
   <223> Any nucleotide
<220>
   <221> misc_feature
   <222> (273) .. (273)
   <223> Any nucleotide
<400> 222
   acancaccnn acnccaaaac gagtccgttc cctntnaaca tggcaccaag ccaaactcta 60
   agttatctct cacaacaaaa cactctcgag tcaagtttcg ttagggaaga agatgagcgt 120
   ccaaaagttg cctacaataa cttcagcaac nagattccaa tcatttctct tgctggaatt 180
   gatgaggttg atggtcgnag aacanagata tgtaacaaga ttgttgaagc ttgtgagaat 240
   tggggtattt ttcangttgt tgatcatggn gtn 273
<210> 223
   <211> 572
   <212> DNA
   <213> Trifolium repens
<220>
   <221> misc_feature
   <222> (3) .. (4)
   <223> Any nucleotides
<220>
   <221> misc_feature
   <222> (8) .. (8)
   <223> Any nucleotide
<220>
   <221> misc_feature
   <222> (16) ..(16)
   <223> Any nucleotide
<220>
   <221> misc_feature
   <222> (18) .. (18)
   <223> Any nucleotide
<220>
   <221> misc_feature
   <222> (39) .. (39)
   <223> Any nucleotide
<220>
   <221> misc_feature
   <222> (41) .. (42)
   <223> Any nucleotides
<400> 223
   gcnncaanca caaacncnaa aacgagcacc gtttcctant nnaacatggc accaagccaa 60
   actctaagtt atctctcaca acaaaaaacc gtcgagtcaa gtttcgttag ggaagaagat 120
   gagcgtccaa aagttgccta caataacttc agcaacgaga ttccaatcat ttctcttgct 180
   ggaattgatg aggttgatgg tcgcagaaca gagatatgta acaagattgt tgaagcttgt 240
   gagaattggg gtatttttca ggttgttgat catggtgttg atacaaagct tgtttctgag 300
   atgactcgtt ttgctagaga gttttttgct ttgccgccgg aagagaagct ccggtttgac 360
   atgtccggtg gtaaaaaggg tggtttcatt gtctctagtc atctccaagg agaagcagtg 420
   aaagattgga gagagctagt gacatatttt tcatacccaa ttaaacaaag agattattca 480
   aggtggccag acaagccaga aggatggaaa gaagtaacag aaaaatacag tgaaaaccta 540
   atgaatttag cttgcaagct attggaagtt tt 572
<210> 224
   <211> 575
   <212> DNA
   <213> Trifolium repens
<220>
   <221> misc_feature
   <222> (2) .. (2)
   <223> Any nucleotide
<220>
   <221> misc_feature
   <222> (10).. (10)
   <223> Any nucleotide
<220>
   <221> misc_feature
   <222> (12) .. (12)
   <223> Any nucleotide
<220>
   <221> misc_feature
   <222> (16) .. (16)
   <223> Any nucleotide
<220>
   <221> misc_feature
   <222> (20) .. (20)
   <223> Any nucleotide
<220>
   <221> misc_feature
   <222> (38) .. (38)
   <223> Any nucleotide
<220>
   <221> misc_feature
   <222> (41) .. (42)
   <223> Any nucleotides
<220>
   <221> misc_feature
   <222> (54) .. (54)
   <223> Any nucleotide
<220>
   <221> misc_feature
   <222> (77) .. (78)
   <223> Any nucleotides
<220>
   <221> misc_feature
   <222> (99) .. (99)
   <223> Any nucleotide
<400> 224
   gnacccacan cnaacnccan aaacgagccc gtttcctnct nnaacatggc accnagccaa
   60
   actctaagtt atctctnnca acaaaacact cttgagtcna gtttcgttag ggaagaagat
   120
   gagcgtccaa aagttgccta caataacttc agcaacgaga ttccaatcat ttctcttgct
   180
   ggaattgatg aggttgatgg tcgtagaaca gagatatgta acaagattgt tgaagcttgt
   240
   gagaattggg gtatttttca ggttgttgat catggtgttg atacaaaact tgtttctgag
   300
   atgacccgtt ttgctagaga gttttttgct ttgccaccgg aagagaagct ccggtttgac
   360
   atgtccggtg gtaaaaaggg tggtttcatt gtctctagtc atctccaagg agaagcagtg
   420
   aaggattgga gagagctagt gacatatttt tcatacccaa ttaaacaaag agattattca
   480
   aggtggccag acaagccaga aggatggaaa gaggtaacag aaaaatacag tgaaaaccta
   540
   atgaatttag cttgcaaact attggaagtt ttatc
   575
<210> 225
   <211> 596
   <212> DNA
   <213> Trifolium repens
<220>
   <221> misc_feature
   <222> (10) .. (11)
   <223> Any nucleotides
<220>
   <221> misc_feature
   <222> (15) .. (15)
   <223> Any nucleotide
<220>
   <221> misc_feature
   <222> (26) .. (26)
   <223> Any nucleotide
<400> 225
   ccacaacacn naacnccaaa acgagnccgt ttcctctcaa catggcacca agccaaactc
   60
   taagttatct ctcacaacaa aacactctcg agtcaagttt cgttagggaa gaagatgagc
   120
   gtccaaaagt tgcctacaat aacttcagca acgagattcc aatcatttct cttgctggaa
   180
   ttgatgaggt tgatggtcgt agaacagaga tatgtaacaa gattgttgaa gcttgtgaga
   240
   attggggtat ttttcaggtt gttgatcatg gtgttgatac aaaacttgtt tctgagatga
   300
   cccgttttgc tagagagttt tttgctttgc caccggaaga gaagctccgg tttgacatgt
   360
   ccggtggtaa aaagggtggt ttcattgtct ctagtcatct ccaaggagaa gcagtgaagg
   420
   attggagaga gctagtgaca tatttttcat acccaattaa acaaagagat tattcaaggt
   480
   ggccagacaa gccagaagga tggaaagagg taacagaaaa atacagtgaa aacctaatga
   540
   atttagcttg caaactattg gaagttttat cagaagcaat gggtttagaa aaagag
   596
<210> 226
   <211> 612
   <212> DNA
   <213> Trifolium repens
<220>
   <221> misc_feature
   <222> (9) .. (9)
   <223> Any nucleotide
<220>
   <221> misc_feature
   <222> (13) .. (13)
   <223> Any nucleotide
<220>
   <221> misc_feature
   <222> (36) .. (36)
   <223> Any nucleotide
<400> 226
   cacaacacnc acnccaaaac gagcccgttt cctctnaaca tggcaccaag ccaaactcta 60
   agttatctct ccaacaaaac actcttgagt caagtttcgt tagggaagaa gatgagcgtc 120
   caaaagttgc ctacaataac ttcagcaacg agattccaat catttctctt gctggaattg 180
   atgaggttga tggtcgtaga acagagatat gtaacaagat tgttgaagct tgtgagaatt 240
   ggggtatttt tcaggttgtt gatcatggtg ttgatacaaa acttgtttct gagatgaccc 300
   gttttgctag agagtttttt gctttgccac cggaagagaa gctccggttt gacatgtccg 360
   gtggtaaaaa gggtggtttc attgtctcta gtcatctcca aggagaagca gtgaaggatt 420
   ggagagagct agtgacatat ttttcatacc caattaaaca aagagattat tcaaggtggc 480
   cagacaagcc agaaggatgg aaagaggtaa cagaaaaata cagtgaaaac ctaatgaatt 540
   tagcttgcaa actattggaa gttttatcag aagcaatggg tttagaaaaa gaagctctaa 600
   caaaagcatg tg 612
<210> 227
   <211> 535
   <212> DNA
   <213> Trifolium repens
<220>
   <221> misc_feature
   <222> (32) .. (32)
   <223> Any nucleotide
<220>
   <221> misc_feature
   <222> (69) .. (69)
   <223> Any nucleotide
<400> 227
   acaacacaaa cccaaaacga gcccgtttcc tntcaacatg gcaccaagcc aaactctaag 60
   ttatctctnc aacaaaacac tcttgagtca agtttcgtta gggaagaaga tgagcgtcca 120
   aaagttgcct acaataactt cagcaacgag attccaatca tttctcttgc tggaattgat 180
   gaggttgatg gtcgtagaac agagatatgt aacaagattg ttgaagcttg tgagaattgg 240
   ggtatttttc aggttgttga tcatggtgtt gatacaaaac ttgtttctga gatgacccgt 300
   tttgctagag agttttttgc tttgccaccg gaagagaagc tccggtttga catgtccggt 360
   ggtaaaaagg gtggtttcat tgtctctagt catctccaag gagaagcagt gaaggattgg 420
   agagagctag tgacatattt ttcataccca attaaacaaa gagattattc aaggtggcca 480
   gacaagccag aaggatggaa agaggtaaca gaaaaataca gtgaaaacct aatgc 535
<210> 228
   <211> 559
   <212> DNA
   <213> Trifolium repens
<220>
   <221> misc_feature
   <222> (23) .. (23)
   <223> Any nucleotide
<220>
   <221> misc_feature
   <222> (33) .. (33)
   <223> Any nucleotide
<220>
   <221> misc_feature
   <222> (559) .. (559)
   <223> Any nucleotide
<400> 228
   gcaacacaaa caccaaaacg agnccgtttc ctntcaacat ggcaccaagc caaactctaa 60
   gttatctctc ccacaaaaca ctcttgagtc aagtttcgtt agggaagaag atgagcgtcc 120
   aaaagttgcc tacaataact tcagcaacga gattccaatc atttctcttg ctggaattga 180
   tgaggttgat ggtcgtagaa cagagatatg taacaagatt gttgaagctt gtgagaattg 240
   gggtattttt caggttgttg atcatggtgt tgatacaaaa cttgtttctg agatgacccg 300
   ttttgctaga gagttttttg ctttgccacc ggaagagaag ctccggtttg acatgtccgg 360
   tggtaaaaag ggtggtttca ttgtctctag tcatctccaa ggagaagcag tgaaggattg 420
   gagagagcta gtgacatatt tttcataccc aattaaacaa agagattatt caaggtggcc 480
   agacaagcca gaaggatgga aagaggtaac agaaaaatac agtgaaaacc taatgaattt 540
   agcttgcaaa ctattggan 559

<210> 229
   <211> 565
   <212> DNA
   <213> Trifolium repens
<220>
   <221> misc_feature
   <222> (3) .. (3)
   <223> Any nucleotide
<400> 229
   gcncaacaca aaccaaaacg agcccgtttc ctatcaacat ggcaccaagc caaaccctaa 60
   gttatctctc acaacaaaag actcttgagt caagtttcgt tagggaagaa gatgagcgtc 120
   caaaagttgc ctacaataac ttcagcaacg agattccaat catttctctt gctggaattg 180
   atgaggttga tggtcgcaga acagagatat gtaacaagat tgttgaagct tgtgagaatt 240
   ggggtatttt tcaggttgtt gatcatggtg ttgatacaaa gcttgtttct gagatgactc 300
   gttttgctag agagtttttt gctttgccgc cggaagagaa gctccggttt gacatgtccg 360
   gtggtaaaaa gggtggtttc attgtctcta gtcatctcca aggagaagca gtgaaagatt 420
   ggagagagct agtgacatat ttttcatacc caattaaaca aagagattat tcaaggtggc 480
   cagacaagcc agaaggatgg aaagaagtaa cagaaaaata cagtgaaaac ctaatgaatt 540
   tagcttgcaa gctattggaa gtttt 565
<210> 230
   <211> 591
   <212> DNA
   <213> Trifolium repens
<220>
   <221> misc_feature
   <222> (6) .. (6)
   <223> Any nucleotide
<220>
   <221> misc_feature
   <222> (12) .. (12)
   <223> Any nucleotide
<220>
   <221> misc_feature
   <222> (16) .. (16)
   <223> Any nucleotide
<220>
   <221> misc_feature
   <222> (32) .. (32)
   <223> Any nucleotide
<220>
   <221> misc_feature
   <222> (34) .. (34)
   <223> Any nucleotide
<220>
   <221> misc_feature
   <222> (38) .. (38)
   <223> Any nucleotide
<220>
   <221> misc_feature
   <222> (74) .. (74)
   <223> Any nucleotide
<220>
   <221> misc_feature
   <222> (591) .. (591)
   <223> Any nucleotide
<400> 230
   caacancaaa cnccanaaac gagcccgttt cntnctcnaa catggcacca agccaaactc 60
   taagttatct ctcncaacaa aacactctcg agtcaagttt cgttagggaa gaagatgagc 120
   gtccaaaagt tgcctacaat aacttcagca acgagattcc aatcatttct cttgctggaa 180
   ttgatgaggt tgatggtcga agaacagaga tatgtaacaa gattgttgaa gcttgtgaga 240
   attggggtat ttttcaggtt gttgatcatg gtgttgatac aaaacttgtt tctgagatga 300
   cccgttttgc tagagagttt tttgctttgc caccggaaga gaagctccgg tttgacatgt 360
   ccggtggtaa aaagggtggt ttcattgtct ctagtcatct ccaaggagaa gcagtgaagg 420
   attggagaga gctagtgaca tatttttcat acccaattaa acaaagagat tattcaaggt 480
   ggccagacaa gccagaagga tggaaagagg taacagaaaa atacagtgaa aacctaatga 540
   atttagcttg caaactattg gaagttttat cagaagcaat gggtttagaa n 591
<210> 231
   <211> 609
   <212> DNA
   <213> Trifolium repens
<220>
   <221> misc_feature
   <222> (14) .. (14)
   <223> Any nucleotide
<220>
   <221> misc_feature
   <222> (66) .. (66)
   <223> Any nucleotide
<220>
   <221> misc_feature
   <222> (76) .. (76)
   <223> Any nucleotide
<220>
   <221> misc_feature
   <222> (84) .. (84)
   <223> Any nucleotide
<220>
   <221> misc_feature
   <222> (94) .. (95)
   <223> Any nucleotides
<220>
   <221> misc_feature
   <222> (98) .. (98)
   <223> Any nucleotide
<400> 231
   gcagttatct ctcncaacaa aacactctcg agtcaagttt cgttagggaa gaagatgagc 60
   gtacancaaa caccanaaac gagnccgttt cctnntcnaa catggcacca agccaaactc 120
   taccaaaagt tgcctacaat aacttcagca acgagattcc aatcatttct cttgctggaa 180
   ttgatgaggt tgatggtcgt agaacagaga tatgtaacaa gattgttgaa gcttgtgaga 240
   attggggtat ttttcaggtt gttgatcatg gtgttgatac aaaacttgtt tctgagatga 300
   cccgttttgc tagagagttt tttgctttgc caccggaaga gaagctccgg tttgacatgt 360
   ccggtggtaa aaagggtggt ttcattgtct ctagtcatct tcaaggagaa gcagtgaagg 420
   attggagaga gctagtgaca tatttttcat acccaattaa acaaagagat tattcaaggt 480
   ggccagacaa gccagaagga tggaaagagg taacagaaaa atacagtgaa aacctaatga 540
   atttagcttg caagctattg gaagttttat cagaagcaat gggtttagaa aaagaagctc 600
   taacaaaag 609
<210> 232
   <211> 597
   <212> DNA
   <213> Trifolium repens
<220>
   <221> misc_feature
   <222> (11) .. (11)
   <223> Any nucleotide
<220>
   <221> misc_feature
   <222> (15) .. (16)
   <223> Any nucleotides
<220>
   <221> misc_feature
   <222> (24) .. (24)
   <223> Any nucleotide
<220>
   <221> misc_feature
   <222> (26) .. (27)
   <223> Any nucleotides
<220>
   <221> misc_feature
   <222> (29) .. (29)
   <223> Any nucleotide
<400> 232
   aacccaaaac ngagnnccgt ttcntnntna acatggccca gccaaactct aagttatctc 60
   tccaacaaaa cactctcgag tcaagtttcg ttagggaaga agatgagcgt ccaaaagttg 120
   cctacaataa cttcagcaac gagattccaa tcatttctct tgctggaatt gatgaggttg 180
   atggtcgtag aacagagata tgtaacaaga ttgttgaagc ttgtgagaat tggggtattt 240
   ttcaggttgt tgatcatggt gttgatacaa aacttgtttc tgagatgacc cgttttgcta 300
   gagagttttt tgctttgcca ccggaagaga agctccggtt tgacatgtcc ggtggtaaaa 360
   agggtggttt cattgtctct agtcatctcc aaggagaagc agtgaaggat tggagagagc 420
   tagtgacata tttttcatac ccaattaaac aaagagatta ttcaaggtgg ccagacaagc 480
   cagaaggatg gaaagaggta acagaaaaat acagtgaaaa cctaatgaat ttagcttgca 540
   agctattgga agttttatca gaagcaatgg gtttagaaaa agaagctcta acaaaag 597
<210> 233
   <211> 418
   <212> DNA
   <213> Trifolium repens
<220>
   <221> misc_feature
   <222> (10) .. (10)
   <223> Any nucleotide
<220>
   <221> misc_feature
   <222> (25) .. (25)
   <223> Any nucleotide
<220>
   <221> misc_feature
   <222> (29) .. (29)
   <223> Any nucleotide
<220>
   <221> misc_feature
   <222> (31) .. (31)
   <223> Any nucleotide
<220>
   <221> misc_feature
   <222> (354) .. (354)
   <223> Any nucleotide
<220>
   <221> misc_feature
   <222> (361) .. (361)
   <223> Any nucleotide
<220>
   <221> misc_feature
   <222> (369) .. (369)
   <223> Any nucleotide
<220>
   <221> misc_feature
   <222> (378) .. (378)
   <223> Any nucleotide
<220>
   <221> misc_feature
   <222> (387) .. (387)
   <223> Any nucleotide
<220>
   <221> misc_feature
   <222> (410) .. (410)
   <223> Any nucleotide
<220>
   <221> misc feature
   <222> (412).. (412)
   <223> Any nucleotide
<220>
   <221> misc_feature
   <222> (415) .. (415)
   <223> Any nucleotide
<220>
   <221> misc_feature
   <222> (418) .. (418)
   <223> Any nucleotide
<400> 233
   accccacctn aaaccgagac cgttncctnt naacatggca ccaagccaaa ctctaagtta 60
   tctctcacaa caaaacactc ttgagtcaag tttcgttagg gaagaagatg agcgtccaaa 120
   agttgcctac aataacttca gcaacgagat tccaatcatt tctcttgctg gaattgatga 180
   ggttgatggt cgtagaacag agatatgtaa caagattgtt gaagcttgtg agaattgggg 240
   tatttttcag gttgttgatc atggtgttga tacaaaactt gtttctgaga tgacccgttt 300
   tgctagagag ttttttgctt tgccaccgga agagaagctc cggtttgaca tgtncggtgg 360
   naaaaaggnt ggctccantg cctgtancca tctccaagga gaaccacccn angantgn 418
<210> 234
   <211> 570
   <212> DNA
   <213> Trifolium repens
<220>
   <221> misc_feature
   <222> (3) .. (4)
   <223> Any nucleotides
<220>
   <221> misc_feature
   <222> (13) .. (13)
   <223> Any nucleotide
<220>
   <221> misc_feature
   <222> (31) .. (32)
   <223> Any nucleotides
<400> 234
   gcnnaaacac canaaacgag cccgtttcct nntcgaacat ggcaccaagc caaactctaa 60
   gttatctctc acaacaaaac actcttgagt caagtttcgt tagggaagaa gatgagcgtc 120
   caaaagttgc ctacaataac ttcagcaacg agattccaat catttctctt gctggaattg 180
   atgaggttga tggtcgtaga acagagatat gtaacaagat tgttgaagct tgtgagaatt 240
   ggggtatttt tcaggttgtt gatcatggtg ttgatacaaa acttgtttct gagatgaccc 300
   gttttgctag agagtttttt gctttgccac cggaagagaa gctccggttt gacatgtccg 360
   gtggtaaaaa gggtggtttc attgtctcta gtcatctcca aggagaagca gtgaaggatt 420
   ggagagagct agtgacatat ttttcatacc caattaaaca aagagattat tcaaggtggc 480
   cagacaagcc agaaggatgg aaagaggtaa cagaaaaata cagtgaaaac ctaatgaatt 540
   tagcttgcaa actattggaa gttttatcag 570
<210> 235
   <211> 608
   <212> DNA
   <213> Trifolium repens
<220>
   <221> misc_feature
   <222> (30).. (30)
   <223> Any nucleotide
<400> 235
   caacaccacc caaaacgagc ccgtttcctn tcaacatggc accaagccaa actctaagtt 60
   atctctcaca acaaaacact cttgagtcaa gtttcgttag ggaagaagat gagcgtccaa 120
   aagttgccta caataacttc agcaacgaga ttccaatcat ttctcttgct ggaattgatg 180
   aggttgatgg tcgtagaaca gagatatgta acaagattgt tgaagcttgt gagaattggg 240
   gtatttttca ggttgttgat catggtgttg atacaaaact tgtttctgag atgacccgtt 300
   ttgctagaga gttttttgct ttgccaccgg aagagaagct ccggtttgac atgtccggtg 360
   gtaaaaaggg tggtttcatt gtctctagtc atctccaagg agaagcagtg aaggattgga 420
   gagagctagt gacatatttt tcatacccaa ttaaataaag agattattca aggtggccag 480
   acaagccaga aggatggaaa gaggtaacag aaaaatacag tgaaaaccta atgaatttag 540
   cttgcaaact attggaagtt ttatcagaag caatgggttt agaaaaagaa gctctaacaa 600
   aagcatgt 608
<210> 236
   <211> 602
   <212> DNA
   <213> Trifolium repens
<220>
   <221> misc_feature
   <222> (12) .. (12)
   <223> Any nucleotide
<220>
   <221> misc_feature
   <222> (16) .. (16)
   <223> Any nucleotide
<220>
   <221> misc_feature
   <222> (28) .. (28)
   <223> Any nucleotide
<220>
   <221> misc_feature
   <222> (506) .. (506)
   <223> Any nucleotide
<220>
   <221> misc_feature
   <222> (536) .. (536)
   <223> Any nucleotide
<220>
   <221> misc_feature
   <222> (569) .. (569)
   <223> Any nucleotide
<220>
   <221> misc_feature
   <222> (597) .. (597)
   <223> Any nucleotide
<220>
   <221> misc_feature
   <222> (599) .. (599)
   <223> Any nucleotide
<220>
   <221> misc_feature
   <222> (601) .. (601)
   <223> Any nucleotide
<400> 236
   caaaccaaaa cngagnccgt ttcctatnaa catggcacca agccaaactc taagttatct
   60
   ctcacaacaa aaaaccgtcg agtcaagttt cgttagggaa gaagatgagc gtccaaaagt
   120
   tgcctacaat aacttcagca acgagattcc aatcatttct cttgctggaa ttgatgaggt
   180
   tgatggtcgc agaacagaga tatgtaacaa gattgttgaa gcttgtgaga attggggtat
   240
   ttttcaggtt gttgatcatg gtgttgatac aaagcttgtt tctgagatga ctcgttttgc
   300
   tagagagttt tttgctttgc cgccggaaga gaagctccgg tttgacatgt ccggtggtaa
   360
   aaagggtggt ttcattgtct ctagtcgtct ccaaggagaa gcagtgaaag attggagaga
   420
   gctagtgaca tatttttcat acccaattaa acaaagagat tattcaaggt ggccagacaa
   480
   gccagaagga tggaaagaag taacanaaaa atacagtgaa aacctaatga atttancttg
   540
   caagctattg gaagttttat cacaagcant gggattacaa aaagaagctg ttacaananc
   600
   nt
   602
<210> 237
   <211> 536
   <212> DNA
   <213> Trifolium repens
<220>
   <221> misc_feature
   <222> (1) .. (1)
   <223> Any nucleotide
<220>
   <221> misc_feature
   <222> (14) .. (14)
   <223> Any nucleotide
<220>
   <221> misc_feature
   <222> (22) .. (22)
   <223> Any nucleotide
<220>
   <221> misc_feature
   <222> (24) .. (24)
   <223> Any nucleotide
<220>
   <221> misc_feature
   <222> (155) .. (155)
   <223> Any nucleotide
<220>
   <221> misc_feature
   <222> (271) .. (271)
   <223> Any nucleotide
<220>
   <221> misc_feature
   <222> (314) .. (314)
   <223> Any nucleotide
<220>
   <221> misc_feature
   <222> (388) .. (388)
   <223> Any nucleotide
<220>
   <221> misc_feature
   <222> (395) .. (395)
   <223> Any nucleotide

<220>
   <221> misc_feature
   <222> (401) .. (401)
   <223> Any nucleotide
<220>
   <221> misc_feature
   <222> (499) .. (499)
   <223> Any nucleotide
<220>
   <221> misc_feature
   <222> (533) .. (533)
   <223> Any nucleotide
<220>
   <221> misc_feature
   <222> (536) .. (536)
   <223> Any nucleotide
<400> 237
   nacccaaaac gagnccgttt cntnctcaac atggctccta gccaaactct aagttatctc 60
   tcccacaaaa cactcttgag tcagtttcgt tagggaagaa gatgagcgtc caaaagttgc 120
   ctacaataac ttcagcaacg agattccaat cattnctctt gctggaattg atgaggttga 180
   tggtcgcaga acagagatat gtaacaagat tgttgaagct tgtgagaatt ggggtatttt 240
   tcaggttgtt gatcatggtg ttgatacaaa ncttgtttct gagatgaccc gttttgctag 300
   agagtttttt gctntgccac cggaagagaa gctccggttt gacatgtccg gtggtaaaaa 360
   gggtggtttc attgtctcta gtcatctnca agganaagca ntgaaggatt ggagagagct 420
   agtgacatat ttttcatacc caattaaaca aagagattat tcaaggtggc cagacaagcc 480
   agaaggatgg aaagaggtna cagaaaaata cagcgaaaac ctaatgaatt tanctn 536
<210> 238
   <211> 572
   <212> DNA
   <213> Trifolium repens
<220>
   <221> misc_feature
   <222> (12) .. (12)
   <223> Any nucleotide
<220>
   <221> misc_feature
   <222> (21) .. (21)
   <223> Any nucleotide
<220>
   <221> misc_feature
   <222> (25) .. (26)
   <223> Any nucleotides
<220>
   <221> misc_feature
   <222> (28) .. (28)
   <223> Any nucleotide
<220>
   <221> misc_feature
   <222> (30) .. (30)
   <223> Any nucleotide
<220>
   <221> misc_feature
   <222> (39) .. (39)
   <223> Any nucleotide
<220>
   <221> misc_feature
   <222> (87) .. (87)
   <223> Any nucleotide
<400> 238
   caaaccaaaa cngagcccgt nttcnntntn aacatggcna ccagccaaac cctaagttat
   60
   ctctcacaac aaaagactct tgagtcnagt ttcgttaggg aagaagatga gcgtccaaaa
   120
   gttgcctaca ataacttcag caacgagatt ccaatcattt ctcttgctgg aattgatgag
   180
   gttgatggtc gcagaacaga gatatgtaac aagattgttg aagcttgtga gaattggggt
   240
   atttttcagg ttgttgatca tggtgttgat acaaagcttg tttctgagat gactcgtttt
   300
   gctagagagt tttttgcttt gccgccggaa gagaagctcc ggtttgacat gtccggtggt
   360
   aaaaagggtg gtttcattgt ctctagtcat ctccaaggag aagcagtgaa agattggagg
   420
   gagctagtga catatttttc atacccaatt aaacaaagag attattcaag gtggccagac
   480
   aagccagaag gatggaaaga agtaacagaa aaatacagtg aaaacctaat gaatttagct
   540
   tgcaagctat tggaagtttt atcagaagca at
   572
<210> 239
   <211> 573
   <212> DNA
   <213> Trifolium repens
<220>
   <221> misc_feature
   <222> (7) .. (7)
   <223> Any nucleotide
<220>
   <221> misc_feature
   <222> (15) .. (15)
   <223> Any nucleotide
<220>
   <221> misc_feature
   <222> (23) .. (23)
   <223> Any nucleotide
<220>
   <221> misc_feature
   <222> (25) .. (26)
   <223> Any nucleotides
<220>
   <221> misc_feature
   <222> (28) .. (29)
   <223> Any nucleotides
<220>
   <221> misc_feature
   <222> (38) .. (38)
   <223> Any nucleotide
<220>
   <221> misc_feature
   <222> (41) .. (41)
   <223> Any nucleotide
<220>
   <221> misc_feature
   <222> (65) .. (65)
   <223> Any nucleotide
<400> 239
   aacaccnaca cgagnccgtt tcntnntnna acatggcncc nagccaaact ctaagttatc
   60
   tctcncaaca aaacactctc gagtcaagtt tcgttaggga agaagatgag cgtccaaaag
   120
   ttgcctacaa taacttcagc aacgagattc caatcatttc tcttgctgga attgatgagg
   180
   ttgatggtcg aagaacagag atatgtaaca agattgttga agcttgtgag aattggggta
   240
   tttttcaggt tgttgatcat ggtgttgata caaaacttgt ttctgagatg acccgttttg
   300
   ctagagagtt ttttgctttg ccaccggaag agaagctccg gtttgacatg tccggtggta
   360
   aaaagggtgg tttcattgtc tctagtcatc tccaaggaga agcagtgaag gattggagag
   420
   agctagtgac atatttttca tacccaatta aacaaagaga ttattcaagg tggccagaca
   480
   agccagaagg atggaaagag gtaacagaaa aatacagtga aaacctaatg aatttagctt
   540
   gcaaactatt ggaagtttta tcagaagcaa tgg
   573
<210> 240
   <211> 573
   <212> DNA
   <213> Trifolium repens
<220>
   <221> misc_feature
   <222> (8) .. (8)
   <223> Any nucleotide
<220>
   <221> misc_feature
   <222> (14) .. (14)
   <223> Any nucleotide
<220>
   <221> misc_feature
   <222> (23) .. (24)
   <223> Any nucleotides
<220>
   <221> misc_feature
   <222> (26) .. (26)
   <223> Any nucleotide
<220>
   <221> misc_feature
   <222> (28) .. (28)
   <223> Any nucleotide
<220>
   <221> misc_feature
   <222> (37) .. (37)
   <223> Any nucleotide
<400> 240
   aaccaaancc gagnccgttt ccnntntnaa catggcncca agccaaaccc taagttatct 60
   ctcacaacaa aagactcttg agtcaagttt cgttagggaa gaagatgagc gtccaaaagt 120
   tgcctacaat aacttcagca acgagattcc aatcatttct cttgctggaa ttgatgaggt 180
   tgatggtcgc agaacagaga tatgtaacaa gattgttgaa gcttgtgaga attggggtat 240
   ttttcaggtt gttgatcatg gtgttgatac aaagcttgtt tctgagatga ctcgttttgc 300
   tagagagttt tttgctttgc cgccggaaga gaagctccgg tttgacatgt ccggtggtaa 360
   aaagggtggt ttcattgtct ctagtcatct ccaaggagaa gcagtgaaag attggaggga 420
   gctagtgaca tatttttcat acccaattaa acaaagagat tattcaaggt ggccagacaa 480
   gccagaagga tggaaagaag taacagaaaa atacagtgaa aacctaatga atttagcttg 540
   caagctattg gaagttttat cagaagcaat ggg 573
<210> 241
   <211> 584
   <212> DNA
   <213> Trifolium repens
<220>
   <221> misc_feature
   <222> (9) .. (9)
   <223> Any nucleotide
<220>
   <221> misc_feature
   <222> (14) ..(14)
   <223> Any nucleotide
<220>
   <221> misc_feature
   <222> (19) .. (19)
   <223> Any nucleotide
<220>
   <221> misc_feature
   <222> (23) .. (24)
   <223> Any nucleotides
<220>
   <221> misc_feature
   <222> (28) .. (28)
   <223> Any nucleotide
<220>
   <221> misc_feature
   <222> (37) .. (37)
   <223> Any nucleotide
<220>
   <221> misc_feature
   <222> (40) .. (40)
   <223> Any nucleotide
<400> 241
   aaccaaaanc gagnccgtnt tcnntatnaa catggcnccn agccaaaccc taagttatct 60
   ctcacaacaa aagactcttg agtcaagttt cgttagggaa gaagatgagc gtccaaaagt 120
   tgcctacaat aacttcagca acgagattcc aatcatttct cttgctggaa ttgatgaggt 180
   tgatggtcgc agaacagaga tatgtaacaa gattgttgaa gcttgtgaga attggggtat 240
   ttttcaggtt gttgatcatg gtgttgatac aaagcttgtt tctgagatga ctcgttttgc 300
   tagagagttt tttgctttgc cgccggaaga gaagctccgg tttgacatgt ccggtggtaa 360
   aaagggtggt ttcattgtct ctagtcatct ccaaggagaa gcagtgaaag attggagaga 420
   gctagtgaca tatttttcat acccaattaa acaaagagat tattcaaggt ggccagacaa 480
   gccagaagga tggaaagaag taacagaaaa atacagtgaa aacctaatga atttagcttg 540
   caagctattg gaagttttat cagaagcaat gggattagaa aaag 584
<210> 242
   <211> 529
   <212> DNA
   <213> Trifolium repens
<220>
   <221> misc_feature
   <222> (2) .. (2)
   <223> Any nucleotide
<220>
   <221> misc_feature
   <222> (6) .. (6)
   <223> Any nucleotide
<220>
   <221> misc_feature
   <222> (8) .. (8)
   <223> Any nucleotide
<220>
   <221> misc_feature
   <222> (12) .. (15)
   <223> Any nucleotides
<220>
   <221> misc_feature
   <222> (18) .. (18)
   <223> Any nucleotide
<220>
   <221> misc_feature
   <222> (23) .. (23)
   <223> Any nucleotide
<220>
   <221> misc_feature
   <222> (27) .. (27)
   <223> Any nucleotide
<220>
   <221> misc_feature
   <222> (33) .. (33)
   <223> Any nucleotide
<220>
   <221> misc_feature
   <222> (35) .. (35)
   <223> Any nucleotide
<220>
   <221> misc_feature
   <222> (49) .. (49)
   <223> Any nucleotide
<220>
   <221> misc_feature
   <222> (103) .. (103)
   <223> Any nucleotide
<220>
   <221> misc_feature
   <222> (332) .. (333)
   <223> Any nucleotides
<220>
   <221> misc_feature
   <222> (354) .. (354)
   <223> Any nucleotide
<220>
   <221> misc_feature
   <222> (368) .. (368)
   <223> Any nucleotide
<220>
   <221> misc_feature
   <222> (385) .. (385)
   <223> Any nucleotide
<220>
   <221> misc_feature
   <222> (413) .. (413)
   <223> Any nucleotide
<220>
   <221> misc_feature
   <222> (425) .. (425)
   <223> Any nucleotide
<220>
   <221> misc_feature
   <222> (428) .. (428)
   <223> Any nucleotide
<220>
   <221> misc_feature
   <222> (488) .. (488)
   <223> Any nucleotide
<220>
   <221> misc_feature
   <222> (515) .. (515)
   <223> Any nucleotide
<220>
   <221> misc_feature
   <222> (517) .. (517)
   <223> Any nucleotide
<220>
   <221> misc_feature
   <222> (522) .. (522)
   <223> Any nucleotide
<400> 242
   tngatntntc tnnnnacnaa agnctcntca gtnangtttc gttgggaang aagatgagcg 60
   tccaaaagtt gcctacaata acttcagcaa cgagattcca atnatttctc ttgctggaat 120
   tgatgaggtt gatggtcgca gaacagagat atgtaacaag attgttgaag cttgtgagaa 180
   ttggggtatt tttcaggttg ctgatcatgg tgttgataca aagcttgttt ctgagatgac 240
   tcgttttgct agagagtttt ttgctttgcc gccggaagag aagctccggt ttgacatgtc 300
   cggtggtaaa aagggtggtt tcattgtctc tnntcatctc caaggagaag cagngaaaga 360
   ttggaganag ctagtgacat atttntcata cccaattaaa caaagagatt atncaaggtg 420
   gtcanacnag ccagaaggat ggaaagaagt aacagaaaaa tacagtgaaa acctaatgaa 480
   tttacctngc aagctattgg aagttttata ataancnatg gnattaaga 529
<210> 243
   <211> 698
   <212> DNA
   <213> Trifolium repens
<220>
   <221> misc_feature
   <222> (3) .. (3)
   <223> Any nucleotide
<220>
   <221> misc_feature
   <222> (9) .. (10)
   <223> Any nucleotides
<220>
   <221> misc_feature
   <222> (17) .. (17)
   <223> Any nucleotide
<220>
   <221> misc_feature
   <222> (26) .. (26)
   <223> Any nucleotide
<220>
   <221> misc_feature
   <222> (685) .. (685)
   <223> Any nucleotide
<400> 243
   gtnaccagnn tgttganctt gtgagnattg gggtattttt caggttgttg atcatggtgt 60
   tgatacaaga cttgtttctg agatgacccg ttttgctaga gagttttttg ctttgccacc 120
   ggaagagaag ctccggtttg acatgtccgg tggtaaaaag ggtggtttca ttgtctctag 180
   tcatctccaa ggagaagcag tgaaggattg gagagagcta gtgacatatt tttcataccc 240
   aattaaacaa agagattatt caaggtggcc agacaagcca gaaggatgga aagaggtaac 300
   agaaaaatac agtgaaaacc taatgaattt agcttgcaaa ctattggaag ttttatcaga 360
   agcaatgggt ttagaaaaag aagctctaac aaaagcatgt gttgatatgg atcaaaaagt 420
   tgttataaat tattacccaa aatgccctga acctgacctc acacttggcc ttaaacgtca 480
   cactgaccct ggcacaatta ctcttttgct tcaagatcaa gttggtggtc ttcaagctac 540
   caaagataat ggtaagacgt ggattacagt tcaaccagtt gaaggtgctt ttgttgttaa 600
   tcttggagac catggtcact atctaagtaa tggacggttc aaaaatgctg accatcaagc 660
   agtggtgaat tcgaactaca gccgnttatc aatagcaa 698
<210> 244
   <211> 579
   <212> DNA
   <213> Trifolium repens
<220>
   <221> misc feature
   <222> (4) .. (4)
   <223> Any nucleotide
<220>
   <221> misc_feature
   <222> (20) .. (20)
   <223> Any nucleotide
<220>
   <221> misc_feature
   <222> (29) .. (29)
   <223> Any nucleotide
<220>
   <221> misc_feature
   <222> (83) .. (83)
   <223> Any nucleotide
<400> 244
   atcnaaaagt tgtgataaan tattacccna aatgccctga acctgacctc acacttggcc 60
   ttaaacgcca cactgaccct ggnacaatta ctcttttgct tcaagatcaa gttggtggcc 120
   ttcaagctac caaagataat ggtaagacgt ggattacagt tcaaccagtt gaaggtgctt 180
   ttgttgttaa tcttggagac catggtcatt atctaagtaa tggacggttc aaaaatgctg 240
   accaccaagc agtggtgaat tcgaactaca gccgtttatc aatagcaaca tttcaaaatc 300
   cagctcccga tgcaactgta taccctttga agattagaga gggtgaaaaa tctgtgttgg 360
   aagaaccaat cacttttgct gaaatgtata gaaggaagat gaccaaagac cttgaaattg 420
   ctaggatgaa gaagttggct aaggaacaac aacttaggga cttggaggag aacaagacta 480
   aatatgaggc caaacctttg aatgagatct ttgcttaatt aattagtctt aatttaaata 540
   attaataaat tttagactta atttacatat aataatttt 579

<210> 245
   <211> 601
   <212> DNA
   <213> Trifolium repens
<220>
   <221> misc_feature
   <222> (2) .. (2)
   <223> Any nucleotide
<220>
   <221> misc_feature
   <222> (22) .. (22)
   <223> Any nucleotide
<220>
   <221> misc_feature
   <222> (30) .. (30)
   <223> Any nucleotide
<400> 245
   gnagcataac ataaaccctg tncccgattn atgtaacaca atctcccctt ttcttattac
   60
   aagtaaaata ccataacaca ataatatgaa taccataatc ttgaatcata caaacaacct
   120
   tggatcaaac aaaacaacaa ccatggttga tctagaaaca gaaccaagtt caccatttat
   180
   tcaatcccca gaacacagac caaaatcctc aataatcatt gctgaaggta tccctctaat
   240
   tgatctcact cctataaact acaaagatga aatcatcacc aacccacttt ccattgaaga
   300
   cttagtcaaa gaaataggca aagcatgtaa agaatggggt ttctttcaag tgattaatca
   360
   caaagttcct ttggataaac gtgaaaggat tgaagaatct tcaaagaagt tttttgaact
   420
   tagtttggag gaaaaactta aggtgagaag agatgaagtt aatttgcttg gttattttga
   480
   agctgagcat acaaaaaatg ttagggactg gaaggaaatt tatgatttta atgtgcaaca
   540
   accaactttt ataccacctt cggatgacca aagttttcag tttcaatggg aaaatcgatg
   600
   g
   601
<210> 246
   <211> 172
   <212> PRT
   <213> Trifolium repens
<400> 246
   Met Asn Thr Ile Ile Leu Asn His Thr Asn Asn Leu Gly Ser Asn Lys
   1 5 10 15
   Thr Thr Thr Met Val Asp Leu Glu Thr Glu Pro Ser Ser Pro Phe Ile
   20 25 30
   Gln Ser Pro Glu His Arg Pro Lys Ser Ser Ile Ile Ile Ala Glu Gly
   35 40 45
   Ile Pro Leu Ile Asp Leu Thr Pro Ile Asn Tyr Lys Asp Glu Ile Ile
   50 55 60
   Thr Asn Pro Leu Ser Ile Glu Asp Leu Val Lys Glu Ile Gly Lys Ala
   65 70 75 80
   Cys Lys Glu Trp Gly Phe Phe Gln Val Ile Asn His Lys Val Pro Leu
   85 90 95
   Asp Lys Arg Glu Arg Ile Glu Glu Ser Ser Lys Lys Phe Phe Glu Leu
   100 105 110
   Ser Leu Glu Glu Lys Leu Lys Val Arg Arg Asp Glu Val Asn Leu Leu
   115 120 125
   Gly Tyr Phe Glu Ala Glu His Thr Lys Asn Val Arg Asp Trp Lys Glu
   130 135 140
   Ile Tyr Asp Phe Asn Val Gln Gln Pro Thr Phe Ile Pro Pro Ser Asp
   145 150 155 160
   Asp Gln Ser Phe Gln Phe Gln Trp Glu Asn Arg Trp
   165 170
<210> 247
   <211> 585
   <212> DNA
   <213> Trifolium repens
<220>
   <221> misc_feature
   <222> (13) .. (13)
   <223> Any nucleotide
<400> 247
   ttacccaaca atnatgtgtg actgatgtta gtgtaccagg aaagatggga gaggtggatc
   60
   cagctttctt caaatccaga aaataggcca aaactttcca taatccaagc tgaaggaatt 120
   cctgtaatca atctctcccc attaattcac cacacagttc aagactcctc tgccattgaa 180
   agcttagtca aagaaatagg aaatgcttgc aaggaatggg gtttcttcca agtaacaaac 240
   catggtgtcc ctctaaatct aaggctcaga ctcgaggaag ctaccaaagt tttctttgca 300
   cagagtttgg aggagaagag gaagcttacc gtagatgata acagtttgcc tggttatcat 360
   gatacagagc acaccaagaa tgtcagagac tggaaagaag tgtttgattt tttatccaaa 420
   gaccccactt tgattcctct gaattctgat gaacatgatg atcgagtcac tcaatggact 480
   aatccatccc ctcaatatcc tccaaacttc aaagttattt tggaagagta tattaaagag 540
   atggaaaagc taggctttaa gttgctagag cttatagctt tgagc 585
<210> 248
   <211> 187
   <212> PRT
   <213> Trifolium repens
<400> 248
   Met Leu Val Tyr Gln Glu Arg Trp Glu Arg Trp Ile Gln Leu Ser Ser
   1               5                   10                  15
   Asn Pro Glu Asn Arg Pro Lys Leu Ser Ile Ile Gln Ala Glu Gly Ile
               20                  25                  30
   Pro Val Ile Asn Leu Ser Pro Leu Ile His His Thr Val Gln Asp Ser
           35                  40                  45
   Ser Ala Ile Glu Ser Leu Val Lys Glu Ile Gly Asn Ala Cys Lys Glu
       50                  55                  60
   Trp Gly Phe Phe Gln Val Thr Asn His Gly Val Pro Leu Asn Leu Arg
   65                  70                  75                  80
   Leu Arg Leu Glu Glu Ala Thr Lys Val Phe Phe Ala Gln Ser Leu Glu
                   85                  90                  95
   Glu Lys Arg Lys Leu Thr Val Asp Asp Asn Ser Leu Pro Gly Tyr His
               100                 105                 110
   Asp Thr Glu His Thr Lys Asn Val Arg Asp Trp Lys Glu Val Phe Asp
           115                 120                 125
   Phe Leu Ser Lys Asp Pro Thr Leu Ile Pro Leu Asn Ser Asp Glu His
       130                 135                 140
   Asp Asp Arg Val Thr Gln Trp Thr Asn Pro Ser Pro Gln Tyr Pro Pro
   145                 150                 155                 160
   Asn Phe Lys Val Ile Leu Glu Glu Tyr Ile Lys Glu Met Glu Lys Leu
                   165                 170                 175
   Gly Phe Lys Leu Leu Glu Leu Ile Ala Leu Ser
               180                 185
<210> 249
   <211> 604
   <212> DNA
   <213> Trifolium repens
<400> 249
   gggaatggtg gaggcgaatg tgaccctagg gctgatgaat tagtaatggt agttgagctt 60
   atggcgttag ctggagtttt caatattggt gattttgttc ctgctttgga atggttagat 120
   attcaaggtg tacaaggaaa aatgaagaaa ttacataaaa gatttgatgc atttttaact 180
   agcattattg aagatcacat gatttccaag agtgagaagc ataatgactt attgagtacg 240
   ttgttatcac taaaagaaaa agttgatgag gatggtgaca aacttaatga tactgagatc 300
   aaagcattac tcttgaacat gttcacagct ggaacagaca catcatcaag cacaacagag 360
   tgggctattg ctgaactaat aaaaaatcca aaactaatga ttcgtgttca aaatgagttg 420
   gacactgttg tgggccgaga caagcttgta actgaacaag acttggccca tcttccttac 480
   ttagaggctg taataaagga gacatttcgt ctccatccat caacccctct ttctctccca 540
   cgtgttgcaa caaatagttg tgaaatcctc gactatcaca ttcccaaagg tgcaactctc 600
   ttgg 604
<210> 250
   <211> 201
   <212> PRT
   <213> Trifolium repens
<400> 250
   Gly Asn Gly Gly Gly Glu Cys Asp Pro Arg Ala Asp Glu Leu Val Met
   1 5 10 15
   Val Val Glu Leu Met Ala Leu Ala Gly Val Phe Asn Ile Gly Asp Phe
   20 25 30
   Val Pro Ala Leu Glu Trp Leu Asp Ile Gln Gly Val Gln Gly Lys Met
   35 40 45
   Lys Lys Leu His Lys Arg Phe Asp Ala Phe Leu Thr Ser Ile Ile Glu
   50 55 60
   Asp His Met Ile Ser Lys Ser Glu Lys His Asn Asp Leu Leu Ser Thr
   65 70 75 80
   Leu Leu Ser Leu Lys Glu Lys Val Asp Glu Asp Gly Asp Lys Leu Asn
   85 90 95
   Asp Thr Glu Ile Lys Ala Leu Leu Leu Asn Met Phe Thr Ala Gly Thr
   100 105 110
   Asp Thr Ser Ser Ser Thr Thr Glu Trp Ala Ile Ala Glu Leu Ile Lys
   115 120 125
   Asn Pro Lys Leu Met Ile Arg Val Gln Asn Glu Leu Asp Thr Val Val
   130 135 140
   Gly Arg Asp Lys Leu Val Thr Glu Gln Asp Leu Ala His Leu Pro Tyr
   145 150 155 160
   Leu Glu Ala Val Ile Lys Glu Thr Phe Arg Leu His Pro Ser Thr Pro
   165 170 175
   Leu Ser Leu Pro Arg Val Ala Thr Asn Ser Cys Glu Ile Leu Asp Tyr
   180 185 190
   His Ile Pro Lys Gly Ala Thr Leu Leu
   195 200
<210> 251
   <211> 581
   <212> DNA
   <213> Trifolium repens
<400> 251
   gggaatggtg gaggcgaatg tgaccctagg gctgatgaat ttaagtaatg gtagttgagc 60
   ttatggcgtt agctggagtt ttcaatattg gtgattttgt tcctgctttg gaatggttag 120
   atattcaagg tgtacaagga aaaatgaaga aattacataa aagatttgat gcatttttaa 180
   ctagcattat tgaagatcac atgatttcca agagtgagaa gcataatgac ttattgagta 240
   cgttgttatc actaaaagaa aaagttgatg aggatggtga caaacttaat gatactgaga 300
   tcaaagcatt actcttgaac atgttcacag ctggaacaga cacatcatca agcacaacag 360
   agtgggctat tgctgaacta ataaaaaatc caaaactaat gattcgtgtt caaaatgagt 420
   tggacactgt tgtgggccga gacaagcttg taactgaaca agacttggcc catcttcctt 480
   acttagaggc tgtaataaag gagacatttc gtctccatcc atcaacccct ctttctctcc 540
   cacgtgttgc aacaaatagt tgtgaaatcc tcgactatca c 581
<210> 252
   <211> 603
   <212> DNA
   <213> Trifolium repens
<220>
   <221> misc_feature
   <222> (44) .. (44)
   <223> Any nucleotide
<400> 252
   aatggtggag gtgaatgtga ccctagggct gatgaattta agtnatggta gttgagctta 60
   tggcgttagc tggagttttc aatattggtg attttgttcc tgctttggaa tggttagata 120
   ttcaaggtgt acaaggaaaa atgaagaaat tacataaaag atttgatgca tttttaacta 180
   gcattattga agatcacatg atttccaaga gtgagaagca taatgactta ttgagtacgt 240
   tgttatcact aaaagaaaaa gttgatgagg atggtgacaa acttaatgat actgagatca 300
   aagcattact cttgaacatg ttcacagctg gaacagacac atcatcaagc acaacagagt 360
   gggctattgc tgaactaata aaaaatccaa aactaatgat tcgtgttcaa aatgagttgc 420
   acactgttgt gggccgagac aagcttgtaa ctgaacaaga cttggcccat cttccttact 480
   tagaggctgt aataaaggag acatttcgtc tccatccatc aacccctctt tctctcccac 540
   gtgttgcaac aaatagttgt gaaatcctcg actatcacat tcccaaaggt gcaactctct 600
   tgg 603
<210> 253
   <211> 621
   <212> DNA
   <213> Trifolium repens
<220>
   <221> misc_feature
   <222> (2) .. (2)
   <223> Any nucleotide
<220>
   <221> misc_feature
   <222> (38) .. (38)
   <223> Any nucleotide
<220>
   <221> misc_feature
   <222> (40) .. (40)
   <223> Any nucleotide
<220>
   <221> misc_feature
   <222> (44) .. (44)
   <223> Any nucleotide
<220>
   <221> misc_feature
   <222> (47) .. (47)
   <223> Any nucleotide
<220>
   <221> misc_feature
   <222> (58) .. (58)
   <223> Any nucleotide
<220>
   <221> misc_feature
   <222> (91) .. (91)
   <223> Any nucleotide
<220>
   <221> misc_feature
   <222> (104) .. (105)
   <223> Any nucleotides
<220>
   <221> misc_feature
   <222> (120) .. (120)
   <223> Any nucleotide
<220>
   <221> misc_feature
   <222> (124) .. (124)
   <223> Any nucleotide
<220>
   <221> misc_feature
   <222> (566) .. (566)
   <223> Any nucleotide
<220>
   <221> misc_feature
   <222> (598) .. (598)
   <223> Any nucleotide
<400> 253
   gnaggaaatt tcaactaaat attgccttta attctttntn atanatnttt gaatttcntt
   60
   ctccctaaaa attctatagc taccacatca ncacaacata acannaatta agaaatattn
   120
   tatntactat tttaagatat ggaagtagta gcagcagcaa tcacaaaaaa caatggcaag
   180
   attgattcat tttgcttgaa tcatgctaat gctaataaca tgaaagtgaa tggtgctgat
   240
   cctttgaatt ggggtgtggc tgctgaggca atgaagggaa gtcacttgga tgaggtgaag
   300
   cgtatggtgg aggaataccg gaaaccggtt gtccgtcttg gtggcgagac actgacgatt
   360
   tctcaggtgg ctgccattgc tgcacacgat ggtgcaacgg tggagctatc ggaatctgct
   420
   agagccggcg ttaaggcgag cagtgactgg gttatggaga gtatgaacaa aggtacagac
   480
   agttatggtg tcactacagg gttcggcgct acctcgcacc gccgaaccaa acaaggtggt
   540
   gctttgcaga aagagctcat aaggtntttt gaatgcagga atatttggaa atggaacntg
   600
   agacaaagcc acacactacc c
   621
<210> 254
   <211> 159
   <212> PRT
   <213> Trifolium repens
<400> 254
   Met Glu Val Val Ala Ala Ala Ile Thr Lys Asn Asn Gly Lys Ile Asp
   1 5 10 15
   Ser Phe Cys Leu Asn His Ala Asn Ala Asn Asn Met Lys Val Asn Gly
   20 25 30
   Ala Asp Pro Leu Asn Trp Gly Val Ala Ala Glu Ala Met Lys Gly Ser
   35 40 45
   His Leu Asp Glu Val Lys Arg Met Val Glu Glu Tyr Arg Lys Pro Val
   50 55 60
   Val Arg Leu Gly Gly Glu Thr Leu Thr Ile Ser Gln Val Ala Ala Ile
   65 70 75 80
   Ala Ala His Asp Gly Ala Thr Val Glu Leu Ser Glu Ser Ala Arg Ala
   85 90 95
   Gly Val Lys Ala Ser Ser Asp Trp Val Met Glu Ser Met Asn Lys Gly
   100 105 110
   Thr Asp Ser Tyr Gly Val Thr Thr Gly Phe Gly Ala Thr Ser His Arg
   115 120 125
   Arg Thr Lys Gln Gly Gly Ala Leu Gln Lys Glu Leu Ile Arg Phe Glu
   130 135 140
   Cys Arg Asn Ile Trp Lys Trp Asn Arg Gln Ser His Thr Leu Pro
   145 150 155
<210> 255
   <211> 582
   <212> DNA
   <213> Trifolium repens
<220>
   <221> misc_feature
   <222> (2) .. (3)
   <223> Any nucleotides
<220>
   <221> misc_feature
   <222> (11) .. (11)
   <223> Any nucleotide
<220>
   <221> misc_feature
   <222> (38) .. (38)
   <223> Any nucleotide
<220>
   <221> misc_feature
   <222> (40) .. (40)
   <223> Any nucleotide
<220>
   <221> misc_feature
   <222> (44) .. (44)
   <223> Any nucleotide
<220>
   <221> misc_feature
   <222> (47) .. (47)
   <223> Any nucleotide
<220>
   <221> misc_feature
   <222> (91) .. (91)
   <223> Any nucleotide
<220>
   <221> misc_feature
   <222> (104) .. (105)
   <223> Any nucleotides
<220>
   <221> misc_feature
   <222> (120) .. (120)
   <223> Any nucleotide
<220>
   <221> misc_feature
   <222> (124) .. (124)
   <223> Any nucleotide
<220>
   <221> misc_feature
   <222> (154) .. (154)
   <223> Any nucleotide
<220>
   <221> misc_feature
   <222> (405) .. (405)
   <223> Any nucleotide
<220>
   <221> misc_feature
   <222> (516) .. (516)
   <223> Any nucleotide
<220>
   <221> misc_feature
   <222> (549) .. (549)
   <223> Any nucleotide
<400> 255
   gnnggaaatt ncaactaaat attgccttta attctttntn atanatnttt gaatttcctt 60
   ctccctaaaa attctatagc taccacatca ncacaacata acannaatta agaaatattn 120
   tatntactat tttaagatat ggaagtagta gcancagcaa tcacaaaaaa caatggcaag 180
   attgattcat tttgcttgaa tcatgctaat gctaataaca tgaaagtgaa tggtgctgat 240
   cctttgaatt ggggtgtggc tgctgaggca atgaagggaa gtcacttgga tgaggtgaag 300
   cgtatggtgg aggaataccg gaaaccggtt gtccgtcttg gtggcgagac gctgacgatt 360
   tctcaggtgg ctgccattgc tgcacacgat ggtgcaacgg tgganctatc ggaatctgct 420
   agagccggcg ttaaggcgag cagtgactgg gttatggaga gcatgaacaa aggtacagac 480
   agttatggtg tcactacggg gttcggcgct acctcncacc gccgaaccaa acaaggtggt 540
   gctttgcana aagagctcat aagctaattt gcttgtgtca at 582

<210> 256
   <211> 621
   <212> DNA
   <213> Trifolium repens
<220>
   <221> misc_feature
   <222> (2) .. (2)
   <223> Any nucleotide
<220>
   <221> misc_feature
   <222> (24) .. (24)
   <223> Any nucleotide
<220>
   <221> misc_feature
   <222> (40) .. (40)
   <223> Any nucleotide
<220>
   <221> misc_feature
   <222> (44) .. (44)
   <223> Any nucleotide
<220>
   <221> misc_feature
   <222> (47) .. (47)
   <223> Any nucleotide
<220>
   <221> misc_feature
   <222> (58) .. (58)
   <223> Any nucleotide
<220>
   <221> misc_feature
   <222> (91) .. (91)
   <223> Any nucleotide
<220>
   <221> misc_feature
   <222> (104) .. (105)
   <223> Any nucleotides
<220>
   <221> misc_feature
   <222> (120) .. (120)
   <223> Any nucleotide
<220>
   <221> misc_feature
   <222> (124) .. (124)
   <223> Any nucleotide
<220>
   <221> misc_feature
   <222> (131) .. (131)
   <223> Any nucleotide
<220>
   <221> misc_feature
   <222> (566) .. (566)
   <223> Any nucleotide
<220>
   <221> misc_feature
   <222> (598) .. (598)
   <223> Any nucleotide
<220>
   <221> misc_feature
   <222> (605) .. (605)
   <223> Any nucleotide
<400> 256
   gnaggaaatt acaactaaat attnccttta attctttatn atanatnttt gaatttcntt
   60
   ctccctaaaa attctatagc taccacatca ncacaacata acannaatta agaaatattn
   120
   tatntactat nttaagatat ggaagtagta gcagcagcaa tcacaaaaaa caatggcaag
   180
   attgattcat tttgcttgaa tcatgctaat gctaataaca tgaaagtgaa tggtgctgat
   240
   cctttgaatt ggggtgtggc tgctgaggca atgaagggaa gtcacttgga tgaggtgaag
   300
   cgtatggtgg aggaataccg gaaaccggtt gtccgtcttg gtggcgagac actaaccatt
   360
   tctcaggtgg ctgccattgc tgcacacgat ggtgcaacgg tggagctatc ggaatctgct
   420
   agagccggcg ttaaggcaag cagtgactgg gttatggaga gtatgaacaa aggtaccgac
   480
   agctacggtg tcccaacagg gttcggcgct acctcgcacc gccgaaccaa acaaggtggt
   540
   gctttgcaga aagagctcat aaggtntttt gaatgctgga atatttggaa atggaacntg
   600
   agtcnaagcc acacactacc c
   621
<210> 257
   <211> 600
   <212> DNA
   <213> Trifolium repens
<220>
   <221> misc_feature
   <222> (20) .. (21)
   <223> Any nucleotides
<220>
   <221> misc_feature
   <222> (28) .. (28)
   <223> Any nucleotide
<220>
   <221> misc_feature
   <222> (33) .. (33)
   <223> Any nucleotide
<220>
   <221> misc_feature
   <222> (42) .. (42)
   <223> Any nucleotide
<220>
   <221> misc_feature
   <222> (48) .. (48)
   <223> Any nucleotide
<220>
   <221> misc_feature
   <222> (57) .. (57)
   <223> Any nucleotide
<220>
   <221> misc_feature
   <222> (127) .. (128)
   <223> Any nucleotides
<220>
   <221> misc_feature
   <222> (556) .. (556)
   <223> Any nucleotide
<220>
   <221> misc_feature
   <222> (592) .. (592)
   <223> Any nucleotide
<400> 257
   tcaagaaatt acactctttn nttctttnta atntttgttt tnatttcntt ctctctncaa
   60
   atactatagt taccaaaaca taacaaagta acacttatta ctagctatta tttgtatcaa
   120
   tttaagnnat ggaagtagta gcagcagcaa tcacaaaaaa caacggaaag attgattcat
   180
   tttgcttgaa tcatgctaat gctaataaca tgaaagtgaa tggtgctgat cctttgaatt
   240
   ggggtgtggc tgctgaggca atgaaaggaa gtcacttgga tgaggtgaag tgtatggtgg
   300
   aggagtatcg aaaaccggtt gtccgtcttg gtggcgagac actgacgatt tctcaagtgg
   360
   ctgccattgc tgcacacgat ggtgcgacgg tggagctatc ggaatctgct agagccggcg
   420
   ttaaggcgag cagtgactgg gttatggaaa gtatgaacaa aggtactgac agttatggtg
   480
   tcactacagg gttcggcgct acctcgcacc gccgaaccaa acaaggtggt gctttgcaga
   540
   aagagctcat aaggtntttt gaatgcagga atatttggaa atggaacttg anacaaatcc
   600
<210> 258
   <211> 693
   <212> DNA
   <213> Trifolium repens
<220>
   <221> misc_feature
   <222> (2) .. (2)
   <223> Any nucleotide
<220>
   <221> misc_feature
   <222> (9) .. (9)
   <223> Any nucleotide
<220>
   <221> misc_feature
   <222> (14) .. (14)
   <223> Any nucleotide
<220>
   <221> misc_feature
   <222> (47) .. (47)
   <223> Any nucleotide
<220>
   <221> misc_feature
   <222> (52) .. (52)
   <223> Any nucleotide
<220>
   <221> misc_feature
   <222> (60) .. (60)
   <223> Any nucleotide
<220>
   <221> misc_feature
   <222> (86) .. (86)
   <223> Any nucleotide
<220>
   <221> misc_feature
   <222> (95) .. (95)
   <223> Any nucleotide
<400> 258
   gnaggaaana attntattgt tattatttcc ccccacacaa cggaaanaat tntattgttn 60
   cttatttccc cccacacaac ataacnaata cattntcctc tcctctcatc acaattatta 120
   ctttctacac acccccctct caactattat taactaacat aatggaggga attaccaatg 180
   gccatgctga agcaactttt tgcgtgacca aaagtgttgg tgatccactc aactggggtg 240
   cagccgcgga gtcgttgatg gggagtcatt tggatgaggt gaagcgtatg gtggaggaat 300
   accgtaatcc attggttaaa attggcggcg agacgcttac cattgctcag gtggctggaa 360
   ttgcttctca tgatagtggt gtgagggtgg agctgtctga gtccgccagg gccggcgtta 420
   aggcgagtag tgattgggtg atggacagca tgaacaatgg gactgatagt tatggtgtta 480
   ccaccggttt cggcgccacc tctcaccgga gaaccaagca gggtggtgcc ttgcagaagg 540
   agctaattag gtttttgaat gctggaatat ttggcaatgg tacagaatct aactgtacac 600
   taccacacac agcaaccaga gctgcaatgc ttgtgagaat caacactctt cttcaagagg 660
   aatatttctt gaatggcctt tgtaaatttt tgg 693
<210> 259
   <211> 177
   <212> PRT
   <213> Trifolium repens
<400> 259
   Met Glu Gly Ile Thr Asn Gly His Ala Glu Ala Thr Phe Cys Val Thr
   1               5                   10                  15
   Lys Ser Val Gly Asp Pro Leu Asn Trp Gly Ala Ala Ala Glu Ser Leu
               20                  25                  30
   Met Gly Ser His Leu Asp Glu Val Lys Arg Met Val Glu Glu Tyr Arg
           35                  40                  45
   Asn Pro Leu Val Lys Ile Gly Gly Glu Thr Leu Thr Ile Ala Gln Val
       50                  55                  60
   Ala Gly Ile Ala Ser His Asp Ser Gly Val Arg Val Glu Leu Ser Glu
   65                  70                  75                  80
   Ser Ala Arg Ala Gly Val Lys Ala Ser Ser Asp Trp Val Met Asp Ser
                   85                  90                  95
   Met Asn Asn Gly Thr Asp Ser Tyr Gly Val Thr Thr Gly Phe Gly Ala
               100                 105                 110
   Thr Ser His Arg Arg Thr Lys Gln Gly Gly Ala Leu Gln Lys Glu Leu
           115                 120                 125
   Ile Arg Phe Leu Asn Ala Gly Ile Phe Gly Asn Gly Thr Glu Ser Asn
       130                 135                 140
   Cys Thr Leu Pro His Thr Ala Thr Arg Ala Ala Met Leu Val Arg Ile
   145                 150                 155                 160
   Asn Thr Leu Leu Gln Glu Glu Tyr Phe Leu Asn Gly Leu Cys Lys Phe
                   165                 170                 175
   Leu
<210> 260
   <211> 592
   <212> DNA
   <213> Trifolium repens
<220>
   <221> misc_feature
   <222> (2) .. (2)
   <223> Any nucleotide
<220>
   <221> misc_feature
   <222> (9) .. (9)
   <223> Any nucleotide
<220>
   <221> misc_feature
   <222> (14) .. (14)
   <223> Any nucleotide
<220>
   <221> misc_feature
   <222> (53) .. (53)
   <223> Any nucleotide
<220>
   <221> misc_feature
   <222> (60) .. (60)
   <223> Any nucleotide
<220>
   <221> misc_feature
   <222> (77) .. (77)
   <223> Any nucleotide
<220>
   <221> misc_feature
   <222> (86) .. (87)
   <223> Any nucleotides
<220>
   <221> misc_feature
   <222> (92) .. (92)
   <223> Any nucleotide
<220>
   <221> misc_feature
   <222> (96) .. (96)
   <223> Any nucleotide
<220>
   <221> misc_feature
   <222> (99) .. (99)
   <223> Any nucleotide
<220>
   <221> misc_feature
   <222> (101) .. (102)
   <223> Any nucleotides
<220>
   <221> misc_feature
   <222> (105) .. (106)
   <223> Any nucleotides
<220>
   <221> misc_feature
   <222> (128) .. (128)
   <223> Any nucleotide
<220>
   <221> misc_feature
   <222> (592) .. (592)
   <223> Any nucleotide
<400> 260
   gnaggaaana attntattgt tattatttcc ccccacacaa cataacaaat acnattattn 60
   ctctcctctc atcacanatt attacnnttt cntacnacnc nnctnncctc tcaactatta 120
   ttaactangc ataatggagg gaattaccaa tggccagctg ctttcaaaaa aaaaccaatg 180
   gccatgctga aacaactttt tgcgtgacca aaagtgttgg tgatccactc aactggggtg 240
   cagccgcgga gtcgttgacg ggtagtcatt tggatgaggt gaagcgtatg gtggaggagt 300
   accgtaatcc gttggttaaa attggcggcg agacgcttac cattgctcag gtggctggaa 360
   ttgcttctca tgatagtggt gtgagggtgg agctgtccga gtccgcaagg gccggcgtta 420
   aggcgagtag tgattgggtg atggatagca tgaacaatgg gactgatagt tacggtgtta 480
   ccaccggttt tggtgccacc tctcaccgga gaaccaagca gggtggtgcc ttgcagaagg 540
   agctaattag gtttttgaat gctggaatat ttggcaatgg tacagaatct an 592
<210> 261
   <211> 618
   <212> DNA
   <213> Trifolium repens
<220>
   <221> misc_feature
   <222> (8) .. (8)
   <223> Any nucloetide
<220>
   <221> misc_feature
   <222> (13) .. (13)
   <223> Any nucloetide
<220>
   <221> misc_feature
   <222> (21) .. (22)
   <223> Any nucloetides
<220>
   <221> misc_feature
   <222> (56) .. (56)
   <223> Any nucloetide
<220>
   <221> misc_feature
   <222> (91) .. (92)
   <223> Any nucloetides
<400> 261
   gaggaaanaa ttntattgtt nntttttcct acccacacaa cataacaaat acattntcct 60
   ctcctctcat cacaattatt actttctaca nncccccctc tcaagtatta ttaactaaca 120
   taatggaggg aattaccaat ggccatgctg aagcaacttt ttgcgtgacc aaaagtgttg 180
   gtgatccact caactggggt gcagccgcgg agtcgttgat ggggagtcat ttggatgagg 240
   tgaagcgtat ggtggaggaa taccgtaatc cattggttaa aattggcggc gagacgctta 300
   ccattgctca ggtggctgga attgcttctc atgatagtgg tgtgagggtg gagctgtctg 360
   agtccgcgag ggccggcgtt aaggcgagta gtgattgggt gatggacagc atgaacaatg 420
   ggactgatag ttatggtgtt accaccggtt tcggtgccac ctctcaccgg agaaccaagc 480
   aaggtggtgc cttgcagaag gagctaatta ggtttttgaa tgctggaata tttggcaatg 540
   gtacagaatc taactgtaca ctaccacaca cagcaaccag agctgcaatg cttgtgagaa 600
   tcaacactct tcttcaag 618
<210> 262
   <211> 654
   <212> DNA
   <213> Trifolium repens
<220>
   <221> misc_feature
   <222> (9) .. (9)
   <223> Any nucleotide
<220>
   <221> misc_feature
   <222> (14) .. (14)
   <223> Any nucleotide
<220>
   <221> misc_feature
   <222> (19) .. (19)
   <223> Any nucleotide
<220>
   <221> misc_feature
   <222> (21) .. (21)
   <223> Any nucleotide
<220>
   <221> misc_feature
   <222> (38) .. (38)
   <223> Any nucleotide
<220>
   <221> misc_feature
   <222> (47) .. (47)
   <223> Any nucleotide
<220>
   <221> misc_feature
   <222> (58) .. (58)
   <223> Any nucleotide
<220>
   <221> misc_feature
   <222> (60) .. (60)
   <223> Any nucleotide
<220>
   <221> misc_feature
   <222> (66) .. (66)
   <223> Any nucleotide
<220>
   <221> misc_feature
   <222> (89) .. (90)
   <223> Any nucleotides
<220>
   <221> misc_feature
   <222> (167) .. (167)
   <223> Any nucleotide
<220>
   <221> misc_feature
   <222> (216) .. (216)
   <223> Any nucleotide
<220>
   <221> misc_feature
   <222> (228) .. (228)
   <223> Any nucleotide
<220>
   <221> misc_feature
   <222> (245) .. (245)
   <223> Any nucleotide
<220>
   <221> misc_feature
   <222> (338) .. (338)
   <223> Any nucleotide

<220>
   <221> misc_feature
   <222> (340) .. (340)
   <223> Any nucleotide
<220>
   <221> misc_feature
   <222> (364) .. (364)
   <223> Any nucleotide
<220>
   <221> misc_feature
   <222> (383) .. (383)
   <223> Any nucleotide
<220>
   <221> misc_feature
   <222> (398) .. (398)
   <223> Any nucleotide
<220>
   <221> misc_feature
   <222> (410) .. (410)
   <223> Any nucleotide
<220>
   <221> misc_feature
   <222> (437) .. (437)
   <223> Any nucleotide
<220>
   <221> misc_feature
   <222> (440) .. (440)
   <223> Any nucleotide
<220>
   <221> misc_feature
   <222> (565) .. (565)
   <223> Any nucleotide
<220>
   <221> misc_feature
   <222> (580) .. (580)
   <223> Any nucleotide
<220>
   <221> misc_feature
   <222> (583) .. (583)
   <223> Any nucleotide
<220>
   <221> misc_feature
   <222> (601) .. (602)
   <223> Any nucleotides
<220>
   <221> misc_feature
   <222> (608) .. (608)
   <223> Any nucleotide
<220>
   <221> misc_feature
   <222> (617) .. (618)
   <223> Any nucleotides
<400> 262
   ccttgaatna ttgntttgnc ncacccaaca caacatanac aaatacnatt tcttgctncn
   60
   ctactngcat cacaattatt actttctann cacccccctc tcaactatta ttaactaaca
   120
   taatggaggg aattactaat ggccatgctg aagcaacttt ttgcgtnacc aaaagtgttg 180
   gtgatccact caactggggt gcagccgcgg agtcgntgat ggggagtnat ttggatgagg 240
   tgaancgtat ggtggaggaa taccgtaatc cattggttaa aattggcggc gagacgctta 300
   ccattgctca ggtggctgga attgcttctc atgatagngn tgtgagggtg gagctgtctg 360
   agtncgccag ggccggcgtt aangcgagta gtgattgngt gatggacagn atgaacaatg 420
   ggactgatag ttatggngtn accaccggtt tcggcgccac ctctcaccgg agaaccaagc 480
   agggtggtgc cttgcaaaag gagctaatta tgtttttgaa tgctggaata tttggcaatg 540
   gtacagaatc taactgtaca cttcnacaca cagcaaccan agntgcattg ctttggtgca 600
   nnatcacnct tcttctnnag gaatatttct tgaatggcct ttgtaaattt ttgg 654
<210> 263
   <211> 590
   <212> DNA
   <213> Trifolium repens
<220>
   <221> misc_feature
   <222> (6) .. (6)
   <223> Any nucleotide
<220>
   <221> misc_feature
   <222> (13) .. (13)
   <223> Any nucleotide
<220>
   <221> misc_feature
   <222> (19) .. (21)
   <223> Any nucleotides
<220>
   <221> misc_feature
   <222> (37) .. (37)
   <223> Any nucleotide
<220>
   <221> misc_feature
   <222> (45) .. (45)
   <223> Any nucleotide
<220>
   <221> misc_feature
   <222> (54) .. (54)
   <323> Any nucleotide
<220>
   <221> misc_feature
   <222> (66) .. (66)
   <223> Any nucleotide
<220>
   <221> misc_feature
   <222> (66) .. (66)
   <223>
<220>
   <221> misc_feature
   <222> (89) .. (90)
   <223> Any nucleotides
<400> 263
   ggaaanaatt atnttgttnn ntttttcccc ccacacnaca taacnaatac attntcttct
   60
   cctctnatca caattattac tttctacann cccccctctc aactattatt aactaacata
   120
   atggagggaa ttactaatgg ccatgctgaa gcaacttttt gcgtgaccaa aagtgttggt
   180
   gatccactca actggggtgc agccgcggag tcgttgatgg ggagtcattt ggatgaggtg
   240
   aagcgtatgg tggaggaata ccgtaatcca ttggttaaaa ttggcggcga gacgcttacc
   300
   attgctcagg tggctggaat tgcttctcat gatagtggtg tgagggtgga gctgtctgag
   360
   tccgccaggg ccggcgttaa ggcgagtagt gattgggtga tggacagcat gaacaatggg
   420
   actgatagtt atggtgttac caccggtttc ggcgccacct ctcaccggag aaccaagcag 480
   ggtggtgcct tgcagaagga gctaattagg tttttgaatg ctggaatatt tggcaatggt 540
   acagaatcta actgtacact accacacacg gcaaccagag ctgcaatgct 590
<210> 264
   <211> 616
   <212> DNA
   <213> Trifolium repens
<220>
   <221> misc_feature
   <222> (6) .. (6)
   <223> Any nucleotide
<220>
   <221> misc_feature
   <222> (29) .. (29)
   <223> Any nucleotide
<220>
   <221> misc_feature
   <222> (54) .. (54)
   <223> Any nucleotide
<400> 264
   ggaaanaatt atattgttcc ttatttccnc ccacacaaca taacaaatac attntcctct 60
   cctctcatca caattattac tttctacacc cccccctctc aactattatt aactaacata 120
   atggagggaa ttaccaatgg ccatgctgaa gcaacttttt gcgtgaccaa aagtgttggt 180
   gatccactca actggggtgc agccgcggag tcgttgatgg ggagtcattt ggatgaggtg 240
   aagcgtatgg tggaggaata ccgtaatcca ttggttaaaa ttggcggcga gacgcttacc 300
   attgctcagg tggctggaat tgcttctcat gatagtggtg tgagggtgga gctgtctgag 360
   tccgccaggg ccggcgttaa ggcgagtagt ggttgggtga tggacagcat gaacaatggg 420
   actgatagtt atggtgttac cactggtttc ggcgccacct ctcaccggag aaccaagcag 480
   ggtggtgcct tgcagaagga gctaattagg tttttgaatg ccggaatatt tggcaatggt 540
   acagaatcta actgtacact accacacaca gcaaccagag ctgcaatgct tgtgagaatc 600
   aacactcttc ttcaag 616
<210> 265
   <211> 441
   <212> DNA
   <213> Trifolium repens
<230>
   <221> misc_feature
   <222> (5) .. (5)
   <223> Any nucleotide
<220>
   <221> misc_feature
   <222> (10) .. (10)
   <223> Any nucleotide
<220>
   <221> misc_feature
   <222> (12) .. (12)
   <223> Any nucleotide
<220>
   <221> misc_feature
   <222> (18) .. (18)
   <223> Any nucleotide
<220>
   <221> misc_feature
   <222> (28) .. (28)
   <223> Any nucleotide
<220>
   <221> misc_feature
   <222> (44) .. (44)
   <223> Any nucleotide
<220>
   <221> misc_feature
   <222> (86) .. (87)
   <223> Any nucleotides
<220>
   <221> misc_feature
   <222> (354) .. (355)
   <223> Any nucleotides
<220>
   <221> misc_feature
   <222> (402) .. (402)
   <223> Any nucleotide
<220>
   <221> misc_feature
   <222> (426) .. (426)
   <223> Any nucleotide
<220>
   <221> misc_feature
   <222> (430) .. (430)
   <223> Any nucleotide
<220>
   <221> misc_feature
   <222> (434) .. (434)
   <223> Any nucleotide
<220>
   <221> misc_feature
   <222> (437) .. (437)
   <223> Any nucleotide
<220>
   <221> misc_feature
   <222> (440) .. (441)
   <223> Any nucleotides
<400> 265
   gaaanaattn tnttgttnat tatttccncc cacacaacat aacnaataca ttattctctc 60
   ctctcatcac aattattact ttctanncac cctcctctca actattatta actagcataa 120
   tggagggaat taccaatggc catgctgaaa caactttttg cgtgaccaaa agtgttggtg 180
   atccactcaa ctggggtgca gccgcggagt cgttgacggg gagtcatttg gatgaggtga 240
   agcgtatggt ggaggagtac cgtaatccgt tggctaaaat tggcggcgag acgcttacca 300
   ttgctcaggt ggctggaatt gcttctcatg atagtggtgt gagggtggag ctgnncgagt 360
   ccgcaagggc cggcgttaag gcgagtactg attgggtgat gnataacatg aacaatggga 420
   ctgatnggtn cggngcnacn n 441
<210> 266
   <211> 567
   <212> DNA
   <213> Trifolium repens
<220>
   <221> misc_feature
   <222> (5) .. (5)
   <223> Any nucleotide
<220>
   <221> misc_feature
   <222> (10) .. (10)
   <223> Any nucleotide
<220>
   <221> misc_feature
   <222> (12) .. (12)
   <223> Any nucleotide
<220>
   <221> misc_feature
   <222> (53) .. (53)
   <223> Any nucleotide
<400> 266
   gaaanaattn tnttgttcct tatttccccc cacacaacat aacaaataca ttntcctctc 60
   ctctcatcac aattattact ttctacaccc ccccctctca actattatta actaacataa 120
   tggagggaat taccaatggc catgctgaag caactttttg cgtgaccaaa agtgttggtg 180
   atccactcaa ctggggtgca gccgcggagt cgttgatggg gagtcatttg gatgaggtga 240
   agcgtatggt ggaggaatac cgtaatccat tggttaaaat tggcggcgag acgcttacca 300
   ttgctcaggt ggctggaatt gcttctcatg atagtggtgt gagggtggag ctgtctgagt 360
   ccgccagggc cggcgttaag gcgagtagtg gttgggtgat ggacagcatg aacaatggga 420
   ctgatagtta tggtgttacc actggtttcg gcgccacctc tcaccggaga accaagcagg 480
   gtggtgcctt gcagaaggag ctaattaggt ttttgaatgc cggaatattt ggcaatggta 540
   cagaatctaa ctgtacacta ccacacc 567
<210> 267
   <211> 532
   <212> DNA
   <213> Trifolium repens
<220>
   <221> misc_feature
   <222> (2) .. (2)
   <223> Any nucleotide
<220>
   <221> misc_feature
   <222> (6) .. (6)
   <223> Any nucleotide
<220>
   <221> misc_feature
   <222> (8) .. (8)
   <223> Any nucleotide
<220>
   <221> misc_feature
   <222> (13) .. (13)
   <223> Any nucleotide
<220>
   <221> misc_feature
   <222> (17) .. (17)
   <223> Any nucleotide
<220>
   <221> misc_feature
   <222> (30) .. (30)
   <223> Any nucleotide
<220>
   <221> misc_feature
   <222> (35) .. (35)
   <223> Any nucleotide
<220>
   <221> misc_feature
   <222> (38) .. (39)
   <223> Any nucleotides
<220>
   <221> misc_feature
   <222> (41) .. (42)
   <223> Any nucleotides
<220>
   <221> misc_feature
   <222> (48) .. (51)
   <223> Any nucleotides
<220>
   <221> misc_feature
   <222> (53) .. (53)
   <223> Any nucleotide
<220>
   <221> misc_feature
   <222> (63) .. (63)
   <223> Any nucleotide
<220>
   <221> misc_feature
   <222> (72) .. (72)
   <223> Any nucleotide
<220>
   <221> misc_feature
   <222> (78) .. (78)
   <223> Any nucleotide
<220>
   <221> misc_feature
   <222> (94) .. (94)
   <223> Any nucleotide
<400> 267
   anatananaa tcntatnttc tctcctctan taacnatnnt nnctttcnnn ncnccctcct 60
   ctnaactatt antaactngc ataatggagg gaantaccaa tggccatgct gaaacaactt 120
   tttgcgtgac caaaagtgtt ggtgatccac tcaactgggg tgcagccgcg gagtcgtcga 180
   cggggagtca tttggatgag gtgaagcgta tggtggagga gtaccgtaat ccgttggtta 240
   aaattggcgg cgagacgctt accattgctc aggtggctgg aattgcttct catgatagtg 300
   gtgtgagggt ggagctgtcc gagtccgcaa gggccggcgt taaggcgagt agtgattggg 360
   tgatggatag catgaacaat gggactgata gttacggtgt taccaccggt tttggtgcca 420
   cctctcaccg gagaaccaag cagggtggtg ccttgcagaa ggagctaatt aggtttttga 480
   atgctggaat atttggcaat ggtacagaat ctaactttac actaccacac ac 532
<210> 268
   <211> 579
   <212> DNA
   <213> Trifolium repens
<400> 268
   aacaagatcg ttatgcctta gaacttcacc tcaatggctt ggtcctttga ttgaagtgat 60
   aagattttca accaaatcaa ttgaaagaga aattaactcg gtcaacgaca accctttgat 120
   cgatgtttca aggaacaagg ccattcatgg tggtaacttt caaggaacac ctattggagt 180
   ttcaatggat aacacacgtt tagctcttgc ttcaattggt aaactcatgt ttgctcaatt 240
   ctctgaactt gttaatgatt tttacaacaa cgggttgcct tcgaatctta ctgctagtag 300
   gaacccgagc ttggactatg gtttcaaggg atcggaaatt gccatggctt cgtattgttc 360
   cgagttacaa tatcttgcta atcctgtcac cacccatgtc caaagtgccg agcaacacaa 420
   ccaagatgtt aactctttgg gtttgatttc atctagaaaa acaaatgaag ctattgagat
   480
   tctcaagctc atgtcttcca ctttcttgat tgcattatgt caagcaatcg acttaaggca
   540
   cttggaggaa aatctcagga acaccgtcaa gaacacggt
   579
<210> 269
   <211> 192
   <212> PRT
   <213> Trifolium repens
<400> 269
   Thr Arg Ser Leu Cys Leu Arg Thr Ser Pro Gln Trp Leu Gly Pro Leu
   1               5                   10                  15
   Ile Glu Val Ile Arg Phe Ser Thr Lys Ser Ile Glu Arg Glu Ile Asn
               20                  25                  30
   Ser Val Asn Asp Asn Pro Leu Ile Asp Val Ser Arg Asn Lys Ala Ile
           35                  40                  45
   His Gly Gly Asn Phe Gln Gly Thr Pro Ile Gly Val Ser Met Asp Asn
       50                  55                  60
   Thr Arg Leu Ala Leu Ala Ser Ile Gly Lys Leu Met Phe Ala Gln Phe
   65                  70                  75                  80
   Ser Glu Leu Val Asn Asp Phe Tyr Asn Asn Gly Leu Pro Ser Asn Leu
                   85                  90                  95
   Thr Ala Ser Arg Asn Pro Ser Leu Asp Tyr Gly Phe Lys Gly Ser Glu
               100                 105                 110
   Ile Ala Met Ala Ser Tyr Cys Ser Glu Leu Gln Tyr Leu Ala Asn Pro
           115                 120                 125
   Val Thr Thr His Val Gln Ser Ala Glu Gln His Asn Gln Asp Val Asn
       130                 135                 140
   Ser Leu Gly Leu Ile Ser Ser Arg Lys Thr Asn Glu Ala Ile Glu Ile
   145                 150                 155                 160
   Leu Lys Leu Met Ser Ser Thr Phe Leu Ile Ala Leu Cys Gln Ala Ile
                   165                 170                 175
   Asp Leu Arg His Leu Glu Glu Asn Leu Arg Asn Thr Val Lys Asn Thr
               180                 185                 190
<210> 270
   <211> 591
   <212> DNA
   <213> Trifolium repens

<220>
   <221> misc_feature
   <222> (8) .. (8)
   <223> Any nucleotide
<220>
   <221> misc_feature
   <222> (15) .. (15)
   <223> Any nucleotide
<220>
   <221> misc_feature
   <222> (41) .. (41)
   <223> Any nucleotide
<220>
   <221> misc_feature
   <222> (66) .. (66)
   <223> Any nucleotide
<400> 270
   ggtcaatnca gcttnggaga tctagtcccc ctttcttact ntgctggttt actaactgga
   60
   agaccnaatt ctaaagctca tgggcctaca ggagaagtac ttaatgcaaa agaagctttt
   120
   caattggctg gaatcaatac cgagttcttt gaattacaac caaaagaagg tcttgcactt
   180
   gttaatggaa ctgctgttgg ttctggttta gcttctattg ttctttttga ggctaacata
   240
   ttggcggtgt tgtctgaagt tctatcggca attttcgctg aagttatgca agggaagccc
   300
   gaatttactg atcatttgac acataagttg aagcaccacc ctggtcaaat tgaggctgct
   360
   gctattatgg aacacatttt ggatgggagt gcttatgtta aagacgcgaa gaagttgcat
   420
   gagatggacc ctttacagaa gccaaagcaa gatagatatg cacttagaac ttcaccacaa
   480
   tggcttggtc ctttgattga agtgattaga ttttcaacca agtcaattga gagagagatc
   540
   aactctgtca atgacaaccc tttgattgat gtttcgagaa acaaggcttt g
   591
<210> 271
   <211> 197
   <212> PRT
   <213> Trifolium repens
<220>
   <221> MISC_FEATURE
   <222> (3) .. (3)
   <223> Any amino acid
<220>
   <221> MISC_FEATURE
   <222> (5) .. (5)
   <223> Any amino acid
<220>
   <221> MISC_FEATURE
   <222> (14) .. (14)
   <223> Any amino acid
<220>
   <221> MISC_FEATURE
   <222> (22) .. (22)
   <223> Any amino acid
<400> 271
   Gly Gln Xaa Ser Xaa Gly Asp Leu Val Pro Leu Ser Tyr Xaa Ala Gly
   1 5 10 15
   Leu Leu Thr Gly Arg Xaa Asn Ser Lys Ala His Gly Pro Thr Gly Glu
   20 25 30
   Val Leu Asn Ala Lys Glu Ala Phe Gln Leu Ala Gly Ile Asn Thr Glu
   35 40 45
   Phe Phe Glu Leu Gln Pro Lys Glu Gly Leu Ala Leu Val Asn Gly Thr
   50 55 60
   Ala Val Gly Ser Gly Leu Ala Ser Ile Val Leu Phe Glu Ala Asn Ile
   65 70 75 80
   Leu Ala Val Leu Ser Glu Val Leu Ser Ala Ile Phe Ala Glu Val Met
   85 90 95
   Gln Gly Lys Pro Glu Phe Thr Asp His Leu Thr His Lys Leu Lys His
   100 105 110
   His Pro Gly Gln Ile Glu Ala Ala Ala Ile Met Glu His Ile Leu Asp
   115 120 125
   Gly Ser Ala Tyr Val Lys Asp Ala Lys Lys Leu His Glu Met Asp Pro
   130 135 140
   Leu Gln Lys Pro Lys Gln Asp Arg Tyr Ala Leu Arg Thr Ser Pro Gln
   145 150 155 160
   Trp Leu Gly Pro Leu Ile Glu Val Ile Arg Phe Ser Thr Lys Ser Ile
   165 170 175
   Glu Arg Glu Ile Asn Ser Val Asn Asp Asn Pro Leu Ile Asp Val Ser
   180 185 190
   Arg Asn Lys Ala Leu
   195
<210> 272
   <211> 592
   <212> DNA
   <213> Trifolium repens
<220>
   <221> misc_feature
   <222> (2) .. (3)
   <223> Any nucleotides
<220>
   <221> misc_feature
   <222> (11) .. (11)
   <223> Any nucleotide
<220>
   <221> misc_feature
   <222> (18) .. (18)
   <223> Any nucleotide
<220>
   <221> misc_feature
   <222> (22) .. (22)
   <223> Any nucleotide
<220>
   <221> misc_feature
   <222> (30) .. (30)
   <223> Any nucleotide
<220>
   <221> misc_feature
   <222> (37) .. (37)
   <223> Any nucleotide
<220>
   <221> misc_feature
   <222> (508) .. (508)
   <223> Any nucleotide
<220>
   <221> misc_feature
   <222> (579) .. (579)
   <223> Any nucleotide
<220>
   <221> misc_feature
   <222> (585) .. (585)
   <223> Any nucleotide
<220>
   <221> misc_feature
   <222> (592) .. (592)
   <223> Any nucleotide
<400> 272
   gnnggaaatt ncaactcnat tntttctttn tataatnttt gaatttcctt ctctctcaaa 60
   ttctatagct actctaccac atcacacaac ataacaaatt aagaaatatt cattactata 120
   ctattaagat atggaagtag tagcagcagc aatcacaaaa aacaacggca agattgattc 180
   attttgcttg aatcatgcta atgctaataa catgaaagtg aatgatgctg atcctttgaa 240
   ttggggtgtg gctgctgagg caatgaaggg aagtcacttg gatgaggtga aacgtatggt 300
   ggaggagtac cggaagccga ttgtccgtct tggtggcgag acgctgacga tttctcaggt 360
   ggctgccatt gctgcacacg atggtgcgat ggttgagctg tcggaatctg ctagagccgg 420
   cgttaaggca agcagtgatt gggttatgga gagtatgaac aaaggtactg acagttatgg 480
   tgtcaccaca gggttcggcg ctacctcnca ccgccgaacc aaacaaggtg gtgctttaca 540
   gaaagggctc ataaggtttt tgaatgctgg aatatttgna aatgnaactg an 592
<210> 273
   <211> 154
   <212> PRT
   <213> Trifolium repens
<220>
   <221> MISC_FEATURE
   <222> (126) .. (126)
   <223> Any amino acid
<220>
   <221> MISC_FEATURE
   <222> (150) .. (150)
   <223> Any amino acid
<220>
   <221> MISC_FEATURE
   <222> (152) .. (152)
   <223> Any amino acid
<220>
   <221> MISC_FEATURE
   <222> (154) .. (154)
   <223> Any amino acid
<400> 273
   Met Glu Val Val Ala Ala Ala Ile Thr Lys Asn Asn Gly Lys Ile Asp
   1               5                   10                  15
   Ser Phe Cys Leu Asn His Ala Asn Ala Asn Asn Met Lys Val Asn Asp
               20                  25                  30
   Ala Asp Pro Leu Asn Trp Gly Val Ala Ala Glu Ala Met Lys Gly Ser
           35                  40                  45
   His Leu Asp Glu Val Lys Arg Met Val Glu Glu Tyr Arg Lys Pro Ile
       50                  55                  60
   Val Arg Leu Gly Gly Glu Thr Leu Thr Ile Ser Gln Val Ala Ala Ile
   65                  70                  75                  80
   Ala Ala His Asp Gly Ala Met Val Glu Leu Ser Glu Ser Ala Arg Ala
                   85                  90                  95
   Gly Val Lys Ala Ser Ser Asp Trp Val Met Glu Ser Met Asn Lys Gly
               100                 105                 110
   Thr Asp Ser Tyr Gly Val Thr Thr Gly Phe Gly Ala Thr Xaa His Arg
           115                 120                 125
   Arg Thr Lys Gln Gly Gly Ala Leu Gln Lys Gly Leu Ile Arg Phe Leu
       130                 135                 140
   Asn Ala Gly Ile Phe Xaa Asn Xaa Thr Xaa
   145                 150
<210> 274
   <211> 566
   <212> DNA
   <213> Trifolium repens
<220>
   <221> misc_feature
   <222> (2) .. (2)
   <223> Any nucleotide
<220>
   <221> misc_feature
   <222> (12) .. (12)
   <223> Any nucleotide
<220>
   <221> misc_feature
   <222> (19) .. (19)
   <223> Any nucleotide
<220>
   <221> misc_feature
   <222> (164) .. (165)
   <223> Any nucleotides
<220>
   <221> misc_feature
   <222> (168) .. (168)
   <223> Any nucleotide
<220>
   <221> misc_feature
   <222> (186) .. (186)
   <223> Any nucleotide
<220>
   <221> misc_feature
   <222> (239) .. (239)
   <223> Any nucleotide
<220>
   <221> misc_feature
   <222> (259) .. (259)
   <223> Any nucleotide
<220>
   <221> misc_feature
   <222> (295) .. (296)
   <223> Any nucleotides
<220>
   <221> misc_feature
   <222> (301) .. (301)
   <223> Any nucleotide
<220>
   <221> misc_feature
   <222> (384) .. (384)
   <223> Any nucleotide
<220>
   <221> misc_feature
   <222> (435) .. (435)
   <223> Any nucleotide
<220>
   <221> misc_feature
   <222> (469) .. (469)
   <223> Any nucleotide
<220>
   <221> misc_feature
   <222> (498) .. (499)
   <223> Any nucleotides
<220>
   <221> misc_feature
   <222> (511) .. (511)
   <223> Any nucleotide
<220>
   <221> misc_feature
   <222> (517) .. (517)
   <223> Any nucleotide
<220>
   <221> misc_feature
   <222> (522) .. (522)
   <223> Any nucleotide
<220>
   <221> misc_feature
   <222> (529) .. (530)
   <223> Any nucleotides
<220>
   <221> misc_feature
   <222> (545) .. (545)
   <223> Any nucleotide
<220>
   <221> misc_feature
   <222> (553) .. (553)
   <223> Any nucleotide
<400> 274
   cnattgttag tngtttccnc ccacccacat aacaaataca taattctctc ctctgatcac
   60
   aattattact ttactacacc ctcctctcaa ctattattaa ctagcataat ggagggaatt
   120
   accaatggcc atgctgaaac aacttttagc gtgaccaaaa gtgnnggnga tccactcaac
   180
   tggcgngcag ccgcggagtc gtcgacgggg agtcatttgg atgaggtgaa gcgtatggng
   240
   gaggagtacc gtaatccgnt ggttaaaatt ggcggcgaga cgcttaccat tgctnnggta
   300
   nctggaattg cttctcatga tagtggagtg agggtggagc tgtccgagtt cgcaagggcc
   360
   ggcgttaagg cgagtagtga ttgngtgatg gatagcatga acaatgggac tgatagttac
   420
   ggtgttacca ccgcntttgg tgccacctgt caccggagaa ccaagccang gtggtgcctt
   480
   gcagaaggag ctaaattnng gtgttttgaa ngctggnaat antttggcnn tggttcagaa
   540
   atctnaactt gtncacttac cacacc
   566
<210> 275
   <211> 152
   <212> PRT
   <213> Trifolium repens
<220>
   <221> MISC_FEATURE
   <222> (19) .. (20)
   <223> Any amino acids
<220>
   <221> MISC_FEATURE
   <222> (26) .. (26)
   <223> Any amino acid
<220>
   <221> MISC_FEATURE
   <222> (44) .. (44)
   <223> Any amino acid
<220>
   <221> MISC_FEATURE
   <222> (51) .. (51)
   <223> Any amino acid
<220>
   <221> MISC_FEATURE
   <222> (63) .. (63)
   <223> Any amino acid
<220>
   <221> MISC_FEATURE
   <222> (65) .. (65)
   <223> Any amino acid
<220>
   <221> MISC_FEATURE
   <222> (92) .. (92)
   <223> Any amino acid
<220>
   <221> MISC_FEATURE
   <222> (109) .. (109)
   <223> Any amino acid
<220>
   <221> MISC_FEATURE
   <222> (121) .. (121)
   <223> Any amino acid
<220>
   <221> MISC_FEATURE
   <222> (130) .. (131)
   <223> Any amino acids
<220>
   <221> MISC_FEATURE
   <222> (135) .. (135)
   <223> Any amino acid
<220>
   <221> MISC_FEATURE
   <222> (137) .. (138)
   <223> Any amino acids
<220>
   <221> MISC_FEATURE
   <222> (141) .. (141)
   <223> Any amino acid
<220>
   <221> MISC_FEATURE
   <222> (146) .. (146)
   <223> Any amino acid
<220>
   <221> MISC_FEATURE
   <222> (149) .. (149)
   <223> Any amino acid
<400> 275
   Met Glu Gly Ile Thr Asn Gly His Ala Glu Thr Thr Phe Ser Val Thr
   1               5                   10                  15
   Lys Ser Xaa Xaa Asp Pro Leu Asn Trp Xaa Ala Ala Ala Glu Ser Ser
               20                  25                  30
   Thr Gly Ser His Leu Asp Glu Val Lys Arg Met Xaa Glu Glu Tyr Arg
           35                  40                  45
   Asn Pro Xaa Val Lys Ile Gly Gly Glu Thr Leu Thr Ile Ala Xaa Val
       50                  55                  60
   Xaa Gly Ile Ala Ser His Asp Ser Gly Val Arg Val Glu Leu Ser Glu
   65                  70                  75                  80
   Phe Ala Arg Ala Gly Val Lys Ala Ser Ser Asp Xaa Val Met Asp Ser
                   85                  90                  95
   Met Asn Asn Gly Thr Asp Ser Tyr Gly Val Thr Thr Xaa Phe Gly Ala
               100                 105                 110
   Thr Cys His Arg Arg Thr Lys Pro Xaa Trp Cys Leu Ala Glu Gly Ala
           115                 120                 125
   Lys Xaa Xaa Cys Phe Glu Xaa Trp Xaa Xaa Phe Gly Xaa Gly Ser Glu
   130 135 140
   Ile Xaa Thr Cys Xaa Leu Thr Thr
   145 150
<210> 276
   <211> 188
   <212> DNA
   <213> Trifolium repens
<220>
   <221> misc_feature
   <222> (2) .. (2)
   <223> Any nucleotide
<220>
   <221> misc_feature
   <222> (12) .. (12)
   <223> Any nucleotide
<220>
   <221> misc_feature
   <222> (19) .. (19)
   <223> Any nucleotide
<220>
   <221> misc_feature
   <222> (26) .. (26)
   <223> Any nucleotide
<220>
   <221> misc_feature
   <222> (34) .. (34)
   <223> Any nucleotide
<220>
   <221> misc_feature
   <222> (40) .. (40)
   <223> Any nucleotide
<220>
   <221> misc_feature
   <222> (43) .. (43)
   <223> Any nucleotide
<220>
   <221> misc_feature
   <222> (73) .. (73)
   <223> Any nucleotide
<220>
   <221> misc_feature
   <222> (165) .. (165)
   <223> Any nucleotide

<220>
   <221> misc_feature
   <222> (168) .. (168)
   <223> Any nucleotide
<220>
   <221> misc_feature
   <222> (172) .. (173)
   <223> Any nucleotides
<220>
   <221> misc_feature
   <222> (175) .. (175)
   <223> Any nucleotide
<220>
   <221> misc_feature
   <222> (178) .. (179)
   <223> Any nucleotides
<220>
   <221> misc_feature
   <222> (186) .. (186)
   <223> Any nucleotide
<220>
   <221> misc_feature
   <222> (188) .. (188)
   <223> Any nucleotide
<400> 276
   cnattgttag tngtttccnc ccaccnacat aacnaatacn tanttctctc ctctgatcac 60
   aattattact ttnctacacc ctcctctcaa ctattattaa ctagcataat ggagggaatt 120
   accaatggcc atgctgaaac aactttttgc gtgaccaaaa gtgtnggnga tnnantgnnc 180
   tgtcgngn 188
<210> 277
   <211> 541
   <212> DNA
   <213> Trifolium repens
<220>
   <221> misc_feature
   <222> (139) .. (139)
   <223> Any nucleotide
<220>
   <221> misc_feature
   <222> (214) .. (214)
   <223> Any nucleotide
<220>
   <221> misc_feature
   <222> (234) .. (234)
   <223> Any nucleotide
<220>
   <221> misc_feature
   <222> (270) .. (271)
   <223> Any nucleotides
<220>
   <221> misc_feature
   <222> (276) .. (276)
   <223> Any nucleotide
<220>
   <221> misc_feature
   <222> (359) .. (359)
   <223> Any nucleotide
<220>
   <221> misc_feature
   <222> (410) .. (410)
   <223> Any nucleotide
<220>
   <221> misc_feature
   <222> (444) .. (444)
   <223> Any nucleotide
<220>
   <221> misc_feature
   <222> (473) .. (474)
   <223> Any nucleotides
<220>
   <221> misc_feature
   <222> (486) .. (486)
   <223> Any nucleotide
<220>
   <221> misc_feature
   <222> (492) .. (492)
   <223> Any nucleotide
<220>
   <221> misc_feature
   <222> (497) .. (497)
   <223> Any nucleotide
<220>
   <221> misc_feature
   <222> (504) .. (505)
   <223> Any nucleotides
<220>
   <221> misc_feature
   <222> (520) .. (520)
   <223> Any nucleotide
<220>
   <221> misc_feature
   <222> (528) .. (528)
   <223> Any nucleotide
<400> 277
   cccataacaa atacattatt ctctcctctg atcacaatta ttactttact acaccctcct
   60
   ctcaactatt attaactagc ataatggagg gaattaccaa tggccatgct gaaacaactt
   120
   ttatcgtgac caaaagtgnt ggtgatccac tcaactgggg tgcagccgcg gagtcgtcga
   180
   cggggagtca tttggatgag gtgaagcgta tggnggagga gtaccgtaat ccgntggtta
   240
   aaattggcgg cgagacgctt accattgctn nggtanctgg aattgcttct catgatagtg
   300
   gagtgagggt ggagctgtcc gagttcgcaa gggccggcgt taaggcgagt agtgattgng
   360
   tgatggatag catgaacaat gggactgata gttacggtgt taccaccgcn tttggtgcca
   420
   cctgtcaccg gagaaccaag ccanggtggt gccttgcaga aggagctaaa ttnnggtgtt
   480
   ttgaangctg gnaatanttt ggcnntggtt cagaaatctn aacttgtnca cttaccacac
   540
   c
   541
<210> 278
   <211> 1185
   <212> DNA
   <213> Trifolium repens
<220>
   <221> misc_feature
   <222> (19) .. (19)
   <223> Any nucleotide
<220>
   <221> misc_feature
   <222> (35) .. (35)
   <223> Any nucleotide
<220>
   <221> misc_feature
   <222> (40) .. (40)
   <223> Any nucleotide
<220>
   <221> misc_feature
   <222> (444) .. (444)
   <223> Any nucleotide
<220>
   <221> misc_feature
   <222> (482) .. (483)
   <223> Any nucleotides
<220>
   <221> misc_feature
   <222> (485) .. (485)
   <223> Any nucleotide
<220>
   <221> misc_feature
   <222> (552) .. (552)
   <223> Any nucleotide
<220>
   <221> misc_feature
   <222> (596) .. (596)
   <223> Any nucleotide
<400> 278
   gtaagagttg agaaaaaana ccaataaagt aaacnctatn tagaaagaga gtcaaaaatg 60
   gctgaaggaa aaggaagggt ttgtgttact ggaggaacag gttttcttgg ttcatggatc 120
   atcaagagtc ttcttgaaaa tggatactct gttaatacca ctattagagc tgatccagaa 180
   cgtaagaggg atgtaagctt cctaacaaat ctacccggcg catccgaaag gctacatttc 240
   ttcaacgccg atctagacga cccagagagt ttcaacgaag caattgaagg ttgtgtcggg 300
   atattccaca ccgcttcacc aatcgatttc gccgtgagtg agccagaaga aatagtgaca 360
   aaaagaacag tggatggagc attaggaatt ttaaaagcat gtgtgaattc aaagacagtg 420
   aagagattta tttacacttc aagnggttct gctgtttcat tcaatggaaa aaacaaagat 480
   gnntnggatg agagtgattg gagtgatgtt gatttgctta gaagtgttaa accatttggt 540
   tggagttatg gngtgttcaa gactttggct gagaaagcag tgcttgaatt tggtcnacaa 600
   aatgggattg atgttgttac tttgattctt ccttttattg ttggaggttt tgtttgtccc 660
   aagcttcctg attctgttga gaaagctctt gttttggtac taggcaaaaa ggaacaaatt 720
   ggtattataa gtttccacat ggtacatgta gatgatgtgg ctagagcaca tatctatcta 780
   cttgagaatc ctgttccagg aggtagatat aattgttcac cattctttgt atctattgaa 840
   gaaatgtcac agcttctctc agccaaatat ccagaatatc aaatactatc agtagatgag 900
   ttgaaggaaa ttaaaggggc aagattgcca gatttgaact cgaagaagct cgtggacgct 960
   ggttttgagt ttaagtatag tgtcgatgat atgttcgatg atgcgattca atgctgcaag 1020
   gaaaaaggct atctctaagc atgtatttga aaattccatg aagttgagaa aacaataatg 1080
   tgcctaaaat caatgatggc taatgagatg tacaagttta tgcattaagt tatttgtgat 1140
   caatcaaata atgaaataat ctgttcattt ttccgaaaaa aaaaa 1185
<210> 279
   <211> 326
   <212> PRT
   <213> Trifolium repens
<220>
   <221> MISC_FEATURE
   <222> (129) .. (129)
   <223> Any amino acid
<220>
   <221> MISC_FEATURE
   <222> (142) .. (143)
   <223> Any amino acids
<220>
   <221> MISC_FEATURE
   <222> (165) .. (165)
   <223> Any amino acid
<220>
   <221> MISC_FEATURE
   <222> (180) .. (180)
   <223> Any amino acid
<400> 279
   Met Ala Glu Gly Lys Gly Arg Val Cys Val Thr Gly Gly Thr Gly Phe
   1 5 10 15
   Leu Gly Ser Trp Ile Ile Lys Ser Leu Leu Glu Asn Gly Tyr Ser Val
   20 25 30
   Asn Thr Thr Ile Arg Ala Asp Pro Glu Arg Lys Arg Asp Val Ser Phe
   35 40 45
   Leu Thr Asn Leu Pro Gly Ala Ser Glu Arg Leu His Phe Phe Asn Ala
   50 55 60
   Asp Leu Asp Asp Pro Glu Ser Phe Asn Glu Ala Ile Glu Gly Cys Val
   65 70 75 80
   Gly Ile Phe His Thr Ala Ser Pro Ile Asp Phe Ala Val Ser Glu Pro
   85 90 95
   Glu Glu Ile Val Thr Lys Arg Thr Val Asp Gly Ala Leu Gly Ile Leu
   100 105 110
   Lys Ala Cys Val Asn Ser Lys Thr Val Lys Arg Phe Ile Tyr Thr Ser
   115 120 125
   Xaa Gly Ser Ala Val Ser Phe Asn Gly Lys Asn Lys Asp Xaa Xaa Asp
   130 135 140
   Glu Ser Asp Trp Ser Asp Val Asp Leu Leu Arg Ser Val Lys Pro Phe
   145 150 155 160
   Gly Trp Ser Tyr Xaa Val Phe Lys Thr Leu Ala Glu Lys Ala Val Leu
   165 170 175
   Glu Phe Gly Xaa Gln Asn Gly Ile Asp Val Val Thr Leu Ile Leu Pro
   180 185 190
   Phe Ile Val Gly Gly Phe Val Cys Pro Lys Leu Pro Asp Ser Val Glu
   195 200 205
   Lys Ala Leu Val Leu Val Leu Gly Lys Lys Glu Gln Ile Gly Ile Ile
   210 215 220
   Ser Phe His Met Val His Val Asp Asp Val Ala Arg Ala His Ile Tyr
   225 230 235 240
   Leu Leu Glu Asn Pro Val Pro Gly Gly Arg Tyr Asn Cys Ser Pro Phe
   245 250 255
   Phe Val Ser Ile Glu Glu Met Ser Gln Leu Leu Ser Ala Lys Tyr Pro
   260 265 270
   Glu Tyr Gln Ile Leu Ser Val Asp Glu Leu Lys Glu Ile Lys Gly Ala
   275 280 285
   Arg Leu Pro Asp Leu Asn Ser Lys Lys Leu Val Asp Ala Gly Phe Glu
       290                 295                 300
   Phe Lys Tyr Ser Val Asp Asp Met Phe Asp Asp Ala Ile Gln Cys Cys
   305                 310                 315                 320
   Lys Glu Lys Gly Tyr Leu
                   325
<210> 280
   <211> 493
   <212> DNA
   <213> Trifolium repens
<220>
   <221> misc_feature
   <222> (22) .. (22)
   <223> Any nucleotide
<220>
   <221> misc_feature
   <222> (35) .. (35)
   <223> Any nucleotide
<220>
   <221> misc_feature
   <222> (40) .. (40)
   <223> Any nucleotide
<220>
   <221> misc_feature
   <222> (444) .. (444)
   <223> Any nucleotide
<220>
   <221> misc_feature
   <222> (467) .. (467)
   <223> Any nucleotide
<220>
   <221> misc_feature
   <222> (473) .. (473)
   <223> Any nucleotide
<220>
   <221> misc_feature
   <222> (482) .. (483)
   <223> Any nucleotides
<220>
   <221> misc_feature
   <222> (485) .. (487)
   <223> Any nucleotides
<220>
   <221> misc_feature
   <222> (492) .. (492)
   <223> Any nucleotide
<400> 280
   gtagtagttg agaaaaaata cnaataaagt aaacnctatn tagaaagaga gtcaaaaatg 60
   gctgaaggaa aaggaagggt ttgtgttact ggaggaacag gttttcttgg ttcatggatc 120
   atcaagagtc ttcttgaaaa tggatactct gttaatacca ctattagagc tgatccagaa 180
   cgtaagaggg atgtaagctt cctaacaaat ctacccggcg catccgaaag gctacatttc 240
   ttcaacgccg atctagacga cccagagagt ttcaacgaag caattgaagg ttgtgtcggg 300
   atattccaca ccgcttcacc aatcgatttc gccgtgagtg agccagaaga aatagtgaca 360
   aaaagaacag tggatggagc attaggaatt ttaaaagcat gtgtgaattc aaagacagtg 420
   aagagattta tttacacttc aagnggttct gctgtttcat tcaatgnaaa aancaaagat 480
   gnntnnnatg ana 493
<210> 281
   <211> 601
   <212> DNA
   <213> Trifolium repens
<220>
   <221> misc_feature
   <222> (16) .. (17)
   <223> Any nucleotides
<220>
   <221> misc_feature
   <222> (32) .. (32)
   <223> Any nucleotide
<220>
   <221> misc_feature
   <222> (37) .. (37)
   <223> Any nucleotide
<220>
   <221> misc_feature
   <222> (50) .. (51)
   <223> Any nucleotides
<220>
   <221> misc_feature
   <222> (552) .. (552)
   <223> Any nucleotide
<400> 281
   agagttgaga aaaaanncca ataaagtaaa cnctatntag aaagagagtn naaaatggct 60
   gaaggaaaag gaagggtttg tgttactgga ggaacaggtt ttcttggttc atggatcatc 120
   aagagtcttc ttgaaaatgg atactctgtt aataccacta ttagagctga tccagaacgt 180
   aagagggatg taagcttcct aacaaatcta cccggcgcat ccgaaaggct acatttcttc 240
   aacgccgatc tagacgaccc agagagtttc aacgaagcaa ttgaaggttg tgtcgggata 300
   ttccacaccg cttcaccaat cgatttcgcc gtgagtgagc cagaagaaat agtgacaaaa 360
   agaacagtgg atggagcatt aggaatttta aaagcatgtg tgaattcaaa gacagtgaag 420
   agatttattt acacttcaag tggttctgct gtttcattca atggaaaaaa caaagatgtt 480
   ttggatgaga gtgattggag tgatgttgat ttgcttagaa gtgttaaacc atttggttgg 540
   agttatggtg tnttcaagac tttggctgag aaagcagtgc ttgaatttgg tcaacaaaat 600
   g 601
<210> 282
   <211> 613
   <212> DNA
   <213> Trifolium repens
<220>
   <221> misc_feature
   <222> (2) .. (2)
   <223> Any nucleotide
<220>
   <221> misc_feature
   <222> (45) .. (46)
   <223> Any nucleotides
<400> 282
   gngtgtttaa gactttagct gagaaagcag tgcttgaatt tggtnnacaa aatgggattg 60
   atgttgttac tttgattctt ccttttattg ttggaggttt tgtttgtcct aagcttcctg 120
   attctgttga gaaagctctt gttttggtac taggcaaaaa ggaacaaatt ggtattataa 180
   gtttccacat ggtacatgtg gatgatgtgg ctagagcaca tatctatcta cttgagaatc 240
   ctgttccagg aggtagatat aattgttcac cattctttgt atctattgaa gaaatgtcac 300
   agcttctttc agccaaatat ccagaatatc aaatactatc tgtagatgag ttgaaggaaa 360
   ttaaaggggc aaggttgcca gatttgaact cgaagaagct cgtggacgct ggttttgagt 420
   ttaagtatag tgtcgatgat atgttcgatg atgcgattca atgctgcaag gaaaaaggct 480
   atctctaagc atgtgtttga aaattccatg aagttgagaa aacaatactg tgcctaaaat 540
   caatgatggc taatgagatg tacaagttta tgcattaagt tatttgtgat caatcaaata 600
   atgaaataat ctg 613
<210> 283
   <211> 602
   <212> DNA
   <213> Trifolium repens
<220>
   <221> misc_feature
   <222> (8) .. (8)
   <223> Any nucleotide
<220>
   <221> misc_feature
   <222> (20) .. (20)
   <223> Any nucleotide
<220>
   <221> misc_feature
   <222> (37) .. (37)
   <223> Any nucleotide
<400> 283
   agaccttngc tgagaaagcn gtgcttgaat ttggtcnaca aaatgggatt gatgttgtta
   60
   ctttgattct tccttttatt gttggaggtt ttgtttgtcc taagcttcct gattctgttg
   120
   agaaagctct tgttttggta ctaggcaaaa aggaacaaat tggtattata agtttccaca
   180
   tggtacatgt ggatgatgtg gctagagcac atatctatct acttgagaat cctgttccag
   240
   gaggtagata taattgttca ccattctttg tatctattga agaaatgtca cagcttcttt
   300
   cagccaaata tccagaatat caaatactat ctgtagatga gttgaaggaa attaaagggg
   360
   caaggttgcc agatttgaac tcgaagaagc tcgtggacgc tggttttgag tttaagtata
   420
   gtgtcgatga tatgttcgat gatgcgattc aatgctgcaa ggaaaaaggc tatctctaag
   480
   catgtgtttg aaaattccat gaagttgaga aaacaatact gtgcctaaaa tcaatgatgg
   540
   ctaatgagat gtacaagttt atgcattaag ttatttgtga tcaatcaaat aatgaaataa
   600
   tc
   602

<210> 284
   <211> 575
   <212> DNA
   <213> Trifolium repens
<220>
   <221> misc_feature
   <222> (1) .. (2)
   <223> Any nucleotides
<220>
   <221> misc_feature
   <222> (13) .. (13)
   <223> Any nucleotide
<220>
   <221> misc_feature
   <222> (15) .. (15)
   <223> Any nucleotide
<220>
   <221> misc_feature
   <222> (56) .. (56)
   <223> Any nucleotide
<220>
   <221> misc_feature
   <222> (575) .. (575)
   <223> Any nucleotide
<400> 284
   nnttgaattt ggncnacaaa atgggattga tgttgttact ttgattcttc cttttnttgt 60
   tggaggtttt gtttgtccca agcttcctga ttctgttgag aaagctcttg ttttggtact 120
   aggcaaaaag gaacaaattg gtattataag tttccacatg gtacatgtag atgatgtggc 180
   tagagcacat atctatctac ttgagaatcc tgttccagga ggtagatata attgttcacc 240
   attctttgta tctattgaag aaatgtcaca gcttctctca gccaaatatc cagaatatca 300
   aatactatca gtagatgagt tgaaggaaat taaaggtgca agattgccag atttgaactc 360
   gaagaagctc gtggacgctg gttttgagtt taagtatagt gtcgatgata tgtttgatga 420
   tgcgattcaa tgctgcaagg aaaaaggcta tctctaagca tgtatttgaa aattccatga 480
   agttgagaaa acaataatgt gcctaaaatc aatgatggct aatgagatgt acaagtttat 540
   gcattaagtt atttgtgatc aatcaaataa tgaan 575
<210> 285
   <211> 604
   <212> DNA
   <213> Trifolium repens
<220>
   <221> misc_feature
   <222> (1) .. (2)
   <223> Any nucleotides
<220>
   <221> misc_feature
   <222> (14) .. (15)
   <223> Any nucleotides
<220>
   <221> misc_feature
   <222> (56) .. (56)
   <223> Any nucleotide
<400> 285
   nnttgaattt ggtnnacaaa atgggattga tgttgttact ttgattcttc cttttnttgt 60
   tggaggtttt gtttgtccca agcttcctga ttctgttgag aaagctcttg ttttggtact 120
   aggcaaaaag gaacaaattg gtattataag tttccacatg gtacatgtag atgatgtggc 180
   tagagcacat atctatctac ttgagaatcc tgttccagga ggtagatata attgttcacc 240
   attctttgta tctattgaag aaatgtcaca gcttctctca gccaaatatc cagaatatca 300
   aatactatca gtagatgagt tgaaggaaat taaaggtgca agattgccag atttgaactc 360
   gaagaagctc gtggacgctg gttttgagtt taagtatagt gtcgatgata tgtttgatga 420
   tgcgattcaa tgctgcaagg aaaaaggcta tctctaagca tgtatttgaa aattccatga 480
   agttgagaaa acaataatgt gcctaaaatc aatgatggct aatgagatgt acaagtttat 540
   gcattaagtt atttgtgatc aatcaaataa tgaaataatc tgttcatttt tccgaaaaaa 600
   aaaa 604
<210> 286
   <211> 695
   <212> DNA
   <213> Lolium perenne
<400> 286
   gtsywttcga gtttgagaga atggcttcca gggcaaggtg tgtgttactg gggcctctgg 60
   ctttgttgct tcttggcttg tcaaaagact actcgagtcc ggttataatg ttctagggac 120
   agtcagagac ccaggcaatc agaagaaggt agcacacctc tggaacttag caggggccaa 180
   ggaaaggttg gagcttgtca aagctgacct cttggaagaa gggagcttcg atgatgctgt 240
   gatggcctgt gagggtgtct tccacactgc atcacctatc atcaccaaat ctgataccaa 300
   ggaagaaatg cttgattctg caattaacgg cactctaaac gtgctgagat cgtgcaagaa 360
   gaatcctttt ctcaaaaggg ttgttctcac gtcatcatcg tcaaccgtga ggctgaggga 420
   tgaagctgaa ttcccaccca acgtgttgct ggatgaaaca tcatggagct ccgtggagtt 480
   ctgtgaaagt atccaggtat ggtatggtgt cgcgaagatc cttgctgaga aatcagcttg 540
   ggagttcgcc aaggagaaca acatcgacct agtggctgtt cttccaacgt tcgtgattgg 600
   acctaatctc tcgtctgaat taggacccac tgttttagat gtccttggct tatttaaagg 660
   agagacagag aagttcacca tgtttkggaa ggatg 695
<210> 287
   <211> 231
   <212> PRT
   <213> Lolium perenne
<400> 287
   Val Phe Ser Ser Leu Arg Glu Trp Leu Pro Gly Gln Val Cys Val Thr
   1 5 10 15
   Gly Ala Ser Gly Phe Val Ala Ser Trp Leu Val Lys Arg Leu Leu Glu
   20 25 30
   Ser Gly Tyr Asn Val Leu Gly Thr Val Arg Asp Pro Gly Asn Gln Lys
   35 40 45
   Lys Val Ala His Leu Trp Asn Leu Ala Gly Ala Lys Glu Arg Leu Glu
   50 55 60
   Leu Val Lys Ala Asp Leu Leu Glu Glu Gly Ser Phe Asp Asp Ala Val
   65 70 75 80
   Met Ala Cys Glu Gly Val Phe His Thr Ala Ser Pro Ile Ile Thr Lys
   85 90 95
   Ser Asp Thr Lys Glu Glu Met Leu Asp Ser Ala Ile Asn Gly Thr Leu
   100 105 110
   Asn Val Leu Arg Ser Cys Lys Lys Asn Pro Phe Leu Lys Arg Val Val
   115 120 125
   Leu Thr Ser Ser Ser Ser Thr Val Arg Leu Arg Asp Glu Ala Glu Phe
   130 135 140
   Pro Pro Asn Val Leu Leu Asp Glu Thr Ser Trp Ser Ser Val Glu Phe
   145 150 155 160
   Cys Glu Ser Ile Gln Val Trp Tyr Gly Val Ala Lys Ile Leu Ala Glu
   165 170 175
   Lys Ser Ala Trp Glu Phe Ala Lys Glu Asn Asn Ile Asp Leu Val Ala
   180 185 190
   Val Leu Pro Thr Phe Val Ile Gly Pro Asn Leu Ser Ser Glu Leu Gly
   195 200 205
   Pro Thr Val Leu Asp Val Leu Gly Leu Phe Lys Gly Glu Thr Glu Lys
   210 215 220
   Phe Thr Met Phe Gly Lys Asp
   225 230
<210> 288
   <211> 667
   <212> DNA
   <213> Lolium perenne
<220>
   <221> misc_feature
   <222> (19) .. (19)
   <223> Any nucleotide
<400> 288
   gtttgagaga atggcttcna gggcaggtgt gtgttactgg ggcctctggc tttgttgctt 60
   cttggcttgt caaaagacta ctcgagtccg gttataatgt tctagggaca gtcagagacc 120
   caggcaatca gaagaaggta gcacacctct ggaacttagc aggggccaag gaaaggttgg 180
   agcttgtcaa agctgacctc ttggaagaag ggagcttcga tgatgctgtg atggcctgtg 240
   agggtgtctt ccacactgca tcacctatca tcaccaaatc tgataccaag gaagaaatgc 300
   ttgattctgc aattaacggc actctaaacg tgctgagatc gtgcaagaag aatccttttc 360
   tcaaaagggt tgttctcacg tcatcatcgt caaccgtgag gctgagggat gaagctgaat 420
   tcccacccaa cgtgttgctg gatgaaacat catggagctc cgtggagttc tgtgaaagta 480
   tccaggtatg gtatggtgtc gcgaagatcc ttgctgagaa atcagcttgg gagttcgcca 540
   aggagaacaa catcgaccta gtggctgttc ttccaacgtt cgtgattgga cctaatctct 600
   cgtctgaatt aggacccact gttttagatg tccttggctt atttaaagga gagacagaga 660
   agttcac 667
<210> 289
   <211> 688
   <212> DNA
   <213> Lolium perenne.
<220>
   <221> misc_feature
   <222> (23) .. (23)
   <223> Any nucleotide
<220>
   <221> misc_feature
   <222> (651) .. (651)
   <223> Any nucleotide
<400> 289
   tcgagtttga gagaatggct tcnagggcag gtgtgtgtta ctggggcctc tggctttgtt 60
   gcttcttggc ttgtcaaaag actactcgag tccggttata atgttctagg gacagtcaga 120
   gacccaggca atcagaagaa ggtagcacac ctctggaact tagcaggggc caaggaaagg 180
   ttggagcttg tcaaagctga cctcttggaa gaagggagct tcgatgatgc tgtgatggcc 240
   tgtgagggtg tcttccacac tgcatcacct atcatcacca aatctgatac caaggaagaa 300
   atgcttgatt ctgcaattaa cggcactcta aacgtgctga gatcgtgcaa gaagaatcct 360
   tttctcaaaa gggttgttct cacgtcatca tcgtcaaccg tgaggctgag ggatgaagct 420
   gaattcccac ccaacgtgtt gctggatgaa acatcatgga gctccgtgga gttctgtgaa 480
   agtatccagg tatggtatgg tgtcgcgaag atccttgctg agaaatcagc ttgggagttc 540
   gccaaggaga acaacatcga cctagtggct gttcttccaa cgttcgtgat tggacctaat 600
   ctctcgtctg aattaggacc cactgtttta gatgtccttg gcttatttaa nggagagaca 660
   gagaagttca ccatgttttg gaaggatg 688
<210> 290
   <211> 425
   <212> DNA
   <213> Lolium perenne
<220>
   <221> misc_feature
   <222> (3) .. (3)
   <223> Any nucleotide
<220>
   <221> misc_feature
   <222> (62) .. (62)
   <223> Any nucleotide
<220>
   <221> misc_feature
   <222> (321) .. (321)
   <223> Any nucleotide
<220>
   <221> misc_feature
   <222> (332) .. (332)
   <223> Any nucleotide
<220>
   <221> misc_feature
   <222> (344) .. (344)
   <223> Any nucleotide
<220>
   <221> misc_feature
   <222> (352) .. (352)
   <223> Any nucleotide
<220>
   <221> misc_feature
   <222> (360) .. (360)
   <223> Any nucleotide
<220>
   <221> misc_feature
   <222> (363) .. (363)
   <223> Any nucleotide
<220>
   <221> misc_feature
   <222> (387) .. (387)
   <223> Any nucleotide
<220>
   <221> misc_feature
   <222> (390) .. (390)
   <223> Any nucleotide
<220>
   <221> misc_feature
   <222> (396) .. (396)
   <223> Any nucleotide
<220>
   <221> misc_feature
   <222> (404) .. (404)
   <223> Any nucleotide
<220>
   <221> misc_feature
   <222> (409) .. (409)
   <223> Any nucleotide
<220>
   <221> misc_feature
   <222> (423) .. (423)
   <223> Any nucleotide
<220>
   <221> misc_feature
   <222> (425) .. (425)
   <223> Any nucleotide
<400> 290
   gtnggcttcc agggccggtg tgtgttactg gggcctctgg ctttgttgct tcttggcttg 60
   tnaaagacta ctcgagtccg gttataatgt tctagggaca gtcagagacc caggcaatca 120
   gaagaaggta gcacacctct ggaacttagc aggggccaag gaaaggttgg agcttgtcaa 180
   agctgacctc ttggaagaag ggagcttcga tgatgctgtg atggcctgtg agggtgtctt 240
   ccacactgca tcacctatca tcaccaaatc tgataccaag gaagaaatgc ttgattctgc 300
   aattaacggc actctaaacg ngctgagatc gngcaagaag aatncttttc tnaaaagggn 360
   tgntctcacg tcatcatcgc cacccgngan gctganggat gaanctgant tcccacccaa 420
   cgngn 425
<210> 291
   <211> 691
   <212> DNA
   <213> Lolium perenne
<220>
   <221> misc_feature
   <222> (691) .. (691)
   <223> Any nucleotide
<400> 291
   gcattcgagt ttgagagaat ggcttccagg gcaggtgtgt gttactgggg cctctggctt
   60
   tgttgcttcc tggcttgtca aaagacttct cgagtccggt tataatgttc tagggacagt
   120
   cagagaccca ggcaatcaga agaaggtagc acacctctgg agcttagcag gggccaagga
   180
   aaggctggag cttgtcagag ctgacctctt ggaagaaggg agcttcgatg atgccgtgat
   240
   ggcctgtgag ggtgtcttcc acactgcatc acctatcatc accaaatctg ataccaagga
   300
   agaaatgctt gattctgcaa taaacggcac tctaaacgtg ctgagatcgt gcaagaagaa
   360
   tccttttctc aaaagggttg ttctcacgtc atcatcgtca accgtgaggc tgagggatga
   420
   agctgaattc ccacccaacg tgttgctgga tgaaacatca tggagctccg tggagttctg
   480
   tgaaagtatc caggtatggt acggtgtcgc aaagatcctt gccgagaaat cagcctggga
   540
   gtttgccaag gagaacaaca tcgacctagt ggctgttctt ccaacattcg tgattggacc
   600
   taatctctcg tctgaattag gacccactgt tttagatgtc cttggcttat ttaaaggaga
   660
   gacagagaag ttcaccatgt ttgggaagga n
   691
<210> 292
   <211> 365
   <212> DNA
   <213> Lolium perenne
<220>
   <221> misc_feature
   <222> (29) .. (29)
   <223> Any nucleotide
<220>
   <221> misc_feature
   <222> (331) .. (331)
   <223> Any nucleotide
<220>
   <221> misc_feature
   <222> (346) .. (347)
   <223> Any nucleotides
<220>
   <221> misc_feature
   <222> (356) .. (356)
   <223> Any nucleotide
<220>
   <221> misc_feature
   <222> (365) .. (365)
   <223> Any nucleotide
<400> 292
   gtctttcgag tttgagcaga atggcttcna gggcaggtgt gtgttactgg ggcctctggc
   60
   tttgttgctt cctggcttgt caaaagactt ctcgagtccg gttataatgt tctagggaca
   120
   gtcagagacc caggcaatca gaagaaggta gcacacctct ggagcttagc aggggccaag
   180
   gaaaggctgg agcttgtcag agctgacctc ttggaagaag ggagcttcga tgatgccgtg
   240
   atggcctgtg agggtgtctt ccacactgca tcacctatca tcaccaaatc tgataccaag
   300
   gaagaaatgc ttgattctgc aataaacggc nctctaaacg tgctgnnatc cgggtnaaaa
   360
   aaaan
   365
<210> 293
   <211> 524
   <212> DNA
   <213> Lolium perenne
<220>
   <221> misc_feature
   <222> (169) .. (169)
   <223> Any nucleotide
<400> 293
   gtcctcgcct acgagcgccc cgacgcccgc ggccgctacc tctgcatcgg ggccgtgctg 60
   caccgcgcgc acttcctaaa gcttctcaag gacctcttcc cgcagtactc cttcaccgcc 120
   aagtgcgaag acgacggcaa gcccatggcg aagccgtaca agttctccna ccagaggctc 180
   agggacctgg gattaaaatt cactccgctg gcggaaagtt tgtacgagac cgtgacgtgc 240
   ctgcaaaaaa atggccacct gcctctgccc gctcccatgg cgccaaagcg tgcataccta 300
   taatactaca aagacacggc cgggatcgac aagccaagaa acagaggatt ctcccgaggt 360
   tcaccatgga attgtgtatt tcacaaagtt tgaattctta ttttttttat tatgaagaaa 420
   tacggaaaac caatactgta taccagaggc aagtgtaaca atgtaaatag tcgtgtaaat 480
   cttgttcaag aatgaatgat aaagtatttt ttgcaaaaaa aaaa 524
<210> 294
   <211> 100
   <212> PRT
   <213> Lolium perenne
<220>
   <221> MISC_FEATURE
   <222> (57) .. (57)
   <223> Any amino acid
<400> 294
   Val Leu Ala Tyr Glu Arg Pro Asp Ala Arg Gly Arg Tyr Leu Cys Ile
   1               5                   10                  15
   Gly Ala Val Leu His Arg Ala His Phe Leu Lys Leu Leu Lys Asp Leu
               20                  25                  30
   Phe Pro Gln Tyr Ser Phe Thr Ala Lys Cys Glu Asp Asp Gly Lys Pro
           35                  40                  45
   Met Ala Lys Pro Tyr Lys Phe Ser Xaa Gln Arg Leu Arg Asp Leu Gly
       50                  55                  60
   Leu Lys Phe Thr Pro Leu Ala Glu Ser Leu Tyr Glu Thr Val Thr Cys
   65                  70                  75                  80
   Leu Gln Lys Asn Gly His Leu Pro Leu Pro Ala Pro Met Ala Pro Lys
                   85                  90                  95
   Arg Ala Tyr Leu
               100

<210> 295
   <211> 524
   <212> DNA
   <213> Lolium perenne
<220>
   <221> misc_feature
   <222> (306) .. (306)
   <223> Any nucleotide
<220>
   <221> misc_feature
   <222> (482) .. (482)
   <223> Any nucleotide
<220>
   <221> misc_feature
   <222> (519) .. (519)
   <223> Any nucleotide
<400> 295
   gtcctcgcct acgagcgccc cgacgcccgc ggccgctacc tctgcatcgg ggccgtgctg 60
   caccgcgcgc acttcctaaa gcttctcaag gacctcttcc cgcagtactc cttcaccgcc 120
   aagtgcgaag acgacggcaa gcccatggcg aagccgtaca agttctccaa ccagaggctc 180
   agggacctgg gattaaaatt cactccgctg gcggaaagtt tgtacgagac cgtgacgtgc 240
   ctgcaaaaaa atggccacct gcctctgccc gctcccgtgg cgccaaagcg tgcataccta 300
   taatantacc aagacacggc cgggatcgac aagccaagaa acagaggatt ctcccgaggt 360
   tcaccatgga attgtgtatt tcagaaagtt tgaattctta ttttttttat tatgaaggaa 420
   tacggataac caatactgta taccagaggc aagtgtaaca atgtaaatag tcgtgtaaat 480
   cntgttcaag attgaatgat aaagtatttt ttgcaaaana aaaa 524
<210> 296
   <211> 374
   <212> DNA
   <213> Lolium perenne
<220>
   <221> misc_feature
   <222> (19) .. (19)
   <223> Any nucleotide
<220>
   <221> misc_feature
   <222> (25) .. (26)
   <223> Any nucleotides
<400> 296
   aagccgtaca agttctccna ccagnngctc agggacctgg gattaaaatt cactccgctg 60
   gcggaaagtt tgtacgagac cgtgacgtgc ctgcaaaaaa atggccacct gcctctgccc 120
   gctcccatgg cgccaaagcg tgcataccta taatactaca aagacacggc cgggatcgac 180
   aagccaagaa acagaggatt ctcccgaggt tcaccatgga attgtgtatt tcacaaagtt 240
   tgaattctta ttttttttat tatgaagaaa tacggaaaac caatactgta taccagaggc 300
   aagtgtaaca atgtaaatag tcgtgtaaat cttgttcaag aatgaatgat aaagtatttt 360
   ttgcaaaaaa aaaa 374
<210> 297
   <211> 363
   <212> DNA
   <213> Lolium perenne
<220>
   <221> misc_feature
   <222> (7) .. (8)
   <223> Any nucleotides
<220>
   <221> misc_feature
   <222> (363) .. (363)
   <223> Any nucleotide
<400> 297
   gttctcnnac cagaggctca gggacctggg attaaaattc actccgctgg cggaaagttt 60
   gtacgagacc gtgacgtgcc tgcaaaaaaa tggccacctg cctctgcccg ctcccatggc 120
   gccaaagcgt gcatacctat aatactacaa agacacggcc gggatcgaca agccaagaaa 180
   cagaggattc tcccgaggtt caccatggaa ttgtgtattt cacaaagttt gaattcttat 240
   tttttttatt atgaagaaat acggaaaacc aatactgtat accagaggca agtgtaacaa 300
   tgtaaatagt cgtgtaaatc ttgttcaaga atgaatgata aagtattttt tgcaaaaaaa
   360
   aan
   363
<210> 298
   <211> 1381
   <212> DNA
   <213> Lolium perenne
<220>
   <221> misc_feature
   <222> (5) .. (5)
   <223> Any nucleotide
<220>
   <221> misc_feature
   <222> (1280) .. (1280)
   <223> Any nucleotide
<220>
   <221> misc_feature
   <222> (1338) .. (1338)
   <223> Any nucleotide
<400> 298
   tctcnagaca cactgtgtaa ccacggtagc gagtggcaag actagcagaa agtacggaca 60
   tcagctaacc attcctcaac tagaataagc atggctccgg cgatgtccaa ccctctcctc 120
   agtgatcggg tggcacgctc caagaaagtc ccatctagcc acgttagagc ggtgggagac 180
   cgcccagacc tcgccaatgt cgaccacgag tccggcgcgg gcattccgct catcgacctg 240
   aagcagctcg aaggtccagg gcgccgcagg gtcgtcgagg ccatcggctc cgcgtgcgag 300
   aacgatgggt ttttcatggt gacgaatcat ggcatcccag aggcggtcgt ggaggggatg 360
   ctgagcgtgg cgagggagtt cttccacctg ccggagtcgg agcggctcaa gtgctactcc 420
   gacgacccca agaaggcggt ccggctgtcg acgagcttca acgtgcgcac ggagaaggtg 480
   agcaactggc gcgacttcct ccggctgcat tgctaccctc ttgagagctt cgtcgaccag 540
   tggccgtcga acccgcccgc cttcaggcaa gtcgtcggca cctactcgac ggaagcgaga 600
   gcgctggcgc tgaggctcct ggaggcgata tcggagagcc tagggctgga gagaggccac 660
   atggtgaagg ccatggggcg gcacgcgcag cacatggcgg tgaactacta cccgccgtgc 720
   ccgcagccgg agctcaccta cggtctgcca gggcacacgg accccaacgc cctcaccatc 780
   ctcctcatgg atccccacgt ctccggcctc caggtcctca gggacggcgc caagtggatc 840
   gccgtccacc cacgccccaa cgccctggtc atcaacctag gcgaccagct acaggcgctg 900
   agcaacggcg cgtacaagag cgtgtggcac cgggcagtgg tgaacgcgga gcaggagcgt 960
   ctgtcggtgg catctttcct gtgcccgtgc aacagcgcgg ttatctgccc cgcgccgagg 1020
   ctcgtcggcg acggggagga ccccgtctac cggagctaca cctacgacga gtactacaag 1080
   aggttttgga gcaggaacct ggatcaggag cactgcctcg agctcttcag gagtcagcac 1140
   tgatgcttga accttgagtt actagctagc tctccttaac agtgcaaatc catggcccaa 1200
   gagggccccg attgcatggt tacttatgtt gtttgaactg gtattgctta agtgcctaat 1260
   aacattgcta cattctactn ctatcttgtc cgtttaaaat tataagatgg cctaaccttt 1320
   ttcttaattg tatgcatnct gaacatattt aagtgtgtgt gttcagacag tttagtctgc 1380
   a 1381
<210> 299
   <211> 346
   <212> PRT
   <213> Lolium perenne
<400> 299
   Met Ser Asn Pro Leu Leu Ser Asp Arg Val Ala Arg Ser Lys Lys Val
   1 5 10 15
   Pro Ser Ser His Val Arg Ala Val Gly Asp Arg Pro Asp Leu Ala Asn
   20 25 30
   Val Asp His Glu Ser Gly Ala Gly Ile Pro Leu Ile Asp Leu Lys Gln
   35 40 45
   Leu Glu Gly Pro Gly Arg Arg Arg Val Val Glu Ala Ile Gly Ser Ala
   50 55 60
   Cys Glu Asn Asp Gly Phe Phe Met Val Thr Asn His Gly Ile Pro Glu
   65 70 75 80
   Ala Val Val Glu Gly Met Leu Ser Val Ala Arg Glu Phe Phe His Leu
   85 90 95
   Pro Glu Ser Glu Arg Leu Lys Cys Tyr Ser Asp Asp Pro Lys Lys Ala
   100 105 110
   Val Arg Leu Ser Thr Ser Phe Asn Val Arg Thr Glu Lys Val Ser Asn
   115 120 125
   Trp Arg Asp Phe Leu Arg Leu His Cys Tyr Pro Leu Glu Ser Phe Val
   130 135 140
   Asp Gln Trp Pro Ser Asn Pro Pro Ala Phe Arg Gln Val Val Gly Thr
   145 150 155 160
   Tyr Ser Thr Glu Ala Arg Ala Leu Ala Leu Arg Leu Leu Glu Ala Ile
   165 170 175
   Ser Glu Ser Leu Gly Leu Glu Arg Gly His Met Val Lys Ala Met Gly
   180 185 190
   Arg His Ala Gln His Met Ala Val Asn Tyr Tyr Pro Pro Cys Pro Gln
   195 200 205
   Pro Glu Leu Thr Tyr Gly Leu Pro Gly His Thr Asp Pro Asn Ala Leu
   210 215 220
   Thr Ile Leu Leu Met Asp Pro His Val Ser Gly Leu Gln Val Leu Arg
   225 230 235 240
   Asp Gly Ala Lys Trp Ile Ala Val His Pro Arg Pro Asn Ala Leu Val
   245 250 255
   Ile Asn Leu Gly Asp Gln Leu Gln Ala Leu Ser Asn Gly Ala Tyr Lys
   260 265 270
   Ser Val Trp His Arg Ala Val Val Asn Ala Glu Gln Glu Arg Leu Ser
   275 280 285
   Val Ala Ser Phe Leu Cys Pro Cys Asn Ser Ala Val Ile Cys Pro Ala
   290 295 300
   Pro Arg Leu Val Gly Asp Gly Glu Asp Pro Val Tyr Arg Ser Tyr Thr
   305 310 315 320
   Tyr Asp Glu Tyr Tyr Lys Arg Phe Trp Ser Arg Asn Leu Asp Gln Glu
   325 330 335
   His Cys Leu Glu Leu Phe Arg Ser Gln His
   340 345
<210> 300
   <211> 755
   <212> DNA
   <213> Lolium perenne
<220>
   <221> misc_feature
   <222> (5) .. (5)
   <223> Any nucleotide
<400> 300
   tctcnagaca cactgtgtaa ccacggtagc gagtggcaag actagcagaa agtacggaca 60
   tcagctaacc attcctcaac tagaataagc atggctccgg cgatgtccaa ccctctcctc 120
   agtgatcggg tggcacgctc caagaaagtc ccatctagcc acgttagagc ggtgggagac 180
   cgcccagacc tcgccaatgt cgaccacgag tccggcgcgg gcattccgct catcgacctg 240
   aagcagctcg aaggtccagg gcgccgcagg gtcgtcgagg ccatcggctc cgcgtgcgag 300
   aacgatgggt ttttcatggt gacgaatcat ggcatcccag aggcggtcgt ggaggggatg 360
   ctgagcgtgg cgagggagtt cttccacctg ccggagtcgg agcggctcaa gtgctactcc 420
   gacgacccca agaaggcggt ccggctgtcg acgagcttca acgtgcgcac ggagaaggtg 480
   agcaactggc gcgacttcct ccggctgcat tgctaccctc ttgagagctt cgtcgaccag 540
   tggccgtcga acccgcccgc cttcaggcaa gtcgtcggca cctactcgac ggaagcgaga 600
   gcgctggcgc tgaggctcct ggaggcgata tcggagagcc tagggctgga gagaggccac 660
   atggtgaagg ccatggggcg gcacgcgcag cacatggcgg tgaactacta cccgccgtgc 720
   ccgcagccgg agctcaccta cggtctgcca gggca 755
<210> 301
   <211> 780
   <212> DNA
   <213> Lolium perenne
<220>
   <221> misc_feature
   <222> (21) .. (21)
   <223> Any nucleotide
<220>
   <221> misc_feature
   <222> (778) .. (778)
   <223> Any nucleotide
<400> 301
   tctagacaca ctgtgtaacc ncggtagcga gtggcaagac tagcagaaag tacggacatc 60
   agctaaccat tcctcaacta gaataagcat ggctccggcg atgtccaacc ctctcctcag 120
   tgatcgggtg gcacgctcca agaaagtccc atctagccac gttagagcgg tgggagaccg 180
   cccagacctc gccaatgtcg accacgagtc cggcgcgggc attccgctca tcgacctgaa 240
   gcagctcgaa ggtccagggc gccgcagggt cgtcgaggcc atcggctccg cgtgcgagaa 300
   cgatgggttt ttcatggtga cgaatcatgg catcccagag gcggtcgtgg aggggatgct 360
   gagcgtggcg agggagttct tccacctgcc ggagtcggag cggctcaagt gctactccga 420
   cgaccccaag aaggcggtcc ggctgtcgac gagcttcaac gtgcgcacgg agaaggtgag 480
   caactggcgc gacttcctcc ggctgcattg ctaccctctt gagagcttcg tcgaccagtg 540
   gccgtcgaac ccgcccgcct tcaggcaagt cgtcggcacc tactcgacgg aagcgagagc 600
   gctggcgctg aggctcctgg aggcgatatc ggagagccta gggctggaga gaggccacat 660
   ggtgaaggcc atggggcggc acgcgcagca catggcggtg aactactacc cgccgtgccc 720
   gcagccggag ctcacctacg gtctgccagg gcacacggac cccaatgccc tcaccatnct 780
<210> 302
   <211> 793
   <212> DNA
   <213> Lolium perenne
<220>
   <221> misc_feature
   <222> (64) .. (64)
   <223> Any nucleotide
<220>
   <221> misc_feature
   <222> (692) .. (692)
   <223> Any nucleotide
<220>
   <221> misc_feature
   <222> (750) .. (750)
   <223> Any nucleotide
<400> 302
   ggggaagtcc ggctgctggg gcttcggctt ctgggcgcga tctcgctggg gctggggctg 60
   gagngagggt atgtggagaa ggtgctgggc gagcaggagc agcacatggc cgtgaactac 120
   tacccgcggt gccccgagcc ggacctcacc tacggcctgc ccaagcacac ggaccccaac 180
   gccctcacca tcctcctcat ggatccccac gtctccggcc tccaggtcct cagggacggc 240
   gccaagtgga tcgccgtcca cccacgcccc aacgccctgg tcatcaacct aggcgaccag 300
   ctacaggcgc tgagcaacgg cgcgtacaag agcgtgtggc accgggcagt ggtgaacgcg 360
   gagcaggagc gtctgtcggt ggcatctttc ctgtgcccgt gcaacagcgc ggttatctgc 420
   cccgcgccga ggctcgtcgg cgacggggag gaccccgtct accggagcta cacctacgac 480
   gagtactaca agaggttttg gagcaggaac ctggatcagg agcactgcct cgagctcttc 540
   aggagtcagc actgatgctt gaaccttgag ttactagcta gctctcctta acagtgcaaa 600
   tccatggccc aagagggccc cgattgcatg gttacttatg ttgtttgaac tggtattgct 660
   taagtgccta ataacattgc tacattctac tnctatcttg tccgtttaaa attataagat 720
   ggcctaacct ttttcttaat tgtatgcatn ctgaacatat ttaagtgtgt gtgttcagac 780
   agtttagtct gca 793
<210> 303
   <211> 1395
   <212> DNA
   <213> Lolium perenne
<400> 303
   gaattcgatt aagcagtggt aacaacgcag agtacgcggg gactctcaga acacactgtg 60
   taaccacggt agcgagtggc aagactagca gaaagtacgg acatcagcta accattcctc 120
   aactagaata agcatggctc cggcgatgtc caaccctctc ctcagtgatc gggtggcacg 180
   ctccaagaaa gtcccatcta gccacgttag agcggtggga gaccgcccag acctcgccaa 240
   tgtcgaccac gagtccggcg cgggcattcc gctcatcgac ctgaagcagc tcgaaggtcc 300
   agggcgccgc agggtcgtcg aggccatcgg ctccgcgtgc gagaacgatg ggtttttcat 360
   ggtgacgaat catggcatcc cagaggcggt cgtggagggg atgctgagcg tggcgaggga 420
   gttcttccac ctgccggagt cggagcggct caagtgctac tccgacgacc ccaagaaggc 480
   ggtccggctg tcgacgagct tcaacgtgcg cacggagaag gtgagcaact ggcgcgactt 540
   cctccggctg cattgctacc ctcttgagag cttcgtcgac cagtggccgt cgaacccgcc 600
   cgccttcagg caagtcgtcg gcacctactc gacggaagcg agagcgctgg cgctgaggct 660
   cctggaggcg atatcggaga gcctagggct ggagagaggc cacatggtga aggccatggg 720
   gcggcacgcg cagcacatgg cggtgaacta ctacccgccg tgcccgcagc cggagctcac 780
   ctacggtctg ccagggcaca aggaccccaa tgccatcacg ctcctcctgc aggacggcgt 840
   ctccggcctg caggtccagc gcgacggccg gtgggtggcc gtcaacccgg tgcccaacgc 900
   cctcgtcatc aacatcggcg atcagttaca ggcgctgagc aacgaccgat acaagagcgt 960
   gaaccacaga gtgatcgtca acagcgcgag cgagaggatt tcggtgccga cgttctactg 1020
   cccgtcgccg gacacggtgg tcgcgccggc cgacgcgctg gtggacgacg cccaccctcg 1080
   ggcctaccag cccttcacgt accaggagta ctacgaggag ttctggaaga tgggccttca 1140
   gtcagcaagt tgcctcgaca ggttccgacg gatcgagtga tggacaagac gtgggccgtt 1200
   gttatctcct gggccatgag cgttgccgca gccgatgtgt cgccatatgg tggagacgtt 1260
   tcctccctcc ggaaaagaaa aataaaacag agtggagacc actagaaccg tcagatagca 1320
   tcccaaaaaa aaaaaaaaaa aaaaaaaaaa aaaagtactc tgcgttgtta ccactgctta 1380
   atcactagtg aattc 1395
<210> 304
   <211> 348
   <212> PRT
   <213> Lolium perenne
<400> 304
   Met Ala Pro Ala Met Ser Asn Pro Leu Leu Ser Asp Arg Val Ala Arg
   1               5                   10                  15
   Ser Lys Lys Val Pro Ser Ser His Val Arg Ala Val Gly Asp Arg Pro
               20                  25                  30
   Asp Leu Ala Asn Val Asp His Glu Ser Gly Ala Gly Ile Pro Leu Ile
           35                  40                  45
   Asp Leu Lys Gln Leu Glu Gly Pro Gly Arg Arg Arg Val Val Glu Ala
       50                  55                  60
   Ile Gly Ser Ala Cys Glu Asn Asp Gly Phe Phe Met Val Thr Asn His
   65                  70                  75                  80
   Gly Ile Pro Glu Ala Val Val Glu Gly Met Leu Ser Val Ala Arg Glu
                   85                  90                  95
   Phe Phe His Leu Pro Glu Ser Glu Arg Leu Lys Cys Tyr Ser Asp Asp
               100                 105                 110
   Pro Lys Lys Ala Val Arg Leu Ser Thr Ser Phe Asn Val Arg Thr Glu
           115                 120                 125
   Lys Val Ser Asn Trp Arg Asp Phe Leu Arg Leu His Cys Tyr Pro Leu
       130                 135                 140
   Glu Ser Phe Val Asp Gln Trp Pro Ser Asn Pro Pro Ala Phe Arg Gln
   145                 150                 155                 160
   Val Val Gly Thr Tyr Ser Thr Glu Ala Arg Ala Leu Ala Leu Arg Leu
                   165                 170                 175
   Leu Glu Ala Ile Ser Glu Ser Leu Gly Leu Glu Arg Gly His Met Val
               180                 185                 190
   Lys Ala Met Gly Arg His Ala Gln His Met Ala Val Asn Tyr Tyr Pro
           195                 200                 205
   Pro Cys Pro Gln Pro Glu Leu Thr Tyr Gly Leu Pro Gly His Lys Asp
       210                 215                 220
   Pro Asn Ala Ile Thr Leu Leu Leu Gln Asp Gly Val Ser Gly Leu Gln
   225                 230                 235                 240
   Val Gln Arg Asp Gly Arg Trp Val Ala Val Asn Pro Val Pro Asn Ala
                   245                 250                 255
   Leu Val Ile Asn Ile Gly Asp Gln Leu Gln Ala Leu Ser Asn Asp Arg
               260                 265                 270
   Tyr Lys Ser Val Asn His Arg Val Ile Val Asn Ser Ala Ser Glu Arg
   275 280 285
   Ile Ser Val Pro Thr Phe Tyr Cys Pro Ser Pro Asp Thr Val Val Ala
   290 295 300
   Pro Ala Asp Ala Leu Val Asp Asp Ala His Pro Arg Ala Tyr Gln Pro
   305 310 315 320
   Phe Thr Tyr Gln Glu Tyr Tyr Glu Glu Phe Trp Lys Met Gly Leu Gln
   325 330 335
   Ser Ala Ser Cys Leu Asp Arg Phe Arg Arg Ile Glu
   340 345

<210> 305
   <211> 1309
   <212> DNA
   <213> Trifolium repens
<400> 305
   gaattcgatt aagcagtggt aacaacgcag agtacgcggg ataaaaactg cactagtgtg 60
   tataagtttc ttggtgaaaa aagagtttgt aaattaacat catggctagt atcaaacaaa 120
   ttggaaacaa gaaagcatgt gtgattggtg gcactggttt tgttgcatct atgttgatca 180
   agcagttact tgaaaagggt tatgctgtta atactaccgt tagagaccca gatagcccta 240
   agaaaatatc tcacctagtg gcactgcaaa gtttggggga actgaatcta tttagagcag 300
   acttaacagt tgaagaagat tttgatgctc ctatagcagg atgtgaactt gtttttcaac 360
   ttgctacacc tgtgaacttt gcttctcaag atcctgagaa tgacatgata aagccagcaa 420
   tcaaaggtgt gttgaatgtg ttgaaagcaa ttgcaagagc aaaagaagtt aaaagagtta 480
   tcttaacatc ttcggcagcc gcggtgacta taaatgaact caaagggaca ggtcatgtta 540
   tggatgaaac caactggtct gatgttgaat ttctcaacac tgcaaaacca cccacttggg 600
   gttatcctgc ctcaaaaatg ctagctgaaa aggctgcatg gaaatttgct gaagaaaatg 660
   acattgatct aatcactgtg atacctagtt taacaactgg tccttctctc acaccagata 720
   tcccatctag tgttggcttg gcaatgtctc taataacagg caatgatttt ctcataaatg 780
   ctttgaaagg aatgcagttt ctgtcgggtt cgttatccat cactcatgtt gaggatattt 840
   gccgagctca tatatttctt gcagagaaag aatcagcttc tggtagatac atttgctgtg 900
   ctcacaatac tagtgttccc gagcttgcaa agtttctcaa caaacgatat cctcagtata 960
   aagttccaac tgaatttgat gattgcccca gcaaggcaaa gttgataatc tcttctgaaa 1020
   agcttatcaa agaagggttc agtttcaagc atggtattgc cgaaactttc gaccagactg 1080
   tcgagtattt taagactaag ggggcactga agaattagat tttgatattt ctaattcaat 1140
   agcaaactct aagcttgtta tgtgtttgtg aagttcagag tgaaatatca aatgaataag 1200
   tggagagagc acaataagag gagagcacaa taattttgga aaaaaaaaaa aaaaaaaaaa 1260
   aaaaaaaagt actctgcgtt gttaccactg cttaatcact agtgaattc 1309
<210> 306
   <211> 338
   <212> PRT
   <213> Trifolium repens
<400> 306
   Met Ala Ser Ile Lys Gln Ile Gly Asn Lys Lys Ala Cys Val Ile Gly
   1               5                   10                  15
   Gly Thr Gly Phe Val Ala Ser Met Leu Ile Lys Gln Leu Leu Glu Lys
               20                  25                  30
   Gly Tyr Ala Val Asn Thr Thr Val Arg Asp Pro Asp Ser Pro Lys Lys
           35                  40                  45
   Ile Ser His Leu Val Ala Leu Gln Ser Leu Gly Glu Leu Asn Leu Phe
       50                  55                  60
   Arg Ala Asp Leu Thr Val Glu Glu Asp Phe Asp Ala Pro Ile Ala Gly
   65                  70                  75                  80
   Cys Glu Leu Val Phe Gln Leu Ala Thr Pro Val Asn Phe Ala Ser Gln
                   85                  90                  95
   Asp Pro Glu Asn Asp Met Ile Lys Pro Ala Ile Lys Gly Val Leu Asn
               100                 105                 110
   Val Leu Lys Ala Ile Ala Arg Ala Lys Glu Val Lys Arg Val Ile Leu
           115                 120                 125
   Thr Ser Ser Ala Ala Ala Val Thr Ile Asn Glu Leu Lys Gly Thr Gly
       130                 135                 140
   His Val Met Asp Glu Thr Asn Trp Ser Asp Val Glu Phe Leu Asn Thr
   145                 150                 155                 160
   Ala Lys Pro Pro Thr Trp Gly Tyr Pro Ala Ser Lys Met Leu Ala Glu
                   165                 170                 175
   Lys Ala Ala Trp Lys Phe Ala Glu Glu Asn Asp Ile Asp Leu Ile Thr
               180                 185                 190
   Val Ile Pro Ser Leu Thr Thr Gly Pro Ser Leu Thr Pro Asp Ile Pro
           195                 200                 205
   Ser Ser Val Gly Leu Ala Met Ser Leu Ile Thr Gly Asn Asp Phe Leu
       210                 215                 220
   Ile Asn Ala Leu Lys Gly Met Gln Phe Leu Ser Gly Ser Leu Ser Ile
   225                 230                 235                 240
   Thr His Val Glu Asp Ile Cys Arg Ala His Ile Phe Leu Ala Glu Lys
                   245                 250                 255
   Glu Ser Ala Ser Gly Arg Tyr Ile Cys Cys Ala His Asn Thr Ser Val
               260                 265                 270
   Pro Glu Leu Ala Lys Phe Leu Asn Lys Arg Tyr Pro Gln Tyr Lys Val
           275                 280                 285
   Pro Thr Glu Phe Asp Asp Cys Pro Ser Lys Ala Lys Leu Ile Ile Ser
       290                 295                 300
   Ser Glu Lys Leu Ile Lys Glu Gly Phe Ser Phe Lys His Gly Ile Ala
   305                 310                 315                 320
   Glu Thr Phe Asp Gln Thr Val Glu Tyr Phe Lys Thr Lys Gly Ala Leu
                   325                 330                 335
   Lys Asn
<210> 307
   <211> 1005
   <212> DNA
   <213> Trifolium repens
<400> 307
   gaattcgatt aagcagtggt aacaacgcag agtacgcggg gacttaaaca ttgacacaag 60
   tcccaaataa aaaagatctg aaacaacata gtcaccccat tttttaacat taaactaaaa 120
   atatgtcggc catcaccgca atccaagtcg agaaccttga atttccggct gtggttactt 180
   ctccggccac cggtaagtca tattttcttg gtggtgcagg ggagagaggt ttgactattg 240
   aaggaaactt catcaagttc actgccatag gagtatattt ggaagatgta gcagtggctt 300
   cacttgccac taaatggaag ggtaaatcct ctgaggagtt gcttgagact cttgacttct 360
   atagagacat catttcagga ccctttgaaa agttgattcg aggatcgaag attagggaat 420
   tgagtggtcc tgagtactca aggaaggtta atgaaaactg cgtggcacac ttaaaatctg 480
   ttgggactta tggagatgct gaagctgaag ctatgcaaaa atttgttgaa gccttcaagc 540
   ctattaattt tccacctggt gcctctgttt tttacaggca atcacctgat ggaatattag 600
   ggcttagttt ctctcaagat gcaagtatac cagaaaagga ggctgcagta atagagaaca 660
   aggcagcttc atcggcagtg ttagaaacta tgattggtga acatgctgtt tctcctgatt 720
   taaagcgttg tttggctgca agattacctg ccttgttgaa cgagggtact ttcaagattg 780
   aatgaaaact gattattatt atctccaaaa gcattgcagc acaagattga gtcatttatg 840
   agcatggaca tttttatgtc cacacatgtt taacttttgt atctctcttt agattctcat 900
   caatatcaat aatactaata tgaaacgaag tcaaaaaaaa aaaaaaaaaa aaaaaaaaaa 960
   aaaagtactc tgcgttgtta ccactgctta atcactagtg aattc 1005
<210> 308
   <211> 220
   <212> PRT
   <213> Trifolium repens
<400> 308
   Met Ser Ala Ile Thr Ala Ile Gln Val Glu Asn Leu Glu Phe Pro Ala
   1               5                   10                  15
   Val Val Thr Ser Pro Ala Thr Gly Lys Ser Tyr Phe Leu Gly Gly Ala
               20                  25                  30
   Gly Glu Arg Gly Leu Thr Ile Glu Gly Asn Phe Ile Lys Phe Thr Ala
           35                  40                  45
   Ile Gly Val Tyr Leu Glu Asp Val Ala Val Ala Ser Leu Ala Thr Lys
       50                  55                  60
   Trp Lys Gly Lys Ser Ser Glu Glu Leu Leu Glu Thr Leu Asp Phe Tyr
   65                  70                  75                  80
   Arg Asp Ile Ile Ser Gly Pro Phe Glu Lys Leu Ile Arg Gly Ser Lys
                   85                  90                  95
   Ile Arg Glu Leu Ser Gly Pro Glu Tyr Ser Arg Lys Val Asn Glu Asn
               100                 105                 110
   Cys Val Ala His Leu Lys Ser Val Gly Thr Tyr Gly Asp Ala Glu Ala
           115                 120                 125
   Glu Ala Met Gln Lys Phe Val Glu Ala Phe Lys Pro Ile Asn Phe Pro
       130                 135                 140
   Pro Gly Ala Ser Val Phe Tyr Arg Gln Ser Pro Asp Gly Ile Leu Gly
   145                 150                 155                 160
   Leu Ser Phe Ser Gln Asp Ala Ser Ile Pro Glu Lys Glu Ala Ala Val
                   165                 170                 175
   Ile Glu Asn Lys Ala Ala Ser Ser Ala Val Leu Glu Thr Met Ile Gly
               180                 185                 190
   Glu His Ala Val Ser Pro Asp Leu Lys Arg Cys Leu Ala Ala Arg Leu
           195                 200                 205
   Pro Ala Leu Leu Asn Glu Gly Thr Phe Lys Ile Glu
       210                 215                 220
<210> 309
   <211> 1105
   <212> DNA
   <213> Trifolium repens
<400> 309
   gaattcacta gtgattaagc agtggtaaca acgcagagta cgcggggaca ttacaactca 60
   caacaccttc tccattacca tctatcttct actaagttca acgagatcaa tggcacttcc 120
   ttctgtcacc gctttgaata tcgagaacaa tctattccct cctaccgtca caccaccggg 180
   atccaccaac aatttcttcc tcggcggtgc aggagagcgg ggtcttcaaa ttcaagacaa 240
   atttgtcaaa ttcaccgcta ttggtgttta tctacaggac attgctgttc cttacctcgc 300
   cactaaatgg aagggtaaga ctgctcaaga gctaacggaa actgttcctt tcttcaggga 360
   catcgttaca ggtccatttg agaaatttat gcaggtgaca atgatcttgc cattgactgg 420
   gcaacaatac tcagagaaag tgtcagaaaa ttgtgtagct atttggaagt ctcttgggat 480
   ttataccgac gaagaagcca aagcaattga gaagtttgtt tctgtcttca aagatgaaac 540
   attcccacca ggctcctcta tccttttcac agtattaccc aaaggattag gatcactaac 600
   gataagtttc tctaaagatg gatccattcc agagaccgag tctgcagtta tagagaataa 660
   gctactctca caagctgtgc ttgagtcgat gataggggcg cacggtgtct cccctgcagc 720
   aaaacagagt ttggccacca ggttatccga gttattcaac gaggttggtg atgctagcaa 780
   ctgattatat caacaaaacg aaaatgaaag tcctttctgc aataaagacc aagcggaaat 840
   tttattttag gtgcactttg aaatgacctc tttggcgact ttttcttgta ctaataataa 900
   agagtgtgtt tgtatcatgt tgtaatttta ttttagaaaa agtgaggtaa gaaaggagtc 960
   cttatgttta tttcaattat tgaaaaatta tttgcatgta taattgattt caactgatgt 1020
   tatttaatca cgttttttct aaaaaaaaaa aaaaaaaaaa aaaaaaaaaa gtactctgcg 1080
   ttgttaccac tgcttaatcg aattc 1105
<210> 310
   <211> 224
   <212> PRT
   <213> Trifolium repens
<400> 310
   Met Ala Leu Pro Ser Val Thr Ala Leu Asn Ile Glu Asn Asn Leu Phe
   1               5                   10                  15
   Pro Pro Thr Val Thr Pro Pro Gly Ser Thr Asn Asn Phe Phe Leu Gly
               20                  25                  30
   Gly Ala Gly Glu Arg Gly Leu Gln Ile Gln Asp Lys Phe Val Lys Phe
           35                  40                  45
   Thr Ala Ile Gly Val Tyr Leu Gln Asp Ile Ala Val Pro Tyr Leu Ala
       50                  55                  60
   Thr Lys Trp Lys Gly Lys Thr Ala Gln Glu Leu Thr Glu Thr Val Pro
   65 70 75 80
   Phe Phe Arg Asp Ile Val Thr Gly Pro Phe Glu Lys Phe Met Gln Val
   85 90 95
   Thr Met Ile Leu Pro Leu Thr Gly Gln Gln Tyr Ser Glu Lys Val Ser
   100 105 110
   Glu Asn Cys Val Ala Ile Trp Lys Ser Leu Gly Ile Tyr Thr Asp Glu
   115 120 125
   Glu Ala Lys Ala Ile Glu Lys Phe Val Ser Val Phe Lys Asp Glu Thr
   130 135 140
   Phe Pro Pro Gly Ser Ser Ile Leu Phe Thr Val Leu Pro Lys Gly Leu
   145 150 155 160
   Gly Ser Leu Thr Ile Ser Phe Ser Lys Asp Gly Ser Ile Pro Glu Thr
   165 170 175
   Glu Ser Ala Val Ile Glu Asn Lys Leu Leu Ser Gln Ala Val Leu Glu
   180 185 190
   Ser Met Ile Gly Ala His Gly Val Ser Pro Ala Ala Lys Gln Ser Leu
   195 200 205
   Ala Thr Arg Leu Ser Glu Leu Phe Asn Glu Val Gly Asp Ala Ser Asn
   210 215 220
<210> 311
   <211> 1272
   <212> DNA
   <213> Trifolium repens
<400> 311
   gaattcgatt aagcagtggt aacaacgcag agtacgcggg gattcaaaca tagctcaaag 60
   tgtgtaacaa atttcttaac ttaaaacatt ttcaacccaa caaaaaaaaa caaagacaaa 120
   aacatgggta gtgttgaaat tccaacaaag gttcttacta acagttctag tcaagtgaaa 180
   atgcctgtgg ttggaatggg atcagcacct gatttcacat gtaagaaaga cacaaaagat 240
   gcaatcattg aagccatcaa acagggttat agacactttg atactgctgc tgcttatggc 300
   tcagaacaag ctcttggtga aggtttgaaa gaagcaattg aacttggtct tgtcactaga 360
   gaagaccttt ttgttacttc taaactttgg gtcactgaaa atcatcctca tcttgttgtt
   420
   cctgctcttc aaaaatctct caagactctt caattggagt acttggactt gtatttgatc
   480
   cattggccac ttagttctca gcctggaaag ttttcatttc caattgatgt ggcagatctc
   540
   ttgccatttg atgtgaaggg tgtttgggaa tccatggaag aaggcttgaa acttggactc
   600
   actaaagcta ttggtgttag taacttctct gtcaagaaac ttcaaaatct tgtctcagtt
   660
   gccactgttc ttcctgctgt caatcaagtg gagatgaacc ttgcatggca acaaaagaag
   720
   cttagagaat tttgcaatgc aaatggaata gtgttaactg cattttcacc attgagaaaa
   780
   ggtgcaagca ggggaccaaa tgaagttatg gaaaatgata tgcttaaaga gattgcagat
   840
   gctcatggaa agtctgttgc acaaatttca ttgagatggt tatatgaaca aggagtcact
   900
   tttgttccca agagctatga taaggaaaga atgggtcaaa atttggctat ctttgattgg
   960
   acattggcaa aagaagatca tgagaaaatt gatcaaatta agcagaaccg tttgatccct
   1020
   ggaccaacca agccaggact cagtgaccta tgggatgatg aaatataaag tggaagatgt
   1080
   taaaagtccc ttaagctcac tcaatatcta tctattgtgt actttttgca tttggggttt
   1140
   gaaattgagt caccccttgt tctgtatcga tttaaaattt aaataatcaa tttttcatta
   1200
   caaaaaaaaa aaaaaaaaaa aaaaaaaaaa agtactctgc gttgttacca ctgcttaatc
   1260
   actagtgaat tc
   1272
<210> 312
   <211> 314
   <212> PRT
   <213> Trifolium repens
<400> 312
   Met Gly Ser Val Glu Ile Pro Thr Lys Val Leu Thr Asn Ser Ser Ser
   1 5 10 15
   Gln Val Lys Met Pro Val Val Gly Met Gly Ser Ala Pro Asp Phe Thr
   20 25 30
   Cys Lys Lys Asp Thr Lys Asp Ala Ile Ile Glu Ala Ile Lys Gln Gly
   35 40 45
   Tyr Arg His Phe Asp Thr Ala Ala Ala Tyr Gly Ser Glu Gln Ala Leu
       50                  55                  60
   Gly Glu Gly Leu Lys Glu Ala Ile Glu Leu Gly Leu Val Thr Arg Glu
   65                  70                  75                  80
   Asp Leu Phe Val Thr Ser Lys Leu Trp Val Thr Glu Asn His Pro His
                   85                  90                  95
   Leu Val Val Pro Ala Leu Gln Lys Ser Leu Lys Thr Leu Gln Leu Glu
               100                 105                 110
   Tyr Leu Asp Leu Tyr Leu Ile His Trp Pro Leu Ser Ser Gln Pro Gly
           115                 120                 125
   Lys Phe Ser Phe Pro Ile Asp Val Ala Asp Leu Leu Pro Phe Asp Val
       130                 135                 140
   Lys Gly Val Trp Glu Ser Met Glu Glu Gly Leu Lys Leu Gly Leu Thr
   145                 150                 155                 160
   Lys Ala Ile Gly Val Ser Asn Phe Ser Val Lys Lys Leu Gln Asn Leu
                   165                 170                 175
   Val Ser Val Ala Thr Val Leu Pro Ala Val Asn Gln Val Glu Met Asn
               180                 185                 190
   Leu Ala Trp Gln Gln Lys Lys Leu Arg Glu Phe Cys Asn Ala Asn Gly
           195                 200                 205
   Ile Val Leu Thr Ala Phe Ser Pro Leu Arg Lys Gly Ala Ser Arg Gly
       210                 215                 220
   Pro Asn Glu Val Met Glu Asn Asp Met Leu Lys Glu Ile Ala Asp Ala
   225                 230                 235                 240
   His Gly Lys Ser Val Ala Gln Ile Ser Leu Arg Trp Leu Tyr Glu Gln
                   245                 250                 255
   Gly Val Thr Phe Val Pro Lys Ser Tyr Asp Lys Glu Arg Met Gly Gln
               260                 265                 270
   Asn Leu Ala Ile Phe Asp Trp Thr Leu Ala Lys Glu Asp His Glu Lys
           275                 280                 285
   Ile Asp Gln Ile Lys Gln Asn Arg Leu Ile Pro Gly Pro Thr Lys Pro
       290                 295                 300
   Gly Leu Ser Asp Leu Trp Asp Asp Glu Ile
   305 310
<210> 313
   <211> 1548
   <212> DNA
   <213> Trifolium repens
<400> 313
   gaattcgatt aagcagtggt atcaacgcag agtacgcggg gacaacaact ataacttcct 60
   gttattaacc aattgagttc aaattacata catagcagga actatactaa agatatcaac 120
   atggttagtg tttctgaaat tcgcaaggct caaagggctg aaggccctgc aactattttg 180
   gccattggta ctgcaaatcc agcaaatcgt gttgaccaga gtacatatcc tgatttctac 240
   ttcaaaatca ctaacagtga gcataaggtt gagcttaaag agaaatttca gcgcatgtgt 300
   gataaatcta tgatcaagag cagatacatg tatctaacag aagagatttt gaaagaaaat 360
   cctagtcttt gtgaatacat ggcaccttca ttggatgcta ggcaagacat ggtggtggtt 420
   gaggtaccta gacttgggaa ggaggctgca gtgaaagcta tcaaagaatg gggtcaacca 480
   aagtcaaaga ttactcactt aatcttttgc accacaagtg gtgttgacat gcctggtgcc 540
   gattaccaac tcacaaaact cttaggtctt cgcccatatg tgaagaggta catgatgtac 600
   caacaagggt gctttgcagg tgggacggtt cttcgtttgg ccaaggattt ggccgagaac 660
   aacaaaggtg ctcgtgtgtt ggttgtttgc tctgaagtaa ccgcagtcac attccgcggc 720
   cccagtgaca ctcatttgga cagtcttgtt ggacaagcac tattcggaga tggagctgct 780
   gcactcattg ttggctcaga cccagtacca gaaattgaga agccaatatt tgagatggtt 840
   tggaccgcac agacaattgc tccagatagt gaaggtgcca ttgatggtca tcttcgtgaa 900
   gctggactaa catttcatct tcttaaagat gttcctggga ttgtctcaaa gaacattgat 960
   aaggcattgg ttgaggcatt ccaaccatta aacatctctg attacaattc aatcttttgg 1020
   attgctcatc caggtggtcc tgcaattcta gaccaagttg agataaagtt gggcttaaaa 1080
   cctgaaaaaa tgaaggccac cagagatgta cttagtgaat atggtaacat gtcaagtgca 1140
   tgtgtattgt tcatcttaga tgagatgaga aagaaatcgg ctgaaaatgg acttaaaacc
   1200
   acaggagaag gacttgactg gggtgtgttg tttggatttg ggcccggact taccattgaa
   1260
   actgttgttc tacatagtgt ggctatatga gaatgagaga cttgatttgt ttttattgta
   1320
   ttgtattgta ttactttaaa tcttggttga acctccattt taagaataaa tatggagttc
   1380
   aatatggacc atcctgttaa aataatatat cgttaatagc tattatttta gtgtctgttt
   1440
   ctttttacta aactatttta ttttagtatt tgtttttgac caaaaaaaaa aaaaaaaaaa
   1500
   aaaaaaagta ctctgcgttg ttaccactgc ttaatcacta gtgaattc
   1548

<210> 314
   <211> 389
   <212> PRT
   <213> Trifolium repens
<400> 314
   Met Val Ser Val Ser Glu Ile Arg Lys Ala Gln Arg Ala Glu Gly Pro
   1               5                   10                  15
   Ala Thr Ile Leu Ala Ile Gly Thr Ala Asn Pro Ala Asn Arg Val Asp
               20                  25                  30
   Gln Ser Thr Tyr Pro Asp Phe Tyr Phe Lys Ile Thr Asn Ser Glu His
           35                  40                  45
   Lys Val Glu Leu Lys Glu Lys Phe Gln Arg Met Cys Asp Lys Ser Met
       50                  55                  60
   Ile Lys Ser Arg Tyr Met Tyr Leu Thr Glu Glu Ile Leu Lys Glu Asn
   65                  70                  75                  80
   Pro Ser Leu Cys Glu Tyr Met Ala Pro Ser Leu Asp Ala Arg Gln Asp
                   85                  90                  95
   Met Val Val Val Glu Val Pro Arg Leu Gly Lys Glu Ala Ala Val Lys
               100                 105                 110
   Ala Ile Lys Glu Trp Gly Gln Pro Lys Ser Lys Ile Thr His Leu Ile
           115                 120                 125
   Phe Cys Thr Thr Ser Gly Val Asp Met Pro Gly Ala Asp Tyr Gln Leu
       130                 135                 140
   Thr Lys Leu Leu Gly Leu Arg Pro Tyr Val Lys Arg Tyr Met Met Tyr
   145                 150                 155                 160
   Gln Gln Gly Cys Phe Ala Gly Gly Thr Val Leu Arg Leu Ala Lys Asp
                   165                 170                 175
   Leu Ala Glu Asn Asn Lys Gly Ala Arg Val Leu Val Val Cys Ser Glu
               180                 185                 190
   Val Thr Ala Val Thr Phe Arg Gly Pro Ser Asp Thr His Leu Asp Ser
           195                 200                 205
   Leu Val Gly Gln Ala Leu Phe Gly Asp Gly Ala Ala Ala Leu Ile Val
       210                 215                 220
   Gly Ser Asp Pro Val Pro Glu Ile Glu Lys Pro Ile Phe Glu Met Val
   225                 230                 235                 240
   Trp Thr Ala Gln Thr Ile Ala Pro Asp Ser Glu Gly Ala Ile Asp Gly
                   245                 250                 255
   His Leu Arg Glu Ala Gly Leu Thr Phe His Leu Leu Lys Asp Val Pro
               260                 265                 270
   Gly Ile Val Ser Lys Asn Ile Asp Lys Ala Leu Val Glu Ala Phe Gln
           275                 280                 285
   Pro Leu Asn Ile Ser Asp Tyr Asn Ser Ile Phe Trp Ile Ala His Pro
       290                 295                 300
   Gly Gly Pro Ala Ile Leu Asp Gln Val Glu Ile Lys Leu Gly Leu Lys
   305                 310                 315                 320
   Pro Glu Lys Met Lys Ala Thr Arg Asp Val Leu Ser Glu Tyr Gly Asn
                   325                 330                 335
   Met Ser Ser Ala Cys Val Leu Phe Ile Leu Asp Glu Met Arg Lys Lys
               340                 345                 350
   Ser Ala Glu Asn Gly Leu Lys Thr Thr Gly Glu Gly Leu Asp Trp Gly
           355                 360                 365
   Val Leu Phe Gly Phe Gly Pro Gly Leu Thr Ile Glu Thr Val Val Leu
       370                 375                 380
   His Ser Val Ala Ile
   385
<210> 315
   <211> 1447
   <212> DNA
   <213> Trifolium repens
<400> 315
   gaattcacta gtgattaagc agtggtaaca acgcagagta cgcggggaac aaaaacaact 60
   acgcatatta tatatatata tatatagtct ataattgaaa gaaactgcta aagatattat 120
   taagatatgg tgagtgtagc tgaaattcgc aaggctcaga gggctgaagg ccctgcaacc 180
   attttggcca ttggcactgc aaatccacca aaccgtgttg agcagagcac atatcctgat 240
   ttctacttca aaattacaaa cagtgagcac aagactgagc tcaaagagaa gttccaacgc 300
   atgtgtgaca aatccatgat caagagcaga tacatgtatc taacagaaga gattttgaaa 360
   gaaaatccta gtctttgtga atacatggca ccttcattgg atgctaggca agacatggtg 420
   gtggttgagg tacctagact tgggaaggag gctgcagtca aggccattaa agaatggggt 480
   caaccaaagt caaagattac tcacttaatc ttttgcacca caagtggtgt tgacatgcct 540
   ggtgctgatt accaactcac aaaactctta ggtcttcgcc catatgtgaa aaggtatatg 600
   atgtaccaac aaggttgttt tgcaggaggc acggtgcttc gtttggcaaa agatttggcc 660
   gagaacaaca aaggtgctcg tgtgctagtt gtttgttctg aagtcaccgc agtcacattt 720
   cgcggcccca gtgatactca cttggacagt cttgttggac aagcattgtt tggagatgga 780
   gccgctgcac taattgttgg ttctgatcca gtgcctgaaa ttgagaaacc aatatttgag 840
   atggtttgga ctgcacaaac aattgctcca gacagtgaag gtgccattga tggtcatctt 900
   cgtgaagctg ggctaacatt tcatcttctt aaagatgttc ctgggattgt atcaaagaac 960
   attaataaag cattggttga ggctttccaa ccattaggaa tttctgacta caactcaatc 1020
   ttttggattg cacacccggg tggacctgca attcttgatc aagtagaaca aaagctagcc 1080
   ttgaagcccg aaaagatgag ggccacgagg gaagttctaa gtgaatatgg aaacatgtca 1140
   agcgcatgtg tattgttcat cttagatgag atgcggaaga aatcggctca aaatggactt 1200
   aagacaactg gagaaggact tgattggggt gtgttgttcg gcttcggacc aggacttacc 1260
   attgaaaccg ttgttcttcg tagcgtggct atataagatg tgtgattgtt tttattttaa
   1320
   tgtattactt ttaatcttgc tgccttgaat ttcgatttaa gaataaataa atatatcttt
   1380
   tgataaaaaa aaaaaaaaaa aaaaaaaaaa aagtactctg cgttgttacc actgcttaat
   1440
   cgaattc
   1447
<210> 316
   <211> 389
   <212> PRT
   <213> Trifolium repens
<400> 316
   Met Val Ser Val Ala Glu Ile Arg Lys Ala Gln Arg Ala Glu Gly Pro
   1               5                   10                  15
   Ala Thr Ile Leu Ala Ile Gly Thr Ala Asn Pro Pro Asn Arg Val Glu
               20                  25                  30
   Gln Ser Thr Tyr Pro Asp Phe Tyr Phe Lys Ile Thr Asn Ser Glu His
           35                  40                  45
   Lys Thr Glu Leu Lys Glu Lys Phe Gln Arg Met Cys Asp Lys Ser Met
       50                  55                  60
   Ile Lys Ser Arg Tyr Met Tyr Leu Thr Glu Glu Ile Leu Lys Glu Asn
   65                  70                  75                  80
   Pro Ser Leu Cys Glu Tyr Met Ala Pro Ser Leu Asp Ala Arg Gln Asp
                   85                  90                  95
   Met Val Val Val Glu Val Pro Arg Leu Gly Lys Glu Ala Ala Val Lys
               100                 105                 110
   Ala Ile Lys Glu Trp Gly Gln Pro Lys Ser Lys Ile Thr His Leu Ile
           115                 120                 125
   Phe Cys Thr Thr Ser Gly Val Asp Met Pro Gly Ala Asp Tyr Gln Leu
       130                 135                 140
   Thr Lys Leu Leu Gly Leu Arg Pro Tyr Val Lys Arg Tyr Met Met Tyr
   145                 150                 155                 160
   Gln Gln Gly Cys Phe Ala Gly Gly Thr Val Leu Arg Leu Ala Lys Asp
                   165                 170                 175
   Leu Ala Glu Asn Asn Lys Gly Ala Arg Val Leu Val Val Cys Ser Glu
               180                 185                 190
   Val Thr Ala Val Thr Phe Arg Gly Pro Ser Asp Thr His Leu Asp Ser
           195                 200                 205
   Leu Val Gly Gln Ala Leu Phe Gly Asp Gly Ala Ala Ala Leu Ile Val
       210                 215                 220
   Gly Ser Asp Pro Val Pro Glu Ile Glu Lys Pro Ile Phe Glu Met Val
   225                 230                 235                 240
   Trp Thr Ala Gln Thr Ile Ala Pro Asp Ser Glu Gly Ala Ile Asp Gly
                   245                 250                 255
   His Leu Arg Glu Ala Gly Leu Thr Phe His Leu Leu Lys Asp Val Pro
               260                 265                 270
   Gly Ile Val Ser Lys Asn Ile Asn Lys Ala Leu Val Glu Ala Phe Gln
           275                 280                 285
   Pro Leu Gly Ile Ser Asp Tyr Asn Ser Ile Phe Trp Ile Ala His Pro
       290                 295                 300
   Gly Gly Pro Ala Ile Leu Asp Gln Val Glu Gln Lys Leu Ala Leu Lys
   305                 310                 315                 320
   Pro Glu Lys Met Arg Ala Thr Arg Glu Val Leu Ser Glu Tyr Gly Asn
                   325                 330                 335
   Met Ser Ser Ala Cys Val Leu Phe Ile Leu Asp Glu Met Arg Lys Lys
               340                 345                 350
   Ser Ala Gln Asn Gly Leu Lys Thr Thr Gly Glu Gly Leu Asp Trp Gly
           355                 360                 365
   Val Leu Phe Gly Phe Gly Pro Gly Leu Thr Ile Glu Thr Val Val Leu
       370                 375                 380
   Arg Ser Val Ala Ile
   385
<210> 317
   <211> 2394
   <212> DNA
   <213> Trifolium repens
<400> 317
   gaattcgatt aagcagtggt aacaacgcag agtacgcggg gattcaatct gttgtgcata 60
   aaattcactc attgcataga aaaccataca catttgatct tgcaaagaag aaatatggga 120
   gacgaaggta tagtgagagg tgtcacaaag cagacaaccc ctgggaaggc tactatattg 180
   gctcttggca aggcattccc tcaccaactt gtgatgcaag agtgtttagt tgatggttat 240
   tttagggaca ctaattgtga caatcctgaa cttaagcaga aacttgctag actttgtaag 300
   acaaccacgg taaaaacaag gtatgttgtt atgaatgagg agatactaaa gaaatatcca 360
   gaacttgttg tcgaaggcgc ctcaactgta aaacaacgtt tagagatatg taatgaggca 420
   gtaacacaaa tggcaattga agcttcccaa gtttgcctaa agaattgggg tagatcctta 480
   tcggacataa ctcatgtggt ttatgtttca tctagtgaag ctagattacc cggtggtgac 540
   ctatacttgt caaaaggact aggactaaac cctaaaattc aaagaaccat gctctatttc 600
   tctggatgct cgggaggcgt agccggcctt cgcgttgcga aagacgtagc tgagaacaac 660
   cctggaagta gagttttgct tgctacttcg gaaactacaa ttattggatt caagccacca 720
   agtgttgata gaccttatga tcttgttggt gtggcactct ttggagatgg tgctggtgca 780
   atgataattg gctcagaccc ggtatttgaa actgagacac cattgtttga gctgcatact 840
   tcagctcagg agtttatacc agacaccgag aagaaaattg atgggcggct gacggaggag 900
   ggcataagtt tcacactagc aagggaactt ccgcagataa tcgaagacaa tgttgaggga 960
   ttctgtaata aactaattga tgttgttggg ttggagaata aggagtacaa taagttgttt 1020
   tgggctgtgc atccaggtgg gcctgcgata ttgaatcgcg tggagaagcg gcttgagttg 1080
   tcgccgcaga agctgaatgc tagtagaaaa gctctaatgg attatggaaa tgctagcagc 1140
   aatactattg tttatgtgct ggaatatatg ctagaagagg aaaagaagat taaaaaggcg 1200
   ggtggaggag attctgaatg gggattgata cttgcttttg gacctggaat tacttttgag 1260
   gggattctag caaggaactt gtgtgcatga agtcttatac aattgtgatg catgacttat 1320
   actcttattt ctactaatta ttatattaag caaattcaga acttttaagt aatgatttaa 1380
   tgaagaatac ttatagtata ttgactttat tcactttcaa agcaagttta tgatcctaag 1440
   acatggtaga acttgagcat gtggaatagt tgtaacaaaa actctaagca aatagagact 1500
   ttatgtagta taaagcattt ccagacatga taaataatgg tacctcagaa cataaaatat 1560
   atttagctat ctttcatccc caactttaca catccaccaa ggtacagaat aagcatatgt 1620
   caacacaaaa tgtactctaa gtctaacatg agtaaccaaa catgatgcct gattaagtta 1680
   aaagaaaaga aaatctgagg gcatagatct tcaatcacac cactccagag ggaaggcgta 1740
   gaacaagctg tccgccgaaa acactgcaat tcaataaata tcattaggac aacagtgcag 1800
   agtcatgcgg gaaatgtctt aagtcactgt actaaaaata taggattata ttatgaacta 1860
   tactaacctt ttcacataat agtaacagaa atcagctaag atgaatgtct ggacaatttc 1920
   tgagataaga accatgacgg ccataagcca taccccaagg caaccaataa atgtccacgg 1980
   gtatctaaca cctgttgcaa gaaatagtaa gttattagga gatgtgcggt tacgaaattc 2040
   aagctacaca acaaaaggag gccagaacaa cagcaatctt gtaaccagat gacaacaata 2100
   aaatgtaaac ttaaagagac cgaacacaca aacattgcaa ctcagatgga attgctgcca 2160
   tgtaactagt aggagatttg ggacgtcaaa tcagtatatt atgcaaatac aaggtatgac 2220
   cgccttgtct attgtagcat acaacaaacg tacagtgggt ttgtccctct caaaatggca 2280
   ggatctttac agcacaatat ttggttttgt catacttata ccataaaaaa aaaaaaaaaa 2340
   aaaaaaaaaa aaagtactct gcgttgttac cactgcttaa tcactagtga attc 2394
<210> 318
   <211> 391
   <212> PRT
   <213> Trifolium repens
<400> 318
   Met Gly Asp Glu Gly Ile Val Arg Gly Val Thr Lys Gln Thr Thr Pro
   1               5                   10                  15
   Gly Lys Ala Thr Ile Leu Ala Leu Gly Lys Ala Phe Pro His Gln Leu
               20                  25                  30
   Val Met Gln Glu Cys Leu Val Asp Gly Tyr Phe Arg Asp Thr Asn Cys
           35                  40                  45
   Asp Asn Pro Glu Leu Lys Gln Lys Leu Ala Arg Leu Cys Lys Thr Thr
       50                  55                  60
   Thr Val Lys Thr Arg Tyr Val Val Met Asn Glu Glu Ile Leu Lys Lys
   65                  70                  75                  80
   Tyr Pro Glu Leu Val Val Glu Gly Ala Ser Thr Val Lys Gln Arg Leu
                   85                  90                  95
   Glu Ile Cys Asn Glu Ala Val Thr Gln Met Ala Ile Glu Ala Ser Gln
               100                 105                 110
   Val Cys Leu Lys Asn Trp Gly Arg Ser Leu Ser Asp Ile Thr His Val
           115                 120                 125
   Val Tyr Val Ser Ser Ser Glu Ala Arg Leu Pro Gly Gly Asp Leu Tyr
       130                 135                 140
   Leu Ser Lys Gly Leu Gly Leu Asn Pro Lys Ile Gln Arg Thr Met Leu
   145                 150                 155                 160
   Tyr Phe Ser Gly Cys Ser Gly Gly Val Ala Gly Leu Arg Val Ala Lys
                   165                 170                 175
   Asp Val Ala Glu Asn Asn Pro Gly Ser Arg Val Leu Leu Ala Thr Ser
               180                 185                 190
   Glu Thr Thr Ile Ile Gly Phe Lys Pro Pro Ser Val Asp Arg Pro Tyr
           195                 200                 205
   Asp Leu Val Gly Val Ala Leu Phe Gly Asp Gly Ala Gly Ala Met Ile
       210                 215                 220
   Ile Gly Ser Asp Pro Val Phe Glu Thr Glu Thr Pro Leu Phe Glu Leu
   225                 230                 235                 240
   His Thr Ser Ala Gln Glu Phe Ile Pro Asp Thr Glu Lys Lys Ile Asp
                   245                 250                 255
   Gly Arg Leu Thr Glu Glu Gly Ile Ser Phe Thr Leu Ala Arg Glu Leu
               260                 265                 270
   Pro Gln Ile Ile Glu Asp Asn Val Glu Gly Phe Cys Asn Lys Leu Ile
           275                 280                 285
   Asp Val Val Gly Leu Glu Asn Lys Glu Tyr Asn Lys Leu Phe Trp Ala
       290                 295                 300
   Val His Pro Gly Gly Pro Ala Ile Leu Asn Arg Val Glu Lys Arg Leu
   305 310 315 320
   Glu Leu Ser Pro Gln Lys Leu Asn Ala Ser Arg Lys Ala Leu Met Asp
   325 330 335
   Tyr Gly Asn Ala Ser Ser Asn Thr Ile Val Tyr Val Leu Glu Tyr Met
   340 345 350
   Leu Glu Glu Glu Lys Lys Ile Lys Lys Ala Gly Gly Gly Asp Ser Glu
   355 360 365
   Trp Gly Leu Ile Leu Ala Phe Gly Pro Gly Ile Thr Phe Glu Gly Ile
   370 375 380
   Leu Ala Arg Asn Leu Cys Ala
   385 390
<210> 319
   <211> 1663
   <212> DNA
   <213> Trifolium repens
<400> 319
   gaattcgatt aagcagtggt aacaacgcag agtacgcggg gatagcaaca cacactttga 60
   tttctttttg agtccttgct acgtggcttt accaaaaaac gttgctaagt catcaaccat 120
   tccaattcct taatataacc tatcagtact caccatcttt tcttcctccc tgctaacttt 180
   agactcagag aagatggtga atgttaatga gatccgccag gcacagagag ctgaaggccc 240
   tgccaccgtg ttggcaatcg gcactgcaac tcctccaaac tgtgtcgatc agagtacata 300
   cccagactac tacttccgca tcacaaacag tgagcacaag acagagctca aagaaaaatt 360
   ccagcgcatg tgtgacaaat ctatgattaa gaagagatac atgcatttga cagaagagat 420
   tttgaaggag aatccaagtt tatgtgagta catggcacct tcattggatg caagacaaga 480
   catggtggtt gtggaagtac caaggctagg aaaagaggct gcaacaaagg ctatcaagga 540
   atggggtcaa cctaagtcca agattactca cctcatcttt tgcaccacaa gtggtgtgga 600
   catgcctggc gccgactatc agcttacaaa gcttttaggc cttcgtccgc atgtgaagcg 660
   ttatatgatg taccaacaag gttgtttcgc tggtggtacg gtgcttcgtt tggctaaaga 720
   cttggctgaa aacaacaaag gtgcccgtgt gttggtggtt tgttcagaga tcactgcggt
   780
   tactttccgt ggacccagtg acactcatct tgatagcctt gtggggcaag cattgtttgg
   840
   agatggtgca gcagctgtga ttgtaggttc agacccatta ccacaagttg agaagccctt
   900
   gtttgaattg gtatggactg ctcaaacaat ccttccagac agtgaaggag ccattgatgg
   960
   gcaccttcgt gaagtcgggc tgacattcca tctcctcaag gatgttcctg gactcatctc
   1020
   aaagaacatt gagaaagctc ttgttgaggc ctttcaacct ttaggtatct ctgattacaa
   1080
   ttctatattt tggatcgcac atcctggtgg acctgcaatt ctggaccaag tggaagccaa
   1140
   attaagctta aagccagaga aaatgcaagc cacccggcat gtgcttagcg agtatggtaa
   1200
   catgtcaagt gcatgtgtgt tatttatctt ggatgagatg aggaggaagt caaaagaaga
   1260
   tggacttgcc acaacaggcg aggggctgga atggggtgta ctattcggtt ttggacccgg
   1320
   actcactgtt gagactgtat tgctccatag tgttgccact taaattgcct agatatgcta
   1380
   taactatatg cttatttaat tctttgtttc tgggggattt tatcttcact tacttcactg
   1440
   agcatttgaa taaagtttgt tttaattatt cataatgtaa tatggtgttg cttaatgtac
   1500
   ccatccatat aatatttgta atacatatat taatcaactt gcaatttcat gaaaaaaaaa
   1560
   aaaaaaaaaa aaaaaaaaaa aaaaaaaaaa aaggaaaaaa aaaaaaaaaa aaaaaaaaaa
   1620
   aagtactctg cgttgttacc actgcttaat cactagtgaa ttc
   1663
<210> 320
   <211> 389
   <212> PRT
   <213> Trifolium repens
<400> 320
   Met Val Asn Val Asn Glu Ile Arg Gln Ala Gln Arg Ala Glu Gly Pro
   1               5                   10                  15
   Ala Thr Val Leu Ala Ile Gly Thr Ala Thr Pro Pro Asn Cys Val Asp
               20                  25                  30
   Gln Ser Thr Tyr Pro Asp Tyr Tyr Phe Arg Ile Thr Asn Ser Glu His
           35                  40                  45
   Lys Thr Glu Leu Lys Glu Lys Phe Gln Arg Met Cys Asp Lys Ser Met
       50                  55                  60
   Ile Lys Lys Arg Tyr Met His Leu Thr Glu Glu Ile Leu Lys Glu Asn
   65                  70                  75                  80
   Pro Ser Leu Cys Glu Tyr Met Ala Pro Ser Leu Asp Ala Arg Gln Asp
                   85                  90                  95
   Met Val Val Val Glu Val Pro Arg Leu Gly Lys Glu Ala Ala Thr Lys
               100                 105                 110
   Ala Ile Lys Glu Trp Gly Gln Pro Lys Ser Lys Ile Thr His Leu Ile
           115                 120                 125
   Phe Cys Thr Thr Ser Gly Val Asp Met Pro Gly Ala Asp Tyr Gln Leu
       130                 135                 140
   Thr Lys Leu Leu Gly Leu Arg Pro His Val Lys Arg Tyr Met Met Tyr
   145                 150                 155                 160
   Gln Gln Gly Cys Phe Ala Gly Gly Thr Val Leu Arg Leu Ala Lys Asp
                   165                 170                 175
   Leu Ala Glu Asn Asn Lys Gly Ala Arg Val Leu Val Val Cys Ser Glu
               180                 185                 190
   Ile Thr Ala Val Thr Phe Arg Gly Pro Ser Asp Thr His Leu Asp Ser
           195                 200                 205
   Leu Val Gly Gln Ala Leu Phe Gly Asp Gly Ala Ala Ala Val Ile Val
       210                 215                 220
   Gly Ser Asp Pro Leu Pro Gln Val Glu Lys Pro Leu Phe Glu Leu Val
   225                 230                 235                 240
   Trp Thr Ala Gln Thr Ile Leu Pro Asp Ser Glu Gly Ala Ile Asp Gly
                   245                 250                 255
   His Leu Arg Glu Val Gly Leu Thr Phe His Leu Leu Lys Asp Val Pro
               260                 265                 270
   Gly Leu Ile Ser Lys Asn Ile Glu Lys Ala Leu Val Glu Ala Phe Gln
           275                 280                 285
   Pro Leu Gly Ile Ser Asp Tyr Asn Ser Ile Phe Trp Ile Ala His Pro
       290                 295                 300
   Gly Gly Pro Ala Ile Leu Asp Gln Val Glu Ala Lys Leu Ser Leu Lys
   305                 310                 315                 320
   Pro Glu Lys Met Gln Ala Thr Arg His Val Leu Ser Glu Tyr Gly Asn
                   325                 330                 335
   Met Ser Ser Ala Cys Val Leu Phe Ile Leu Asp Glu Met Arg Arg Lys
               340                 345                 350
   Ser Lys Glu Asp Gly Leu Ala Thr Thr Gly Glu Gly Leu Glu Trp Gly
           355                 360                 365
   Val Leu Phe Gly Phe Gly Pro Gly Leu Thr Val Glu Thr Val Leu Leu
       370                 375                 380
   His Ser Val Ala Thr
   385

<210> 321
   <211> 1653
   <212> DNA
   <213> Trifolium repens
<400> 321
   gaattcgatt aagcagtggt aacaacgcag agtacgcggg actaagcctt gattcattgt 60
   ttgtttccat aacacaagaa ctagtgtttg cttgaatctt aagaaaaaat gcctcaaggt 120
   gatttgaatg gaagttcctc ggtgaatgga gcacgtgcta gacgtgctcc tactcaggga 180
   aaggcaacga tacttgcatt aggaaaggct ttccccgccc aggtcctccc tcaagagtgc 240
   ttggtggaag gattcattcg cgacactaag tgtgacgata cttatattaa ggagaaattg 300
   gagcgtcttt gcaaaaacac aactgtgaaa acaagataca cagtaatgtc aaaggagatc 360
   ttagacaact atccagagct agccatagat ggaacaccaa caataaggca aaagcttgaa 420
   atagcaaatc cagcagtagt tgaaatggca acaagagcaa gcaaagattg catcaaagaa 480
   tggggaaggt cacctcaaga tatcacacac atagtctatg tttcctcgag cgaaattcgt 540
   ctacccggtg gtgaccttta tcttgcaaat gaactcggct taaacagcga tgttaatcgc 600
   gtaatgctct atttcctcgg ttgctacggc ggtgtcactg gcttacgtgt cgccaaagac 660
   atcgccgaaa ataaccctgg tagtagggtg ttactcacaa catccgagac cactattctc 720
   ggttttcgac caccgagtaa agctagacct tatgacctcg ttggcgctgc acttttcggt
   780
   gatggcgccg ctgctgcaat aattggaaca gaccctatat tgaatcaaga atcacctttc
   840
   atggaattga accatgcagt ccaaaaattc ttgcctgata cacaaaatgt gattgatggt
   900
   agaatcactg aagagggtat taattttaag cttggaagag accttcctca aaaaattgaa
   960
   gacaatattg aagaattttg caagaaaatt atggctaaaa gtgatgttaa ggaatttaat
   1020
   gacttatttt gggctgttca tcctggtggg ccagctatac tcaataagct agaaaatata
   1080
   ctcaaattga aaagtgataa attggattgt agtaggaagg cattaatgga ttatggaaat
   1140
   gttagtagca atactatatt ctatgtgatg gagtatatga gagattattt gaaggaagat
   1200
   ggaagtgaag aatggggatt aggattggct tttggaccag ggattacttt tgaaggggtt
   1260
   ctcctccgta gcctttaatc ttgaaataat aattcatatg aaattacttg tcttaagatt
   1320
   gtgataggaa gatgaatatg tattggatta atattgatat ggtgttattt taagttgatt
   1380
   ttaaaaaaag tttattaata aagtatgatg taacaattgt tgtttgaatg ttaaaaggga
   1440
   agtatactat tttaagttct tgaccatact gattttttct ttacacattt tcatatctaa
   1500
   aattgttcta tgatatcttc attgttgata ctgtaataat ataatatcta atttggctgg
   1560
   caaaatgaaa gatttttcac cgaaaaaaaa aaaaaaaaaa aaaaaaaaaa aagtactctg
   1620
   cgttgttacc actgcttaat cactagtgaa ttc
   1653
<210> 322
   <211> 389
   <212> PRT
   <213> Trifolium repens
<400> 322
   Met Pro Gln Gly Asp Leu Asn Gly Ser Ser Ser Val Asn Gly Ala Arg
   1               5                   10                  15
   Ala Arg Arg Ala Pro Thr Gln Gly Lys Ala Thr Ile Leu Ala Leu Gly
               20                  25                  30
   Lys Ala Phe Pro Ala Gln Val Leu Pro Gln Glu Cys Leu Val Glu Gly
           35                  40                  45
   Phe Ile Arg Asp Thr Lys Cys Asp Asp Thr Tyr Ile Lys Glu Lys Leu
   50 55 60
   Glu Arg Leu Cys Lys Asn Thr Thr Val Lys Thr Arg Tyr Thr Val Met
   65 70 75 80
   Ser Lys Glu Ile Leu Asp Asn Tyr Pro Glu Leu Ala Ile Asp Gly Thr
   85 90 95
   Pro Thr Ile Arg Gln Lys Leu Glu Ile Ala Asn Pro Ala Val Val Glu
   100 105 110
   Met Ala Thr Arg Ala Ser Lys Asp Cys Ile Lys Glu Trp Gly Arg Ser
   115 120 125
   Pro Gln Asp Ile Thr His Ile Val Tyr Val Ser Ser Ser Glu Ile Arg
   130 135 140
   Leu Pro Gly Gly Asp Leu Tyr Leu Ala Asn Glu Leu Gly Leu Asn Ser
   145 150 155 160
   Asp Val Asn Arg Val Met Leu Tyr Phe Leu Gly Cys Tyr Gly Gly Val
   165 170 175
   Thr Gly Leu Arg Val Ala Lys Asp Ile Ala Glu Asn Asn Pro Gly Ser
   180 185 190
   Arg Val Leu Leu Thr Thr Ser Glu Thr Thr Ile Leu Gly Phe Arg Pro
   195 200 205
   Pro Ser Lys Ala Arg Pro Tyr Asp Leu Val Gly Ala Ala Leu Phe Gly
   210 215 220
   Asp Gly Ala Ala Ala Ala Ile Ile Gly Thr Asp Pro Ile Leu Asn Gln
   225 230 235 240
   Glu Ser Pro Phe Met Glu Leu Asn His Ala Val Gln Lys Phe Leu Pro
   245 250 255
   Asp Thr Gln Asn Val Ile Asp Gly Arg Ile Thr Glu Glu Gly Ile Asn
   260 265 270
   Phe Lys Leu Gly Arg Asp Leu Pro Gln Lys Ile Glu Asp Asn Ile Glu
   275 280 285
   Glu Phe Cys Lys Lys Ile Met Ala Lys Ser Asp Val Lys Glu Phe Asn
   290 295 300
   Asp Leu Phe Trp Ala Val His Pro Gly Gly Pro Ala Ile Leu Asn Lys
   305 310 315 320
   Leu Glu Asn Ile Leu Lys Leu Lys Ser Asp Lys Leu Asp Cys Ser Arg
   325 330 335
   Lys Ala Leu Met Asp Tyr Gly Asn Val Ser Ser Asn Thr Ile Phe Tyr
   340 345 350
   Val Met Glu Tyr Met Arg Asp Tyr Leu Lys Glu Asp Gly Ser Glu Glu
   355 360 365
   Trp Gly Leu Gly Leu Ala Phe Gly Pro Gly Ile Thr Phe Glu Gly Val
   370 375 380
   Leu Leu Arg Ser Leu
   385
<210> 323
   <211> 1600
   <212> DNA
   <213> Trifolium repens
<400> 323
   gaattcacta gtgattaagc agtggtaaca acgcagagta cgcgggggaa tccaccaaat 60
   caacaccatt aataaccttc caaattctcg ttacctcacc aaatctcatt tttcattata 120
   tatcttgggt acatcttttg ttacctccaa caaaaaaatg gtgaccgtag aagagattcg 180
   taacgcccaa cgttcaaatg gccctgccac tatcttagct tttggcacag ccactccttc 240
   taactgtgtc actcaagctg attatcctga ttactacttt cgtatcacca acagcgaaca 300
   tatgactgat cttaaggaaa aattcaagcg gatgtgtgat agatcaatga taaagaaacg 360
   ttacatgcac ctaacagaag actttctgaa ggagaatcca aatatgtgtg aatacatggc 420
   accatcacta gatgtaagac gagacatagt ggttgttgaa gtaccaaagc taggtaaaga 480
   agcagcaaaa aaagccatat gtgaatgggg acaaccaaaa tccaaaatca cacatcttgt 540
   tttctgcacc acttccggtg ttgacatgcc gggagccgat taccaactca ccaaactttt 600
   aggcttaaaa ccttctgtca agcgtctcat gatgtatcaa caaggttgtt tcgctggcgg 660
   cacagttctc cgcttagcaa aagaccttgt tgagaataac aaaaatgcaa gagttcttgt 720
   tgtttgttct gaaattactg cggttacttt tcgtggacca tcggatactc atcttgattc 780
   gctcgtggga caggcgcttt ttggtgatgg agccgcagca atgattattg gtgcggatcc 840
   tgatttaacc gtggagcgtc cgattttcga gattgtttcg gctgctcaga ctattcttcc 900
   tgattctgat ggcgcaattg atggacatct tcgtgaagtg gggctcactt ttcatttatt 960
   gaaagatgtt ccggggatta tttcaaagaa cattgaaaaa agtttagttg aagcttttgc 1020
   gcctattggg attaatgatt ggaactcaat attttgggtt gcacatccag gtggaccggc 1080
   tattttagac caggttgaag agaaactcca tcttaaagag gagaaactcc ggtccacccg 1140
   gcatgtgctt agtgaatatg gaaatatgtc aagtgcatgt gttttattta ttttggatga 1200
   aatgagaaag aggtctaaag aggaagggat gattacaact ggtgaagggt tggaatgggg 1260
   tgtgttgttt gggtttggac cgggtttaac tgttgaaacc gttgtgcttc atagtgttcc 1320
   ggttcagggt tgaatttatt atacatagat tggaaaataa aatttgcctg ccgagagatg 1380
   tgaactaact ttgtaggcaa gctcaaatta aagtttgaga taatattgtg ctttagttat 1440
   tatggtatgt aatgtaatgt ttttactttt ttcgaaattc atgtaatttg atatgtaaag 1500
   taatatgttt gggttggaat ataattattt gttaactaaa aaaaaaaaaa aaaaaaaaaa 1560
   aaaaagtact ctgcgttgtt accactgctt aatcgaattc 1600
<210> 324
   <211> 391
   <212> PRT
   <213> Trifolium repens
<400> 324
   Met Val Thr Val Glu Glu Ile Arg Asn Ala Gln Arg Ser Asn Gly Pro
   1               5                   10                  15
   Ala Thr Ile Leu Ala Phe Gly Thr Ala Thr Pro Ser Asn Cys Val Thr
               20                  25                  30
   Gln Ala Asp Tyr Pro Asp Tyr Tyr Phe Arg Ile Thr Asn Ser Glu His
           35                  40                  45
   Met Thr Asp Leu Lys Glu Lys Phe Lys Arg Met Cys Asp Arg Ser Met
   50 55 60
   Ile Lys Lys Arg Tyr Met His Leu Thr Glu Asp Phe Leu Lys Glu Asn
   65 70 75 80
   Pro Asn Met Cys Glu Tyr Met Ala Pro Ser Leu Asp Val Arg Arg Asp
   85 90 95
   Ile Val Val Val Glu Val Pro Lys Leu Gly Lys Glu Ala Ala Lys Lys
   100 105 110
   Ala Ile Cys Glu Trp Gly Gln Pro Lys Ser Lys Ile Thr His Leu Val
   115 120 125
   Phe Cys Thr Thr Ser Gly Val Asp Met Pro Gly Ala Asp Tyr Gln Leu
   130 135 140
   Thr Lys Leu Leu Gly Leu Lys Pro Ser Val Lys Arg Leu Met Met Tyr
   145 150 155 160
   Gln Gln Gly Cys Phe Ala Gly Gly Thr Val Leu Arg Leu Ala Lys Asp
   165 170 175
   Leu Val Glu Asn Asn Lys Asn Ala Arg Val Leu Val Val Cys Ser Glu
   180 185 190
   Ile Thr Ala Val Thr Phe Arg Gly Pro Ser Asp Thr His Leu Asp Ser
   195 200 205
   Leu Val Gly Gln Ala Leu Phe Gly Asp Gly Ala Ala Ala Met Ile Ile
   210 215 220
   Gly Ala Asp Pro Asp Leu Thr Val Glu Arg Pro Ile Phe Glu Ile Val
   225 230 235 240
   Ser Ala Ala Gln Thr Ile Leu Pro Asp Ser Asp Gly Ala Ile Asp Gly
   245 250 255
   His Leu Arg Glu Val Gly Leu Thr Phe His Leu Leu Lys Asp Val Pro
   260 265 270
   Gly Ile Ile Ser Lys Asn Ile Glu Lys Ser Leu Val Glu Ala Phe Ala
   275 280 285
   Pro Ile Gly Ile Asn Asp Trp Asn Ser Ile Phe Trp Val Ala His Pro
   290 295 300
   Gly Gly Pro Ala Ile Leu Asp Gln Val Glu Glu Lys Leu His Leu Lys
   305 310 315 320
   Glu Glu Lys Leu Arg Ser Thr Arg His Val Leu Ser Glu Tyr Gly Asn
   325 330 335
   Met Ser Ser Ala Cys Val Leu Phe Ile Leu Asp Glu Met Arg Lys Arg
   340 345 350
   Ser Lys Glu Glu Gly Met Ile Thr Thr Gly Glu Gly Leu Glu Trp Gly
   355 360 365
   Val Leu Phe Gly Phe Gly Pro Gly Leu Thr Val Glu Thr Val Val Leu
   370 375 380
   His Ser Val Pro Val Gln Gly
   385 390
<210> 325
   <211> 1333
   <212> DNA
   <213> Trifolium repens
<400> 325
   gaattcacta gtgattaagc agtggtaaca acgcagagta cgcgggggtg acttgatcta 60
   gcagttatca aacacaacag tcttccactt gagctctgtt tctccacatg tcgaagctag 120
   tttgcgtcac cggcggcagc ggatgcatcg gttcatggct agtccatctc cttctcctcc 180
   gcggctacac tgttcacgcc accgtccaaa atctcaatga tgagaacgaa acgaagcatc 240
   tagaagctct cgaaggagca caaactaatc tccgtctctt ccagatcgat ctccttaact 300
   acgacacaat cctcgctgct gtccgcggtt gcgtcggaat tttccacctc gcttcacctt 360
   gcactgtaga caaagttcat gatcctcaga aggagctttt ggatcctgca attaaaggga 420
   ctttgaatgt gcttactgca gctaaggaag taggggtgaa gcgtgtggtt gttacctcgt 480
   ctgtctcggc gattactcct agtcctgatt ggccttctga tgttgttaaa agagaggatt 540
   gttggactga tgttgaatat tgcaagaaaa aagagttgtg gtatccgttg tccaaaacat 600
   tggctgagaa agctgcgtgg gatttttcca aagaaaatgg tttggatgtt gttgtggtga 660
   atcccggtac tgtgatgggt cctgttattc caccacggca taatgcaagc atgctcatgc 720
   ttgtgagact tcttgaaggc tgcgctgaaa catttgaaga ctattttatg ggattggtcc 780
   acttcaaaga tgtagcattg gcgcatattt tggtgtatga gaacaaagaa gcatctggta
   840
   gacatgtgtg tgttgaaact atctctcact acggtgattt tgtggcaaaa gttgctgaac
   900
   tttatccaga atatagtgtt cctaggatgc agcgagatac gcaacctgga ttgttgagag
   960
   cgaatgatgg atcaaagaag ctcatagatt tgggtttgga attcattcca atggagcaaa
   1020
   ttatcaagga tgctgtagag agtttgaaga acaaaggatt catttcttga atgatgttac
   1080
   tgttctttgg agaaccctat agttaccaga gtatagacta aataatatat aggtgatggg
   1140
   tcagagaatg agtacttatg tcatgagttg tgtctgtata atatgttttc tcaattctta
   1200
   tatgttaaat tgctaatgtt aacttcaata tttatcagcc agtattgttt ttttaataaa
   1260
   atattgaagc aaaaaaaaaa aaaaaaaaaa aaaaaaaagt actctgcgtt gttaccactg
   1320
   cttaatcgaa ttc
   1333
<210> 326
   <211> 320
   <212> PRT
   <213> Trifolium repens
<400> 326
   Met Ser Lys Leu Val Cys Val Thr Gly Gly Ser Gly Cys Ile Gly Ser
   1               5                   10                  15
   Trp Leu Val His Leu Leu Leu Leu Arg Gly Tyr Thr Val His Ala Thr
               20                  25                  30
   Val Gln Asn Leu Asn Asp Glu Asn Glu Thr Lys His Leu Glu Ala Leu
           35                  40                  45
   Glu Gly Ala Gln Thr Asn Leu Arg Leu Phe Gln Ile Asp Leu Leu Asn
       50                  55                  60
   Tyr Asp Thr Ile Leu Ala Ala Val Arg Gly Cys Val Gly Ile Phe His
   65                  70                  75                  80
   Leu Ala Ser Pro Cys Thr Val Asp Lys Val His Asp Pro Gln Lys Glu
                   85                  90                  95
   Leu Leu Asp Pro Ala Ile Lys Gly Thr Leu Asn Val Leu Thr Ala Ala
               100                 105                 110
   Lys Glu Val Gly Val Lys Arg Val Val Val Thr Ser Ser Val Ser Ala
   115 120 125
   Ile Thr Pro Ser Pro Asp Trp Pro Ser Asp Val Val Lys Arg Glu Asp
   130 135 140
   Cys Trp Thr Asp Val Glu Tyr Cys Lys Lys Lys Glu Leu Trp Tyr Pro
   145 150 155 160
   Leu Ser Lys Thr Leu Ala Glu Lys Ala Ala Trp Asp Phe Ser Lys Glu
   165 170 175
   Asn Gly Leu Asp Val Val Val Val Asn Pro Gly Thr Val Met Gly Pro
   180 185 190
   Val Ile Pro Pro Arg His Asn Ala Ser Met Leu Met Leu Val Arg Leu
   195 200 205
   Leu Glu Gly Cys Ala Glu Thr Phe Glu Asp Tyr Phe Met Gly Leu Val
   210 215 220
   His Phe Lys Asp Val Ala Leu Ala His Ile Leu Val Tyr Glu Asn Lys
   225 230 235 240
   Glu Ala Ser Gly Arg His Val Cys Val Glu Thr Ile Ser His Tyr Gly
   245 250 255
   Asp Phe Val Ala Lys Val Ala Glu Leu Tyr Pro Glu Tyr Ser Val Pro
   260 265 270
   Arg Met Gln Arg Asp Thr Gln Pro Gly Leu Leu Arg Ala Asn Asp Gly
   275 280 285
   Ser Lys Lys Leu Ile Asp Leu Gly Leu Glu Phe Ile Pro Met Glu Gln
   290 295 300
   Ile Ile Lys Asp Ala Val Glu Ser Leu Lys Asn Lys Gly Phe Ile Ser
   305 310 315 320
<210> 327
   <211> 1470
   <212> DNA
   <213> Trifolium repens
<400> 327
   gaattcgatt aagcagtggt aacaacgcag agtacgcggg gaccactcta tttatttcta 60
   cttaaacctc acaaaaaata aaccacacaa cacacaaaca ccaaaaacag agcaccgttt 120
   ccatcatcaa acaatggcac cagccaaaac tctaagttat ctctcacaac aaaacactct 180
   cgagtcaagt ttcgttaggg aagaagatga gcgtccaaaa gttgcctaca ataacttcag
   240
   caacgagatt ccaatcattt ctcttgctgg aattgatgag gttgatggtc gtagaacaga
   300
   gatatgtaac aagattgttg aagcttgtga gaattggggt atttttcagg ttgttgatca
   360
   tggtgttgat acaaaacttg tttctgagat gacccgtttt gctagagagt tttttgcttt
   420
   gccaccggaa gagaagctcc ggtttgacat gtccggtggt aaaaagggtg gtttcattgt
   480
   ctctagtcat cttcaaggag aagcagtgaa ggattggaga gagctagtga catatttttc
   540
   atacccaatt aaacaaagag attattcaag gtggccagac aagccagaag gatggaaaga
   600
   ggtaacagaa aaatacagtg aaaacctaat gaatttagct tgcaagctat tggaagtttt
   660
   atcagaagca atgggtttag aaaaagaagc tctaacaaaa gcatgtgttg atatggatca
   720
   aaaagttgtt ataaattatt acccaaaatg ccctgaacct gacctcacac ttggccttaa
   780
   acgtcacact gaccctggca caattactct tttgcttcaa gatcaagttg gtggtcttca
   840
   agctaccaaa gataatggta agacgtggat tacagttcaa ccagttgaag gtgcttttgt
   900
   tgttaatctt ggagaccatg gtcactatct aagtaatgga cggttcaaaa atgctgacca
   960
   tcaagcagtg gtgaattcga actacagccg tttatcaata gcaacatttc aaaatccagc
   1020
   tccagatgca actgtgtacc ctttgaagat tagagatggt gaaaaatctg tgttggaaga
   1080
   accaatcact tttgctgaaa tgtatagaag gaagatgacc aaagaccttg aaattgctag
   1140
   gatgaagaag ttggctaagg aacaacaact tagggacttg gaggagaaca agactaaata
   1200
   tgaggccaaa cctttgaatg agatctttgc ttaattaatt agtcttaatt taaataataa
   1260
   attttagact taatttacat ataataattt taattttttg ttcaattaat ctatgtttaa
   1320
   tttgtcgtta ttgtccacgt gtattaagct gcttggttgt gtgtgccttg gagaataatc
   1380
   aataatatta catctatgtt taattataaa aaaaaaaaaa aaaaaaaaaa gtatctgcgt
   1440
   tgttaccact gcttaatcac tagtgaattc
   1470

<210> 328
   <211> 366
   <212> PRT
   <213> Trifolium repens
<400> 328
   Met Ala Pro Ala Lys Thr Leu Ser Tyr Leu Ser Gln Gln Asn Thr Leu
   1               5                   10                  15
   Glu Ser Ser Phe Val Arg Glu Glu Asp Glu Arg Pro Lys Val Ala Tyr
               20                  25                  30
   Asn Asn Phe Ser Asn Glu Ile Pro Ile Ile Ser Leu Ala Gly Ile Asp
           35                  40                  45
   Glu Val Asp Gly Arg Arg Thr Glu Ile Cys Asn Lys Ile Val Glu Ala
       50                  55                  60
   Cys Glu Asn Trp Gly Ile Phe Gln Val Val Asp His Gly Val Asp Thr
   65                  70                  75                  80
   Lys Leu Val Ser Glu Met Thr Arg Phe Ala Arg Glu Phe Phe Ala Leu
                   85                  90                  95
   Pro Pro Glu Glu Lys Leu Arg Phe Asp Met Ser Gly Gly Lys Lys Gly
               100                 105                 110
   Gly Phe Ile Val Ser Ser His Leu Gln Gly Glu Ala Val Lys Asp Trp
           115                 120                 125
   Arg Glu Leu Val Thr Tyr Phe Ser Tyr Pro Ile Lys Gln Arg Asp Tyr
       130                 135                 140
   Ser Arg Trp Pro Asp Lys Pro Glu Gly Trp Lys Glu Val Thr Glu Lys
   145                 150                 155                 160
   Tyr Ser Glu Asn Leu Met Asn Leu Ala Cys Lys Leu Leu Glu Val Leu
                   165                 170                 175
   Ser Glu Ala Met Gly Leu Glu Lys Glu Ala Leu Thr Lys Ala Cys Val
               180                 185                 190
   Asp Met Asp Gln Lys Val Val Ile Asn Tyr Tyr Pro Lys Cys Pro Glu
           195                 200                 205
   Pro Asp Leu Thr Leu Gly Leu Lys Arg His Thr Asp Pro Gly Thr Ile
       210                 215                 220
   Thr Leu Leu Leu Gln Asp Gln Val Gly Gly Leu Gln Ala Thr Lys Asp
   225                 230                 235                 240
   Asn Gly Lys Thr Trp Ile Thr Val Gln Pro Val Glu Gly Ala Phe Val
                   245                 250                 255
   Val Asn Leu Gly Asp His Gly His Tyr Leu Ser Asn Gly Arg Phe Lys
               260                 265                 270
   Asn Ala Asp His Gln Ala Val Val Asn Ser Asn Tyr Ser Arg Leu Ser
           275                 280                 285
   Ile Ala Thr Phe Gln Asn Pro Ala Pro Asp Ala Thr Val Tyr Pro Leu
       290                 295                 300
   Lys Ile Arg Asp Gly Glu Lys Ser Val Leu Glu Glu Pro Ile Thr Phe
   305                 310                 315                 320
   Ala Glu Met Tyr Arg Arg Lys Met Thr Lys Asp Leu Glu Ile Ala Arg
                   325                 330                 335
   Met Lys Lys Leu Ala Lys Glu Gln Gln Leu Arg Asp Leu Glu Glu Asn
               340                 345                 350
   Lys Thr Lys Tyr Glu Ala Lys Pro Leu Asn Glu Ile Phe Ala
           355                 360                 365
<210> 329
   <211> 2515
   <212> DNA
   <213> Trifolium repens
<400> 329
   gaattcgatt aagcagtggt aacaacgcag agtacgcggg gaggaaattc acaactcaaa
   60
   tattccttta attctttcat ataatcattt gaatttccat tctccctaaa aattctatag
   120
   ctaccacatc acacaacata acaaattaag aaatatttat tactatatta agatatggaa
   180
   gtagtagcag cagcaatcac aaaaaacaat ggcaagattg attcattttg cttgaatcat
   240
   gctaatgcta ataacatgaa agtgaatggt gctgatcctt tgaattgggg tgtggctgct
   300
   gaggcaatga agggaagtca cttggatgag gtgaagcgta tggtggagga ataccggaaa
   360
   ccggttgtcc gtcttggtgg cgagacacta accatttctc aggtggctgc cattgctgca
   420
   cacgatggtg caacggtgga gctatcggaa tctgctagag ccggcgttaa ggcaagcagt
   480
   gactgggtta tggagagtat gaacaaaggt accgacagct acggtgtccc aacagggttc
   540
   ggcgctacct cgcaccgccg aaccaaacaa ggtggtgctt tgcagaaaga gctcataagg
   600
   tttttgaatg ctggaatatt tggaaatgga actgagtcaa gccacacact accacacaca
   660
   gccacaagag ctgccatgct agtgagaatc aacacacttc tccaaggcta ttcaggaatt
   720
   agatttgaaa tcttagaagc tatcaccaag cttcttaaca acaatgtcac cccatgttta
   780
   ccgcttcgcg gtacaatcac agcttcagga gatttagtcc ctctttctta cattgctggt
   840
   ttactaaccg gacgaccaaa ttccaaggct catggacctt ctggagaagt acttaatgca
   900
   aaacaagctt ttcaatcagc tggaatcgat gccgagttct ttgaattaca accaaaagaa
   960
   ggccttgccc ttgttaacgg aaccgctgtt ggttctggtt tagcttctat tgttcttttt
   1020
   gaggctaata tattggcggt gttgtctgaa gttctatctg caattttcgc tgaagttatg
   1080
   caagggaagc ccgaatttac cgatcatttg acacataagt tgaaacatca ccctggtcaa
   1140
   attgaggctg ctgctattat ggaacacatt ttggatggga gtgcttatgt taaagacgct
   1200
   aagaagttgc atgagatgga tcctttacag aagccaaaac aagatagata tgcacttagg
   1260
   acttcgccac aatggcttgg tcctttgatt gaagtgatta gattctctac caagtcaatt
   1320
   gagagagaga tcaactctgt caatgacaat cctttgattg atgtttcaag gaacaaggct
   1380
   ttgcatggtg gaaattttca aggaacacct atcggagtat ccatggataa tacacgtttg
   1440
   gctcttgcat caattggcaa acttatgttt gctcaattct ctgagcttgt caatgatttt
   1500
   tacaacaatg gattgccatc aaatctctct gctagtagaa atccgagctt ggattatggg
   1560
   ttcaagggat ccgaaattgc catggcttct tattgttccg agttgcaata tcttgcaaat
   1620
   ccggttacaa ctcatgtcca aagtgcggaa caacacaacc aagatgtcaa ctctttgggt
   1680
   ttgatttctt ctagaaaaac ttatgaagca attgagatcc ttcaattgat gtcttccaca
   1740
   ttcttgattg cactttgtca agcaattgat ttaagacatt tggaggagaa tttgaaaaac
   1800
   tcggtcaaaa ataccgtaag ccaagtggcc aaaaagacac taaccatagg tgtcaatgga
   1860
   gaacttcatc cttcaagatt ttgtgaaaaa gacttattga aagtggttga tagggaacat
   1920
   gtctttgcct acattgatga tccttgtagt gctacatacc cattgatgca aaaactcagg
   1980
   caagtactag tggatcatgc attagttaat ggagaaagtg agaagaattt gaacacatca
   2040
   atcttccaaa agattgcaac ttttgaggaa gagttgaaaa acctttgcca aaagaggttg
   2100
   aaagtgcaag gattgcatat gaaagtggaa attcaacaat tccaaacaag atcaatggat
   2160
   gcagatctta tccactctac aattttgtga gaaaggagtt gggaactggt ttgctaactg
   2220
   gagaaaatgt catttcaccg ggtgaagagt gtgacaaact attcacagct atgtgtcaag
   2280
   gaaaaatcat tgatcctctt cttgaatgct tgggagagtg gaacggtgct cctcttccaa
   2340
   tttgttaact ttgattgtta gttcataaaa tgttttattt gtatttatca tttgtattta
   2400
   tgcgagtgta gtaataatga ttaggtgttt tgtgccttta atgaaaaaaa aaaaaaaaaa
   2460
   aaaaaaaaaa aaaagtactc tgcgttgtta ccactgctta atcactagtg aattc
   2515
<210> 330
   <211> 671
   <212> PRT
   <213> Trifolium repens
<400> 330
   Met Glu Val Val Ala Ala Ala Ile Thr Lys Asn Asn Gly Lys Ile Asp
   1 5 10 15
   Ser Phe Cys Leu Asn His Ala Asn Ala Asn Asn Met Lys Val Asn Gly
   20 25 30
   Ala Asp Pro Leu Asn Trp Gly Val Ala Ala Glu Ala Met Lys Gly Ser
   35 40 45
   His Leu Asp Glu Val Lys Arg Met Val Glu Glu Tyr Arg Lys Pro Val
   50 55 60
   Val Arg Leu Gly Gly Glu Thr Leu Thr Ile Ser Gln Val Ala Ala Ile
   65 70 75 80
   Ala Ala His Asp Gly Ala Thr Val Glu Leu Ser Glu Ser Ala Arg Ala
   85 90 95
   Gly Val Lys Ala Ser Ser Asp Trp Val Met Glu Ser Met Asn Lys Gly
   100 105 110
   Thr Asp Ser Tyr Gly Val Pro Thr Gly Phe Gly Ala Thr Ser His Arg
   115 120 125
   Arg Thr Lys Gln Gly Gly Ala Leu Gln Lys Glu Leu Ile Arg Phe Leu
   130 135 140
   Asn Ala Gly Ile Phe Gly Asn Gly Thr Glu Ser Ser His Thr Leu Pro
   145 150 155 160
   His Thr Ala Thr Arg Ala Ala Met Leu Val Arg Ile Asn Thr Leu Leu
   165 170 175
   Gln Gly Tyr Ser Gly Ile Arg Phe Glu Ile Leu Glu Ala Ile Thr Lys
   180 185 190
   Leu Leu Asn Asn Asn Val Thr Pro Cys Leu Pro Leu Arg Gly Thr Ile
   195 200 205
   Thr Ala Ser Gly Asp Leu Val Pro Leu Ser Tyr Ile Ala Gly Leu Leu
   210 215 220
   Thr Gly Arg Pro Asn Ser Lys Ala His Gly Pro Ser Gly Glu Val Leu
   225 230 235 240
   Asn Ala Lys Gln Ala Phe Gln Ser Ala Gly Ile Asp Ala Glu Phe Phe
   245 250 255
   Glu Leu Gln Pro Lys Glu Gly Leu Ala Leu Val Asn Gly Thr Ala Val
   260 265 270
   Gly Ser Gly Leu Ala Ser Ile Val Leu Phe Glu Ala Asn Ile Leu Ala
   275 280 285
   Val Leu Ser Glu Val Leu Ser Ala Ile Phe Ala Glu Val Met Gln Gly
   290 295 300
   Lys Pro Glu Phe Thr Asp His Leu Thr His Lys Leu Lys His His Pro
   305 310 315 320
   Gly Gln Ile Glu Ala Ala Ala Ile Met Glu His Ile Leu Asp Gly Ser
   325 330 335
   Ala Tyr Val Lys Asp Ala Lys Lys Leu His Glu Met Asp Pro Leu Gln
   340 345 350
   Lys Pro Lys Gln Asp Arg Tyr Ala Leu Arg Thr Ser Pro Gln Trp Leu
   355 360 365
   Gly Pro Leu Ile Glu Val Ile Arg Phe Ser Thr Lys Ser Ile Glu Arg
   370 375 380
   Glu Ile Asn Ser Val Asn Asp Asn Pro Leu Ile Asp Val Ser Arg Asn
   385 390 395 400
   Lys Ala Leu His Gly Gly Asn Phe Gln Gly Thr Pro Ile Gly Val Ser
   405 410 415
   Met Asp Asn Thr Arg Leu Ala Leu Ala Ser Ile Gly Lys Leu Met Phe
   420 425 430
   Ala Gln Phe Ser Glu Leu Val Asn Asp Phe Tyr Asn Asn Gly Leu Pro
   435 440 445
   Ser Asn Leu Ser Ala Ser Arg Asn Pro Ser Leu Asp Tyr Gly Phe Lys
   450 455 460
   Gly Ser Glu Ile Ala Met Ala Ser Tyr Cys Ser Glu Leu Gln Tyr Leu
   465 470 475 480
   Ala Asn Pro Val Thr Thr His Val Gln Ser Ala Glu Gln His Asn Gln
   485 490 495
   Asp Val Asn Ser Leu Gly Leu Ile Ser Ser Arg Lys Thr Tyr Glu Ala
   500 505 510
   Ile Glu Ile Leu Gln Leu Met Ser Ser Thr Phe Leu Ile Ala Leu Cys
   515 520 525
   Gln Ala Ile Asp Leu Arg His Leu Glu Glu Asn Leu Lys Asn Ser Val
   530 535 540
   Lys Asn Thr Val Ser Gln Val Ala Lys Lys Thr Leu Thr Ile Gly Val
   545 550 555 560
   Asn Gly Glu Leu His Pro Ser Arg Phe Cys Glu Lys Asp Leu Leu Lys
   565 570 575
   Val Val Asp Arg Glu His Val Phe Ala Tyr Ile Asp Asp Pro Cys Ser
   580 585 590
   Ala Thr Tyr Pro Leu Met Gln Lys Leu Arg Gln Val Leu Val Asp His
   595 600 605
   Ala Leu Val Asn Gly Glu Ser Glu Lys Asn Leu Asn Thr Ser Ile Phe
   610 615 620
   Gln Lys Ile Ala Thr Phe Glu Glu Glu Leu Lys Asn Leu Cys Gln Lys
   625 630 635 640
   Arg Leu Lys Val Gln Gly Leu His Met Lys Val Glu Ile Gln Gln Phe
   645 650 655
   Gln Thr Arg Ser Met Asp Ala Asp Leu Ile His Ser Thr Ile Leu
   660 665 670
<210> 331
   <211> 2667
   <212> DNA
   <213> Trifolium repens
<400> 331
   gaattcgatt aagcagtggt aacaacgcag agtacgcggg aggaaataaa ttcatcattg 60
   ttccttattt cccacccaac acaacataac aaatacattt cctctcctct catcacaatt 120
   attactttct acaccccccc ctctcaacta ttattaacta acataatgga gggaattacc 180
   aatggccatg ctgaagcaac tttttgcgtg accaaaagtg ttggtgatcc actcaactgg 240
   ggtgcagccg cggagtcgtt gatggggagt catttggatg aggtgaagcg tatggtggag 300
   gaataccgta atccattggt taaaattggc ggcgagacgc ttaccattgc tcaggtggct 360
   ggaattgctt ctcatgatag tggtgtgagg gtggagctgt ctgagtccgc cagggccggc 420
   gttaaggcga gtagtggttg ggtgatggac agcatgaaca atgggactga tagttatggt 480
   gttaccactg gtttcggcgc cacctctcac cggagaacca agcagggtgg tgccttgcag 540
   aaggagctaa ttaggttttt gaatgccgga atatttggca atggtacaga atctaactgt 600
   acactaccac acacagcaac cagagctgca atgcttgtga gaatcaacac tcttcttcaa 660
   ggatattctg gaattagatt tgaaattttg gaagctatca caaagcttct aaacaacaac 720
   attaccccat gtttaccact tcgtggtaca atcacggctt ccggtgatct cgttccgctt 780
   tcctacattg ccggtttgtt aaccggtaga ccgaactcca aagccgttgg accctccgga 840
   gaaattctca atgcaaaaga agcttttcaa cttgccggca ttggttctga gttttttgaa 900
   ttgcagccaa aagaaggtct tgctcttgtt aatggtactg ctgttggttc tggtttagct 960
   tctattgttc tgtttgaagc aaatgtacta gctgttttgt ctgaagttat gtcggcgatt 1020
   ttcgctgaag ttatgcaagg gaaaccagaa ttcactgatc atttgactca taagttgaaa 1080
   catcaccctg gtcaaattga agctgctgca attatggaac atattttgga tggaagtgct 1140
   tatgttaaag cagctaagaa attacacgaa accgatcctt tacaaaagcc gaaacaagat 1200
   cgttatgcac ttagaacttc acctcaatgg cttggtcctt tgattgaagt gataagattt 1260
   tcaactaagt caattgagag agaaattaac tctgtcaatg ataacccttt gattgatgtt 1320
   tcaaggaaca aggccattca cggtggtaat tttcaaggaa cacctattgg agtttcaatg 1380
   gataacacac gtttagctct tgcttcaatt ggtaaactca tgtttgctca attctctgaa 1440
   cttgttaatg atttttacaa caacgggtta ccttcgaatc ttactgctag taggaaccca 1500
   agcttggatt acggtttcaa gggatcggaa attgccatgg cttcttattg ttctgagtta 1560
   caatatcttg ctaatcctgt caccacccat gtccaaagtg cggagcaaca caatcaagat 1620
   gttaactctt tgggtttgat ttcttcaaga aaaacaaatg aagctattga gatcctaaag 1680
   ctcatgtctt cgacatttct gattgcactt tgtcaagcaa ttgatttaag gcatttggag 1740
   gaaaatctga ggaacactgt caagaacacg gtaagccaag tagcgaagag aacactcacc 1800
   accggtgtta atggagaact tcatccttct agattttgtg agaaagattt gctcaaagtt 1860
   gttgataggg agtatgtatt tgcctatgtc gacgatcctt gtctagctac ataccctttg 1920
   atgcaaaagt tgagacaagt gcttgtggat catgcattgg taaatgctga tggagagaag 1980
   aatttgaaca catcaatctt tcaaaagatt gcaacttttg aggatgaatt gaaagctatc 2040
   ttgccaaagg aagttgaaag tacaagaact gcatatgaaa atggacaatg tggaatttca 2100
   aacaagatta aggaatgcag gtcttatcca ttgtacaagt ttgttagaga ggagttagga 2160
   accgcgttgc taaccggaga aaaaacgata tcgctgggcg aagagtgtga caaattgttc 2220
   acagctatgt gccaaggtaa aattgttgat cctcttttgg aatgccttgg agagtggaat 2280
   ggtgctcctc taccaatatg ttaattagca gaattaatat gtttctttga gaagtgattt 2340
   ctttatatat ttgtagtata ctatagtagt tgcattgaga agcaattggt ttgtctataa 2400
   gcctatggaa aatggcaaaa caattttctg ctcaaagcat cgtttattaa gttttcctta 2460
   aagtgttaag gaacttttaa ttgtttttgt aatagaattt catttgtttg ccacaacttt 2520
   gggtgcaaat atcacgtgat acatgtggtg tttgatgtaa atggtgtttt ctcaattaat 2580
   aaatagtgtt tcagccatga aaaaaaaaaa aaaaaaaaaa aaaaaagtac tctgcgttgt 2640
   taccactgct taatcactag tgaattc 2667
<210> 332
   <211> 712
   <212> PRT
   <213> Trifolium repens
<400> 332
   Met Glu Gly Ile Thr Asn Gly His Ala Glu Ala Thr Phe Cys Val Thr
   1 5 10 15
   Lys Ser Val Gly Asp Pro Leu Asn Trp Gly Ala Ala Ala Glu Ser Leu
   20 25 30
   Met Gly Ser His Leu Asp Glu Val Lys Arg Met Val Glu Glu Tyr Arg
   35 40 45
   Asn Pro Leu Val Lys Ile Gly Gly Glu Thr Leu Thr Ile Ala Gln Val
   50 55 60
   Ala Gly Ile Ala Ser His Asp Ser Gly Val Arg Val Glu Leu Ser Glu
   65 70 75 80
   Ser Ala Arg Ala Gly Val Lys Ala Ser Ser Gly Trp Val Met Asp Ser
   85 90 95
   Met Asn Asn Gly Thr Asp Ser Tyr Gly Val Thr Thr Gly Phe Gly Ala
   100 105 110
   Thr Ser His Arg Arg Thr Lys Gln Gly Gly Ala Leu Gln Lys Glu Leu
   115 120 125
   Ile Arg Phe Leu Asn Ala Gly Ile Phe Gly Asn Gly Thr Glu Ser Asn
   130 135 140
   Cys Thr Leu Pro His Thr Ala Thr Arg Ala Ala Met Leu Val Arg Ile
   145 150 155 160
   Asn Thr Leu Leu Gln Gly Tyr Ser Gly Ile Arg Phe Glu Ile Leu Glu
   165 170 175
   Ala Ile Thr Lys Leu Leu Asn Asn Asn Ile Thr Pro Cys Leu Pro Leu
   180 185 190
   Arg Gly Thr Ile Thr Ala Ser Gly Asp Leu Val Pro Leu Ser Tyr Ile
   195 200 205
   Ala Gly Leu Leu Thr Gly Arg Pro Asn Ser Lys Ala Val Gly Pro Ser
   210 215 220
   Gly Glu Ile Leu Asn Ala Lys Glu Ala Phe Gln Leu Ala Gly Ile Gly
   225 230 235 240
   Ser Glu Phe Phe Glu Leu Gln Pro Lys Glu Gly Leu Ala Leu Val Asn
   245 250 255
   Gly Thr Ala Val Gly Ser Gly Leu Ala Ser Ile Val Leu Phe Glu Ala
   260 265 270
   Asn Val Leu Ala Val Leu Ser Glu Val Met Ser Ala Ile Phe Ala Glu
   275 280 285
   Val Met Gln Gly Lys Pro Glu Phe Thr Asp His Leu Thr His Lys Leu
   290 295 300
   Lys His His Pro Gly Gln Ile Glu Ala Ala Ala Ile Met Glu His Ile
   305 310 315 320
   Leu Asp Gly Ser Ala Tyr Val Lys Ala Ala Lys Lys Leu His Glu Thr
   325 330 335
   Asp Pro Leu Gln Lys Pro Lys Gln Asp Arg Tyr Ala Leu Arg Thr Ser
   340 345 350
   Pro Gln Trp Leu Gly Pro Leu Ile Glu Val Ile Arg Phe Ser Thr Lys
   355 360 365
   Ser Ile Glu Arg Glu Ile Asn Ser Val Asn Asp Asn Pro Leu Ile Asp
   370 375 380
   Val Ser Arg Asn Lys Ala Ile His Gly Gly Asn Phe Gln Gly Thr Pro
   385 390 395 400
   Ile Gly Val Ser Met Asp Asn Thr Arg Leu Ala Leu Ala Ser Ile Gly
   405 410 415
   Lys Leu Met Phe Ala Gln Phe Ser Glu Leu Val Asn Asp Phe Tyr Asn
   420 425 430
   Asn Gly Leu Pro Ser Asn Leu Thr Ala Ser Arg Asn Pro Ser Leu Asp
   435 440 445
   Tyr Gly Phe Lys Gly Ser Glu Ile Ala Met Ala Ser Tyr Cys Ser Glu
   450 455 460
   Leu Gln Tyr Leu Ala Asn Pro Val Thr Thr His Val Gln Ser Ala Glu
   465 470 475 480
   Gln His Asn Gln Asp Val Asn Ser Leu Gly Leu Ile Ser Ser Arg Lys
   485 490 495
   Thr Asn Glu Ala Ile Glu Ile Leu Lys Leu Met Ser Ser Thr Phe Leu
   500 505 510
   Ile Ala Leu Cys Gln Ala Ile Asp Leu Arg His Leu Glu Glu Asn Leu
   515 520 525
   Arg Asn Thr Val Lys Asn Thr Val Ser Gln Val Ala Lys Arg Thr Leu
   530 535 540
   Thr Thr Gly Val Asn Gly Glu Leu His Pro Ser Arg Phe Cys Glu Lys
   545 550 555 560
   Asp Leu Leu Lys Val Val Asp Arg Glu Tyr Val Phe Ala Tyr Val Asp
   565 570 575
   Asp Pro Cys Leu Ala Thr Tyr Pro Leu Met Gln Lys Leu Arg Gln Val
   580 585 590
   Leu Val Asp His Ala Leu Val Asn Ala Asp Gly Glu Lys Asn Leu Asn
   595 600 605
   Thr Ser Ile Phe Gln Lys Ile Ala Thr Phe Glu Asp Glu Leu Lys Ala
   610 615 620
   Ile Leu Pro Lys Glu Val Glu Ser Thr Arg Thr Ala Tyr Glu Asn Gly
   625 630 635 640
   Gln Cys Gly Ile Ser Asn Lys Ile Lys Glu Cys Arg Ser Tyr Pro Leu
   645 650 655
   Tyr Lys Phe Val Arg Glu Glu Leu Gly Thr Ala Leu Leu Thr Gly Glu
   660 665 670
   Lys Thr Ile Ser Leu Gly Glu Glu Cys Asp Lys Leu Phe Thr Ala Met
           675                     680                     685
   Cys Gln Gly Lys Ile Val Asp Pro Leu Leu Glu Cys Leu Gly Glu Trp
       690                     695                     700
   Asn Gly Ala Pro Leu Pro Ile Cys
   705                     710

<210> 333
   <211> 2664
   <212> DNA
   <213> Trifolium repens
<400> 333
   gaattcacta gtgattaagc agtggtaaca acgcagagta cgcggggagg aaataaattc 60
   atcattgttc attatttccc acccaacaca acataacaaa tacattattc tctcctctga 120
   tcacaattat tactttctac accctcctct caactattat taactagcat aatggaggga 180
   attaccaatg gccatgctga aacaactttt tgcgtgacca aaagtgttgg tgatccactc 240
   aactggggtg cagccgcgga gtcgttgacg gggagtcatt tggatgaggt gaagcgtatg 300
   gtggaggagt accgtaatcc gttggctaaa attggcggcg agacgcttac cattgctcag 360
   gtggctggaa ttgcttctca tgatagtggt gtgagggtgg agctgtccga gtccgcaagg 420
   gccggcgtta aggcgagtag tgattgggtg atggatagca tgaacaatgg gactgatagt 480
   tacggtgtta ccaccggttt tggtgccacc tctcaccgga gaaccaagca gggtggtgct 540
   ttgcagaagg agctaattag gtttttgaat gctggaatat ttggcaatgg tacagaatct 600
   aactgtacac taccacacac agcaactaga gctgcaatgc ttgtgagaat caacactctt 660
   cttcaagggt actctggtat tagatttgaa attttggaag ctatcacaaa gcttccaaac 720
   aacaacatta ccccatgttt accacttcgt ggtacaatca cggcttccgg tgatcttgtt 780
   ccgctttcct acattgccgg tttgttaacc ggaagaccca actccaaagc agttggacct 840
   tccggagaaa ttttgagtgc taaagaagct tttcaactcg ccggcattgg ttctgagttt 900
   tttgaattgc aaccaaaaga aggtcttgct cttgttaatg gtactgctgt tggctctggt 960
   ttagcttcta ttgttctgtt tgaagcaaat gtactagctg ttttatccga agttatgtcg 1020
   gcgattttcg ctgaagttat gcaagggaaa ccggaattta ccgatcattt gactcataag 1080
   ttgaaacatc accctggtca aattgaagct gctgcaatta tggaacatat tttggatgga 1140
   agtgcttatg ttaaagcagc taagaagtta cacgaaaccg atcctttaca aaaaccgaaa 1200
   caagatcgtt atgcacttag aacttcacct caatggcttg gtcctttgat tgaagtgata 1260
   agattttcaa ccaaatcgat tgaaagagaa attaactcgg tcaacgacaa ccctttgatc 1320
   gatgtttcaa ggaacaaggc cattcatggt ggtaactttc aaggaacacc tattggagtt 1380
   tcaatggata acacacgttt agctcttgct tcaattggta aactcatgtt tgctcaattc 1440
   tctgaacttg ttaatgattt ttacaacaac gggttgcctt cgtatcttac tgctagtagg 1500
   aacccgagct tggactatgg tttcaaggga tcggaaattg ccatggcttc gtattgttcc 1560
   gagttacaat atcttgctaa tcctgtcacc acccatgtcc aaagtgccga gcaacacaac 1620
   caagatgtta actctttggg tttgatttct tctagaaaaa caaatgaagc tattgagatt 1680
   ctcaagctca tgtcttccac tttcttgatt gcattatgtc aagcaatcga cttaaggcac 1740
   ttggaggaaa atctcaggaa caccgtcaag aacacggtaa gccaagtagc gaagagaaca 1800
   ctcaccaccg gcgtcaacgg agaacttcat tcttctagat tttgtgagaa agatttgctt 1860
   aaagttgttg atagggagta tgtatttgcc tatgccgacg atccttgtct agctacatac 1920
   cctttgatgc aaaagttgag acaagtgctt gtggatcatg cattggtaaa tgttgatgga 1980
   gagaagaatt tgaacacatc aatctttcaa aagattgcaa cttttgagga tgagttgaaa 2040
   gctattttgc caaaggaggt tgaaagtaca agaactgcat atgaaaatgg acaatgtgga 2100
   atttcaaaca agattaagga atgcaggtct tatccattgt acaagtttgt tagagaggag 2160
   ttaggaaccg cgttgctaac cggagaaaaa actatatcgc cgggcgaaga gtgcgataaa 2220
   ttgttcacag ctatgtgcca aggtaaaatt gttgatcctc ttatggaatg cctcggagag 2280
   tggaatggtg ctcctctacc aatatgttaa ttagcataat atgttttctt tgagaagtga 2340
   ttactttata tatttgtagt atactatagt agttgcattg agaagaaatt ggtttgttta 2400
   taagcctatg gaaaatggca aatcaatttt ctgctcaaag catcgtttat taagttttcc 2460
   ttaaagtgtt aaggaacttt taattgtttt tgtaatagaa tttcatttgt ttgccacaac 2520
   tttgggtgca aatatcacat gatacatgtg gtgtttgatg taaatggtgt tttttcaata 2580
   aataaatagt gtttcaacta aaaaaaaaaa aaaaaaaaaa aaaaaaaaag tactctgcgt 2640
   tgttaccact gcttaatcga attc 2664
<210> 334
   <211> 712
   <212> PRT
   <213> Trifolium repens
<400> 334
   Met Glu Gly Ile Thr Asn Gly His Ala Glu Thr Thr Phe Cys Val Thr
   1               5                   10                  15
   Lys Ser Val Gly Asp Pro Leu Asn Trp Gly Ala Ala Ala Glu Ser Leu
               20                  25                  30
   Thr Gly Ser His Leu Asp Glu Val Lys Arg Met Val Glu Glu Tyr Arg
           35                  40                  45
   Asn Pro Leu Ala Lys Ile Gly Gly Glu Thr Leu Thr Ile Ala Gln Val
       50                  55                  60
   Ala Gly Ile Ala Ser His Asp Ser Gly Val Arg Val Glu Leu Ser Glu
   65                  70                  75                  80
   Ser Ala Arg Ala Gly Val Lys Ala Ser Ser Asp Trp Val Met Asp Ser
                   85                  90                  95
   Met Asn Asn Gly Thr Asp Ser Tyr Gly Val Thr Thr Gly Phe Gly Ala
               100                 105                 110
   Thr Ser His Arg Arg Thr Lys Gln Gly Gly Ala Leu Gln Lys Glu Leu
           115                 120                 125
   Ile Arg Phe Leu Asn Ala Gly Ile Phe Gly Asn Gly Thr Glu Ser Asn
       130                 135                 140
   Cys Thr Leu Pro His Thr Ala Thr Arg Ala Ala Met Leu Val Arg Ile
   145 150 155 160
   Asn Thr Leu Leu Gln Gly Tyr Ser Gly Ile Arg Phe Glu Ile Leu Glu
   165 170 175
   Ala Ile Thr Lys Leu Pro Asn Asn Asn Ile Thr Pro Cys Leu Pro Leu
   180 185 190
   Arg Gly Thr Ile Thr Ala Ser Gly Asp Leu Val Pro Leu Ser Tyr Ile
   195 200 205
   Ala Gly Leu Leu Thr Gly Arg Pro Asn Ser Lys Ala Val Gly Pro Ser
   210 215 220
   Gly Glu Ile Leu Ser Ala Lys Glu Ala Phe Gln Leu Ala Gly Ile Gly
   225 230 235 240
   Ser Glu Phe Phe Glu Leu Gln Pro Lys Glu Gly Leu Ala Leu Val Asn
   245 250 255
   Gly Thr Ala Val Gly Ser Gly Leu Ala Ser Ile Val Leu Phe Glu Ala
   260 265 270
   Asn Val Leu Ala Val Leu Ser Glu Val Met Ser Ala Ile Phe Ala Glu
   275 280 285
   Val Met Gln Gly Lys Pro Glu Phe Thr Asp His Leu Thr His Lys Leu
   290 295 300
   Lys His His Pro Gly Gln Ile Glu Ala Ala Ala Ile Met Glu His Ile
   305 310 315 320
   Leu Asp Gly Ser Ala Tyr Val Lys Ala Ala Lys Lys Leu His Glu Thr
   325 330 335
   Asp Pro Leu Gln Lys Pro Lys Gln Asp Arg Tyr Ala Leu Arg Thr Ser
   340 345 350
   Pro Gln Trp Leu Gly Pro Leu Ile Glu Val Ile Arg Phe Ser Thr Lys
   355 360 365
   Ser Ile Glu Arg Glu Ile Asn Ser Val Asn Asp Asn Pro Leu Ile Asp
   370 375 380
   Val Ser Arg Asn Lys Ala Ile His Gly Gly Asn Phe Gln Gly Thr Pro
   385 390 395 400
   Ile Gly Val Ser Met Asp Asn Thr Arg Leu Ala Leu Ala Ser Ile Gly
   405 410 415
   Lys Leu Met Phe Ala Gln Phe Ser Glu Leu Val Asn Asp Phe Tyr Asn
   420 425 430
   Asn Gly Leu Pro Ser Tyr Leu Thr Ala Ser Arg Asn Pro Ser Leu Asp
   435 440 445
   Tyr Gly Phe Lys Gly Ser Glu Ile Ala Met Ala Ser Tyr Cys Ser Glu
   450 455 460
   Leu Gln Tyr Leu Ala Asn Pro Val Thr Thr His Val Gln Ser Ala Glu
   465 470 475 480
   Gln His Asn Gln Asp Val Asn Ser Leu Gly Leu Ile Ser Ser Arg Lys
   485 490 495
   Thr Asn Glu Ala Ile Glu Ile Leu Lys Leu Met Ser Ser Thr Phe Leu
   500 505 510
   Ile Ala Leu Cys Gln Ala Ile Asp Leu Arg His Leu Glu Glu Asn Leu
   515 520 525
   Arg Asn Thr Val Lys Asn Thr Val Ser Gln Val Ala Lys Arg Thr Leu
   530 535 540
   Thr Thr Gly Val Asn Gly Glu Leu His Ser Ser Arg Phe Cys Glu Lys
   545 550 555 560
   Asp Leu Leu Lys Val Val Asp Arg Glu Tyr Val Phe Ala Tyr Ala Asp
   565 570 575
   Asp Pro Cys Leu Ala Thr Tyr Pro Leu Met Gln Lys Leu Arg Gln Val
   580 585 590
   Leu Val Asp His Ala Leu Val Asn Val Asp Gly Glu Lys Asn Leu Asn
   595 600 605
   Thr Ser Ile Phe Gln Lys Ile Ala Thr Phe Glu Asp Glu Leu Lys Ala
   610 615 620
   Ile Leu Pro Lys Glu Val Glu Ser Thr Arg Thr Ala Tyr Glu Asn Gly
   625 630 635 640
   Gln Cys Gly Ile Ser Asn Lys Ile Lys Glu Cys Arg Ser Tyr Pro Leu
   645 650 655
   Tyr Lys Phe Val Arg Glu Glu Leu Gly Thr Ala Leu Leu Thr Gly Glu
   660 665 670
   Lys Thr Ile Ser Pro Gly Glu Glu Cys Asp Lys Leu Phe Thr Ala Met
   675 680 685
   Cys Gln Gly Lys Ile Val Asp Pro Leu Met Glu Cys Leu Gly Glu Trp
   690 695 700
   Asn Gly Ala Pro Leu Pro Ile Cys
   705 710
<210> 335
   <211> 1296
   <212> DNA
   <213> Trifolium repens
<400> 335
   gaattcgatt aagcagtggt aacaacgcag agtacgcggg atagtagtag ttgagaaaaa
   60
   atacacaaat aaagtaaaca ctatcataga aagagagtca aaaatggctg aaggaaaagg
   120
   aagggtttgt gttactggag gaacaggttt tcttggttca tggatcatca agagtcttct
   180
   tgaaaatgga tactctgtta ataccactat tagagctgat ccagaacgta agagggatgt
   240
   aagcttccta acaaatctac ccggcgcatc cgaaaggcta catttcttca acgccgatct
   300
   agacgaccca gagagtttca acgaagcaat tgaaggttgt gtcgggatat tccacaccgc
   360
   ttcaccaatc gatttcgccg tgagtgagcc agaagaaata gtgacaaaaa gaacagtgga
   420
   tggagcatta ggaattttaa aagcatgtgt gaattcaaag acagtgaaga gatttattta
   480
   cacttcaagt ggttctgctg tttcattcaa tggaaaaaac aaagatgttt tggatgagag
   540
   tgattggagt gatgttgatt tgcttagaag tgttaaacca tttggttgga gttatggtgt
   600
   ttcaaagact ttggctgaga aagcagtgct tgaatttggt caacaaaatg ggattgatgt
   660
   tgttactttg attcttcctt ttattgttgg aagttttgtt tgtcctaagc ttcctgattc
   720
   tgttgagaaa gctcttgttt tggtactagg caaaaaggaa caaattggta ttataagttt
   780
   ccacatggta catgtggatg atgtggctag agcacatatt tatctacttg agaatcctgt
   840
   tccaggaggt agatataatt gttcaccatt ctttgtatct attgaagaaa tgtcacaact
   900
   tctttcagcc aaatatccag aatatcaaat actatcagta gatgagttga aggaaatcaa
   960
   aggtgcaagg ttgccagatt tgaactcgaa aaagctcgtg gacgctggtt ttgagtttaa
   1020
   gtatagtgtc ggtgatatgt tcgatgatgc gattcaatgc tgcaaggaaa aaggctatct
   1080
   ctaagtatgt gtttgaaaaa aattccatga agctgagaaa acaataatat gcctaaaatc
   1140
   aatgatggct aatgaaatgt acaagtttat gcataaagtt atttgtgatg aatcaaataa
   1200
   tgaaataatc ggttcatttt tccgaaaaaa aaaaaaaaaa aaaaaaaaaa aaaaagtact
   1260
   ctgcgttgtt accactgctt aatcactagt gaattc
   1296
<210> 336
   <211> 326
   <212> PRT
   <213> Trifolium repens
<400> 336
   Met Ala Glu Gly Lys Gly Arg Val Cys Val Thr Gly Gly Thr Gly Phe
   1               5                   10                  15
   Leu Gly Ser Trp Ile Ile Lys Ser Leu Leu Glu Asn Gly Tyr Ser Val
               20                  25                  30
   Asn Thr Thr Ile Arg Ala Asp Pro Glu Arg Lys Arg Asp Val Ser Phe
           35                  40                  45
   Leu Thr Asn Leu Pro Gly Ala Ser Glu Arg Leu His Phe Phe Asn Ala
       50                  55                  60
   Asp Leu Asp Asp Pro Glu Ser Phe Asn Glu Ala Ile Glu Gly Cys Val
   65                  70                  75                  80
   Gly Ile Phe His Thr Ala Ser Pro Ile Asp Phe Ala Val Ser Glu Pro
                   85                  90                  95
   Glu Glu Ile Val Thr Lys Arg Thr Val Asp Gly Ala Leu Gly Ile Leu
               100                 105                 110
   Lys Ala Cys Val Asn Ser Lys Thr Val Lys Arg Phe Ile Tyr Thr Ser
           115                 120                 125
   Ser Gly Ser Ala Val Ser Phe Asn Gly Lys Asn Lys Asp Val Leu Asp
       130                 135                 140
   Glu Ser Asp Trp Ser Asp Val Asp Leu Leu Arg Ser Val Lys Pro Phe
   145                 150                 155                 160
   Gly Trp Ser Tyr Gly Val Ser Lys Thr Leu Ala Glu Lys Ala Val Leu
                   165                     170                     175
   Glu Phe Gly Gln Gln Asn Gly Ile Asp Val Val Thr Leu Ile Leu Pro
               180                     185                     190
   Phe Ile Val Gly Ser Phe Val Cys Pro Lys Leu Pro Asp Ser Val Glu
           195                     200                     205
   Lys Ala Leu Val Leu Val Leu Gly Lys Lys Glu Gln Ile Gly Ile Ile
       210                     215                     220
   Ser Phe His Met Val His Val Asp Asp Val Ala Arg Ala His Ile Tyr
   225                     230                     235                     240
   Leu Leu Glu Asn Pro Val Pro Gly Gly Arg Tyr Asn Cys Ser Pro Phe
                   245                     250                     255
   Phe Val Ser Ile Glu Glu Met Ser Gln Leu Leu Ser Ala Lys Tyr Pro
               260                     265                     270
   Glu Tyr Gln Ile Leu Ser Val Asp Glu Leu Lys Glu Ile Lys Gly Ala
           275                     280                     285
   Arg Leu Pro Asp Leu Asn Ser Lys Lys Leu Val Asp Ala Gly Phe Glu
       290                     295                     300
   Phe Lys Tyr Ser Val Gly Asp Met Phe Asp Asp Ala Ile Gln Cys Cys
   305                     310                     315                     320
   Lys Glu Lys Gly Tyr Leu
                   325